# EUROPEAN PATENT SPECIFICATION

(11) **EP 3 824 100 B1**
(45) Date of publication and mention of the grant of the patent: **29.04.2026**
(21) Application number: 19748892.7
(22) Date of filing: 19.07.2019
(51) Int. Cl.: C12Q 1/6844, B01L 3/00

(54) **POLYNUCLEOTIDE SYNTHESIS METHOD**
POLYNUKLEOTIDSYNTHESEVERFAHREN
PROCÉDÉ DE SYNTHÈSE DE POLYNUCLÉOTIDES

(30) Priority: 19.07.2018 GB 201811810
(43) Date of publication of application: 26.05.2021
(73) Proprietor: Oxford Nanopore Technologies plc, Oxford Science Park Oxford OX4 4DQ (GB)
(72) Inventor: MILTON, John, Cambridge Cambridgeshire CB10 1XL (GB); NAYYAR, Sobia, Cambridge Cambridgeshire CB10 1XL (GB); RIEDL, Jan, Cambridge Cambridgeshire CB10 1XL (GB); OGAKI, Ryosuke, Cambridge Cambridgeshire CB10 1XL (GB)
(74) Representative: J A Kemp LLP
(86) International application number: PCT/GB2019/052035
(87) International publication number: WO 2020/016604

(56) References cited:
- WO-A1-2018/134616
- WO-A2-01/88173
- WO-A2-2012/078312
- US-A1- 2013 085 073
- SRIRAM KOSURI ET AL: "Large-scale de novo DNA synthesis: technologies and applications", NATURE METHODS, vol. 11, no. 5, 1 May 2014 (2014-05-01), New York, pages 499 - 507, XP055574038, ISSN: 1548-7091, DOI: 10.1038/nmeth.2918
- JIAYUAN QUAN ET AL: "Parallel on-chip gene synthesis and application to optimization of protein expression", NATURE BIOTECHNOLOGY, vol. 29, no. 5, 24 April 2011 (2011-04-24), pages 449 - 452, XP055162152, ISSN: 1087-0156, DOI: 10.1038/nbt.1847

## Description

### FIELD OF THE INVENTION

The invention relates to new methods for synthesising polynucleotide molecules according to a predefined nucleotide sequence. The invention also relates to methods for the assembly of synthetic polynucleotides following synthesis, as well as systems and kits for performing the synthesis and/or assembly methods. The invention is defined in the appended claims.

### BACKGROUND TO THE INVENTION

Two primary methods exist for the synthesis and assembly of polynucleotide molecules, particularly DNA.

Phosphoramidite chemistry is a synthetic approach that assembles monomers of chemically activated T, C, A or G into oligonucleotides of approximately 100/150 bases in length via a stepwise process. The chemical reaction steps are highly sensitive and the conditions alternate between fully anhydrous (complete absence of water), aqueous oxidative and acidic (Roy and Caruthers, Molecules, 2013, 18, 14268-14284). If the reagents from the previous reaction step have not been completely removed this will be detrimental to future steps of synthesis. Accordingly, this synthesis method is limited to the production of polynucleotides of length of approximately 100 nucleotides.

The Polymerase Synthetic approach uses a polymerase to synthesise a complementary strand to a DNA template using T, C, A and G triphosphates. The reaction conditions are aqueous and mild and this approach can be used to synthesise DNA polynucleotides which are many thousands of bases in length. The main disadvantage of this method is that single- and double-stranded DNA cannot be synthesised *de novo* by this method, it requires a DNA template from which a copy is made. (Kosuri and Church, Nature Methods, 2014, 11, 499-507). Further methods of de novo nucleic acid synthesis are described in WO0188173, WO2012078312 and US2013085073.

Thus previous methods cannot be used to synthesise double-stranded DNA *de novo* without the aid of a pre-existing template molecule which is copied.

The inventors have developed new methodologies by which single- and double-stranded polynucleotide molecules can be synthesised *de novo* in a stepwise manner without the need to copy a pre-existing template molecule. Such methods also avoid the extreme conditions associated with phosphoramidite chemistry techniques and in contrast are carried out under mild, aqueous conditions around neutral pH. Such methods also enable *de novo* synthesis of single- or double-stranded polynucleotide molecules with a potential 10⁸ improvement on current synthesis methods with nucleotide lengths of >100mers to full genomes, providing a wide range of possibly applications in synthetic biology.

### SUMMARY OF THE INVENTION

The invention provides an *in vitro* method of synthesising a double-stranded polynucleotide having a predefined sequence, the method comprising performing cycles of synthesis wherein in each cycle:
(A) a first strand of a double-stranded polynucleotide is extended by the addition of a first nucleotide of the predefined sequence by the action of a ligase enzyme;
(B) the double-stranded polynucleotide is then cleaved at a cleavage site; and
(C) the second strand which is hybridized to the first strand is then extended by the addition of a second nucleotide of the predefined sequence by a nucleotide transferase or polymerase enzyme; and
wherein the first and second nucleotides of the predefined sequence of each cycle are retained in the double-stranded polynucleotide following cleavage.

In any of the above-described methods, the cleavage site may be defined by a polynucleotide sequence comprising a universal nucleotide.

In any of the above-described methods, in each cycle a cleavage site may be created in the double-stranded polynucleotide before extension of the second strand

In any of the above-described methods, during step (A) by the action of the ligase enzyme a universal nucleotide may be incorporated into the first strand of the double-stranded polynucleotide to define the cleavage site.

In any of the above-described methods, in a given cycle of synthesis the second nucleotide of that cycle which is added to the second strand of the double-stranded polynucleotide may comprise a reversible terminator group which prevents further extension by the enzyme, and wherein the reversible terminator group is removed from the incorporated second nucleotide of that cycle prior to the addition in the next cycle of synthesis of the second nucleotide of the next cycle.

In the above-described methods, in each cycle the first nucleotide may be a partner nucleotide for the second nucleotide, and wherein upon incorporation into the double-stranded polynucleotide the first and second nucleotides form a nucleotide pair.

In such methods the ligation reaction may comprise a blunt-ended ligation reaction.

In such methods involving a blunt-ended ligation reaction, the first nucleotide and the universal nucleotide may be components of a polynucleotide ligation molecule, and wherein the polynucleotide ligation molecule is ligated to the double-stranded polynucleotide during step (A) by the action of the ligase enzyme in a blunt-ended ligation reaction, and wherein upon ligation of the polynucleotide ligation molecule to the double-stranded polynucleotide the first strand of the double-stranded polynucleotide is extended and the cleavage site is created.

In any such methods involving a blunt-ended ligation reaction, the method may comprise performing a first cycle of synthesis comprising:
(1) providing a scaffold polynucleotide comprising a synthesis strand and a support strand hybridized thereto, wherein the synthesis strand comprises a primer strand portion, and wherein the support strand is the first strand of the double-stranded polynucleotide and the synthesis strand is the strand is the second strand of the double-stranded polynucleotide;
(2) ligating a double-stranded polynucleotide ligation molecule to the scaffold polynucleotide by the action of the ligase enzyme in a blunt-ended ligation reaction, the polynucleotide ligation molecule comprising a support strand and a helper strand hybridised thereto and further comprising a complementary ligation end, the ligation end comprising:
   (i) in the support strand a universal nucleotide and a first nucleotide of the predefined sequence; and
   (ii) in the helper strand a non-ligatable terminal nucleotide;
   wherein upon ligation the first strand of the double-stranded polynucleotide is extended with the first nucleotide and the cleavage site is created by the incorporation of the universal nucleotide into the first strand;
(3) cleaving the ligated scaffold polynucleotide at the cleavage site, wherein cleavage comprises cleaving the support strand and removing the universal nucleotide from the scaffold polynucleotide to provide a cleaved double-stranded scaffold polynucleotide comprising the incorporated first nucleotide;
(4) extending the terminal end of the primer strand portion of the synthesis strand of the double-stranded scaffold polynucleotide by the incorporation of a second nucleotide of the predefined sequence by the action of the nucleotide transferase or polymerase enzyme, the second nucleotide comprising a reversible terminator group which prevents further extension by the enzyme, wherein the second nucleotide is a partner for first nucleotide and wherein upon incorporation the second nucleotide and the first nucleotide form a nucleotide pair; and
(5) removing the reversible terminator group from the second nucleotide;
the method further comprising performing a further cycle of synthesis comprising:
(6) ligating a further double-stranded polynucleotide ligation molecule to the cleaved scaffold polynucleotide by the action of the ligase enzyme in a blunt-ended ligation reaction, the polynucleotide ligation molecule comprising a support strand and a helper strand hybridised thereto and further comprising a complementary ligation end, the ligation end comprising:
   (i) in the support strand a universal nucleotide and the first nucleotide of the further cycle of synthesis; and
   (ii) in the helper strand a non-ligatable terminal nucleotide;
   wherein upon ligation the first strand of the double-stranded polynucleotide is extended with the first nucleotide of the further cycle of synthesis and the cleavage site is created by the incorporation of the universal nucleotide into the first strand;
(7) cleaving the ligated scaffold polynucleotide at the cleavage site, wherein cleavage comprises cleaving the support strand and removing the universal nucleotide from the scaffold polynucleotide to provide a cleaved double-stranded scaffold polynucleotide comprising the nucleotide pair(s) of the previous cycle(s) and the first nucleotide of the further cycle of synthesis;
(8) extending the terminal end of the primer strand portion of the synthesis strand of the double-stranded scaffold polynucleotide by the incorporation of the second nucleotide of the further cycle of synthesis by the action of the nucleotide transferase or polymerase enzyme, the second nucleotide comprising a reversible terminator group which prevents further extension by the enzyme, wherein the second nucleotide of the further cycle of synthesis is a partner for the first nucleotide of the further cycle of synthesis, and wherein upon incorporation the second and first nucleotides of the further cycle of synthesis form a further nucleotide pair;
(9) removing the reversible terminator group from the second nucleotide; and
(10) repeating steps 6 to 9 multiple times to provide the double-stranded polynucleotide having a predefined nucleotide sequence.

In any of these particular methods wherein the first and second nucleotides form a nucleotide pair and involving a blunt-ended ligation reaction, the method may be performed as follows:
(a) in the ligation step of the first cycle (step 2) and in ligation steps of all further cycles the complementary ligation end of the polynucleotide ligation molecule is structured such that:
   i. the first nucleotide of the predefined sequence of that cycle is the terminal nucleotide of the support strand, occupies nucleotide position n in the support strand and is paired with the terminal nucleotide of the helper strand;
   ii. the universal nucleotide is the penultimate nucleotide of the support strand, occupies nucleotide position n+1 in the support strand and is paired with the penultimate nucleotide of the helper strand; and
   iii. the terminal nucleotide of the helper strand is a non-ligatable nucleotide;
   wherein position n is the nucleotide position which is opposite the second nucleotide of the predefined sequence of that cycle upon its incorporation, and wherein position n+1 is the next nucleotide position in the support strand relative to position n in the direction distal to the complementary ligation end; and wherein upon ligation the terminal nucleotide of the support strand of the polynucleotide ligation molecule is ligated to the terminal nucleotide of the scaffold polynucleotide proximal to the primer strand portion of the synthesis strand and a single-strand break is created between the terminal nucleotides of the helper strand and the primer strand portion of the synthesis strand;
(b) in the cleavage step of the first cycle (step 3) and in all further cycles the support strand of the ligated scaffold polynucleotide is cleaved between positions n+1 and n, thereby releasing the polynucleotide ligation molecule from the scaffold polynucleotide and retaining the first nucleotide of that cycle attached to the first strand of the cleaved scaffold polynucleotide; and
(c) in the extension step of the first cycle (step 4) and in all further cycles the second nucleotide of that cycle is incorporated into the second strand opposite the first nucleotide in the first strand and is paired therewith; and whereupon the position occupied by the first nucleotide of that cycle in the support strand of the cleaved scaffold polynucleotide is defined as nucleotide position n-1 in the next cycle of synthesis.

In any of these particular methods wherein the first and second nucleotides form a nucleotide pair and involving a blunt-ended ligation reaction, the method may alternatively be performed as follows:
(a) in the ligation step of the first cycle (step 2) and in ligation steps of all further cycles the complementary ligation end of the polynucleotide ligation molecule is structured such that:
   i. the first nucleotide of the predefined sequence of that cycle is the terminal nucleotide of the support strand, occupies nucleotide position n in the support strand and is paired with the terminal nucleotide of the helper strand;
   ii. the universal nucleotide occupies nucleotide position n+2 in the support strand and is paired with a partner nucleotide in the helper strand; and
   iii. the terminal nucleotide of the helper strand is a non-ligatable nucleotide;
   wherein position n is the nucleotide position which is opposite the second nucleotide of the predefined sequence of that cycle upon its incorporation, and wherein position n+2 is the second nucleotide position in the support strand relative to position n in the direction distal to the complementary ligation end; and wherein upon ligation the terminal nucleotide of the support strand of the polynucleotide ligation molecule is ligated to the terminal nucleotide of the scaffold polynucleotide proximal to the primer strand portion of the synthesis strand and a single-strand break is created between the terminal nucleotides of the helper strand and the primer strand portion of the synthesis strand;
(b) in the cleavage step of the first cycle (step 3) and in all further cycles the support strand of the ligated scaffold polynucleotide is cleaved between positions n+1 and n, thereby releasing the polynucleotide ligation molecule from the scaffold polynucleotide and retaining the first nucleotide of that cycle attached to the first strand of the cleaved scaffold polynucleotide; and
(c) in the extension step of the first cycle (step 4) and in all further cycles the second nucleotide of that cycle is incorporated into the second strand opposite the first nucleotide in the first strand and is paired therewith, and whereupon the position occupied by the first nucleotide of that cycle in the support strand of the cleaved scaffold polynucleotide is defined as nucleotide position n-1 in the next cycle of synthesis.

In any of these particular methods wherein the first and second nucleotides form a nucleotide pair and involving a blunt-ended ligation reaction, the method may alternatively still be performed as follows:
(a) in the ligation step of the first cycle (step 2) and in ligation steps of all further cycles the complementary ligation end of the polynucleotide ligation molecule is structured such that:
   i. the first nucleotide of the predefined sequence of that cycle is the terminal nucleotide of the support strand, occupies nucleotide position n in the support strand and is paired with the terminal nucleotide of the helper strand;
   ii. the universal nucleotide occupies nucleotide position n+2+x in the support strand and is paired with a partner nucleotide in the helper strand; and
   iii. the terminal nucleotide of the helper strand is a non-ligatable nucleotide;
   wherein position n is the nucleotide position which is opposite the second nucleotide of the predefined sequence of that cycle upon its incorporation, and wherein position n+2 is the second nucleotide position in the support strand relative to position n in the direction distal to the complementary ligation end and wherein x is a number of nucleotide positions relative to position n+2 in the direction distal to the complementary ligation end wherein the number is a whole number from 1 to 10 or more; and wherein upon ligation the terminal nucleotide of the support strand of the polynucleotide ligation molecule is ligated to the terminal nucleotide of the scaffold polynucleotide proximal to the primer strand portion of the synthesis strand and a single-strand break is created between the terminal nucleotides of the helper strand and the primer strand portion of the synthesis strand;
(b) in the cleavage step of the first cycle (step 3) and in all further cycles the support strand of the ligated scaffold polynucleotide is cleaved between positions n+1 and n, thereby releasing the polynucleotide ligation molecule from the scaffold polynucleotide and retaining the first nucleotide of that cycle attached to the first strand of the cleaved scaffold polynucleotide; and
(c) in the extension step of the first cycle (step 4) and in all further cycles the second nucleotide of that cycle is incorporated into the second strand opposite the first nucleotide in the first strand and is paired therewith, and whereupon the position occupied by the first nucleotide of that cycle in the support strand of the cleaved scaffold polynucleotide is defined as nucleotide position n-1 in the next cycle of synthesis.

In certain of the above-described methods of the invention, in each cycle upon incorporation the first nucleotide and the second nucleotide may alternatively become partner nucleotides in different nucleotide pairs in the synthesised double-stranded polynucleotide.

In such methods the ligation reaction may comprise a sticky-ended ligation reaction.

In such methods involving a sticky-ended ligation reaction, the first nucleotide and the universal nucleotide may be components of a polynucleotide ligation molecule, and wherein the polynucleotide ligation molecule is ligated to the double-stranded polynucleotide during step (A) by the action of the ligase enzyme in a sticky-ended ligation reaction, and wherein upon ligation of the polynucleotide ligation molecule to the double-stranded polynucleotide the first strand of the double-stranded polynucleotide is extended and the cleavage site is created.

In any such methods involving a sticky-ended ligation reaction, the method may comprise performing a first cycle of synthesis comprising:
(1) providing a scaffold polynucleotide comprising a synthesis strand and a support strand hybridized thereto, wherein the synthesis strand comprises a primer strand portion;
(2) ligating a double-stranded polynucleotide ligation molecule to the scaffold polynucleotide by the action of the ligase enzyme in a sticky-ended ligation reaction, the polynucleotide ligation molecule comprising a support strand and a helper strand hybridised thereto and further comprising a complementary ligation end, the ligation end comprising:
   (i) in the support strand a universal nucleotide and a first nucleotide of the predefined sequence; and
   (ii) in the helper strand a non-ligatable terminal nucleotide;
   wherein upon ligation the first strand of the double-stranded polynucleotide is extended with the first nucleotide and the cleavage site is created by the incorporation of the universal nucleotide into the first strand;
(3) cleaving the ligated scaffold polynucleotide at the cleavage site, wherein cleavage comprises cleaving the support strand and removing the universal nucleotide from the scaffold polynucleotide to provide a cleaved double-stranded scaffold polynucleotide comprising the incorporated first nucleotide;
(4) extending the terminal end of the primer strand portion of the synthesis strand of the double-stranded scaffold polynucleotide by the incorporation of a second nucleotide of the predefined sequence by the action of the nucleotide transferase or polymerase enzyme, the second nucleotide comprising a reversible terminator group which prevents further extension by the enzyme, wherein the second nucleotide; and
(5) removing the reversible terminator group from the second nucleotide;
the method further comprising performing a further cycle of synthesis comprising:
(6) ligating a further double-stranded polynucleotide ligation molecule to the cleaved scaffold polynucleotide by the action of the ligase enzyme in a sticky-ended ligation reaction, the polynucleotide ligation molecule comprising a support strand and a helper strand hybridised thereto and further comprising a complementary ligation end, the ligation end comprising:
   (i) in the support strand a universal nucleotide and the first nucleotide of the further cycle of synthesis; and
   (ii) in the helper strand a non-ligatable terminal nucleotide;
   wherein upon ligation the first strand of the double-stranded polynucleotide is extended with the first nucleotide of the further cycle of synthesis and the cleavage site is created by the incorporation of the universal nucleotide into the first strand;
(7) cleaving the ligated scaffold polynucleotide at the cleavage site, wherein cleavage comprises cleaving the support strand and removing the universal nucleotide from the scaffold polynucleotide to provide a cleaved double-stranded scaffold polynucleotide comprising the nucleotide pair(s) of the previous cycle(s) and the first nucleotide of the further cycle of synthesis;
(8) extending the terminal end of the primer strand portion of the synthesis strand of the double-stranded scaffold polynucleotide by the incorporation of the second nucleotide of the further cycle of synthesis by the action of the nucleotide transferase or polymerase enzyme, the second nucleotide comprising a reversible terminator group which prevents further extension by the enzyme;
(9) removing the reversible terminator group from the second nucleotide; and
(10) repeating steps 6 to 9 multiple times to provide the double-stranded polynucleotide having a predefined nucleotide sequence.

Any of the above-described methods involving a sticky-ended ligation reaction may be performed as follows: in step (1) the terminal end of the support strand of the scaffold polynucleotide proximal to the primer strand portion comprises a nucleotide overhang, wherein the terminal nucleotide of the support strand overhangs the terminal nucleotide of the primer strand portion, and wherein the terminal nucleotide of the support strand is the partner nucleotide for the second nucleotide of that cycle;
wherein in step (2) at the complementary ligation end of the polynucleotide ligation molecule the terminal end of the helper strand comprises a nucleotide overhang, wherein the terminal nucleotide of the helper strand overhangs the terminal nucleotide of the support strand, and wherein the terminal nucleotide of the support strand is the first nucleotide of that cycle and is a partner nucleotide in a different nucleotide pair formed in the next cycle of synthesis;
wherein in step (6) in the cleaved scaffold polynucleotide the terminal end of the support strand proximal to the primer strand portion comprises a nucleotide overhang, wherein the terminal and penultimate nucleotides of the support strand overhang the terminal nucleotide of the primer strand portion, and wherein the penultimate nucleotide of the support strand is the partner nucleotide for the second nucleotide of the further cycle of synthesis incorporated in step (8);
wherein in step (6) at the complementary ligation end of the polynucleotide ligation molecule the terminal end of the helper strand comprises a nucleotide overhang, wherein the terminal nucleotide of the helper strand overhangs the terminal nucleotide of the support strand, and wherein the terminal nucleotide of the support strand is the first nucleotide of the further cycle of synthesis and is a partner nucleotide in a different nucleotide pair formed in the next cycle of synthesis; and
wherein in first and further cycles of synthesis the nucleotide overhang in the complimentary ligation end of the polynucleotide ligation molecule and the nucleotide overhang in the scaffold polynucleotide comprises the same number of nucleotides, wherein the number is one or more, provided that the number of nucleotides in the overhang in the scaffold polynucleotide is one more than the number of nucleotides in the overhang in the complimentary ligation end.

In any of these particular methods wherein the first and second nucleotides become partner nucleotides in different nucleotide pairs in the synthesised double-stranded polynucleotide and involving a sticky-ended ligation reaction, the method may be performed as follows:
(a) in the ligation step of the first cycle (step 2) and in ligation steps of all further cycles the complementary ligation end of the polynucleotide ligation molecule is structured such that:
   i. the first nucleotide of the predefined sequence of that cycle is the terminal nucleotide of the support strand, occupies nucleotide position n+1 in the support strand and is paired with the penultimate nucleotide of the helper strand;
   ii. the universal nucleotide is the penultimate nucleotide of the support strand, occupies nucleotide position n+2 in the support strand and is paired with a partner nucleotide in the helper strand;
   iii. the overhang comprises a one nucleotide overhang comprises the terminal nucleotide of the helper strand overhanging the first nucleotide of the predefined sequence of that cycle; and
   iv. the terminal nucleotide of the helper strand is a non-ligatable nucleotide;
   wherein position n is the nucleotide position which is opposite the second nucleotide of the predefined sequence of that cycle upon its incorporation, and positions n+1 and n+2 are respectively the first/next and second nucleotide positions in the support strand relative to position n in the direction distal to the complementary ligation end; and wherein upon ligation the terminal nucleotide of the support strand of the polynucleotide ligation molecule is ligated to the terminal nucleotide of the scaffold polynucleotide proximal to the primer strand portion of the synthesis strand and a single-strand break is created between the terminal nucleotides of the helper strand and the primer strand portion of the synthesis strand;
(b) in the cleavage step of the first cycle (step 3) and in all further cycles the support strand of the ligated scaffold polynucleotide is cleaved between positions n+2 and n+1, thereby releasing the polynucleotide ligation molecule from the scaffold polynucleotide and retaining the first nucleotide of that cycle unpaired and attached to the first strand of the cleaved scaffold polynucleotide, wherein upon cleavage a double-nucleotide overhang is created in the support strand of the cleaved scaffold polynucleotide wherein the terminal and penultimate nucleotides of the support strand overhang the terminal nucleotide of the primer strand portion of the synthesis strand; and
(c) in the extension step of the first cycle (step 4) and in all further cycles the second nucleotide of that cycle is incorporated into the second strand and is paired with the penultimate nucleotide of the overhanging support strand, and wherein following steps (8) and (9) the position occupied by the first nucleotide of that further cycle in the support strand of the cleaved scaffold polynucleotide is defined as nucleotide position n in the next cycle of synthesis.

In any of these particular methods wherein the first and second nucleotides become partner nucleotides in different nucleotide pairs in the synthesised double-stranded polynucleotide and involving a sticky-ended ligation reaction, the method may alternatively be performed as follows:
(a) in the ligation step of the first cycle (step 2) and in ligation steps of all further cycles the complementary ligation end of the polynucleotide ligation molecule is structured such that:
   i. the first nucleotide of the predefined sequence of that cycle is the terminal nucleotide of the support strand, occupies nucleotide position n+1 in the support strand and is paired with the penultimate nucleotide of the helper strand;
   ii. the universal nucleotide occupies nucleotide position n+3 in the support strand and is paired with a partner nucleotide in the helper strand;
   iii. the overhang comprises a one nucleotide overhang comprises the terminal nucleotide of the helper strand overhanging the first nucleotide of the predefined sequence of that cycle; and
   iv. the terminal nucleotide of the helper strand is a non-ligatable nucleotide;
   wherein position n is the nucleotide position which is opposite the second nucleotide of the predefined sequence of that cycle upon its incorporation, and positions n+1, n+2 and n+3 are respectively the next, second and third nucleotide positions in the support strand relative to position n in the direction distal to the complementary ligation end; and wherein upon ligation the terminal nucleotide of the support strand of the polynucleotide ligation molecule is ligated to the terminal nucleotide of the scaffold polynucleotide proximal to the primer strand portion of the synthesis strand and a single-strand break is created between the terminal nucleotides of the helper strand and the primer strand portion of the synthesis strand;
(b) in the cleavage step of the first cycle (step 3) and in all further cycles the support strand of the ligated scaffold polynucleotide is cleaved between positions n+2 and n+1, thereby releasing the polynucleotide ligation molecule from the scaffold polynucleotide and retaining the first nucleotide of that cycle unpaired and attached to the first strand of the cleaved scaffold polynucleotide, wherein upon cleavage a double-nucleotide overhang is created in the support strand of the cleaved scaffold polynucleotide wherein the terminal and penultimate nucleotides of the support strand overhang the terminal nucleotide of the primer strand portion of the synthesis strand; and
(c) in the extension step of the first cycle (step 4) and in all further cycles the second nucleotide of that cycle is incorporated into the second strand and is paired with the penultimate nucleotide of the overhanging support strand, and wherein following steps (8) and (9) the position occupied by the first nucleotide of that further cycle in the support strand of the cleaved scaffold polynucleotide is defined as nucleotide position n in the next cycle of synthesis.

In any of these particular methods wherein the first and second nucleotides become partner nucleotides in different nucleotide pairs in the synthesised double-stranded polynucleotide and involving a sticky-ended ligation reaction, the method may alternatively still be performed as follows:
(a) in the ligation step of the first cycle (step 2) and in ligation steps of all further cycles the complementary ligation end of the polynucleotide ligation molecule is structured such that:
   i. the first nucleotide of the predefined sequence of that cycle is the terminal nucleotide of the support strand, occupies nucleotide position n+1 in the support strand and is paired with the penultimate nucleotide of the helper strand;
   ii. the universal nucleotide occupies nucleotide position n+3+x in the support strand and is paired with a partner nucleotide in the helper strand;
   iii. the overhang comprises a one nucleotide overhang comprises the terminal nucleotide of the helper strand overhanging the first nucleotide of the predefined sequence of that cycle; and
   iv. the terminal nucleotide of the helper strand is a non-ligatable nucleotide;
   wherein position n is the nucleotide position which is opposite the second nucleotide of the predefined sequence of that cycle upon its incorporation, and positions n+1 and n+3 are respectively the first/next and third nucleotide positions in the support strand relative to position n in the direction distal to the complementary ligation end, and wherein x is a number of nucleotide positions relative to position n+3 in the direction distal to the complementary ligation end wherein the number is a whole number from 1 to 10 or more; and wherein upon ligation the terminal nucleotide of the support strand of the polynucleotide ligation molecule is ligated to the terminal nucleotide of the scaffold polynucleotide proximal to the primer strand portion of the synthesis strand and a single-strand break is created between the terminal nucleotides of the helper strand and the primer strand portion of the synthesis strand;
(b) in the cleavage step of the first cycle (step 3) and in all further cycles the support strand of the ligated scaffold polynucleotide is cleaved between positions n+2 and n+1, thereby releasing the polynucleotide ligation molecule from the scaffold polynucleotide and retaining the first nucleotide of that cycle unpaired and attached to the first strand of the cleaved scaffold polynucleotide, wherein upon cleavage a double-nucleotide overhang is created in the support strand of the cleaved scaffold polynucleotide wherein the terminal and penultimate nucleotides of the support strand overhang the terminal nucleotide of the primer strand portion of the synthesis strand; and
(c) in the extension step of the first cycle (step 4) and in all further cycles the second nucleotide of that cycle is incorporated into the second strand and is paired with the penultimate nucleotide of the overhanging support strand, and wherein following steps (8) and (9) the position occupied by the first nucleotide of that further cycle in the support strand of the cleaved scaffold polynucleotide is defined as nucleotide position n in the next cycle of synthesis.

In certain methods described above wherein in each cycle upon incorporation the first nucleotide and the second nucleotide become partner nucleotides in different nucleotide pairs in the synthesised double-stranded polynucleotide and wherein the ligation reaction comprises a sticky-ended ligation reaction, the methods may be performed as follows: in step (1) the terminal end of the support strand of the scaffold polynucleotide proximal to the primer strand portion comprises a nucleotide overhang comprising 1+y nucleotides, wherein the 1+y nucleotides of the support strand overhang the terminal nucleotide of the primer strand portion, and wherein the first nucleotide of the overhang occupies a position in the overhang distal to the terminal end of the overhang, occupies nucleotide position n and is the partner nucleotide for the second nucleotide of that first cycle incorporated in step (4), and wherein the nucleotide of the overhang which occupies position n+1 is the partner nucleotide for the second nucleotide of the next/second cycle of synthesis incorporated in step (8);
wherein in step (2) at the complementary ligation end of the polynucleotide ligation molecule the terminal end of the helper strand comprises a nucleotide overhang comprising 1+y nucleotides, wherein the 1+y nucleotides of the helper strand overhang the terminal nucleotide of the support strand and are partner nucleotides for the 1+y overhanging nucleotides of the support strand of the scaffold polynucleotide, and wherein the terminal nucleotide of the support strand of the complementary ligation end is the first nucleotide of that cycle, occupies nucleotide position n+2+x and is a partner nucleotide in a different nucleotide pair formed in the third cycle of synthesis;
wherein in step (6) in the cleaved scaffold polynucleotide the terminal end of the support strand proximal to the primer strand portion comprises a nucleotide overhang comprising 1+y nucleotides, wherein the 1+y nucleotides of the support strand overhang the terminal nucleotide of the primer strand portion, and wherein the first nucleotide of the overhang occupies a position in the overhang distal to the terminal end of the overhang, occupies nucleotide position n and is the partner nucleotide for the second nucleotide of the further cycle of synthesis incorporated in step (8), and wherein the nucleotide of the overhang which occupies position n+1 is the partner nucleotide for the second nucleotide of the next cycle of synthesis;
wherein in step (6) at the complementary ligation end of the polynucleotide ligation molecule the terminal end of the helper strand comprises a nucleotide overhang comprising 1+y nucleotides, wherein the 1+y nucleotides of the helper strand overhang the terminal nucleotide of the support strand and are partner nucleotides for the 1+y overhanging nucleotides of the support strand of the scaffold polynucleotide, and wherein the terminal nucleotide of the support strand of the complementary ligation end is the first nucleotide of the further cycle of synthesis, occupies nucleotide position n+2+x and is a partner nucleotide in a different nucleotide pair formed in the next cycle of synthesis; and wherein:
   i. position n is the nucleotide position which is opposite the second nucleotide of the predefined sequence of that cycle upon its incorporation;
   ii. following ligation positions n+1 and n+2 are the first and second nucleotide positions in the support strand relative to position n in the direction proximal to the helper strand/distal to the primer strand portion;
   iii. y is a whole number which is one or more;
   iv. x is a whole number which is zero or more;
   v. the number for y in the scaffold polynucleotide is preferably the same number as the number for y in the complementary ligation end of the polynucleotide ligation molecule, and wherein if y is a different number the number for y in the complementary ligation end of the polynucleotide ligation molecule is preferably less than the number for y in the scaffold polynucleotide; and
   vi. in any given series of first and further cycles the number selected for x is the number selected for y in the scaffold polynucleotide minus one.

In any of these particular methods, in both first and further cycles in both the polynucleotide ligation molecule and in the ligated scaffold polynucleotide the universal nucleotide occupies position n+3+x and the scaffold polynucleotide is cleaved between positions n+3+x and n+2+x. Alternatively, in both first and further cycles in both the polynucleotide ligation molecule and in the ligated scaffold polynucleotide the universal nucleotide occupies position n+4+x and the scaffold polynucleotide is cleaved between positions n+3+x and n+2+x.

In any of the above-described methods involving blunt- and sticky-ended ligation reactions, the method may be modified such that:
(i) in step (2) the polynucleotide ligation molecule is provided with a complementary ligation end comprising a first nucleotide of the predefined sequence of the first cycle and further comprising one or more further nucleotides of the predefined sequence of the first cycle;
(ii) in step (3) following cleavage the first and further nucleotides of the predefined sequence of the first cycle are retained in the cleaved scaffold polynucleotide;
(iii) in step (4) the terminal end of the primer strand portion of the synthesis strand of the double-stranded scaffold polynucleotide is extended by the incorporation of a second nucleotide of the predefined sequence of the first cycle by the action of the nucleotide transferase or polymerase enzyme, and wherein the terminal end of the primer strand portion is further extended by the incorporation of one or more further nucleotides of the predefined sequence of the first cycle by the action of the nucleotide transferase or polymerase enzyme, wherein each one of the second and further nucleotides of the first cycle comprises a reversible terminator group which prevents further extension by the enzyme, and wherein following each further extension the reversible terminator group is removed from a nucleotide before the incorporation of the next nucleotide;
(iv) in step (6) the polynucleotide ligation molecule is provided with a complementary ligation end comprising a first nucleotide of the predefined sequence of the further cycle and further comprising one or more further nucleotides of the predefined sequence of the further cycle;
(v) in step (7) following cleavage the first and further nucleotides of the predefined sequence of the further cycle are retained in the cleaved scaffold polynucleotide;
(vi) in step (8) the terminal end of the primer strand portion of the synthesis strand of the double-stranded scaffold polynucleotide is extended by the incorporation of a second nucleotide of the predefined sequence of the further cycle by the action of the nucleotide transferase or polymerase enzyme, and wherein the terminal end of the primer strand portion is further extended by the incorporation of one or more further nucleotides of the predefined sequence of the further cycle by the action of the nucleotide transferase or polymerase enzyme, wherein each one of the second and further nucleotides of the further cycle comprises a reversible terminator group which prevents further extension by the enzyme, and wherein following each further extension the reversible terminator group is removed from a nucleotide before the incorporation of the next nucleotide.

In any of these particular methods, the complementary ligation end of the polynucleotide ligation molecule may be structured such that in steps (3) and (7) prior to cleavage the universal nucleotide occupies a position in the support strand which is the next nucleotide position in the support strand after the nucleotide positions of the first and further nucleotides in the direction distal to the complementary ligation end, and the support strand is cleaved between the position occupied by the last further nucleotide and the position occupied by the universal nucleotide. In any of these particular methods, the complementary ligation end of the polynucleotide ligation molecule may alternatively be structured such that in steps (3) and (7) prior to cleavage the universal nucleotide occupies a position in the support strand which is the next+1 nucleotide position in the support strand after the nucleotide positions of the first and further nucleotides in the direction distal to the complementary ligation end, and the support strand is cleaved between the position occupied by the last further nucleotide and the position occupied by the next nucleotide in the support strand.

In any of the methods described above and herein, in any one, more or all cycles of synthesis a partner nucleotide which pairs with the first nucleotide of the predefined sequence may be a nucleotide which is complementary with the first nucleotide, preferably naturally complementary.

In any of the methods described above and herein, in any one, more or all cycles of synthesis, prior to step (3) and/or (7) the scaffold polynucleotide may be provided comprising a synthesis strand and a support strand hybridized thereto, and wherein the synthesis strand is provided without a helper strand. In any one, more or all cycles of synthesis, prior to step (3) and/or (7) the synthesis strand may be removed from the scaffold polynucleotide.

In any of the methods described above and herein, in any one, more or all cycles of synthesis, after the step of ligating the double-stranded polynucleotide ligation molecule to the cleaved scaffold polynucleotide and before the step of incorporating the next nucleotide of the predefined nucleotide sequence into the synthesis strand of the scaffold polynucleotide, the helper strand portion of the synthesis strand may be removed from the scaffold polynucleotide. In any such method the helper strand portion of the synthesis strand may be removed from the scaffold polynucleotide by: (i) heating the scaffold polynucleotide to a temperature of about 80°C to about 95°C and separating the helper strand portion from the scaffold polynucleotide, (ii) treating the scaffold polynucleotide with urea solution, such as 8M urea and separating the helper strand portion from the scaffold polynucleotide, (iii) treating the scaffold polynucleotide with formamide or formamide solution, such as 100% formamide and separating the helper strand portion from the scaffold polynucleotide, or (iv) contacting the scaffold polynucleotide with a single-stranded polynucleotide molecule which comprises a region of nucleotide sequence which is complementary with the sequence of the helper strand portion, thereby competitively inhibiting the hybridisation of the helper strand portion to the scaffold polynucleotide.

In any such method described above and herein wherein the support strand of the scaffold polynucleotide is cleaved between the position occupied by the universal nucleotide and the position occupied by the next nucleotide in the support strand relative to the universal nucleotide in the direction proximal to the primer strand portion of the synthesis strand, each cleavage step may comprise a two-step cleavage process wherein each cleavage step may comprise a first step comprising removing the universal nucleotide thus forming an abasic site, and a second step comprising cleaving the support strand at the abasic site. In any such method the first step may be performed with a nucleotide-excising enzyme. The nucleotide-excising enzyme may be a 3-methyladenine DNA glycosylase enzyme. The nucleotide-excising enzyme may be human alkyladenine DNA glycosylase (hAAG) or uracil DNA glycosylase (UDG). In any such method the second step may be performed with a chemical which is a base. The base may be NaOH. In any such method the second step may be performed with an organic chemical having abasic site cleavage activity. The organic chemical may be N,N'-dimethylethylenediamine. In any such method the second step may be performed with an enzyme having abasic site lyase activity such as AP Endonuclease, Endonuclease III (Nth) or Endonuclease VIII.

In any such method described above and herein wherein the support strand of the scaffold polynucleotide is cleaved between the position occupied by the universal nucleotide and the position occupied by the next nucleotide in the support strand relative to the universal nucleotide in the direction proximal to the primer strand portion of the synthesis strand, each cleavage step may comprise a one step cleavage process comprising removing the universal nucleotide with a cleavage enzyme, wherein the enzyme is Endonuclease III, Endonuclease VIII, formamidopyrimidine DNA glycosylase (Fpg) or 8-oxoguanine DNA glycosylase (hOGG1).

In any such method described above and herein wherein the support strand of the scaffold polynucleotide is cleaved between the position occupied by the next nucleotide in the support strand relative to the universal nucleotide in the direction proximal to the primer strand portion and the position occupied by the second nucleotide the support strand relative to the universal nucleotide in the direction proximal to the primer strand portion, the cleavage step may comprise cleaving the support strand with an enzyme. Such an enzyme may be Endonuclease V.

In any of the methods described above and herein, both strands of the synthesised double-stranded polynucleotide may be DNA strands. The synthesis strand and the support strand may be DNA strands. In such cases incorporated nucleotides are preferably dNTPs, preferably dNTPs comprising a reversible terminator group. In any such method any one or more or all of the incorporated nucleotides comprising a reversible terminator group may comprise 3'-*O*-allyl-dNTPs or 3'-*O*-azidomethyl-dNTPs.

In any of the methods described above and herein, one strand of the synthesised double-stranded polynucleotide may be a DNA strand and the other strand of the synthesised double-stranded polynucleotide may be an RNA strand. The synthesis strand may be an RNA strand and the support strand may be an RNA or a DNA strand. In such cases nucleotides incorporated by the transferase enzyme or the polymerase enzyme are preferably NTPs, preferably NTPs comprising a reversible terminator group. In any such method any one or more or all of the incorporated nucleotides comprising a reversible terminator group may be 3'-*O*-allyl-NTPs or 3'-*O*-azidomethyl-NTPs.

In any of the methods described above and herein involving incorporation of a nucleotide into a synthesis strand comprising DNA *e.g*. incorporation of one or more dNTPs, the enzyme may be a polymerase enzyme, preferably a DNA polymerase enzyme, more preferably a modified DNA polymerase enzyme having an enhanced ability to incorporate a dNTP comprising a reversible terminator group compared to an unmodified polymerase. The polymerase may be a variant of the native DNA polymerase from *Thermococcus* species 9°N, preferably species 9°N-7.

In any of the methods described above and herein involving incorporation of a nucleotide into a synthesis strand comprising RNA *e.g*. incorporation of one or more NTPs, the enzyme may be a polymerase enzyme, preferably an RNA polymerase enzyme such as T3 or T7 RNA polymerase, more preferably a modified RNA polymerase enzyme having an enhanced ability to incorporate an NTP comprising a reversible terminator group compared to an unmodified polymerase.

In any of the methods described above and herein, a first strand of the synthesised double-stranded polynucleotide may be a DNA strand and the second strand of the synthesised double-stranded polynucleotide may be an RNA strand. Alternatively, a first strand of the synthesised double-stranded polynucleotide may be an RNA strand and the second strand of the synthesised double-stranded polynucleotide may be a DNA strand.

In any of the methods described above and herein, the enzyme enzyme has a terminal transferase activity, optionally wherein the enzyme is a terminal nucleotidyl transferase, a terminal deoxynucleotidyl transferase, terminal deoxynucleotidyl transferase (TdT), pol lambda, pol micro or Φ29 DNA polymerase.

In any of the methods described above and herein, the step of removing the reversible terminator group from the first/next nucleotide may be performed with tris(carboxyethyl)phosphine (TCEP).

In any of the methods described above and herein, the step of ligating a double-stranded polynucleotide ligation molecule to the cleaved scaffold polynucleotide is preferably performed with a ligase enzyme. The ligase enzyme may be a T3 DNA ligase or a T4 DNA ligase.

In any of the methods described above and herein, in any one, more or all cycles of synthesis in step (1)/(6) in the scaffold polynucleotide the synthesis strand comprising the primer strand portion and the portion of the support strand hybridized thereto may be connected by a hairpin loop.

In any of the methods described above and herein, in any one, more or all cycles of synthesis in steps (2)/(6) in the polynucleotide ligation molecule the helper strand and the portion of the support strand hybridized thereto may be connected by a hairpin loop at the end opposite the complementary ligation end.

In any of the methods described above and herein, in any one, more or all cycles of synthesis:
a) in steps (1)/(6) in the scaffold polynucleotide the synthesis strand comprising the primer strand portion and the portion of the support strand hybridized thereto are connected by a hairpin loop; and
b) in steps (2)/(6) in the polynucleotide ligation molecule the helper strand and the portion of the support strand hybridized thereto are connected by a hairpin loop at the end opposite the complementary ligation end.

In any of the methods described above and herein, at least one or more or all of the polynucleotide ligation molecules may be provided as a single molecule comprising a hairpin loop connecting the support strand and the helper strand at the end opposite the complementary ligation end. In any of the methods described above and herein, the polynucleotide ligation molecules of each synthesis cycle may be provided as single molecules each comprising a hairpin loop connecting the support strand and the helper strand at the end opposite the complementary ligation end.

In any of the methods described above and herein, in steps (1) and (6) the synthesis strand of the scaffold polynucleotide comprising the primer strand portion and/or the portion of the support strand hybridized thereto may be tethered to a common surface. The synthesis strand of the scaffold polynucleotide comprising the primer strand portion and/or the portion of the support strand hybridized thereto may comprise a cleavable linker, wherein the linker may be cleaved to detach the double-stranded polynucleotide from the surface following synthesis.

In any of the methods described above and herein, in steps (1) and (6) the primer strand portion of the synthesis strand and the portion of the support strand hybridized thereto may be connected by a hairpin loop, and wherein the hairpin loop is tethered to a surface.

In any of the methods described above and herein, a hairpin loop may be tethered to a surface via a cleavable linker, wherein the linker may be cleaved to detach the double-stranded polynucleotide from the surface following synthesis. The cleavable linker may be a UV cleavable linker.

In any of the methods described above and herein, the surface to which polynucleotides are attached may be the surface of a microparticle or a planar surface.

In any of the methods described above and herein, the surface to which polynucleotides are attached may comprise a gel. The surface may comprise a polyacrylamide surface, such as about 2% polyacrylamide, preferably wherein the polyacrylamide surface is coupled to a solid support such as glass.

In any of the methods described above and herein, the synthesis strand comprising the primer strand portion and the portion of the support strand hybridized thereto may tethered to the common surface via one or more covalent bonds. The one or more covalent bonds may be formed between a functional group on the common surface and a functional group on the scaffold molecule, wherein the functional group on the scaffold molecule may be an amine group, a thiol group, a thiophosphate group or a thioamide group. The functional group on the common surface may be a bromoacetyl group, optionally wherein the bromoacetyl group is provided on a polyacrylamide surface derived using N- (5-bromoacetamidylpentyl) acrylamide (BRAPA).

In any of the methods described above and herein, reactions relating to any of the synthesis cycles described above and herein may be performed in droplets within a microfluidic system. The microfluidic system may be an electrowetting system. The microfluidic system may be an electrowetting-on-dielectric system (EWOD).

In any of the methods described above and herein, following synthesis the strands of the double-stranded polynucleotides may be separated to provide a single-stranded polynucleotide having a predefined sequence.

In any of the methods described above and herein, following synthesis the double-stranded polynucleotide or a region thereof is amplified, preferably by PCR.

The invention also provides a method of assembling a polynucleotide having a predefined sequence, the method comprising performing any of the synthesis methods described above and herein to synthesise a first polynucleotide having a predefined sequence and one or more additional polynucleotides having a predefined sequence and joining together the first and the one or more additional polynucleotides. The first and the one or more additional polynucleotides may preferably comprise different predefined sequences. The first polynucleotide and the one or more additional polynucleotides may be double-stranded or may be single-stranded. The first polynucleotide and the one or more additional polynucleotides may first be cleaved to create compatible termini and then joined together, *e.g.* by ligation. The first polynucleotide and the one or more additional polynucleotides may be cleaved by a restriction enzyme at a cleavage site to create compatible termini.

Any of the *in vitro* methods for synthesising a double-stranded polynucleotide having a predefined sequence as described above and herein, and/or any of the *in vitro* methods of assembling a polynucleotide having a predefined sequence as described above and herein may be performed in droplets within a microfluidic system. In any such methods, the assembly methods may comprise assembly steps which comprise providing a first droplet comprising a first synthesised polynucleotide having a predefined sequence and a second droplet comprising an additional one or more synthesised polynucleotides having a predefined sequence, wherein the droplets are brought in contact with each other and wherein the synthesised polynucleotides are joined together thereby assembling a polynucleotide comprising the first and additional one or more polynucleotides. In any such methods the synthesis steps may be performed by providing a plurality of droplets each droplet comprising reaction reagents corresponding to a step of the synthesis cycle, and sequentially delivering the droplets to the scaffold polynucleotide in accordance with the steps of the synthesis cycles. In any such methods, following delivery of a droplet and prior to the delivery of a next droplet, a washing step may be carried out to remove excess reaction reagents. In any such methods the microfluidic system may be an electrowetting system. In any such methods the microfluidic system may be an electrowetting-on-dielectric system (EWOD). In any such methods the synthesis and assembly steps may be performed within the same system.

In any of the *in vitro* methods for synthesising a double-stranded polynucleotide having a predefined sequence as described above and herein, the universal nucleotide is inosine, or an analogue, variant or derivative thereof, and the partner nucleotide for the universal nucleotide in the helper strand is cytosine.

In a related aspect, the invention further provides an *in vitro* method of extending a double-stranded polynucleotide to synthesise a double-stranded polynucleotide having a predefined sequence, the method comprising one or more cycles of synthesis wherein in each cycle of synthesis a universal nucleotide and a first nucleotide of the predefined sequence are added to a first strand of a double-stranded scaffold polynucleotide in a ligation reaction; the scaffold polynucleotide is cleaved at a cleavage site defined by a sequence comprising the universal nucleotide, wherein upon cleavage the universal nucleotide is released from the scaffold polynucleotide; and then a second nucleotide of the predefined sequence is added to the opposite strand of the scaffold polynucleotide.

In a related aspect, the invention further provides the use of a universal nucleotide in an *in vitro* method of synthesising a double-stranded polynucleotide having a predefined sequence, wherein the universal nucleotide is used in cycles of synthesis to create a polynucleotide cleavage site in a double-stranded scaffold polynucleotide, and wherein cleavage of the scaffold polynucleotide provides a site in each strand of the scaffold polynucleotide for the incorporation of one or more nucleotides of the predefined sequence, wherein in each cycle of synthesis said use comprises: providing a double-stranded scaffold polynucleotide comprising a synthesis strand and a support strand hybridized thereto, wherein the synthesis strand comprises a primer strand portion; providing a double-stranded polynucleotide ligation molecule comprising a support strand and a helper strand hybridized thereto and further comprising a complementary ligation end, wherein the terminal nucleotide of the helper strand at the complementary ligation end comprises a non-ligatable nucleotide, wherein the terminal nucleotide of the support strand at the complementary ligation end comprises a ligatable first nucleotide of the predefined sequence, and wherein the support strand comprises a universal nucleotide for use in creating a polynucleotide cleavage site; ligating the support strand of the polynucleotide ligation molecule to the support strand of the scaffold polynucleotide in a ligation reaction, whereupon the support strand of the scaffold polynucleotide is extended with the first nucleotide of the predefined sequence and a single-strand break is created between the helper strand and the primer strand portion of the synthesis strand, and then optionally removing the helper strand; cleaving the support strand of the scaffold polynucleotide at a cleavage site defined by a sequence comprising the universal nucleotide, whereupon the polynucleotide ligation molecule comprising the universal nucleotide is removed from the scaffold polynucleotide and the first nucleotide of the predefined sequence is retained in the cleaved scaffold polynucleotide; adding to the terminal end of the primer strand portion of the synthesis strand of the scaffold polynucleotide by a polymerase enzyme or a transferase enzyme a second nucleotide of the predefined sequence comprising a reversible terminator group; and removing the reversible terminator group from the second nucleotide.

In any such method of synthesising a double-stranded polynucleotide having a predefined sequence, the support strand of the polynucleotide ligation molecule may further comprise one or more further nucleotides of the predefined sequence next to the first nucleotide of the predefined sequence, wherein the first nucleotide of the predefined sequence is the terminal nucleotide of the support strand of the polynucleotide ligation molecule and is ligated to the terminal nucleotide of the support strand of the scaffold polynucleotide; and wherein following the addition of the second nucleotide of the predefined sequence the method further comprises adding to the terminal end of the primer strand portion of the synthesis strand by the action of a polymerase enzyme or a transferase enzyme one or more further nucleotides of the predefined sequence by performing one or more cycles of adding a further nucleotide comprising a reversible terminator group and then removing the reversible terminator group, and wherein upon cleavage the first and further nucleotides of the predefined sequence are retained in the cleaved scaffold polynucleotide.

Such use of a universal nucleotide in a method of synthesising a double-stranded polynucleotide having a predefined sequence may be implemented using any of the specific methods defined and described above and herein.

In a related aspect, the invention further provides an *in vitro* method of extending with a predefined nucleotide each strand of a double-stranded polynucleotide molecule at the same terminal end, the method comprising: providing a double-stranded scaffold polynucleotide comprising a synthesis strand and a support strand hybridized thereto, wherein the synthesis strand comprises a primer strand portion; adding to the terminal end of the support strand of the scaffold polynucleotide by the action of a ligase enzyme in a ligation reaction a first nucleotide of the predefined sequence, wherein the first nucleotide is the terminal nucleotide in a support strand of a double-stranded polynucleotide ligation molecule, the support strand further comprising a universal nucleotide, wherein the first nucleotide is ligated to the terminal nucleotide of the support strand of the scaffold polynucleotide and a single-strand break is created between the opposite/helper strand of the polynucleotide ligation molecule and the primer strand portion of the synthesis strand, and optionally removing the helper strand of the polynucleotide ligation molecule; cleaving the support strand of the scaffold polynucleotide at a cleavage site defined by a sequence comprising the universal nucleotide whereupon the polynucleotide ligation molecule comprising the universal nucleotide is removed from the scaffold polynucleotide, wherein following cleavage the first nucleotide is retained in the support strand; adding to the terminal end of the primer strand portion of the synthesis strand by the action of a polymerase enzyme or a transferase enzyme a second nucleotide of the predefined sequence comprising a reversible terminator group; and removing the reversible terminator group from the second nucleotide.

In any such method of extending each strand of a double-stranded polynucleotide molecule, the support strand of the polynucleotide ligation molecule may further comprise one or more further nucleotides of the predefined sequence next to the first nucleotide of the predefined sequence, wherein the first nucleotide of the predefined sequence is the terminal nucleotide of the support strand of the polynucleotide ligation molecule and is ligated to the terminal nucleotide of the support strand of the scaffold polynucleotide; and wherein following the addition of the second nucleotide of the predefined sequence the method further comprises adding to the terminal end of the primer strand portion of the synthesis strand by the action of a polymerase enzyme or a transferase enzyme one or more further nucleotides of the predefined sequence by performing one or more cycles of adding a further nucleotide comprising a reversible terminator group and then removing the reversible terminator group, and wherein upon cleavage the first and further nucleotides of the predefined sequence are retained in the cleaved scaffold polynucleotide.

The invention provides an *in vitro* method of synthesising a double-stranded polynucleotide having a predefined sequence, the method comprising preforming one or more extension cycles according to the aforementioned extension method.

In any such method of extending with a predefined nucleotide each strand of a double-stranded polynucleotide molecule, or in any such method of synthesising a double-stranded polynucleotide having a predefined sequence, the method may be implemented using any of the specific methods defined and described above and herein.

In a related aspect, the invention further provides an *in vitro* method of ligating a polynucleotide ligation molecule comprising a universal nucleotide to a double-stranded scaffold polynucleotide during a cycle of extending with a predefined nucleotide each strand of the double-stranded scaffold polynucleotide at the same terminal end, the method comprising: providing a double-stranded scaffold polynucleotide comprising a support strand and a synthesis strand hybridized thereto, wherein the synthesis strand comprises a primer strand portion; and ligating a double-stranded polynucleotide ligation molecule to the double-stranded scaffold polynucleotide, wherein the polynucleotide ligation molecule comprises a support strand and a helper strand hybridized thereto and further comprises a complementary ligation end, wherein the terminal nucleotide of the helper strand at the complementary ligation end comprises a non-ligatable nucleotide, wherein the terminal nucleotide of the support strand at the complementary ligation end comprises a ligatable first nucleotide of the predefined sequence, and wherein the support strand comprises a universal nucleotide for use in creating a polynucleotide cleavage site, wherein the ligation reaction comprises ligating the support strand of the polynucleotide ligation molecule to the support strand of the double-stranded scaffold polynucleotide in a ligation reaction, whereupon the support strand of the scaffold polynucleotide is extended with the first nucleotide of the predefined sequence and a single-strand break is created between the helper strand and the primer strand portion of the synthesis strand, and then optionally removing the helper strand; the method further comprising: cleaving the support strand of the scaffold polynucleotide at a cleavage site defined by a sequence comprising the universal nucleotide, whereupon the polynucleotide ligation molecule comprising the universal nucleotide is removed from the scaffold polynucleotide and the first nucleotide of the predefined sequence is retained in the cleaved scaffold polynucleotide; adding to the terminal end of the primer strand portion of the synthesis strand of the scaffold polynucleotide by a polymerase enzyme or a transferase enzyme a second nucleotide of the predefined sequence comprising a reversible terminator group; and removing the reversible terminator group from the second nucleotide.

In any such method of ligating a polynucleotide ligation molecule comprising a universal nucleotide to a double-stranded scaffold polynucleotide, the support strand of the polynucleotide ligation molecule may further comprise one or more further nucleotides of the predefined sequence next to the first nucleotide of the predefined sequence, wherein the first nucleotide of the predefined sequence is the terminal nucleotide of the support strand of the polynucleotide ligation molecule and is ligated to the terminal nucleotide of the support strand of the scaffold polynucleotide; and wherein following the addition of the second nucleotide of the predefined sequence the method further comprises adding to the terminal end of the primer strand portion of the synthesis strand by the action of a polymerase enzyme or a transferase enzyme one or more further nucleotides of the predefined sequence by performing one or more cycles of adding a further nucleotide comprising a reversible terminator group and then removing the reversible terminator group, and wherein upon cleavage the first and further nucleotides of the predefined sequence are retained in the cleaved scaffold polynucleotide.

The invention provides an *in vitro* method of synthesising a double-stranded polynucleotide having a predefined sequence, the method comprising preforming one or more extension cycles according to the aforementioned ligation method.

In any such method of ligating a ligation polynucleotide comprising a universal nucleotide to a double-stranded polynucleotide during a cycle of synthesising a double-stranded polynucleotide having a predefined sequence, the method may be implemented using any of the specific methods defined and described above and herein.

The invention additionally provides a polynucleotide synthesis system for carrying out any of the synthesis and/or assembly methods described above and herein, comprising (a) an array of reaction areas, wherein each reaction area comprises at least one scaffold polynucleotide; and (b) means for the delivery of the reaction reagents to the reaction areas and optionally, (c) means to cleave the synthesised double-stranded polynucleotide from the scaffold polynucleotide. Such a system may further comprise means for providing the reaction reagents in droplets and means for delivering the droplets to the scaffold polynucleotide in accordance with the synthesis cycles.

The invention further provides a kit for use with any of the systems described above and herein, and for carrying out any of the synthesis methods described above and herein, the kit comprising volumes of reaction reagents corresponding to the steps of the synthesis cycles.

The invention also provides a method of making a polynucleotide microarray, wherein the microarray comprises a plurality of reaction areas, each area comprising one or more polynucleotides having a predefined sequence, the method comprising:
a) providing a surface comprising a plurality of reaction areas, each area comprising one or more double-stranded anchor or scaffold polynucleotides, and
b) performing cycles of synthesis according to any of the methods described above and herein at each reaction area, thereby synthesising at each area one or more double-stranded polynucleotides having a predefined sequence.

In such methods, following synthesis the strands of the double-stranded polynucleotides may be separated to provide a microarray wherein each area comprises one or more single-stranded polynucleotides having a predefined sequence.

### DESCRIPTION OF THE FIGURES

Relevant Figures presented herein and described below show some or all of the steps of a cycle of synthesis using methods, including methods of the invention, as well as means for performing aspects of the methods, such as oligonucleotides, surfaces, surface attachment chemistries, linkers etc. These Figures as well as all descriptions thereof and all associated methods, reagents and protocols are presented for illustration only and are not to be construed as limiting.

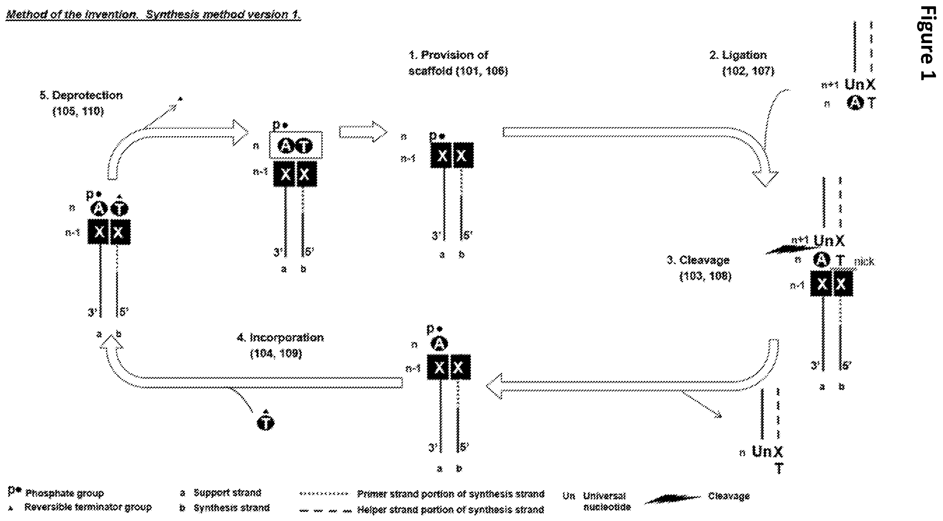

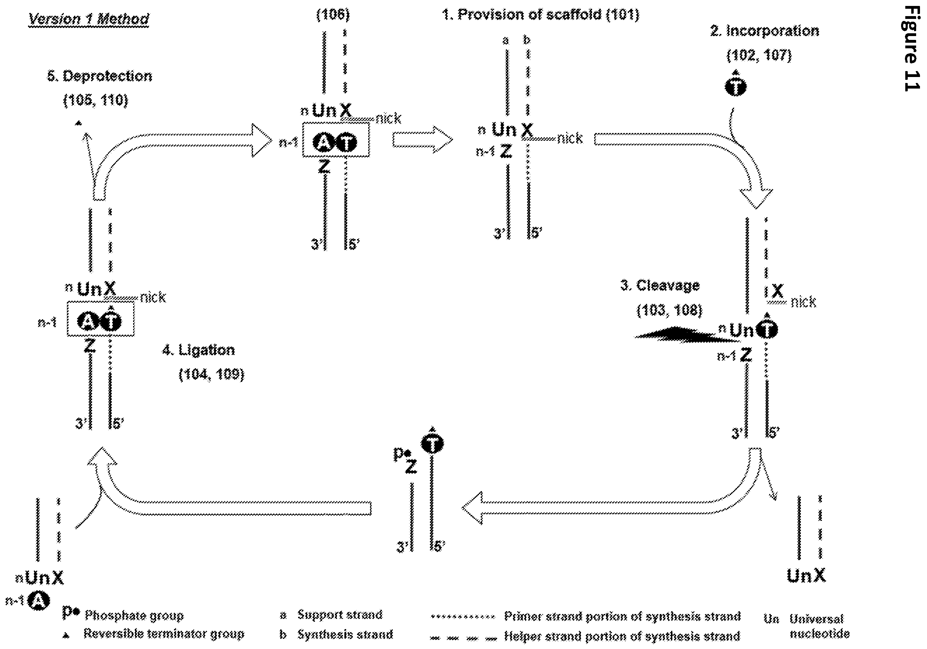

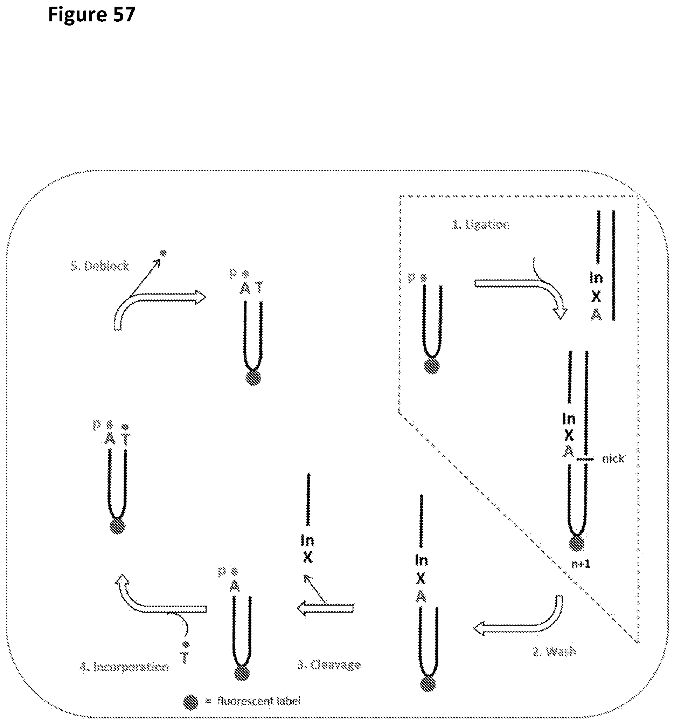

Relevant Figures, such as *e.g.* Figures 11, 12, 13, 14, 15, 18a, 19a, 20a etc. show some or all of the steps of a cycle of synthesis including incorporation of a nucleotide (*e.g*., a nucleotide comprising a reversible terminator group), cleavage (*e.g*., cleaving the scaffold polynucleotide into a first portion and a second portion, wherein the first portion comprises an universal nucleotide, and the second portion comprises the incorporated nucleotide), ligation (*e.g*., ligating to the second portion of the cleaved scaffold polynucleotide comprising the incorporated nucleotide, a polynucleotide construct comprising a single-stranded portion, wherein the single-stranded portion comprises a partner nucleotide that is complementary to the incorporated nucleotide) and deprotection (*e.g*., removing the reversible terminator group from the incorporated nucleotide). These methods are provided for illustrative support only and are not within the scope of the claimed invention. Method schemes shown in Figures 1 to 10, as well as in Figures 57, 60 and 61, are methods of the invention.

### Figure 1. Scheme of Exemplary Method Version 1 of the Invention.

Scheme showing a first synthesis cycle according to exemplary method version 1 of the invention.

The method comprises a cycle of provision of a scaffold polynucleotide, ligation of a polynucleotide ligation molecule to the scaffold polynucleotide, cleavage, incorporation of a nucleotide comprising a reversible terminator group or blocking group and deprotection.

The scheme shows the provision of a scaffold polynucleotide (101, 106) comprising a support strand (labelled "a") and a synthesis strand (labelled "b") hybridised thereto. The synthesis strand comprises a primer strand portion (dotted line). The terminal nucleotide of the support strand proximal to the primer strand portion comprises a ligatable group, preferably a terminal phosphate group as depicted in the Figure. The terminal nucleotide of the primer strand portion and the nucleotide paired therewith are depicted as "X". These two nucleotides can be any two nucleotides or analogs or derivatives thereof, and are not limited to being a naturally complementary pair of nucleotides.

The scheme shows the provision of a polynucleotide ligation molecule (102, 107; structure in the top right of the Figure). The polynucleotide ligation molecule comprises a helper strand (dashed line), a support strand hybridised thereto and a complementary ligation end. The terminal nucleotide of the support strand of the complementary ligation end is a first nucleotide of the predefined sequence and is depicted as "A" (adenosine). The terminal nucleotide of the helper strand of the complementary ligation end is depicted as "T" (thymine). The terminal nucleotide of the helper strand of the complementary ligation end comprises a non-ligatable nucleotide. The complementary ligation end comprises a universal nucleotide (depicted as "Un") in the support strand and which is paired with a partner nucleotide in the helper strand (depicted as "X"). A and T are depicted purely for illustration and can be any nucleotides or analogs or derivatives thereof. X can be any nucleotide or analog or derivative thereof. It is not necessary for paired nucleotides to comprise naturally complementary nucleotides.

The scheme shows the ligation of the support strand of the polynucleotide ligation molecule (102, 107) to the support strand of the scaffold polynucleotide and the creation of single-stranded break ("nick") in the synthesis strand between the helper strand and primer strand portion.

The scheme shows a cleavage step (103, 108) comprising cleaving the support strand (jagged arrowhead) at a cleavage site defined by a sequence comprising the universal nucleotide. Cleavage releases the polynucleotide ligation molecule comprising the universal nucleotide and leads to the retention of the first nucleotide of the predefined sequence in the scaffold polynucleotide. Following cleavage, the first nucleotide is the terminal nucleotide of the support strand of the cleaved scaffold polynucleotide and overhangs the terminal nucleotide of the primer strand portion in a single-nucleotide overhang. In synthesis method of the invention version 1 the support strand is cleaved between the position occupied by the universal nucleotide and the nucleotide which occupies the next nucleotide position in the support strand in the direction proximal to the primer strand portion/distal to the helper strand portion.

The scheme shows the incorporation (104, 109) of a second nucleotide of the predefined sequence. This nucleotide comprises a reversible terminator group (triangle) and is depicted as "T" (thymine) purely for illustration, it can be any nucleotide or analog or derivative thereof.

The scheme shows a deprotection step (105, 110) comprising removal of the reversible terminator group from the second nucleotide of the predefined sequence.

In synthesis method of the invention version 1 in each cycle the first nucleotide of the predefined sequence and the second nucleotide of the predefined sequence form a nucleotide pair.

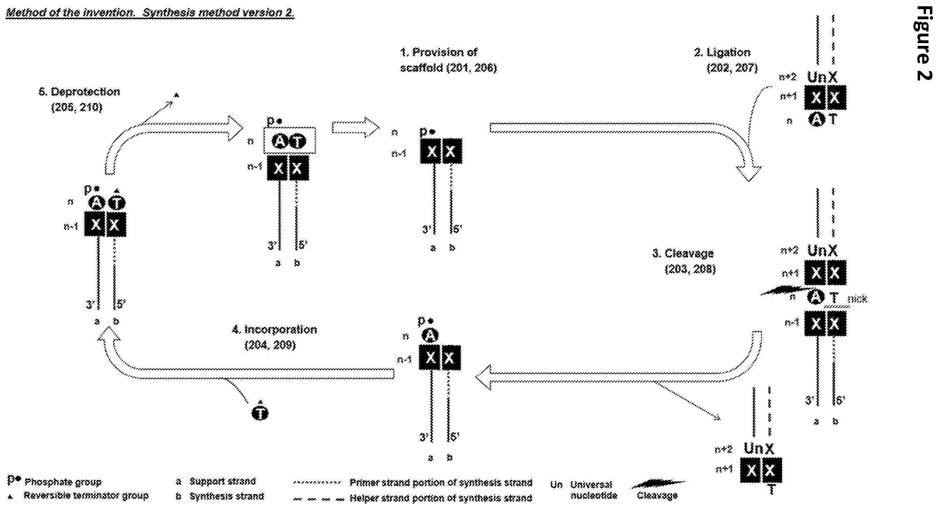

### Figure 2. Scheme of Exemplary Method Version 2 of the Invention.

Scheme showing a first synthesis cycle according to exemplary method version 2 of the invention.

The method comprises a cycle of provision of a scaffold polynucleotide, ligation of a polynucleotide ligation molecule to the scaffold polynucleotide, cleavage, incorporation of a nucleotide comprising a reversible terminator group or blocking group and deprotection.

The scheme shows the provision of a scaffold polynucleotide (201, 206) comprising a support strand (labelled "a") and a synthesis strand (labelled "b") hybridised thereto. The synthesis strand comprises a primer strand portion (dotted line). The terminal nucleotide of the support strand proximal to the primer strand portion comprises a ligatable group, preferably a terminal phosphate group as depicted in the Figure. The terminal nucleotide of the primer strand portion and the nucleotide paired therewith are depicted as "X". These two nucleotides can be any two nucleotides or analogs or derivatives thereof, and are not limited to being a naturally complementary pair of nucleotides.

The scheme shows the provision of a polynucleotide ligation molecule (202, 207; structure in the top right of the Figure). The polynucleotide ligation molecule comprises a helper strand (dashed line), a support strand hybridised thereto and a complementary ligation end. The terminal nucleotide of the support strand of the complementary ligation end is a first nucleotide of the predefined sequence and is depicted as "A" (adenosine). The terminal nucleotide of the helper strand of the complementary ligation end is depicted as "T" (thymine). The terminal nucleotide of the helper strand of the complementary ligation end comprises a non-ligatable nucleotide. The complementary ligation end comprises a universal nucleotide (depicted as "Un") in the support strand and which is paired with a partner nucleotide in the helper strand (depicted as "X"). The penultimate nucleotides of both the support strand and helper strand at the complementary ligation end are depicted as "X". A and T are depicted purely for illustration and can be any nucleotides or analogs or derivatives thereof. The nucleotides depicted as "X" can be any nucleotides or analogs or derivatives thereof. It is not necessary for paired nucleotides to comprise naturally complementary nucleotides.

The scheme shows the ligation of the support strand of the polynucleotide ligation molecule (202, 207) to the support strand of the scaffold polynucleotide and the creation of single-stranded break ("nick") in the synthesis strand between the helper strand and primer strand portion.

The scheme shows a cleavage step (203, 208) comprising cleaving the support strand (jagged arrowhead) at a cleavage site defined by a sequence comprising the universal nucleotide. Cleavage releases the polynucleotide ligation molecule comprising the universal nucleotide and leads to the retention of the first nucleotide of the predefined sequence in the scaffold polynucleotide. Following cleavage, the first nucleotide is the terminal nucleotide of the support strand of the cleaved scaffold polynucleotide and overhangs the terminal nucleotide of the primer strand portion in a single-nucleotide overhang. In synthesis method of the invention version 2 the support strand is cleaved between the nucleotide which occupies the next nucleotide position relative to the universal nucleotide in the direction proximal to the primer strand portion/distal to the helper strand portion and the nucleotide which occupies the second nucleotide position relative to the universal nucleotide in the direction proximal to the primer strand portion/distal to the helper strand portion.

The scheme shows the incorporation (204, 209) of a second nucleotide of the predefined sequence. This nucleotide comprises a reversible terminator group (triangle) and is depicted as "T" (thymine) purely for illustration, it can be any nucleotide or analog or derivative thereof.

The scheme shows a deprotection step (205, 210) comprising removal of the reversible terminator group from the second nucleotide of the predefined sequence.

In synthesis method of the invention version 2 in each cycle the first nucleotide of the predefined sequence and the second nucleotide of the predefined sequence form a nucleotide pair.

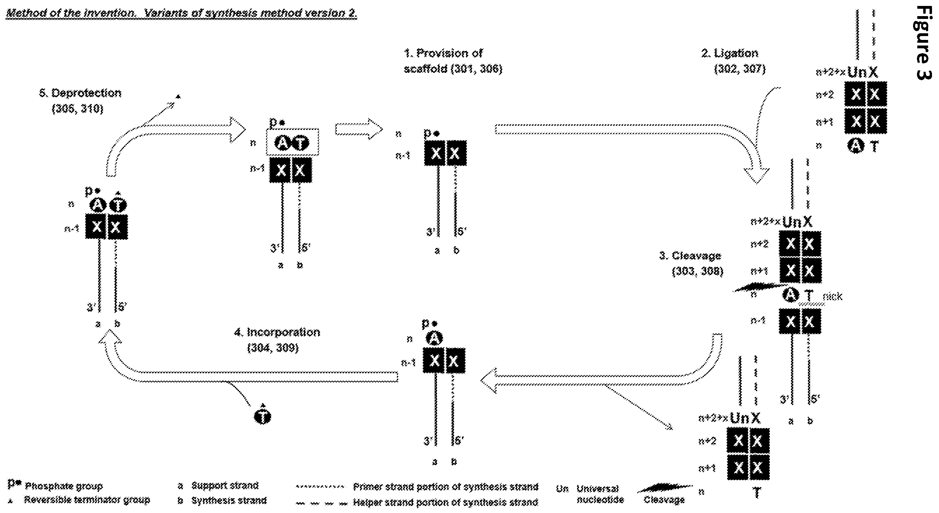

### Figure 3. Scheme Showing Variants of Exemplary Method Version 2 of the Invention.

Scheme showing a first synthesis cycle according to variants of exemplary method version 2 of the invention.

The method comprises a cycle of provision of a scaffold polynucleotide, ligation of a polynucleotide ligation molecule to the scaffold polynucleotide, cleavage, incorporation of a nucleotide comprising a reversible terminator group or blocking group and deprotection.

The scheme shows the provision of a scaffold polynucleotide (301, 306) comprising a support strand (labelled "a") and a synthesis strand (labelled "b") hybridised thereto. The synthesis strand comprises a primer strand portion (dotted line). The terminal nucleotide of the support strand proximal to the primer strand portion comprises a ligatable group, preferably a terminal phosphate group as depicted in the Figure. The terminal nucleotide of the primer strand portion and the nucleotide paired therewith are depicted as "X". These two nucleotides can be any two nucleotides or analogs or derivatives thereof, and are not limited to being a naturally complementary pair of nucleotides.

The scheme shows the provision of a polynucleotide ligation molecule (302, 307; structure in the top right of the Figure). The polynucleotide ligation molecule comprises a helper strand (dashed line), a support strand hybridised thereto and a complementary ligation end. The terminal nucleotide of the support strand of the complementary ligation end is a first nucleotide of the predefined sequence and is depicted as "A" (adenosine). The terminal nucleotide of the helper strand of the complementary ligation end is depicted as "T" (thymine). The terminal nucleotide of the helper strand of the complementary ligation end comprises a non-ligatable nucleotide. The complementary ligation end comprises a universal nucleotide (depicted as "Un") in the support strand and which is paired with a partner nucleotide in the helper strand (depicted as "X"). At the complementary ligation end two nucleotides, depicted as "X", are positioned between the universal nucleotide and the terminal nucleotide of the support strand and are paired with partner nucleotides in the helper strand, also depicted as "X". A and T are depicted purely for illustration and can be any nucleotides or analogs or derivatives thereof. The nucleotides depicted as "X" can be any nucleotides or analogs or derivatives thereof. It is not necessary for paired nucleotides to comprise naturally complementary nucleotides.

The scheme shows the ligation of the support strand of the polynucleotide ligation molecule (302, 307) to the support strand of the scaffold polynucleotide and the creation of single-stranded break ("nick") in the synthesis strand between the helper strand and primer strand portion.

The scheme shows a cleavage step (303, 308) comprising cleaving the support strand (jagged arrowhead) at a cleavage site defined by a sequence comprising the universal nucleotide. Cleavage releases the polynucleotide ligation molecule comprising the universal nucleotide and leads to the retention of the first nucleotide in the scaffold polynucleotide. In these variants of synthesis method of the invention version 2 the support strand is always cleaved between the position occupied by the first nucleotide of the predefined sequence and the position occupied by the next nucleotide in the support strand in the direction proximal to the helper strand/distal to the primer strand portion.

The scheme shows the incorporation (304, 309) of a second nucleotide of the predefined sequence. This nucleotide comprises a reversible terminator group (triangle) and is depicted as "T" (thymine) purely for illustration, it can be any nucleotide or analog or derivative thereof.

The scheme shows a deprotection step (305, 310) comprising removal of the reversible terminator group from the second nucleotide of the predefined sequence.

In all of these particular variants of synthesis method of the invention version 2, in each cycle the first nucleotide of the predefined sequence and the second nucleotide of the predefined sequence form a nucleotide pair.

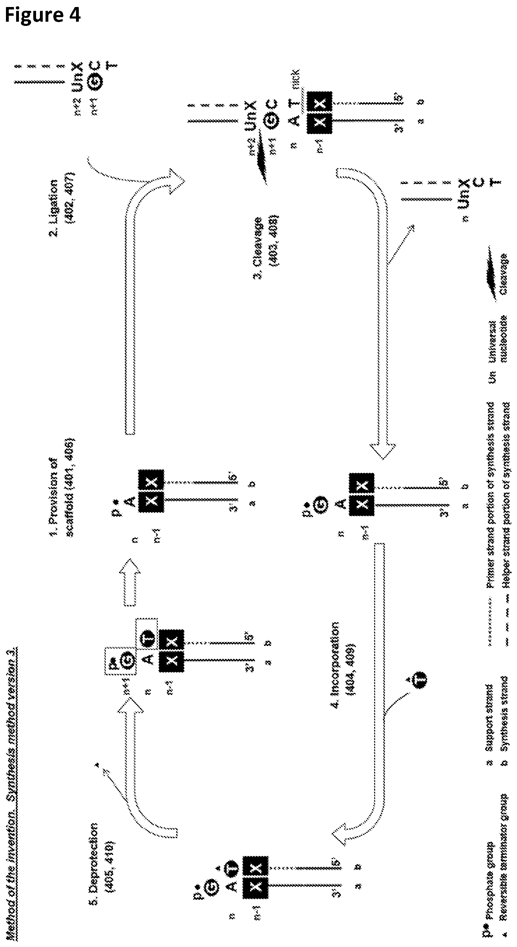

### Figure 4. Scheme of Exemplary Method Version 3 of the Invention.

Scheme showing a first synthesis cycle according to exemplary method version 3 of the invention.

The method comprises a cycle of provision of a scaffold polynucleotide, ligation of a polynucleotide ligation molecule to the scaffold polynucleotide, cleavage, incorporation of a nucleotide comprising a reversible terminator group or blocking group and deprotection.

The scheme shows the provision of a scaffold polynucleotide (401, 406) comprising a support strand (labelled "a") and a synthesis strand (labelled "b") hybridised thereto. The synthesis strand comprises a primer strand portion (dotted line). The terminal nucleotide of the support strand proximal to the primer strand portion comprises a ligatable group, preferably a terminal phosphate group as depicted in the Figure. The terminal nucleotide of the support strand proximal to the primer strand portion, depicted as "A" (adenosine) overhangs the terminal nucleotide of the primer strand portion in a single-nucleotide overhang. The terminal nucleotide of the primer strand portion is paired with the penultimate nucleotide of the support strand in a nucleotide pair. Both nucleotides of the pair are depicted as "X". These two nucleotides can be any two nucleotides or analogs or derivatives thereof, and are not limited to being a naturally complementary pair of nucleotides. The overhanging terminal nucleotide of the support strand can be any nucleotide or analog or derivative thereof.

The scheme shows the provision of a polynucleotide ligation molecule (402, 407; structure in the top right of the Figure). The polynucleotide ligation molecule comprises a helper strand (dashed line), a support strand hybridised thereto and a complementary ligation end. The terminal nucleotide of the support strand of the complementary ligation end is a first nucleotide of the predefined sequence and is depicted as "G" (guanine) and is paired with the penultimate nucleotide of the helper strand which is depicted as "C" (cytosine). The terminal nucleotide of the helper strand of the complementary ligation end is depicted as "T" (thymine) and overhangs the terminal nucleotide of the support strand of the complementary ligation end in a single-nucleotide overhang. The terminal nucleotide of the helper strand of the complementary ligation end comprises a non-ligatable nucleotide. The complementary ligation end comprises a universal nucleotide (depicted as "Un") in the support strand and which is paired with a partner nucleotide in the helper strand (depicted as "X"). G, C and T are depicted purely for illustration and can be any nucleotides or analogs or derivatives thereof. X can be any nucleotide or analog or derivative thereof. It is not necessary for paired nucleotides to comprise naturally complementary nucleotides.

The scheme shows the ligation of the support strand of the polynucleotide ligation molecule (402, 407) to the support strand of the scaffold polynucleotide and the creation of single-stranded break ("nick") in the synthesis strand between the helper strand and primer strand portion.

The scheme shows a cleavage step (403, 408) comprising cleaving the support strand (jagged arrowhead) at a cleavage site defined by a sequence comprising the universal nucleotide. Cleavage releases the polynucleotide ligation molecule comprising the universal nucleotide and leads to the retention of the first nucleotide in the scaffold polynucleotide. In synthesis method of the invention version 3 the support strand is cleaved between the position occupied by the universal nucleotide and the nucleotide which occupies the next nucleotide position in the support strand in the direction proximal to the primer strand portion/distal to the helper strand portion.

The scheme shows the incorporation (404, 409) of a second nucleotide of the predefined sequence. This nucleotide comprises a reversible terminator group (triangle) and is depicted as "T" (thymine) purely for illustration, it can be any nucleotide or analog or derivative thereof.

The scheme shows a deprotection step (405, 410) comprising removal of the reversible terminator group from the second nucleotide of the predefined sequence.

In synthesis method of the invention version 3 in each cycle following cleavage and incorporation the first nucleotide of the predefined sequence is unpaired, is the terminal nucleotide of the support strand proximal to the primer strand portion and is provided in a single-nucleotide overhang overhanging the second nucleotide of the predefined sequence which is the terminal nucleotide of the primer strand portion. Consequently, the first nucleotide of the predefined sequence and the second nucleotide of the predefined sequence do not form a nucleotide pair with each other.

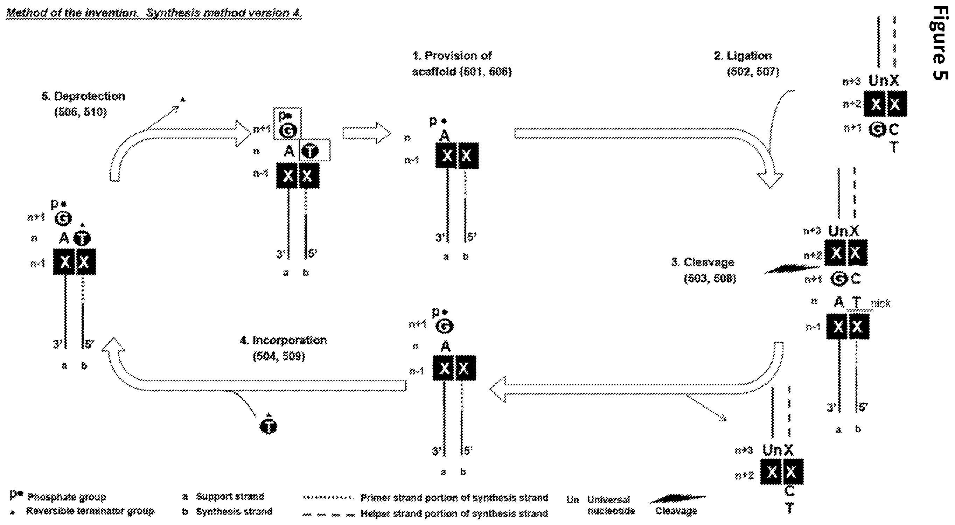

### Figure 5. Scheme of Exemplary Method Version 4 of the Invention.

Scheme showing a first synthesis cycle according to exemplary method version 4 of the invention.

The method comprises a cycle of provision of a scaffold polynucleotide, ligation of a polynucleotide ligation molecule to the scaffold polynucleotide, cleavage, incorporation of a nucleotide comprising a reversible terminator group or blocking group and deprotection.

The scheme shows the provision of a scaffold polynucleotide (501, 506) comprising a support strand (labelled "a") and a synthesis strand (labelled "b") hybridised thereto. The synthesis strand comprises a primer strand portion (dotted line). The terminal nucleotide of the support strand proximal to the primer strand portion comprises a ligatable group, preferably a terminal phosphate group as depicted in the Figure. The terminal nucleotide of the support strand proximal to the primer strand portion, depicted as "A" (adenosine) overhangs the terminal nucleotide of the primer strand portion in a single-nucleotide overhang. The terminal nucleotide of the primer strand portion is paired with the penultimate nucleotide of the support strand in a nucleotide pair. Both nucleotides of the pair are depicted as "X". These two nucleotides can be any two nucleotides or analogs or derivatives thereof, and are not limited to being a naturally complementary pair of nucleotides. The overhanging terminal nucleotide of the support strand can be any nucleotide or analog or derivative thereof.

The scheme shows the provision of a polynucleotide ligation molecule (502, 507; structure in the top right of the Figure). The polynucleotide ligation molecule comprises a helper strand (dashed line), a support strand hybridised thereto and a complementary ligation end. The terminal nucleotide of the support strand of the complementary ligation end is a first nucleotide of the predefined sequence and is depicted as "G" (guanine) and is paired with the penultimate nucleotide of the helper strand which is depicted as "C" (cytosine). The terminal nucleotide of the helper strand of the complementary ligation end is depicted as "T" (thymine) and overhangs the terminal nucleotide of the support strand of the complementary ligation end in a single-nucleotide overhang. The terminal nucleotide of the helper strand of the complementary ligation end comprises a non-ligatable nucleotide. The complementary ligation end comprises a universal nucleotide (depicted as "Un") in the support strand and which is paired with a partner nucleotide in the helper strand (depicted as "X"). The penultimate nucleotide of the support strand is depicted as "X" and is paired with a partner nucleotide in the helper strand. The partner nucleotide is also depicted as "X". G, C and T are depicted purely for illustration and can be any nucleotides or analogs or derivatives thereof. The nucleotides depicted as "X" can be any nucleotides or analogs or derivatives thereof. It is not necessary for paired nucleotides to comprise naturally complementary nucleotides.

The scheme shows the ligation of the support strand of the polynucleotide ligation molecule (502, 507) to the support strand of the scaffold polynucleotide and the creation of single-stranded break ("nick") in the synthesis strand between the helper strand and primer strand portion.

The scheme shows a cleavage step (503, 508) comprising cleaving the support strand (jagged arrowhead) at a cleavage site defined by a sequence comprising the universal nucleotide. Cleavage releases the polynucleotide ligation molecule comprising the universal nucleotide and leads to the retention of the first nucleotide in the scaffold polynucleotide. In synthesis method of the invention version 4 the support strand is cleaved between the nucleotide which occupies the next nucleotide position relative to the universal nucleotide in the direction proximal to the primer strand portion/distal to the helper strand portion and the nucleotide which occupies the second nucleotide position relative to the universal nucleotide in the direction proximal to the primer strand portion/distal to the helper strand portion.

The scheme shows the incorporation (504, 509) of a second nucleotide of the predefined sequence. This nucleotide comprises a reversible terminator group (triangle) and is depicted as "T" (thymine) purely for illustration, it can be any nucleotide or analog or derivative thereof.

The scheme shows a deprotection step (505, 510) comprising removal of the reversible terminator group from the second nucleotide of the predefined sequence.

In synthesis method of the invention version 4 in each cycle following cleavage and incorporation the first nucleotide of the predefined sequence is unpaired, is the terminal nucleotide of the support strand proximal to the primer strand portion and is provided in a single-nucleotide overhang overhanging the second nucleotide of the predefined sequence which is the terminal nucleotide of the primer strand portion. Consequently, the first nucleotide of the predefined sequence and the second nucleotide of the predefined sequence do not form a nucleotide pair with each other.

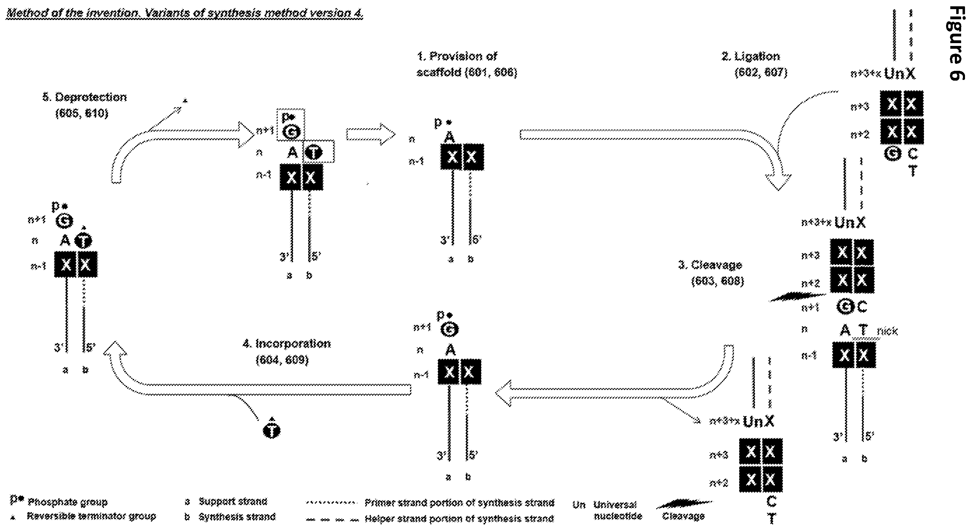

### Figure 6. Scheme Showing Variants of Exemplary Method Version 4 of the Invention.

Scheme showing a first synthesis cycle according to variants of exemplary method version 4 of the invention.

The method comprises a cycle of provision of a scaffold polynucleotide, ligation of a polynucleotide ligation molecule to the scaffold polynucleotide, cleavage, incorporation of a nucleotide comprising a reversible terminator group or blocking group and deprotection.

The scheme shows the provision of a scaffold polynucleotide (601, 606) comprising a support strand (labelled "a") and a synthesis strand (labelled "b") hybridised thereto. The synthesis strand comprises a primer strand portion (dotted line). The terminal nucleotide of the support strand proximal to the primer strand portion comprises a ligatable group, preferably a terminal phosphate group as depicted in the Figure. The terminal nucleotide of the support strand proximal to the primer strand portion, depicted as "A" (adenosine) overhangs the terminal nucleotide of the primer strand portion in a single-nucleotide overhang. The terminal nucleotide of the primer strand portion is paired with the penultimate nucleotide of the support strand in a nucleotide pair. Both nucleotides of the pair are depicted as "X". These two nucleotides can be any two nucleotides or analogs or derivatives thereof, and are not limited to being a naturally complementary pair of nucleotides. The overhanging terminal nucleotide of the support strand can be any nucleotide or analog or derivative thereof.

The scheme shows the provision of a polynucleotide ligation molecule (602, 607; structure in the top right of the Figure). The polynucleotide ligation molecule comprises a helper strand (dashed line), a support strand hybridised thereto and a complementary ligation end. The terminal nucleotide of the support strand of the complementary ligation end is a first nucleotide of the predefined sequence and is depicted as "G" (guanine) and is paired with the penultimate nucleotide of the helper strand which is depicted as "C" (cytosine). The terminal nucleotide of the helper strand of the complementary ligation end is depicted as "T" (thymine) and overhangs the terminal nucleotide of the support strand of the complementary ligation end in a single-nucleotide overhang. The terminal nucleotide of the helper strand of the complementary ligation end comprises a non-ligatable nucleotide. The complementary ligation end comprises a universal nucleotide (depicted as "Un") in the support strand and which is paired with a partner nucleotide in the helper strand (depicted as "X"). At the complementary ligation end two nucleotides, depicted as "X", are positioned between the universal nucleotide and the terminal nucleotide in the support strand and are paired with partner nucleotides in the helper strand. The partner nucleotides are also depicted as "X". G, C and T are depicted purely for illustration and can be any nucleotides or analogs or derivatives thereof. The nucleotides depicted as "X" can be any nucleotides or analogs or derivatives thereof. It is not necessary for paired nucleotides to comprise naturally complementary nucleotides.

The scheme shows the ligation of the support strand of the polynucleotide ligation molecule (602, 607) to the support strand of the scaffold polynucleotide and the creation of single-stranded break ("nick") in the synthesis strand between the helper strand and primer strand portion.

The scheme shows a cleavage step (603, 608) comprising cleaving the support strand (jagged arrowhead) at a cleavage site defined by a sequence comprising the universal nucleotide. Cleavage releases the polynucleotide ligation molecule comprising the universal nucleotide and leads to the retention of the first nucleotide in the scaffold polynucleotide. In these variants of synthesis method of the invention version 4 the support strand is always cleaved between the position occupied by the first nucleotide of the predefined sequence and the position occupied by the next nucleotide in the support strand in the direction proximal to the helper strand/distal to the primer strand portion.

The scheme shows the incorporation (604, 609) of a second nucleotide of the predefined sequence. This nucleotide comprises a reversible terminator group (triangle) and is depicted as "T" (thymine) purely for illustration, it can be any nucleotide or analog or derivative thereof.

The scheme shows a deprotection step (605, 610) comprising removal of the reversible terminator group from the second nucleotide of the predefined sequence.

In these variants of synthesis method of the invention version 4 in each cycle following cleavage and incorporation the first nucleotide of the predefined sequence is unpaired, is the terminal nucleotide of the support strand proximal to the primer strand portion and is provided in a single-nucleotide overhang overhanging the second nucleotide of the predefined sequence which is the terminal nucleotide of the primer strand portion. Consequently, the first nucleotide of the predefined sequence and the second nucleotide of the predefined sequence do not form a nucleotide pair with each other.

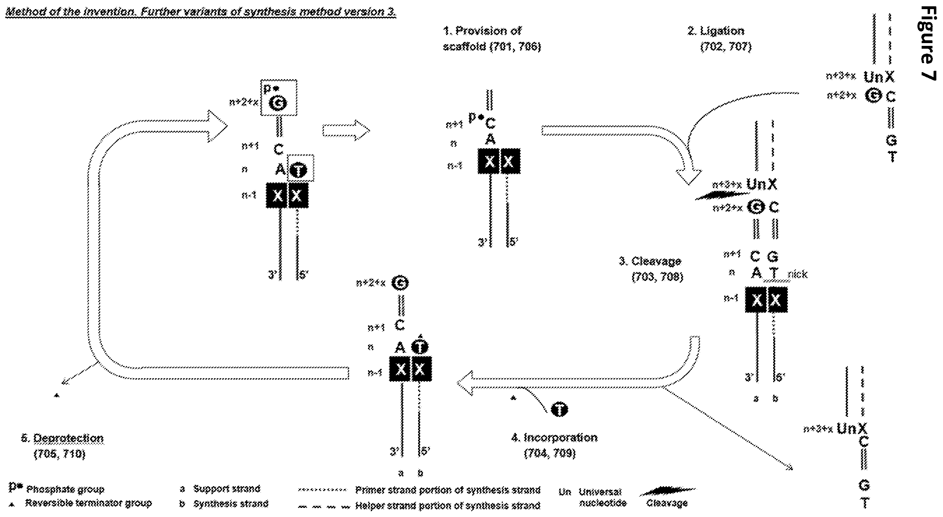

### Figure 7. Scheme Showing Further Variants of Exemplary Method Version 3 of the Invention.

Scheme showing a first synthesis cycle according to further variants of exemplary method version 3 of the invention.

The method comprises a cycle of provision of a scaffold polynucleotide, ligation of a polynucleotide ligation molecule to the scaffold polynucleotide, cleavage, incorporation of a nucleotide comprising a reversible terminator group or blocking group and deprotection.

The scheme shows the provision of a scaffold polynucleotide (701, 706) comprising a support strand (labelled "a") and a synthesis strand (labelled "b") hybridised thereto. The synthesis strand comprises a primer strand portion (dotted line). The terminal nucleotide of the support strand proximal to the primer strand portion comprises a ligatable group, preferably a terminal phosphate group as depicted in the Figure. The terminal nucleotide of the support strand proximal to the primer strand portion is depicted as "C" (cytosine). The penultimate nucleotide of the support strand is depicted as "A" (adenosine). The terminal and penultimate nucleotides of the support strand overhang the terminal nucleotide of the primer strand portion in a multiple-nucleotide overhang. The support strand may optionally comprise one or more further nucleotides, depicted by parallel vertical lines. The terminal nucleotide of the primer strand portion is paired with a partner nucleotide in the support strand in a nucleotide pair. Both nucleotides of the pair are depicted as "X". These two nucleotides can be any two nucleotides or analogs or derivatives thereof, and are not limited to being a naturally complementary pair of nucleotides. The overhanging nucleotides of the support strand can be any nucleotides or analogs or derivatives thereof.

The scheme shows the provision of a polynucleotide ligation molecule (702, 707; structure in the top right of the Figure). The polynucleotide ligation molecule comprises a helper strand (dashed line), a support strand hybridised thereto and a complementary ligation end. The terminal nucleotide of the support strand of the complementary ligation end is a first nucleotide of the predefined sequence and is depicted as "G" (guanine) and is paired with a partner nucleotide in the helper strand which is depicted as "C" (cytosine). The terminal nucleotide of the helper strand of the complementary ligation end is depicted as "T" (thymine). The penultimate nucleotide of the helper strand is depicted as "G" (guanine). The terminal and penultimate nucleotides of the helper strand overhang the terminal nucleotide of the support strand of the complementary ligation end in a multiple-nucleotide overhang. The helper strand may optionally comprise one or more further nucleotides prior to the terminal and penultimate nucleotides, depicted by parallel vertical lines. The terminal nucleotide of the helper strand of the complementary ligation end comprises a non-ligatable nucleotide. The complementary ligation end comprises a universal nucleotide (depicted as "Un") in the support strand and which is paired with a partner nucleotide in the helper strand (depicted as "X"). G, C and T are depicted purely for illustration and can be any nucleotides or analogs or derivatives thereof. X can be any nucleotide or analog or derivative thereof. It is not necessary for paired nucleotides to comprise naturally complementary nucleotides.

The scheme shows the ligation of the support strand of the polynucleotide ligation molecule (702, 707) to the support strand of the scaffold polynucleotide and the creation of single-stranded break ("nick") in the synthesis strand between the helper strand and primer strand portion.

The scheme shows a cleavage step (703, 708) comprising cleaving the support strand (jagged arrowhead) at a cleavage site defined by a sequence comprising the universal nucleotide. Cleavage releases the polynucleotide ligation molecule comprising the universal nucleotide and leads to the retention of the first nucleotide in the scaffold polynucleotide. In these particular variants of synthesis method of the invention version 3 the support strand is always cleaved between the position occupied by the universal nucleotide and the nucleotide which occupies the next nucleotide position in the support strand in the direction proximal to the primer strand portion/distal to the helper strand portion.

The scheme shows the incorporation (704, 709) of a second nucleotide of the predefined sequence. This nucleotide comprises a reversible terminator group (triangle) and is depicted as "T" (thymine) purely for illustration, it can be any nucleotide or analog or derivative thereof.

The scheme shows a deprotection step (705, 710) comprising removal of the reversible terminator group from the second nucleotide of the predefined sequence.

In these particular variants of synthesis method of the invention version 3, in each cycle following cleavage and incorporation the first nucleotide of the predefined sequence is unpaired, is the terminal nucleotide of the support strand proximal to the primer strand portion and is provided in a multiple-nucleotide overhang overhanging the second nucleotide of the predefined sequence which is the terminal nucleotide of the primer strand portion. Consequently, the first nucleotide of the predefined sequence and the second nucleotide of the predefined sequence do not form a nucleotide pair with each other.

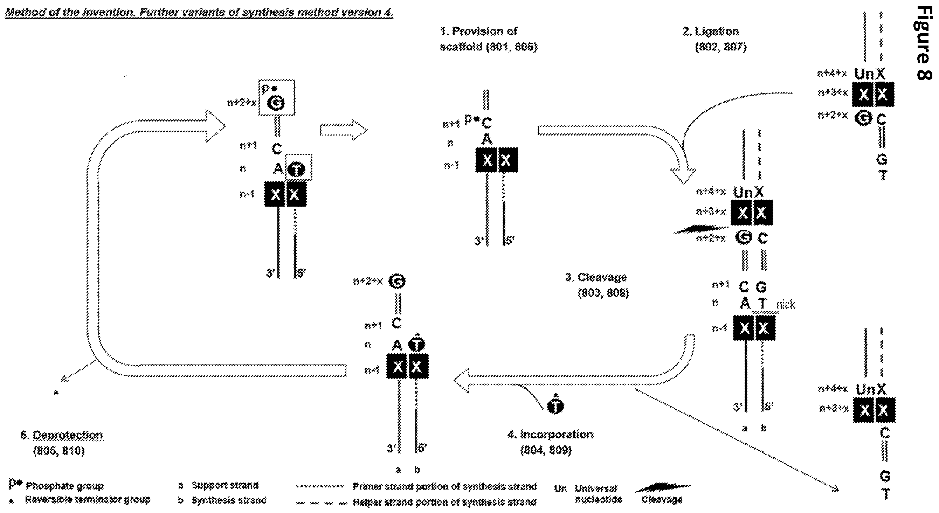

### Figure 8. Scheme Showing Further Variants of Exemplary Method Version 4 of the Invention.

Scheme showing a first synthesis cycle according to further variants of exemplary method version 4 of the invention.

The method comprises a cycle of provision of a scaffold polynucleotide, ligation of a polynucleotide ligation molecule to the scaffold polynucleotide, cleavage, incorporation of a nucleotide comprising a reversible terminator group or blocking group and deprotection.

The scheme shows the provision of a scaffold polynucleotide (801, 806) comprising a support strand (labelled "a") and a synthesis strand (labelled "b") hybridised thereto. The synthesis strand comprises a primer strand portion (dotted line). The terminal nucleotide of the support strand proximal to the primer strand portion comprises a ligatable group, preferably a terminal phosphate group as depicted in the Figure. The terminal nucleotide of the support strand proximal to the primer strand portion is depicted as "C" (cytosine). The penultimate nucleotide of the support strand is depicted as "A" (adenosine). The terminal and penultimate nucleotides of the support strand overhang the terminal nucleotide of the primer strand portion in a multiple-nucleotide overhang. The support strand may optionally comprise one or more further nucleotides, depicted by parallel vertical lines. The terminal nucleotide of the primer strand portion is paired with a partner nucleotide in the support strand in a nucleotide pair. Both nucleotides of the pair are depicted as "X". These two nucleotides can be any two nucleotides or analogs or derivatives thereof, and are not limited to being a naturally complementary pair of nucleotides. The overhanging nucleotides of the support strand can be any nucleotides or analogs or derivatives thereof.

The scheme shows the provision of a polynucleotide ligation molecule (802, 807; structure in the top right of the Figure). The polynucleotide ligation molecule comprises a helper strand (dashed line), a support strand hybridised thereto and a complementary ligation end. The terminal nucleotide of the support strand of the complementary ligation end is a first nucleotide of the predefined sequence and is depicted as "G" (guanine) and is paired with a partner nucleotide in the helper strand which is depicted as "C" (cytosine). The terminal nucleotide of the helper strand of the complementary ligation end is depicted as "T" (thymine). The penultimate nucleotide of the helper strand is depicted as "G" (guanine). The terminal and penultimate nucleotides of the helper strand overhang the terminal nucleotide of the support strand of the complementary ligation end in a multiple-nucleotide overhang. The helper strand may optionally comprise one or more further nucleotides prior to the terminal and penultimate nucleotides, depicted by parallel vertical lines. The terminal nucleotide of the helper strand of the complementary ligation end comprises a non-ligatable nucleotide. The complementary ligation end comprises a universal nucleotide (depicted as "Un") in the support strand and which is paired with a partner nucleotide in the helper strand (depicted as "X"). A further nucleotide (depicted as "X") is positioned between the first nucleotide of the predefined sequence and the universal nucleotide in the support strand of the complementary ligation end and is paired with a partner nucleotide in the helper strand (also depicted as "X"). G, C and T are depicted purely for illustration and can be any nucleotides or analogs or derivatives thereof. X can be any nucleotide or analog or derivative thereof. It is not necessary for paired nucleotides to comprise naturally complementary nucleotides.

The scheme shows the ligation of the support strand of the polynucleotide ligation molecule (802, 807) to the support strand of the scaffold polynucleotide and the creation of single-stranded break ("nick") in the synthesis strand between the helper strand and primer strand portion.

The scheme shows a cleavage step (803, 808) comprising cleaving the support strand (jagged arrowhead) at a cleavage site defined by a sequence comprising the universal nucleotide. Cleavage releases the polynucleotide ligation molecule comprising the universal nucleotide and leads to the retention of the first nucleotide in the scaffold polynucleotide. In these particular variants of synthesis method of the invention version 4 the support strand is always cleaved between the nucleotide which occupies the next nucleotide position relative to the universal nucleotide in the direction proximal to the primer strand portion/distal to the helper strand portion and the nucleotide which occupies the second nucleotide position relative to the universal nucleotide in the direction proximal to the primer strand portion/distal to the helper strand portion.

The scheme shows the incorporation (804, 809) of a second nucleotide of the predefined sequence. This nucleotide comprises a reversible terminator group (triangle) and is depicted as "T" (thymine) purely for illustration, it can be any nucleotide or analog or derivative thereof.

The scheme shows a deprotection step (805, 810) comprising removal of the reversible terminator group from the second nucleotide of the predefined sequence.

In these particular variants of synthesis method of the invention version 4 in each cycle following cleavage and incorporation the first nucleotide of the predefined sequence is unpaired, is the terminal nucleotide of the support strand proximal to the primer strand portion and is provided in a multiple-nucleotide overhang overhanging the second nucleotide of the predefined sequence which is the terminal nucleotide of the primer strand portion. Consequently, the first nucleotide of the predefined sequence and the second nucleotide of the predefined sequence do not form a nucleotide pair with each other.

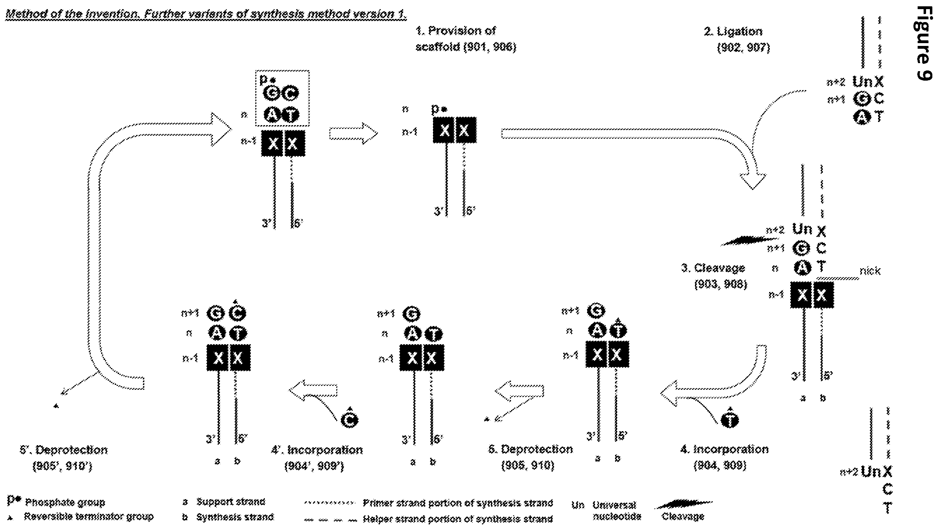

### Figure 9. Scheme Showing Variants of Exemplary Method Version 1 of the Invention Involving Incorporation of More Than Two Nucleotides Per Cycle.

Scheme showing a first synthesis cycle according to variants of exemplary method version 1 of the invention involving the incorporation of multiple nucleotides in both steps of ligation and incorporation.

The method comprises a cycle of provision of a scaffold polynucleotide, ligation of a polynucleotide ligation molecule to the scaffold polynucleotide, cleavage and multiple steps of (a) incorporation of a nucleotide comprising a reversible terminator group or blocking group followed by (b) deprotection.

The scheme shows the provision of a scaffold polynucleotide (901, 906) comprising a support strand (labelled "a") and a synthesis strand (labelled "b") hybridised thereto. The synthesis strand comprises a primer strand portion (dotted line). The terminal nucleotide of the support strand proximal to the primer strand portion comprises a ligatable group, preferably a terminal phosphate group as depicted in the Figure. The terminal nucleotide of the primer strand portion and the nucleotide paired therewith are depicted as "X". These two nucleotides can be any two nucleotides or analogs or derivatives thereof, and are not limited to being a naturally complementary pair of nucleotides.

The scheme shows the provision of a polynucleotide ligation molecule (902, 907; structure in the top right of the Figure). The polynucleotide ligation molecule comprises a helper strand (dashed line), a support strand hybridised thereto and a complementary ligation end. The terminal nucleotide of the support strand of the complementary ligation end is a first nucleotide of the predefined sequence and is depicted as "A" (adenosine). The terminal nucleotide of the helper strand of the complementary ligation end is depicted as "T" (thymine). The terminal nucleotide of the helper strand of the complementary ligation end comprises a non-ligatable nucleotide. The penultimate nucleotide of the support strand is a further nucleotide of the predefined sequence and is depicted as "G" (guanine) and is paired with the penultimate nucleotide of the helper strand which is depicted as "C" (cytosine). The complementary ligation end comprises a universal nucleotide (depicted as "Un") in the support strand and which is paired with a partner nucleotide in the helper strand (depicted as "X"). A, T, G and C are depicted purely for illustration and can be any nucleotides or analogs or derivatives thereof. X can be any nucleotide or analog or derivative thereof. It is not necessary for paired nucleotides to comprise naturally complementary nucleotides.

The scheme shows the ligation of the support strand of the polynucleotide ligation molecule (902, 907) to the support strand of the scaffold polynucleotide and the creation of single-stranded break ("nick") in the synthesis strand between the helper strand and primer strand portion.

The scheme shows a cleavage step (903, 908) comprising cleaving the support strand (jagged arrowhead) at a cleavage site defined by a sequence comprising the universal nucleotide. Cleavage releases the polynucleotide ligation molecule comprising the universal nucleotide and leads to the retention of the first and further nucleotides in the scaffold polynucleotide. In this particular variant of synthesis method of the invention version 1 the support strand is cleaved between the position occupied by the universal nucleotide and the nucleotide which occupies the next nucleotide position in the support strand in the direction proximal to the primer strand portion/distal to the helper strand portion.

The scheme shows the incorporation (904, 909) of a second nucleotide of the predefined sequence. This nucleotide comprises a reversible terminator group (triangle) and is depicted as "T" (thymine) purely for illustration, it can be any nucleotide or analog or derivative thereof. Upon incorporation the second nucleotide forms a nucleotide pair with the first nucleotide.

The scheme shows a deprotection step following incorporation of the second nucleotide (905, 910) comprising removal of the reversible terminator group from the second nucleotide of the predefined sequence.

The scheme shows the incorporation (904', 909') of a further nucleotide of the predefined sequence. This nucleotide comprises a reversible terminator group (triangle) and is depicted as "C" (cytosine). Upon incorporation the further nucleotide forms a nucleotide pair with the further nucleotide provided by the polynucleotide ligation molecule in step (2), depicted as "G" (guanine). Cytosine and guanine are depicted purely for illustration, these nucleotides can be any nucleotides or analogs or derivatives thereof and are not limited to being a naturally complementary pair of nucleotides.

The scheme shows a second deprotection step following incorporation of the further nucleotide (905', 910') comprising removal of the reversible terminator group from the further nucleotide of the predefined sequence.

In all of these particular variants of synthesis method of the invention version 1, in each cycle the first nucleotide of the predefined sequence and the second nucleotide of the predefined sequence form a nucleotide pair, the first further nucleotide provided by the polynucleotide ligation molecule in step (2) and the first further nucleotide incorporated in step (4') form a nucleotide pair, and so on and so forth.

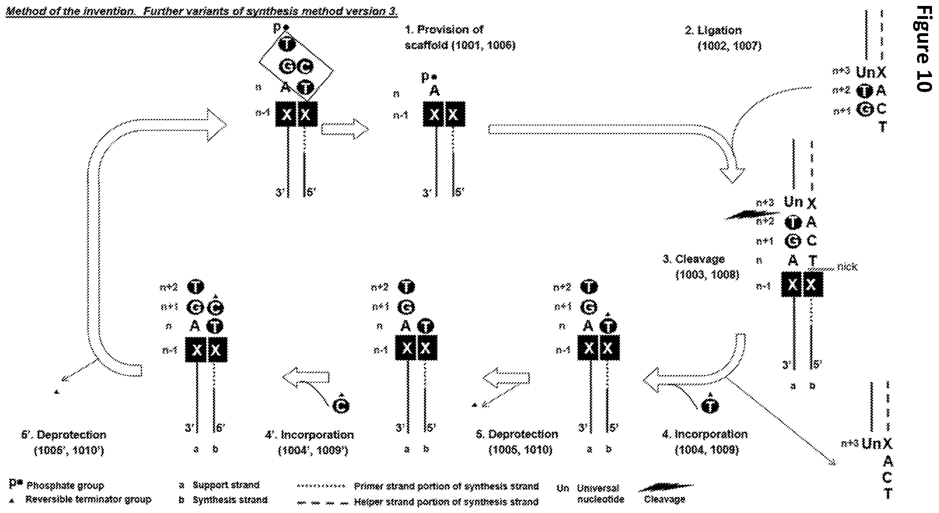

### Figure 10. Scheme Showing Variants of Exemplary Method Version 3 of the Invention Involving Incorporation of More Than Two Nucleotides Per Cycle.

Scheme showing a first synthesis cycle according to variants of exemplary method version 3 of the invention involving the incorporation of multiple nucleotides in both steps of ligation and incorporation.

The method comprises a cycle of provision of a scaffold polynucleotide, ligation of a polynucleotide ligation molecule to the scaffold polynucleotide, cleavage and then multiple steps of (a) incorporation of a nucleotide comprising a reversible terminator group or blocking group followed by (b) deprotection.

The scheme shows the provision of a scaffold polynucleotide (1001, 1006) comprising a support strand (labelled "a") and a synthesis strand (labelled "b") hybridised thereto. The synthesis strand comprises a primer strand portion (dotted line). The terminal nucleotide of the support strand proximal to the primer strand portion comprises a ligatable group, preferably a terminal phosphate group as depicted in the Figure. The terminal nucleotide of the support strand proximal to the primer strand portion, depicted as "A" (adenosine) overhangs the terminal nucleotide of the primer strand portion in a single-nucleotide overhang. The terminal nucleotide of the primer strand portion is paired with the penultimate nucleotide of the support strand in a nucleotide pair. Both nucleotides of the pair are depicted as "X". These two nucleotides can be any two nucleotides or analogs or derivatives thereof, and are not limited to being a naturally complementary pair of nucleotides. The overhanging terminal nucleotide of the support strand can be any nucleotide or analog or derivative thereof.

The scheme shows the provision of a polynucleotide ligation molecule (1002, 1007; structure in the top right of the Figure). The polynucleotide ligation molecule comprises a helper strand (dashed line), a support strand hybridised thereto and a complementary ligation end. The terminal nucleotide of the support strand of the complementary ligation end is a first nucleotide of the predefined sequence and is depicted as "G" (guanine) and is paired with the penultimate nucleotide of the helper strand which is depicted as "C" (cytosine). The terminal nucleotide of the helper strand of the complementary ligation end is depicted as "T" (thymine) and overhangs the terminal nucleotide of the support strand of the complementary ligation end in a single-nucleotide overhang. The terminal nucleotide of the helper strand of the complementary ligation end comprises a non-ligatable nucleotide. The penultimate nucleotide of the support strand is a further nucleotide of the predefined sequence and is depicted as "T" (thymine) and is paired with a partner nucleotide in the helper strand which is depicted as "A" (adenine). The complementary ligation end comprises a universal nucleotide (depicted as "Un") in the support strand and which is paired with a partner nucleotide in the helper strand (depicted as "X"). A, T, G and C are depicted purely for illustration and can be any nucleotides or analogs or derivatives thereof. X can be any nucleotide or analog or derivative thereof. It is not necessary for paired nucleotides to comprise naturally complementary nucleotides.

The scheme shows the ligation of the support strand of the polynucleotide ligation molecule (1002, 1007) to the support strand of the scaffold polynucleotide and the creation of single-stranded break ("nick") in the synthesis strand between the helper strand and primer strand portion.

The scheme shows a cleavage step (1003, 1008) comprising cleaving the support strand (jagged arrowhead) at a cleavage site defined by a sequence comprising the universal nucleotide. Cleavage releases the polynucleotide ligation molecule comprising the universal nucleotide and leads to the retention of the first and further nucleotides in the scaffold polynucleotide. In this particular variant of synthesis method of the invention version 3 the support strand is cleaved between the position occupied by the universal nucleotide and the nucleotide which occupies the next nucleotide position in the support strand in the direction proximal to the primer strand portion/distal to the helper strand portion.

The scheme shows the incorporation (1004, 1009) of a second nucleotide of the predefined sequence. This nucleotide comprises a reversible terminator group (triangle) and is depicted as "T" (thymine) purely for illustration, it can be any nucleotide or analog or derivative thereof. Upon incorporation the second nucleotide forms a nucleotide pair with the first nucleotide.

The scheme shows a deprotection step following incorporation of the second nucleotide (1005, 1010) comprising removal of the reversible terminator group from the second nucleotide of the predefined sequence.

The scheme shows the incorporation (1004', 1009') of a further nucleotide of the predefined sequence. This nucleotide comprises a reversible terminator group (triangle) and is depicted as "C" (cytosine). Upon incorporation the further nucleotide forms a nucleotide pair with the first nucleotide of the predefined sequence which was provided by the polynucleotide ligation molecule in step (2), depicted as "G" (guanine). Cytosine and guanine are depicted purely for illustration, these nucleotides can be any nucleotides or analogs or derivatives thereof and are not limited to being a naturally complementary pair of nucleotides.

The scheme shows a second deprotection step following incorporation of the further nucleotide (1005', 1010') comprising removal of the reversible terminator group from the further nucleotide of the predefined sequence.

In all of these particular variants of synthesis method of the invention version 3, following cleavage and incorporation the further nucleotide of the pred efined sequence provided by the polynucleotide ligation molecule at step (2) is unpaired, is the terminal nucleotide of the support strand proximal to the primer strand portion and is provided in a single-nucleotide overhang overhanging the further nucleotide of the predefined sequence provided at incorporation step (4') which is the terminal nucleotide of the primer strand portion. Consequently, the first nucleotide of the predefined sequence and the second nucleotide of the predefined sequence do not form a nucleotide pair with each other, the first further nucleotide provided by the polynucleotide ligation molecule in step (2) and the first further nucleotide incorporated in step (4') do not form a nucleotide pair with each other, and so on and so forth.

### Figure 11. Scheme of Exemplary Method Version 1.

Scheme showing a first synthesis cycle according to exemplary method version 1 of the Examples section. This method is provided for illustrative support only and is not within the scope of the claimed invention. The method comprises a cycle of provision of a scaffold polynucleotide, incorporation, cleavage, ligation and deprotection. The scheme shows the incorporation of a thymine nucleotide in the first synthesis cycle (101, 102) and its pairing opposite a partner adenine nucleotide (104), as well as the provision of a scaffold polynucleotide (106) for use in the next synthesis cycle. This pair is shown for illustration purposes only and is not limiting, it can be any pair depending on the required predefined sequence. Nucleotide Z can be any nucleotide. Nucleotide X can be any appropriate nucleotide. The Figure also shows reference signs corresponding to a second synthesis cycle.

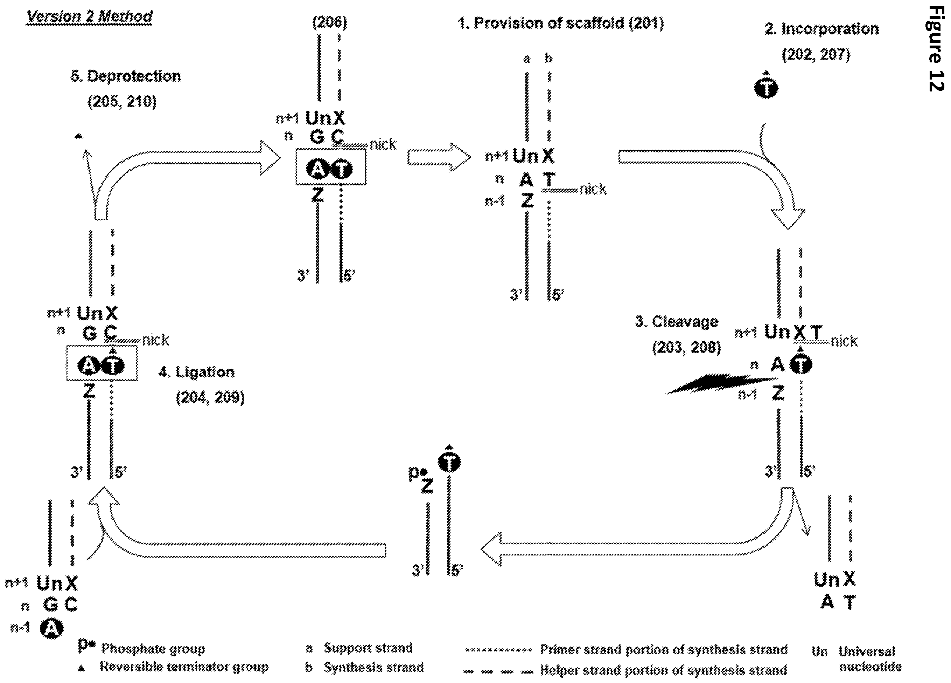

### Figure 12. Scheme of Exemplary Method Version 2.

Scheme showing a first synthesis cycle according to exemplary method version 2 of the Examples section. This method is provided for illustrative support only and is not within the scope of the claimed invention. The method comprises a cycle of provision of a scaffold polynucleotide, incorporation, cleavage, ligation and deprotection. The scheme shows the incorporation in the first cycle (201, 202) of a thymine nucleotide and its pairing opposite a partner adenine nucleotide (204), as well as the provision of a scaffold polynucleotide (206) comprising a guanine for pairing with a cytosine in the next synthesis cycle. These pairs are shown for illustration purposes only and are not limiting, they can be any pairs depending on the required predefined sequence. Nucleotide Z can be any nucleotide. Nucleotide X can be any appropriate nucleotide. The Figure also shows reference signs corresponding to a second synthesis cycle.

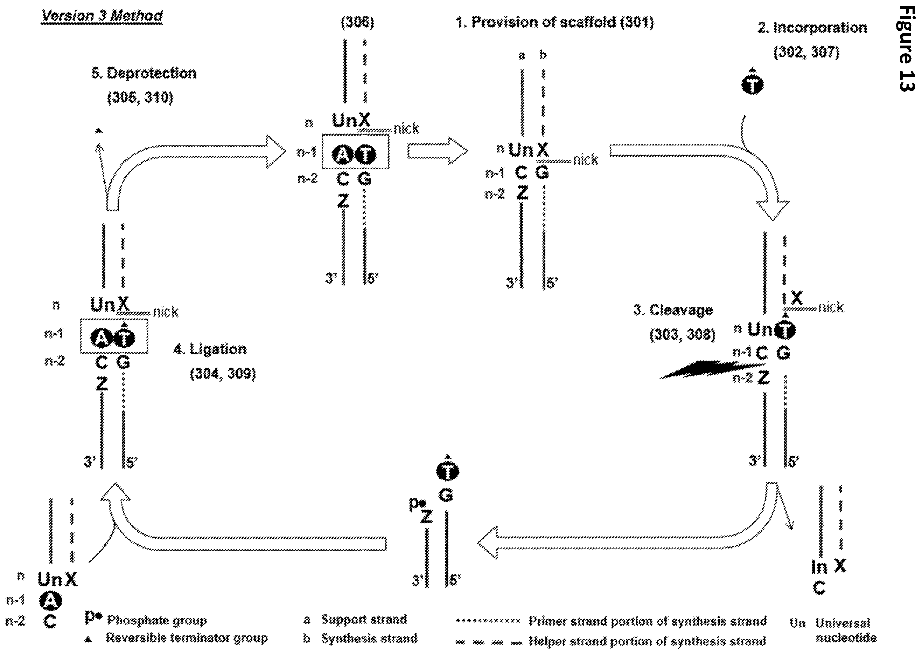

### Figure 13. Scheme of Exemplary Method Version 3.

Scheme showing a first synthesis cycle according to exemplary method version 3 of the Examples section. This method is provided for illustrative support only and is not within the scope of the claimed invention. The method comprises a cycle of provision of a scaffold polynucleotide, incorporation, cleavage, ligation and deprotection. The scheme shows the incorporation in the first cycle (301, 302) of a thymine nucleotide and its pairing opposite a partner adenine nucleotide (304), as well as the provision of a scaffold polynucleotide (306) for use in the next synthesis cycle. This pair is shown for illustration purposes only and is not limiting, it can be any pair depending on the required predefined sequence. The scheme also shows a cytosine-guanine pair as a component of the scaffold polynucleotide and which is not part of the predefined sequence. This pair is also shown for illustration purposes only and is not limiting, it can be any pair. Nucleotide Z can be any nucleotide. Nucleotide X can be any appropriate nucleotide.

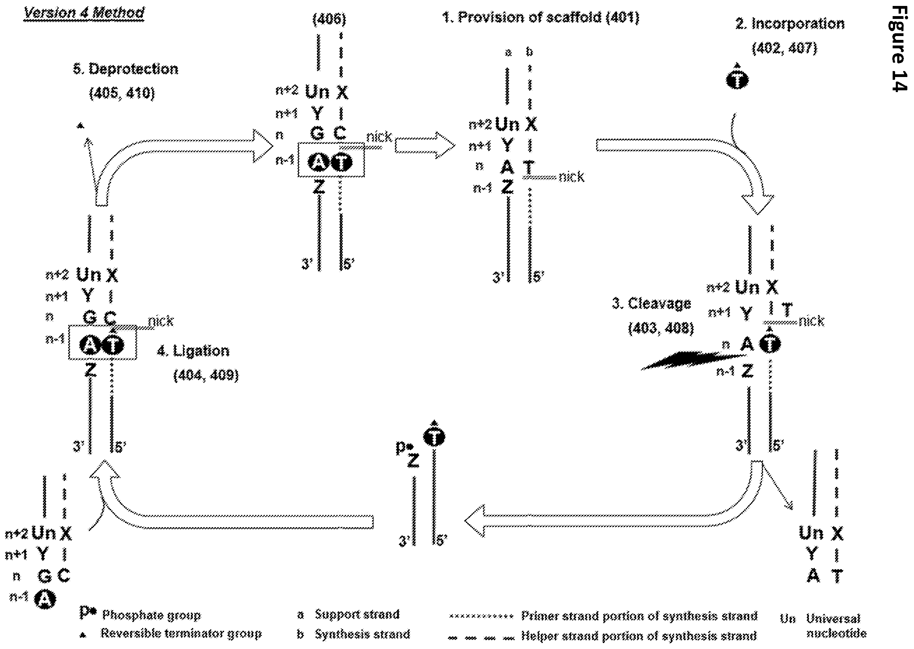

### Figure 14. Scheme of Exemplary Method Version 4.

Scheme showing a first synthesis cycle according to exemplary method version 4 of the Examples section. This method is provided for illustrative support only and is not within the scope of the claimed invention. The method comprises a cycle of provision of a scaffold polynucleotide, incorporation, cleavage, ligation and deprotection. The scheme shows the incorporation in the first cycle (401, 402) of a thymine nucleotide and its pairing opposite a partner universal nucleotide (404), as well as the provision of a scaffold polynucleotide (406) comprising a guanine for pairing with a cytosine in the next synthesis cycle. These pairs are shown for illustration purposes only and are not limiting, they can be any pairs depending on the required predefined sequence. Nucleotides X, Y and Z can be any nucleotide.

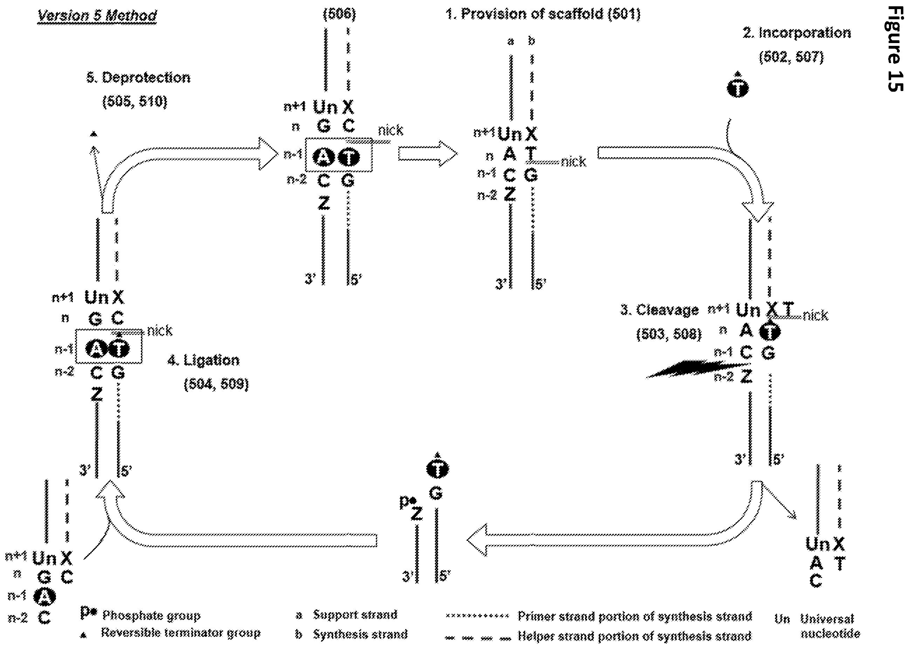

### Figure 15. Scheme of Exemplary Method Version 5.

Scheme showing a first synthesis cycle according to exemplary method version 5 of the Examples section. This method is provided for illustrative support only and is not within the scope of the claimed invention. The method comprises a cycle of provision of a scaffold polynucleotide, incorporation, cleavage, ligation and deprotection. The scheme shows the incorporation in the first cycle (501, 502) of a thymine nucleotide and its pairing opposite a partner adenine nucleotide (504), as well as the provision of a scaffold polynucleotide (506) comprising a guanine for pairing with a cytosine in the next synthesis cycle. The scheme also shows a cytosine-guanine pair (position n-2) as a component of the scaffold polynucleotide and which is not part of the predefined sequence. These pairs are shown for illustration purposes only and are not limiting, they can be any pairs depending on the required predefined sequence. Nucleotides X, Y and Z can be any nucleotide.

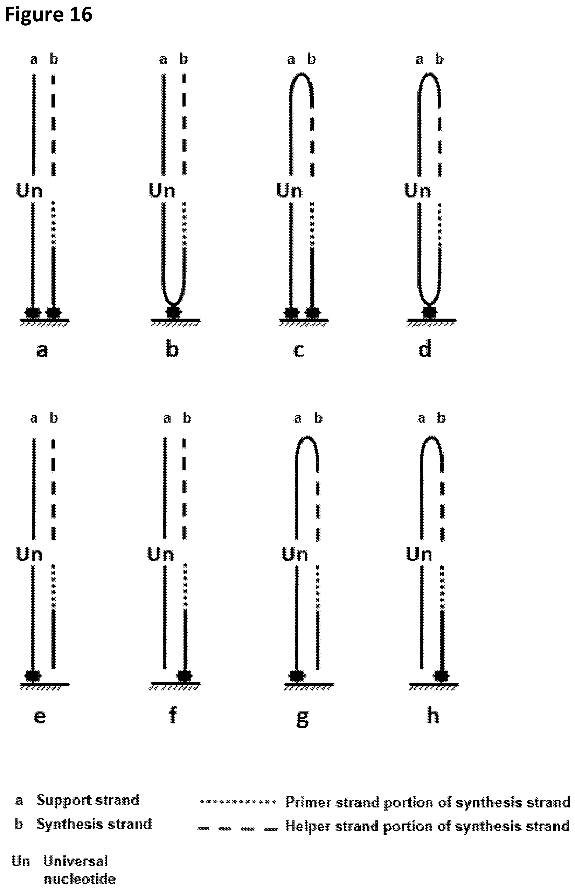

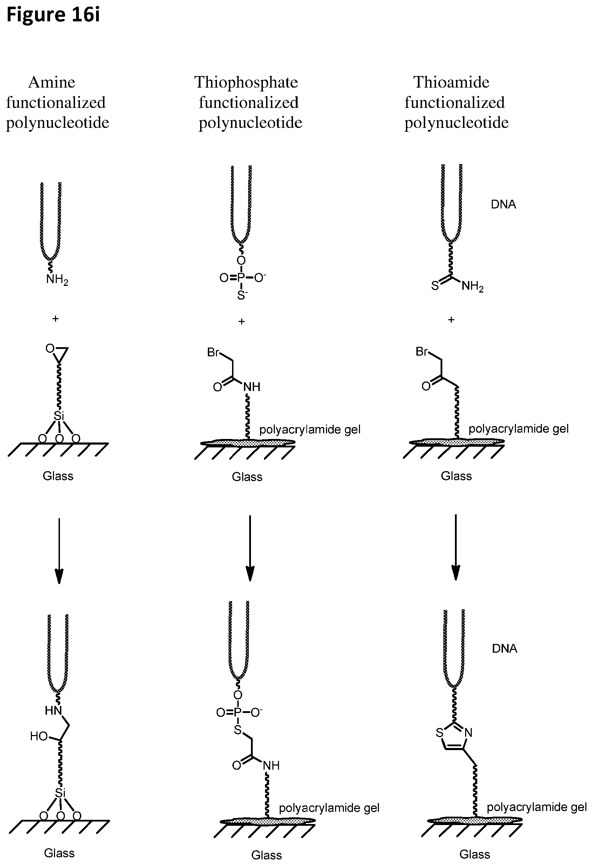

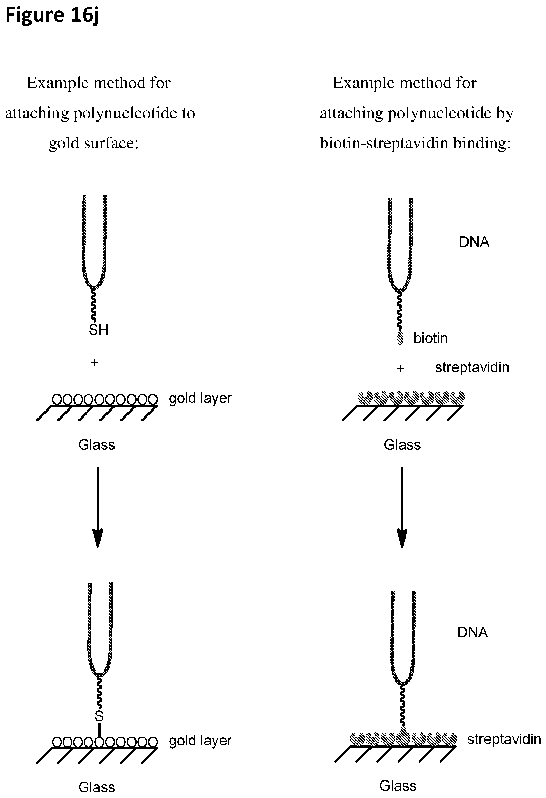

### Figure 16. Scheme Showing Surface Immobilization of Scaffold Polynucleotides.

Schemes show (a to h) possible example hairpin loop configurations of scaffold polynucleotides and their immobilisation to surfaces.

Schemes (i and j) show examples of surface chemistries for attaching polynucleotides to surfaces. The examples show double-stranded embodiments wherein both strands are connected via a hairpin, but the same chemistries may be used for attaching one or both strands of an unconnected double-stranded polynucleotide.

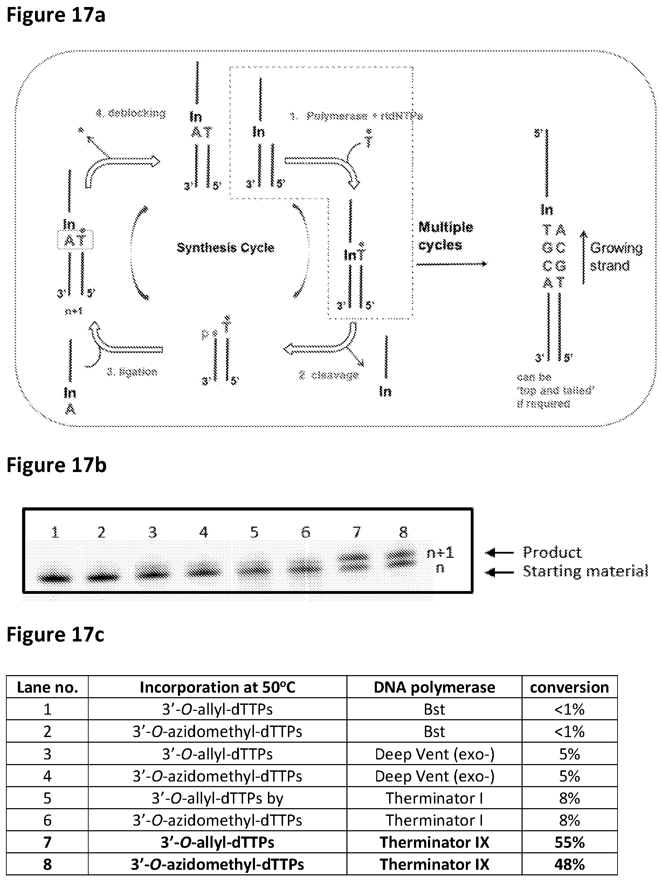

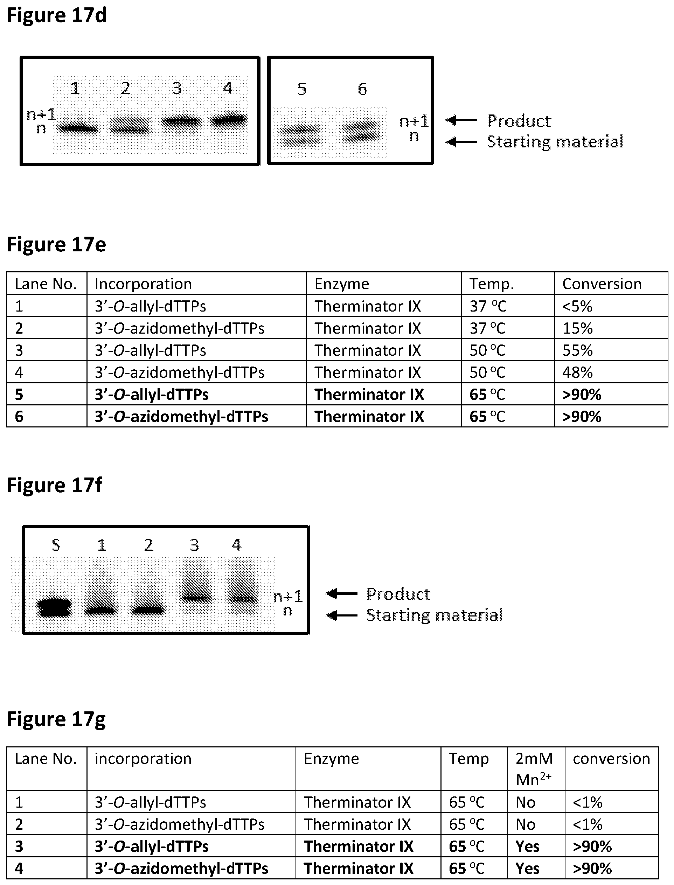

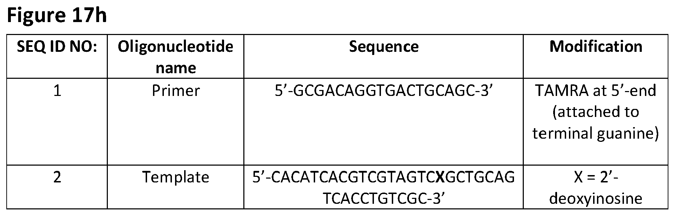

### Figure 17. Absence of Helper Strand - Incorporation.

a) Scheme showing incorporation step highlighted in dashed box.
b) Evaluation of DNA polymerases for incorporation of 3'-*O*-modified-dTTPs opposite inosine. The Figure depicts a gel showing results of incorporation of 3'-*O-*modified-dTTPs by various DNA polymerases (Bst, Deep Vent (Exo-), Therminator I and Therminator IX) in presence of Mn²⁺ ions at 50°C. Lane 1: Incorporation of 3'-*O*-allyl-dTTPs using Bst DNA polymerase. Lane 2: Incorporation of 3'-*O*-azidomethyl-dTTPs using Bst DNA polymerase. Lane 3: Incorporation of 3'-*O*-allyl-dTTPs using Deep vent (exo-) DNA polymerase. Lane 4: Incorporation of 3'-*O*-azidomethyl-dTTPs using Deep vent (exo-) DNA polymerase. Lane 5: Incorporation of 3'-*O*-allyl-dTTPs using Therminator I DNA polymerase. Lane 6: Incorporation of 3'-*O*-azidomethyl-dTTPs using Therminator I DNA polymerase. Lane 7: Incorporation of 3'-*O*-allyl-dTTPs using Therminator IX DNA polymerase. Lane 8: Incorporation of 3'-*O*-azidomethyl-dTTPs using Therminator IX DNA polymerase.
c) Evaluation of DNA polymerases for incorporation of 3'-*O*-modified-dTTPs opposite inosine. Results of incorporation using various DNA polymerases.
d) Evaluation of the temperature on the incorporation using Therminator IX DNA polymerase. The Figure depicts a gel showing results of incorporation of 3'-modified-dTTP opposite inosine in presence of Mn²⁺ ions using Therminator IX DNA polymerase at various temperatures. Lane 1: Incorporation of 3'-*O*-allyl-dTTPs at 37°C. Lane 2: Incorporation of 3'-*O*-azidomethyl-dTTPs at 37°C. Lane 3: Incorporation of 3'-*O*-allyl-dTTPs at 50°C. Lane 4: Incorporation of 3'-*O*-azidomethyl-dTTPs at 50°C. Lane 5: Incorporation of 3'-*O*-allyl-dTTPs at 65°C. Lane 6: Incorporation of 3'-*O*-azidomethyl-dTTPs at 65°C.
e) Evaluation of the temperature on the incorporation using Therminator IX DNA polymerase. Results of incorporation performed at different temperatures.
f) Evaluation of the presence of Mn²⁺ on the incorporation using Therminator IX DNA polymerase. The Figure depicts a gel showing results of incorporation of 3'-*O-*modified-dTTP opposite inosine at 65°C. Lane S: Standards. Lane 1: Incorporation of 3'-*O*-allyl-dTTPs without Mn²⁺ ions. Lane 2: Incorporation of 3'-*O*-azidomethyl-dTTPs without Mn²⁺ ions. Lane 3: Incorporation of 3'-*O*-allyl-dTTPs in presence of Mn²⁺ ions. Lane 4: Incorporation of 3'-*O*-azidomethyl-dTTPs in presence of Mn²⁺ ions.
g) Evaluation of the presence of Mn²⁺ on the incorporation using Therminator IX DNA polymerase. Results of incorporation in presence and absence of Mn²⁺ ions.
h) Oligonucleotides used for study of the incorporation step.

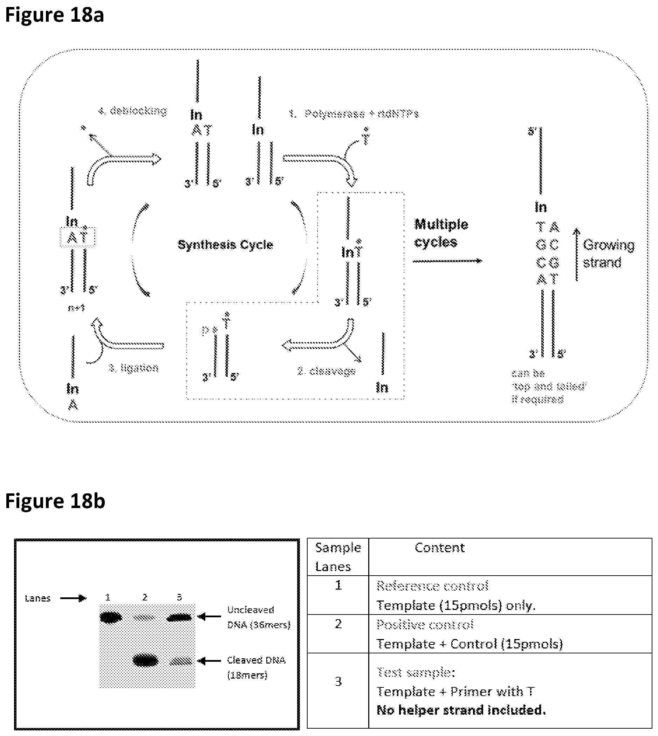

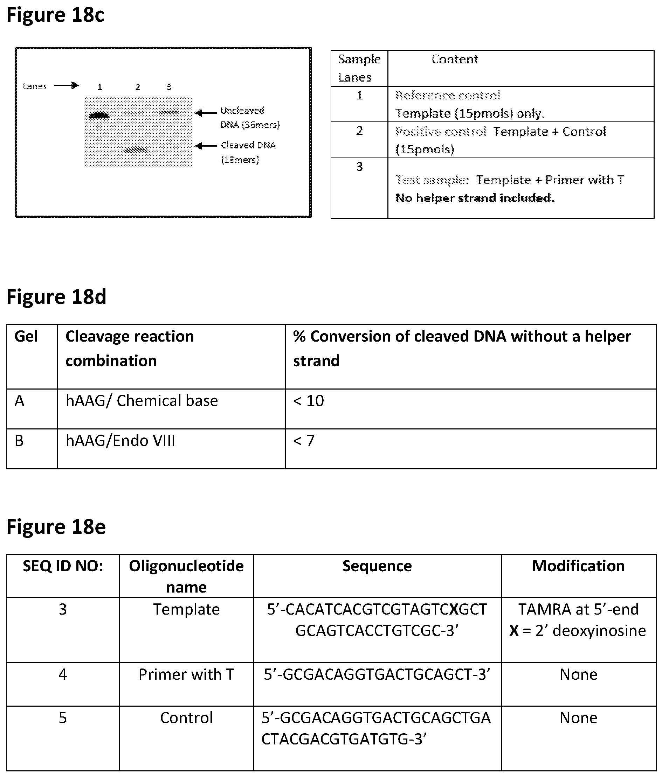

### Figure 18. Absence of Helper Strand - Cleavage.

a) Scheme showing cleavage of hybridized polynucleotide strands in the absence of a helper strand. Cleavage step is highlighted in dashed box.
b) Gel showing cleavage of oligonucleotide with hAAG and 0.2M NaOH (strong base) at 37°C and room temperature 24°C respectively. Lane 1. Starting oligonucleotide. Lane 2 which was a positive control that contained both full length strands showed a higher yield of cleaved to uncleaved DNA ratio of 90% : 10%. Lane 3 which included the cleavage reaction without a helper strand showed a low percentage yield of cleaved to uncleaved DNA ratio of 10 % : 90%.
c) Gel showing cleavage of oligonucleotide with hAAG and Endo VIII at 37°C. Lane 2 which was a positive control that contained both full length strands showed a higher yield of cleaved to uncleaved DNA ratio of ~ 90% : 10%. Lane 3 which included the cleavage reaction without a helper strand showed a low percentage yield of cleaved to uncleaved DNA ratio of ~7% : 93%.
d) A summary of cleavage of oligonucleotide with hAAG/Endo VIII and hAAG/Chemical base.
e) Oligonucleotides used for study of the cleavage step.

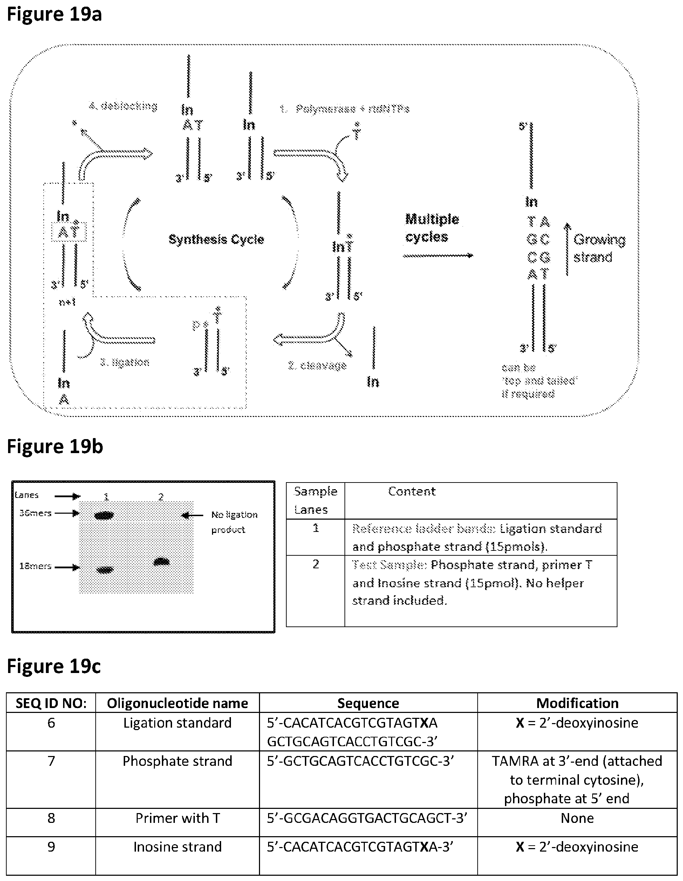

### Figure 19. Absence of Helper Strand - Ligation.

a) Scheme showing ligation of hybridized polynucleotide strands in the absence of a helper strand. Ligation step highlighted in dashed box.
b) Gel showing ligation of Oligonucleotides with Quick T4 DNA ligase at room temperature (24°C) in the absence of a helper strand. Lane 1 contained a mixture of the 36mers TAMRA single stranded oligos and 18mers TAMRA single stranded oligos. These oligos served reference bands.
c) Oligonucleotides used for study of the ligation step.

### Figure 20. Version 1 Chemistry with Helper Strand - Incorporation.

a) Scheme showing incorporation step highlighted in dashed box.
b) Oligonucleotides applicable for study of the incorporation step.

### Figure 21. Version 1 Chemistry with Helper Strand - Cleavage.

a) Scheme showing cleavage of hybridized polynucleotide strands in the absence of a helper strand. Cleavage step is highlighted in dashed box.
b) Gel showing cleavage of Oligonucleotide with hAAG and 0.2M NaOH (strong base) at 37°C and room temperature 24°C respectively. Lane 1. Starting oligonucleotide. Lane 2 which was a positive control that contained both full length strands showed a higher yield of cleaved to uncleaved DNA ratio of 90% : 10%. Lane 3 which included the cleavage reaction without a helper strand showed a low percentage yield of cleaved to uncleaved DNA ratio of 10 % : 90%. Lane 4 which included the cleavage reaction with a helper strand showed an equal percentage yield of cleaved to uncleaved DNA ratio of 50 % : 50%.
c) Evaluation of Endonuclease VIII for cleavage of abasic sites. Gel shows cleavage of oligonucleotide with hAAG and Endo VIII at 37°C. Lane 2 which was a positive control that contained both full length strands showed a higher yield of cleaved to uncleaved DNA ratio of ~ 90% : 10%. Lane 3 which included the cleavage reaction without a helper strand showed a low percentage yield of cleaved to uncleaved DNA ratio of ~7% : 93%. Lane 4 which included the cleavage reaction with a helper strand showed an low percentage yield of cleaved to uncleaved DNA ratio of 10 % : 90%.
d) Evaluation of N,N'-dimethylethylenediamine for cleavage of abasic sites. Gel shows cleavage of oligonucleotide with hAAG and 100mM N,N'-dimethylethylenediamine at 37°C. Lane 1. Starting oligonucleotide. Lane 2 which was a positive control that contained both full length strands showed a 100% cleaved DNA. Lane 3 which included the cleavage reaction with a helper strand showed a higher percentage yield of cleaved to uncleaved DNA ratio of 90 % : 10%.
e) A summary of cleavage of oligonucleotide with hAAG/Endo VIII, hAAG/chemical base and hAAG/ alternative chemical base.
f) Oligonucleotides used for study of the cleavage step.

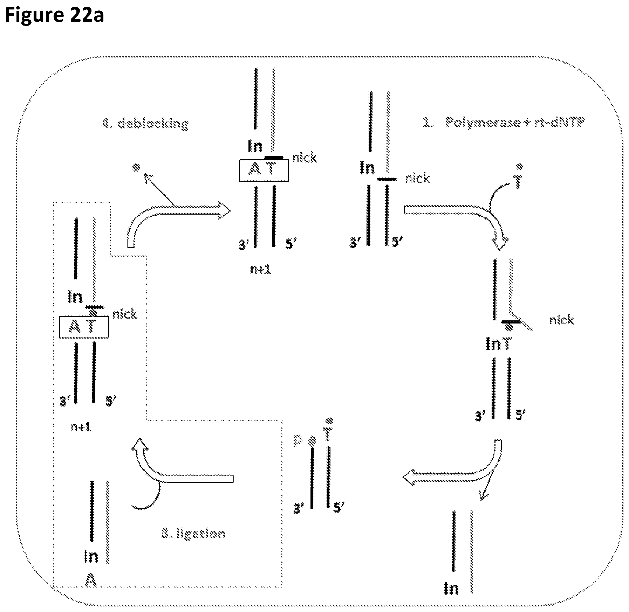

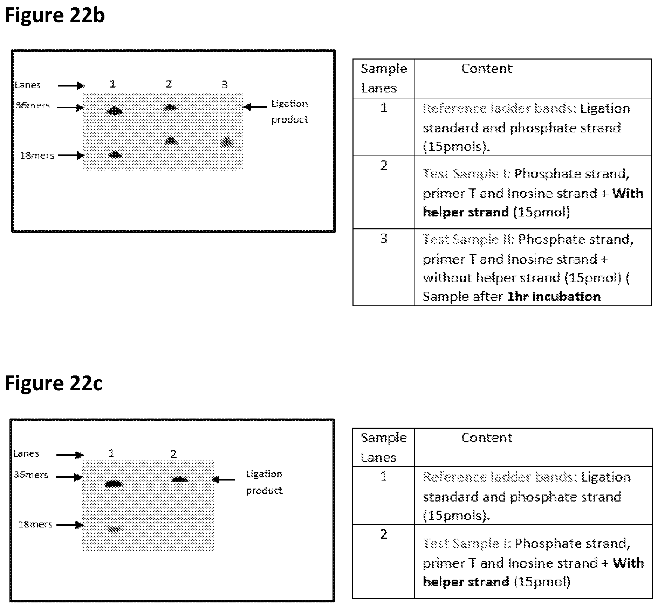

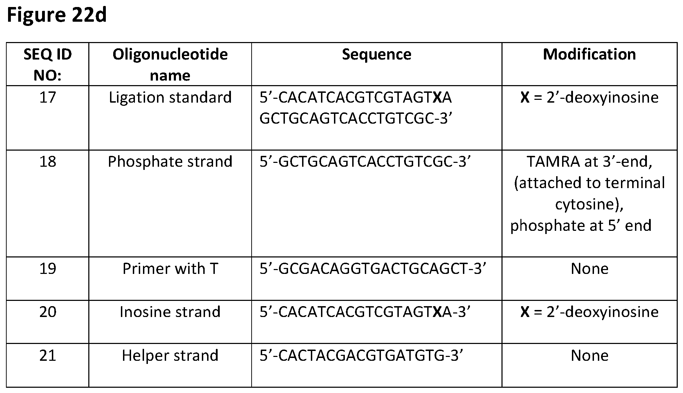

### Figure 22. Version 1 Chemistry with Helper Strand - Ligation.

a) Scheme showing ligation of hybridized polynucleotide strands in the presence of a helper strand. Ligation step highlighted in dashed box.
b) Gel showing ligation of oligonucleotides with Quick T4 DNA ligase at room temperature (24°C) in the presence of a helper strand. Lane 1 contained a mixture of the 36mers TAMRA single stranded oligos and 18mers TAMRA single stranded oligos. These oligos served reference bands. In lane 2 there was an observable ligation product of expected band size 36mers after 20 minutes.
c) Gel showing ligation of oligonucleotides with Quick T4 DNA ligase at room temperature (24°C) after overnight incubation in the presence of a helper strand. Lane 1 contained a mixture of the 36mers TAMRA single stranded oligos and 18mers TAMRA single stranded oligos. These oligos served as reference bands. In lane 2 there was an observable completely ligated product of expected band size of 36mers.
d) Oligonucleotides used for study of the ligation step.

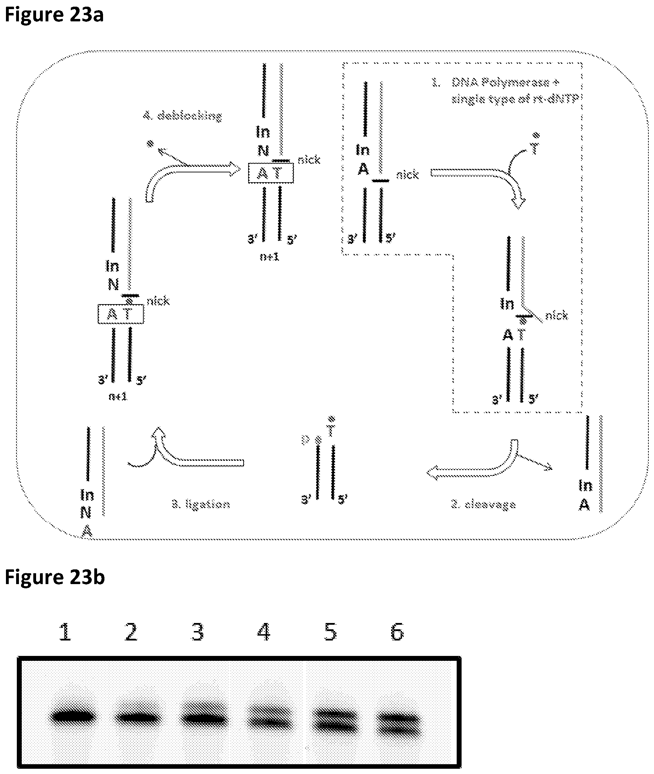

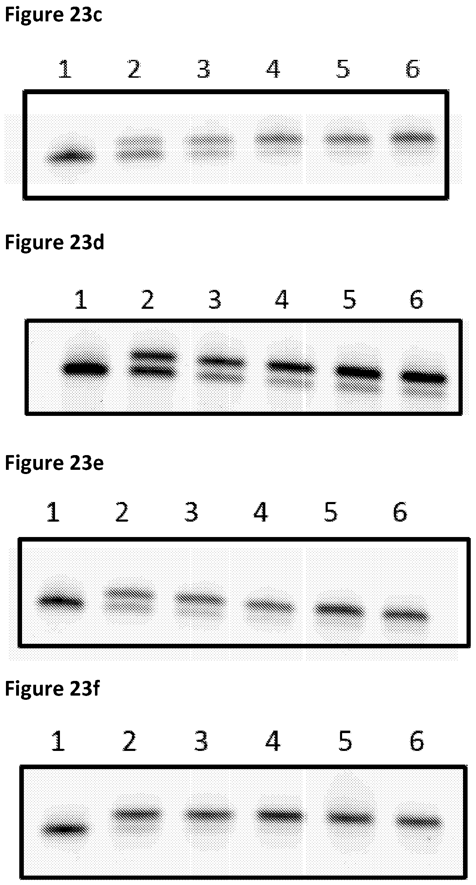

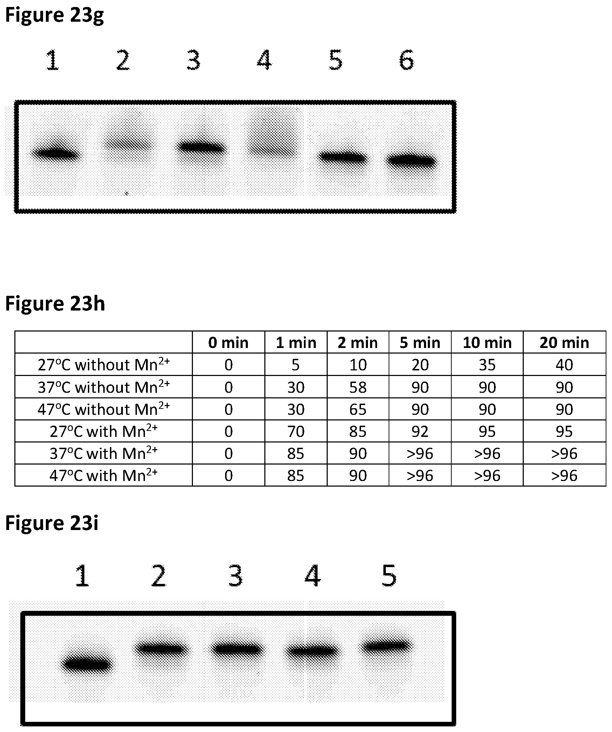

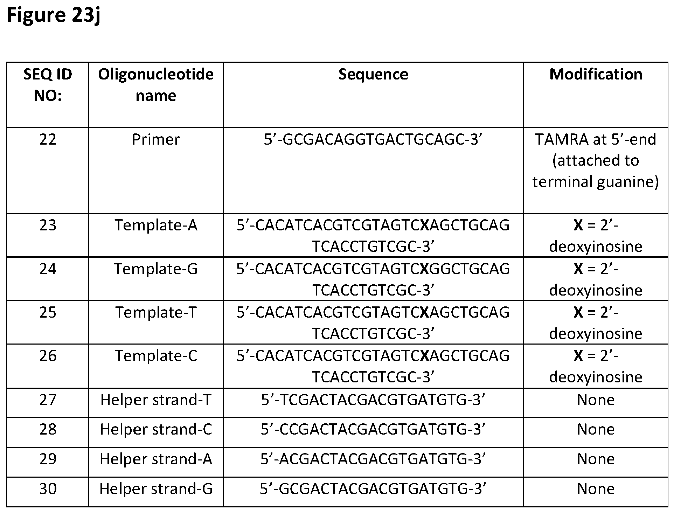

### Figure 23. Version 2 Chemistry with Helper Strand - Incorporation.

a) Scheme showing incorporation step highlighted in orange dashed box
b) Gel showing results of incorporation of 3'-*O*-modified-dTTPs by Therminator IX DNA polymerase at 27°C. Lane 1: Starting material. Lane 2: Incorporation after 1 minute, conversion 5%. Lane 3: Incorporation after 2 minutes, conversion 10%. Lane 4: Incorporation after 5 minutes, conversion 20%. Lane 5: Incorporation after 10 minutes, conversion 30%. Lane 6: Incorporation after 20 minutes, conversion 35%.
c) The Figure depicts a gel showing results of incorporation of 3'-*O*-modified-dTTPs by Therminator IX DNA polymerase at 37°C. Lane 1: Starting material. Lane 2: Incorporation after 1 minute, conversion 30%. Lane 3: Incorporation after 2 minutes, conversion 60%. Lane 4: Incorporation after 5 minutes, conversion 90%. Lane 5: Incorporation after 10 minutes, conversion 90%. Lane 6: Incorporation after 20 minutes, conversion 90%.
d) Gel showing results of incorporation of 3'-*O*-modified-dTTPs by Therminator IX DNA polymerase at 47°C. Lane 1: Starting material. Lane 2: Incorporation after 1 minute, conversion 30%. Lane 3: Incorporation after 2 minutes, conversion 65%. Lane 4: Incorporation after 5 minutes, conversion 90%. Lane 5: Incorporation after 10 minutes, conversion 90%. Lane 6: Incorporation after 20 minutes, conversion 90%.
e) Gel showing results of incorporation of 3'-*O*-modified-dTTPs by Therminator IX DNA polymerase at 27°C. Lane 1: Starting material. Lane 2: Incorporation after 1 minute, conversion 70%. Lane 3: Incorporation after 2 minutes, conversion 85%. Lane 4: Incorporation after 5 minutes, conversion 92%. Lane 5: Incorporation after 10 minutes, conversion 96%. Lane 6: Incorporation after 20 minutes, conversion 96%.
f) Gel showing results of incorporation of 3'-*O*-modified-dTTPs by Therminator IX DNA polymerase at 37°C. Lane 1: Starting material. Lane 2: Incorporation after 1 minute, conversion 85%. Lane 3: Incorporation after 2 minutes, conversion 95%. Lane 4: Incorporation after 5 minutes, conversion 96%. Lane 5: Incorporation after 10 minutes, conversion 96%. Lane 6: Incorporation after 20 minutes, conversion 96%.
g) Gel showing results of incorporation of 3'-*O*-modified-dTTPs by Therminator IX DNA polymerase at 47°C. Lane 1: Starting material. Lane 2: Incorporation after 1 minute, conversion 85%. Lane 3: Incorporation after 2 minutes, conversion 90%. Lane 4: Incorporation after 5 minutes, conversion 96%. Lane 5: Incorporation after 10 minutes, conversion 96%. Lane 6: Incorporation after 20 minutes, conversion 96%.
h) Summary of incorporation of 3'-*O*-azidomethyl-dTTP at various temperatures and presence of Mn²⁺ ions.
i) Gel showing results of incorporation of 3'-*O*-modified-dNTPs opposite complementary base by Therminator IX DNA polymerase in presence of Mn²⁺ at 37°C. Lane 1: Starting material. Lane 2: Incorporation of 3'-*O*-azidomethyl-dTTP for 5 minutes. Lane 3: Incorporation of 3'-*O*-azidomethyl-dATP for 5 minutes. Lane 4: Incorporation of 3'-*O*-azidomethyl-dCTP for 5 minutes. Lane 5: Incorporation of 3'-*O*-azidomethyl-dGTP for 5 minutes.
j) Oligonucleotides used for study of the incorporation step.

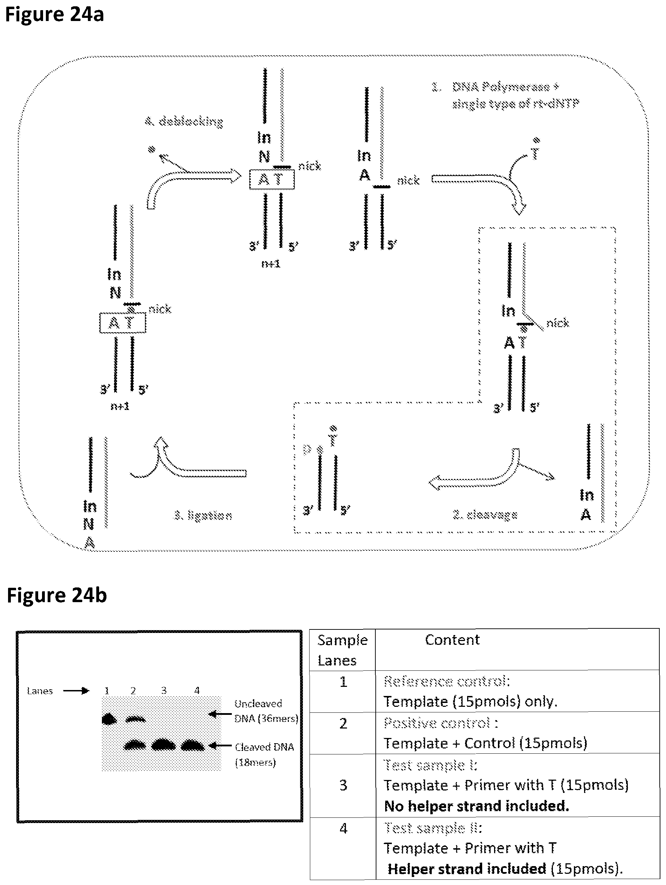

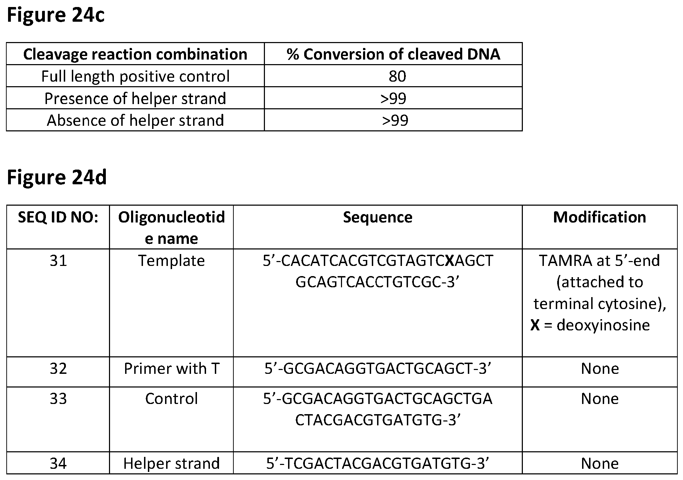

### Figure 24. Version 2 Chemistry with Helper Strand - Cleavage.

a) Scheme showing cleavage of hybridized polynucleotide strand in the presence of a helper strand. Cleavage step is highlighted in orange dashed box.
b) Gel shows cleavage of Oligonucleotide with Endo V at 37°C. Lane 1. Starting oligonucleotide. Lane 2 which was a positive control that contained both full length strands showed a yield of cleaved to uncleaved DNA ratio of 80% : 20%. Lane 3 which included the cleavage reaction without a helper strand showed a much higher yield of cleaved DNA of >99%. Lane 4 which included the cleavage reaction with a helper strand also showed a DNA cleavage yield of >99%.
c) A summary of cleavage study with Endonuclease V.
d) Oligonucleotides used for study of the cleavage step.

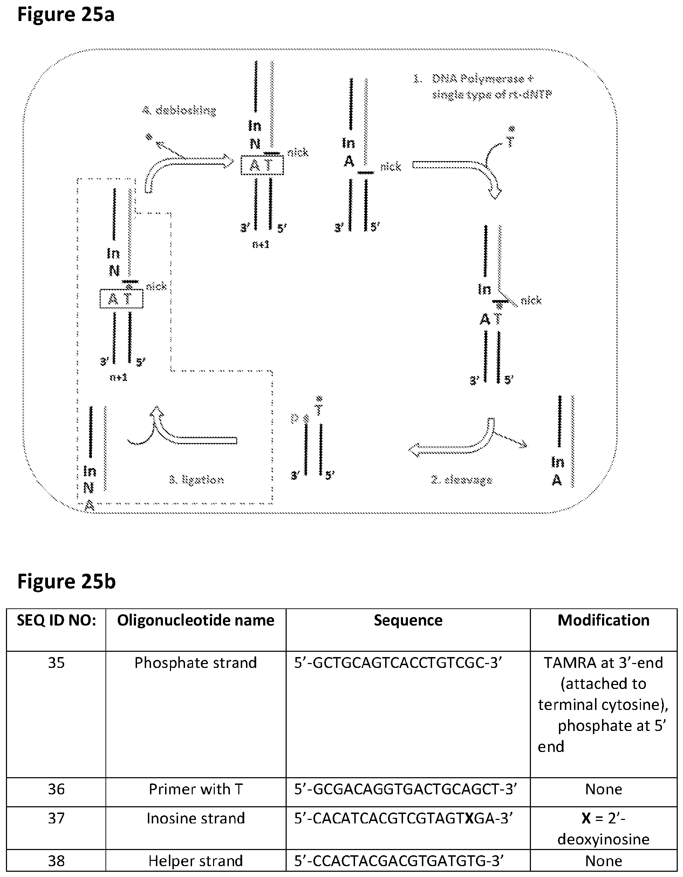

### Figure 25. Version 2 Chemistry with Helper Strand - Ligation.

a) Scheme showing ligation of hybridized polynucleotide strands in the absence of a helper strand. Ligation step highlighted in orange dashed box.
b) Oligonucleotides for study of the ligation step.

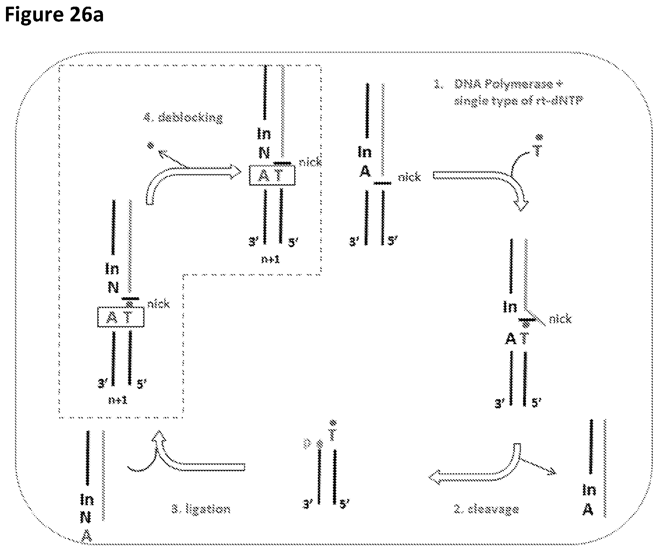

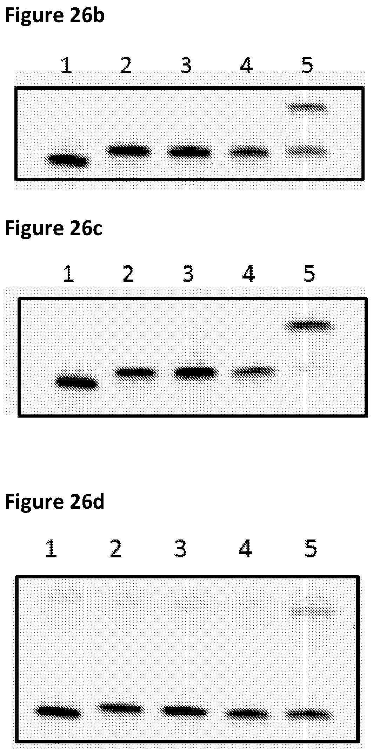

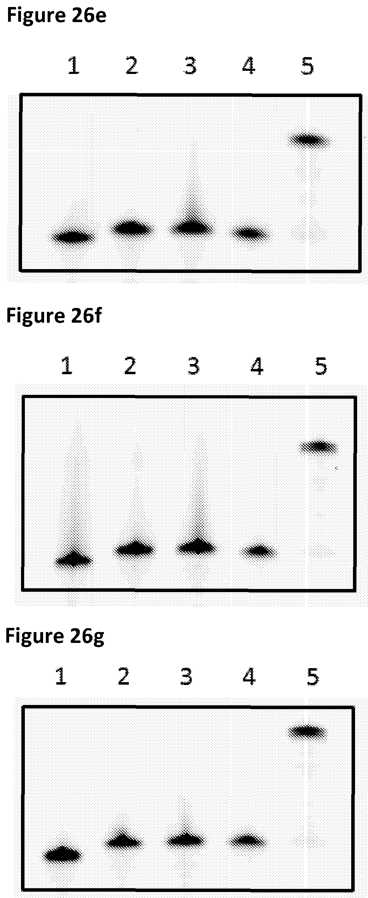

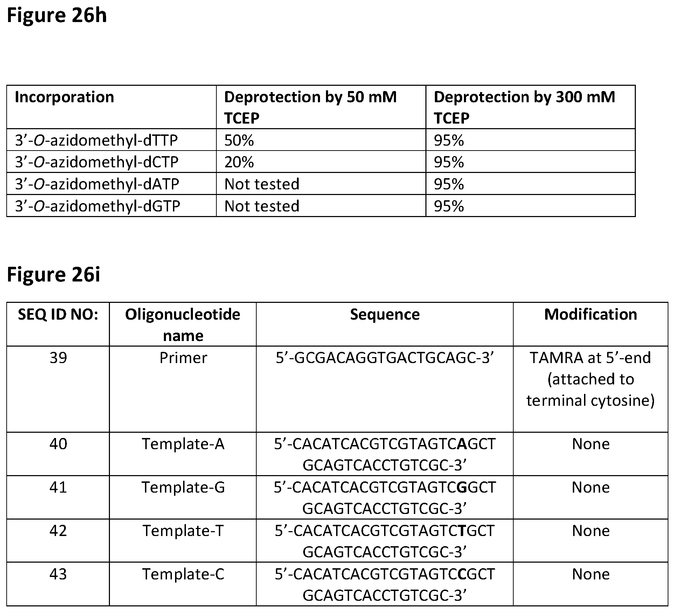

### Figure 26. Version 2 Chemistry with Helper Strand - Deprotection.

a) Scheme showing deprotection step highlighted in orange dashed box.
b) The Figure depicts a gel showing results of 3'-*O*-azidomethyl group deprotection by 50mM TCEP after incorporation of 3'-*O*-azidomethyl-dTTP. Lane 1: Starting primer Lane 2: Incorporation of 3'-*O*-azidomethyl-dTTPs in presence Mn²⁺. Lane 3: Extension of the product in lane 2 by addition of all natural dNTPs. Lane 4: Deprotection of the product (0.5 µM) in lane 2 by 50 mM TCEP. Lane 5: Extension of the product in lane 4 by addition of all natural dNTPs.
c) The Figure depicts a gel showing results of 3'-*O*-azidomethyl group deprotection by 300mM TCEP after incorporation of 3'-*O*-azidomethyl-dTTP. Lane 1: Starting primer. Lane 2: Incorporation of 3-*O*-azidomethyl-dTTPs in presence Mn²⁺. Lane 3: Extension of the product in lane 2 by addition of all natural dNTPs. Lane 4: Deprotection of the product (0.5 µM) in lane 2 by 300mM TCEP. Lane 5: Extension of the product in lane 4 by addition of all natural dNTPs.
d) The Figure depicts a gel showing results of 3'-*O*-azidomethyl group deprotection by 50mM TCEP after incorporation of 3'-*O*-azidomethyl-dCTP. Lane 1: Starting primer. Lane 2: Incorporation of 3-*O*-azidomethyl-dCTPs in presence Mn²⁺. Lane 3: Extension of the product in lane 2 by addition of all natural dNTPs. Lane 4: Deprotection of the product (0.5 µM) in lane 2 by 300mM TCEP. Lane 5: Extension of the product in lane 4 by addition of all natural dNTPs.
e) The Figure depicts a gel showing results of 3'-O-azidomethyl group deprotection by 300mM TCEP after incorporation of 3'-*O*-azidomethyl-dCTP. Lane 1: Starting primer Lane 2: Incorporation of 3-*O*-azidomethyl-dCTPs in presence Mn²⁺. Lane 3: Extension of the product in lane 1 by addition of all natural dNTPs. Lane 4: Deprotection of the product (0.5 µM) in lane 1 by 300mM TCEP. Lane 5: Extension of the product in lane 3 by addition of all natural dNTPs.
f) The Figure depicts a gel showing results of 3'-*O*-azidomethyl group deprotection by 300mM TCEP after incorporation of 3'-*O*-azidomethyl-dATP.
   Lane 1: Starting primer
   Lane 2: Incorporation of 3-*O*-azidomethyl-dATPs in presence Mn²⁺. Lane 3: Extension of the product in lane 2 by addition of all natural dNTPs. Lane 4: Deprotection of the product (0.5 µM) in lane 2 by 300mM TCEP. Lane 5: Extension of the product in lane 4 by addition of all natural dNTPs.
g) The Figure depicts a gel showing results of 3'-*O*-azidomethyl group deprotection by 300mM TCEP after incorporation of 3'-*O*-azidomethyl-dGTP. Lane 1: Starting primer. Lane 2: Incorporation of 3-*O*-azidomethyl-dGTPs in presence Mn²⁺. Lane 3: Extension of the product in lane 2 by addition of all natural dNTPs. Lane 4: Deprotection of the product (0.5 µM) in lane 2 by 300mM TCEP. Lane 5: Extension of the product in lane 4 by addition of all natural dNTPs.
h) Efficiency of deprotection by TCEP on 0.2 µM DNA.
i) Oligonucleotides used for study of the cleavage step.

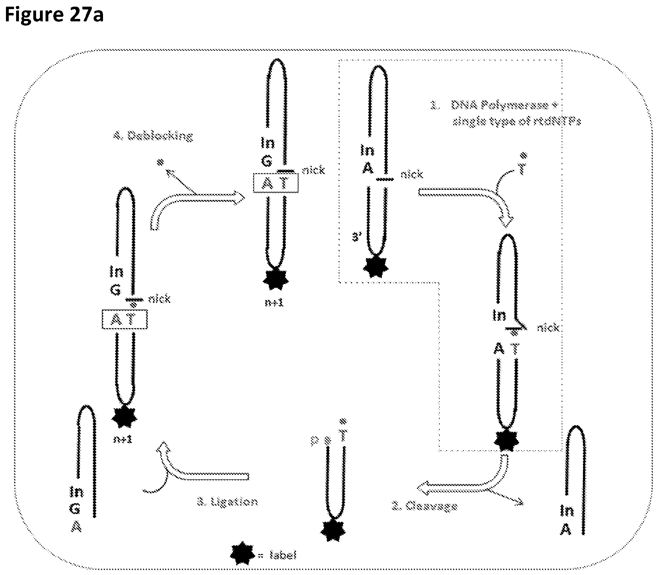

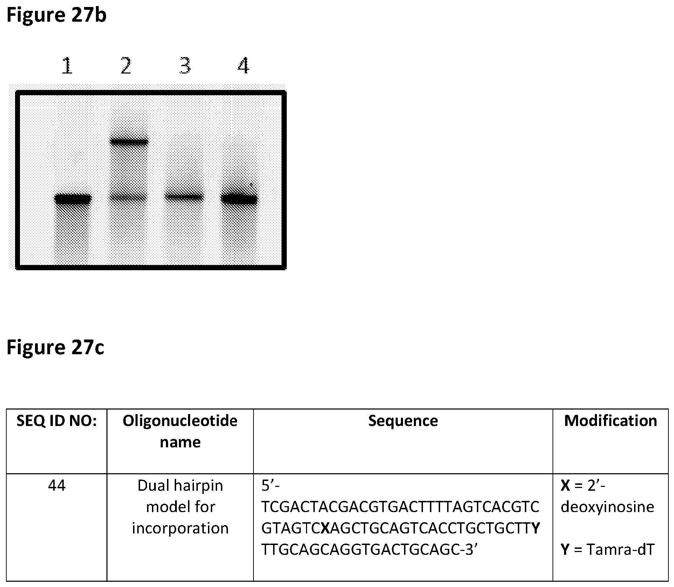

### Figure 27. Version 2 Chemistry with Double Hairpin Model - Incorporation.

a) Scheme showing incorporation step highlighted in dashed box.
b) Evaluation of DNA polymerases for incorporation of 3'-*O*-modified-dTTPs opposite its natural counterpart. The Figure depicts a gel showing results of incorporation of 3'-*O*-modified-dTTPs by Therminator IX DNA polymerase at 37°C. Lane 1: Starting material. Lane 2: Incorporation of natural dNTP mix. Lane 3: Incorporation of 3'-*O-*azidomethyl-dTTP by Therminator IX DNA polymerase. Lane 4: Extension of the product in lane 3 by addition of all natural dNTPs.
c) Evaluation of DNA polymerases for incorporation of 3'-*O*-modified-dTTPs opposite its natural counterpart. Oligonucleotides applicable for study of the incorporation step.

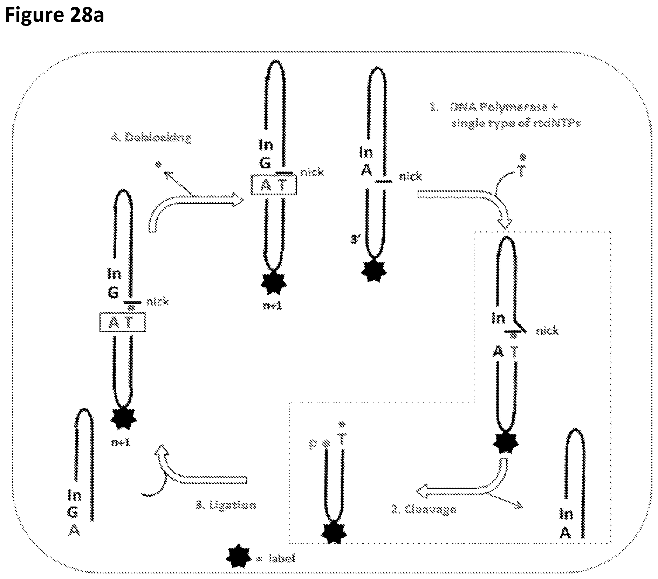

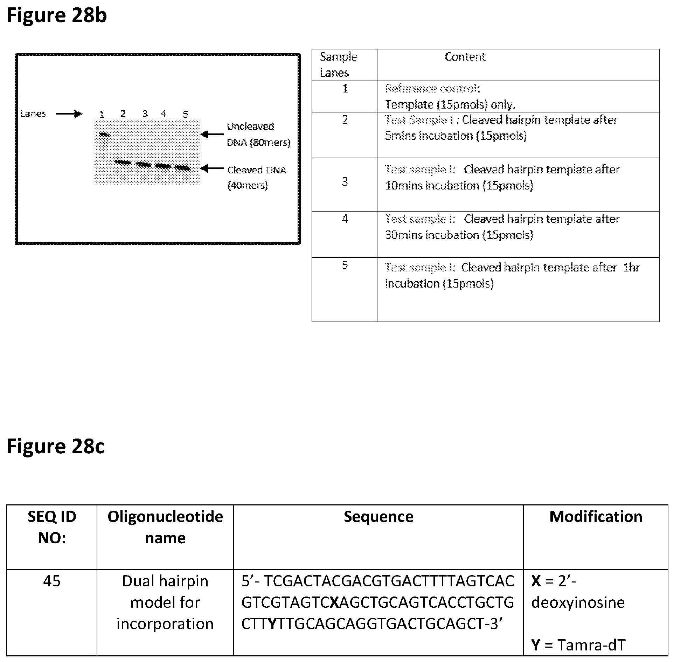

### Figure 28. Version 2 Chemistry with Double Hairpin Model - Cleavage.

a) Scheme showing cleavage of a hairpin Oligonucleotide. Cleavage step is highlighted in dashed box.
b) Gel showing cleavage of Hairpin Oligonucleotide with Endo V at 37°C. Lane 1. Starting hairpin oligonucleotide. Lane 2 which was the cleaved hairpin oligonucleotide after 5 minutes showed a high yield of digested DNA with a ratio of ~ 98%. Lane 3 which was the cleaved hairpin oligonucleotide after 10 minutes showed a high yield of digested DNA with a ratio of ~ 99%. Lane 4 which was the cleaved hairpin oligonucleotide after 30 minutes showed a high yield of digested DNA with a ratio of ~ 99% and in lane 5 which was the cleaved hairpin oligonucleotide after 1hr showed a high yield of digested DNA with a ratio of ~ 99%.
c) Oligonucleotides used for study of the cleavage step.

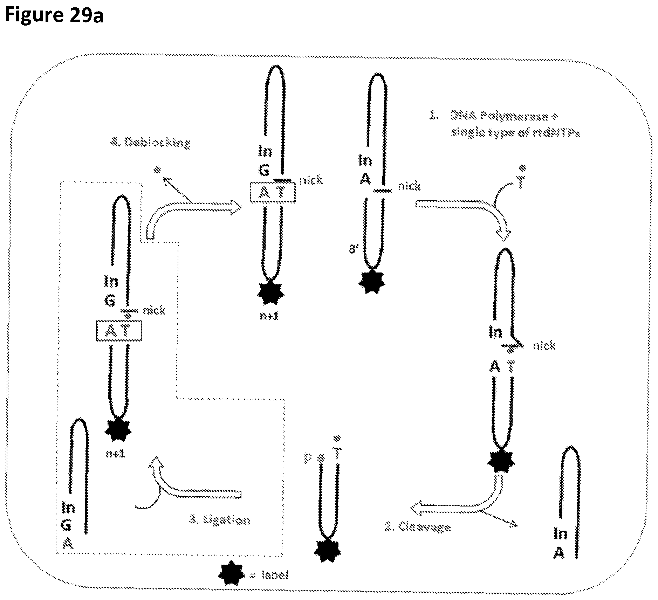

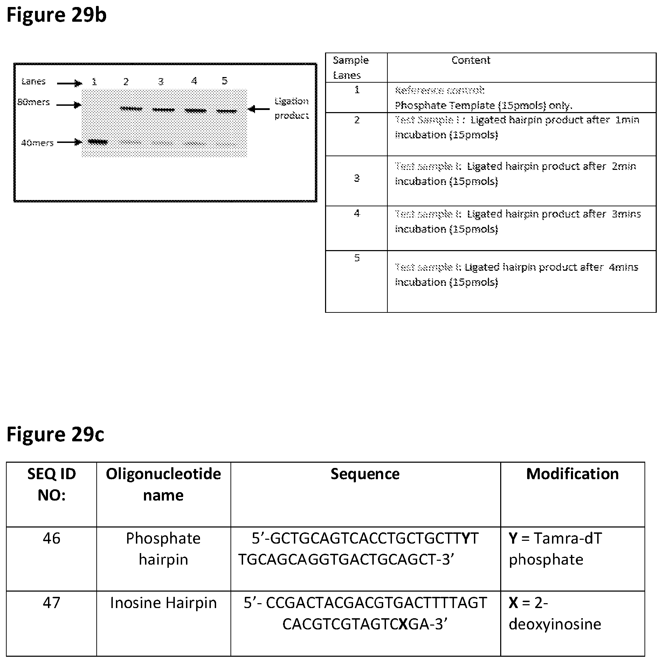

### Figure 29. Version 2 Chemistry with Double Hairpin Model - Ligation.

a) Scheme showing ligation of hybridized hairpins. Ligation step highlighted in dashed box.
b) The gel shows ligation of Hairpin Oligonucleotides with Blunt/TA DNA ligase at room temperature (24°C) in the presence of a helper strand. Lane 1 contained a starting hairpin Oligonucleotide. Lane 2 which was the ligated hairpin oligonucleotide after 1 minute showed a high yield of ligated DNA product with a ratio of ~ 85%. Lane 3 which was the ligated hairpin oligonucleotide after 2 minutes showed a high yield of digested DNA with a ratio of ~ 85%. Lane 4 which was the ligated hairpin oligonucleotide after 3 minutes showed a high yield of ligated DNA product with a ratio of ~ 85%. Lane 5 which was the ligated hairpin oligonucleotide after 4 minutes showed a high yield of ligated DNA product with a ratio of ~ >85%.
c) Hairpin Oligonucleotides used for study of the Ligation step.

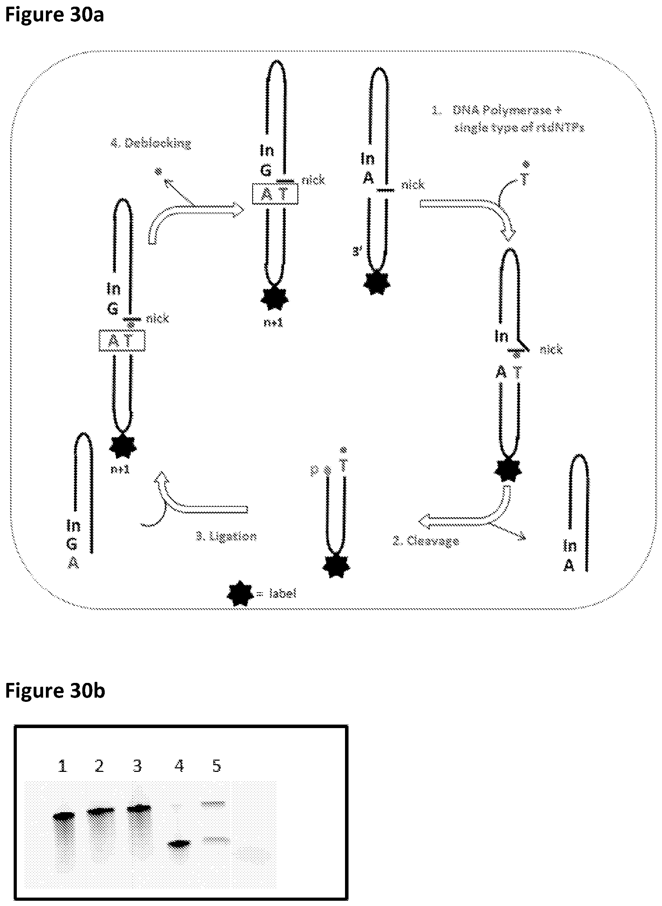

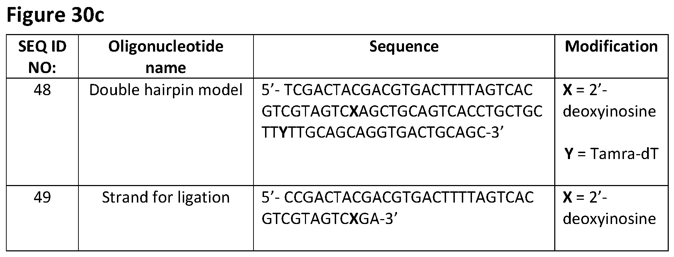

### Figure 30. Version 2 Chemistry - Complete Cycle on Double Hairpin Model.

a) Scheme showing full cycle involving enzymatic incorporation, cleavage, ligation and deprotection steps.
b) Evaluation of DNA polymerases for incorporation of 3'-*O*-modified-dTTPs opposite its natural counterpart. The Figure depicts a gel showing results of incorporation of 3'-*O*-modified-dTTPs by Therminator IX DNA polymerase at 37°C. Lane 1: Starting material. Lane 2: Incorporation of 3'-*O*-azidomethyl-dTTP by Therminator IX DNA polymerase. Lane 3: Extension of the product in lane 2 by addition of all natural dNTPs. Lane 4: Cleavage of the product in lane 2 by Endonuclease V. Lane 5: Ligation of the product in lane 4 by blunt TA ligase kit.
c) Oligonucleotides applicable for study of the incorporation step.

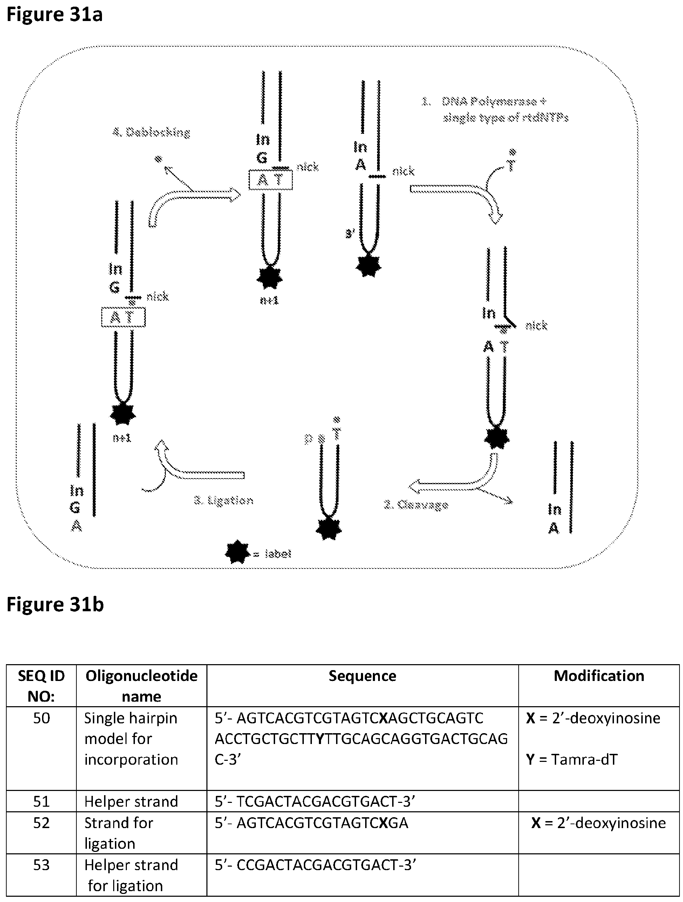

### Figure 31. Version 2 Chemistry - Complete Cycle on Single Hairpin Model using Helper Strand.

a) Scheme showing full cycle involving enzymatic incorporation, cleavage, ligation and deprotection steps.
b) Oligonucleotides applicable for study of the incorporation step.

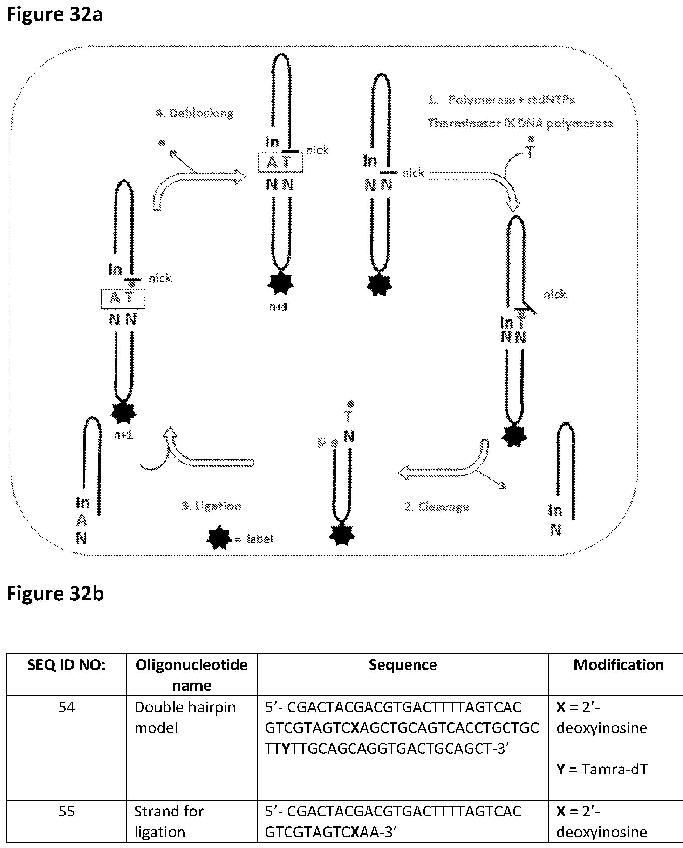

### Figure 32. Version 3 Chemistry - Complete Cycle on Double-Hairpin Model.

a) Scheme showing full cycle involving enzymatic incorporation, cleavage, ligation and deprotection steps.
b) Oligonucleotides applicable for study of the incorporation step.

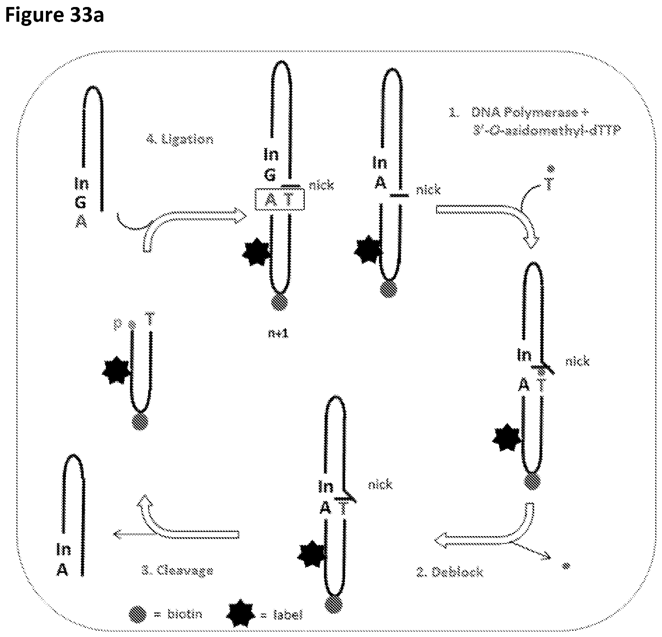

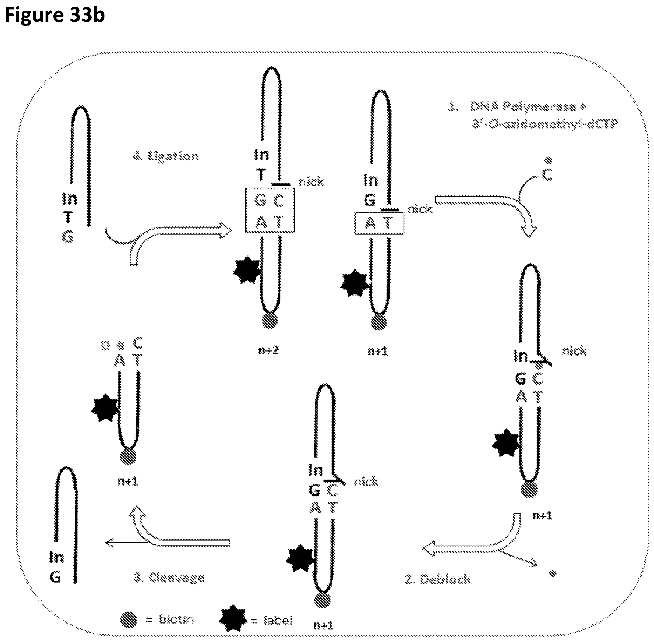

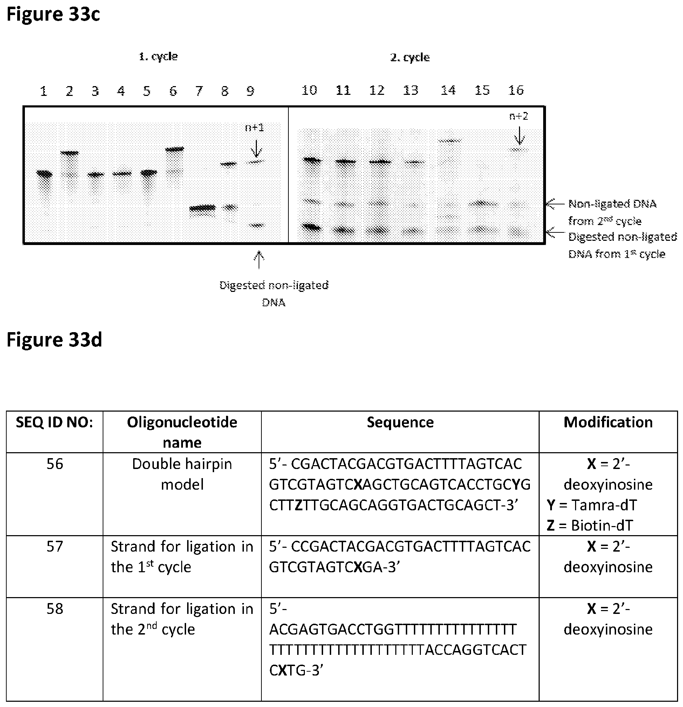

### Figure 33. Version 2 Chemistry - Complete Two-Cycle on Double-Hairpin Model.

a) Scheme showing the first full cycle involving enzymatic incorporation, deprotection, cleavage and ligation steps.
b) Scheme showing the second full cycle, following the first full cycle, involving enzymatic incorporation, deprotection, cleavage and ligation steps.
c) The Figure depicts a gel showing full two-cycle experiment comprising: incorporation, deprotection, cleavage and ligation steps.
   Lane 1. Starting material.
   Lane 2. Extension of starting material with natural dNTPs.
   Lane 3. Incorporation of 3'-O-azidomethyl-dTTP by Therminator IX DNA polymerase.
   Lane 4. Extension of the product in lane 3 by addition of all natural dNTPs.
   Lane 5. Deprotection of the product in lane 3 by TCEP.
   Lane 6. Extension of the product in lane 5 by addition of all natural dNTPs.
   Lane 7. Cleavage of the product in lane 5 by Endonuclease V.
   Lane 8. Ligation of the product in lane 7 by blunt TA ligase kit.
   Lane 9. Cleavage of the product in lane 8 by Lambda exonuclease.
   Lane 10. Starting material for second cycle - the same material as in lane 9.
   Lane 11. Incorporation of 3'-*O*-azidomethyl-dTTP by Therminator IX DNA polymerase.
   Lane 12. Extension of the product in lane 11 by addition of all natural dNTPs.
   Lane 13. Deprotection of the product in lane 11 by TCEP.
   Lane 14. Extension of the product in lane 13 by addition of all natural dNTPs.
   Lane 15. Cleavage of the product in lane 13 by Endonuclease V.
   Lane 16. Ligation of the product in lane 15 by blunt TA ligase kit.
d) Oligonucleotides used for study.

### Figure 34.

Example showing a mechanism of release from a scaffold polynucleotide of a polynucleotide of predefined sequence, as synthesised in accordance with the methods described herein.

### Figure 35.

Schematic of an exemplary method for the synthesis of RNA according to the invention. The exemplary method shows synthesis in the absence of a helper strand.

### Figure 36.

Schematic of an exemplary method for the synthesis of RNA according to the invention. The exemplary method shows synthesis in the presence of a helper strand.

### Figure 37.

Schematic of an exemplary method for the synthesis of RNA according to the invention. The exemplary method shows synthesis in the presence of a helper strand.

### Figure 38.

Schematic of the 1st full cycle of an exemplary method for the synthesis of DNA according to synthesis method version 2 with single hairpin model, involving a step of denaturing the helper strand prior to the incorporation step.

### Figure 39.

Schematic of the 2nd full cycle of an exemplary method for the synthesis of DNA according to synthesis method version 2 with single hairpin model, involving a step of denaturing the helper strand prior to the incorporation step.

### Figure 40.

Schematic of the 3rd full cycle of an exemplary method for the synthesis of DNA according to synthesis method version 2 with single hairpin model, involving a step of denaturing the helper strand prior to the incorporation step.

### Figure 41.

Oligonucleotides used in the experiments detailed in Example 9.

### Figure 42.

Gel showing reaction products corresponding to a full three-cycle experiment as detailed in Example 9.

The Figure depicts a gel showing the results of a full three-cycle experiment comprising: incorporation, deblock, cleavage and ligation steps.
Lane 1: Starting material.
Lane 2. Extension of starting material with natural dNTPs
Lane 3: Incorporation of 3'-O-azidomethyl-dTTP by Therminator X DNA polymerase.
Lane 4: Extension of the product in lane 3 by addition of all natural dNTPs.¬
Lane 5: Deblock of the product in lane 3 by TCEP
Lane 6: Extension of the product in lane 5 by addition of all natural dNTPs.¬
Lane 7: Cleavage of the product in lane 5 by Endonuclease V.
Lane 8: Ligation of the product in lane 7 by T3 DNA ligase
Lane 9: Starting material for 2nd cycle - the same material as in lane 9
Lane 10: Extension of the product in lane 9 by addition of all natural dNTPs.
Lane 11: Incorporation of 3'-*O*-azidomethyl-dTTP by Therminator X DNA polymerase.
Lane 12: Extension of the product in lane 11 by addition of all natural dNTPs.
Lane 13: Deblock of the product in lane 11 by TCEP
Lane 14: Extension of the product in lane 13 by addition of all natural dNTPs.
Lane 15: Cleavage of the product in lane 13 by Endonuclease V
Lane 16: Ligation of the product in lane 15 by T3 DNA ligase
Lane 17: Starting material for 3rd cycle - the same material as in lane 16
Lane 18: Extension of the product in lane 17 by addition of all natural dNTPs.
Lane 19: Incorporation of 3'-*O*-azidomethyl-dTTP by Therminator X DNA polymerase.
Lane 20: Extension of the product in lane 19 by addition of all natural dNTPs.
Lane 21: Deblock of the product in lane 19 by TCEP
Lane 22: Extension of the product in lane 21 by addition of all natural dNTPs.
Lane 23: Cleavage of the product in lane 21 by Endonuclease V
Lane 24: Ligation of the product in lane 23 by T3 DNA ligase

### Figure 43.

Fluorescence signals from polyacrylamide gel surfaces spiked with different amount of BRAPA exposed to FITC-PEG-SH and FITC-PEG-COOH.

### Figure 44.

Measured fluorescence signals from fluorescein channel on polyacrylamide gel surfaces spiked with different amount of BRAPA that are exposed to FITC-PEG-SH and FITC-PEG-COOH.

### Figure 45.

(a) Shows sequences of hairpin DNA without linker immobilised on different samples.
(b) Shows sequences of hairpin DNA with linker immobilised on different samples.

### Figure 46.

Fluorescence signals from hairpin DNA oligomers with and without linker immobilised onto bromoacetyl functionalised polyacrylamide surfaces.

### Figure 47.

Measured fluorescence from hairpin DNA oligomers with and without linker immobilised onto bromoacetyl functionalised polyacrylamide surfaces.

### Figure 48.

Fluorescence signals from hairpin DNA oligomers with and without linker immobilised onto bromoacetyl functionalised polyacrylamide surfaces following incorporation of triphosphates.

### Figure 49.

Measured fluorescence from hairpin DNA oligomers with and without linker immobilised onto bromoacetyl functionalised polyacrylamide surfaces following incorporation of triphosphates.

### Figure 50.

(a) Experimental overview and outcome for each reaction step as detailed in Example 12.
(b) Oligonucleotides used in the experiments detailed in Example 12.

### Figure 51.

Shows fluorescence signals from hairpin DNA oligomers before and after cleavage reactions (Example 12).

### Figure 52.

Shows measured fluorescence signals from hairpin DNA oligomers before and after cleavage reactions (Example 12).

### Figure 53.

Shows the sequences for the inosine-containing strand and the complimentary 'helper' strand for ligation reactions (Example 12).

### Figure 54.

Results relating to fluorescence signals from hairpin DNA oligomers corresponding to the monitoring of ligation reactions (Example 12).

### Figure 55.

Results relating to measured fluorescence from hairpin DNA oligomers corresponding to the monitoring of ligation reactions (Example 12).

### Figure 56.

Results relating to incorporation of 3'-*O*-modified-dNTPs by Therminator X DNA polymerase using an incorporation step according to methods of the invention, *e.g.* synthesis method versions of the invention 1, 2, 3 and 4 and variants thereof (Figures 1 to 10 and Example 13).

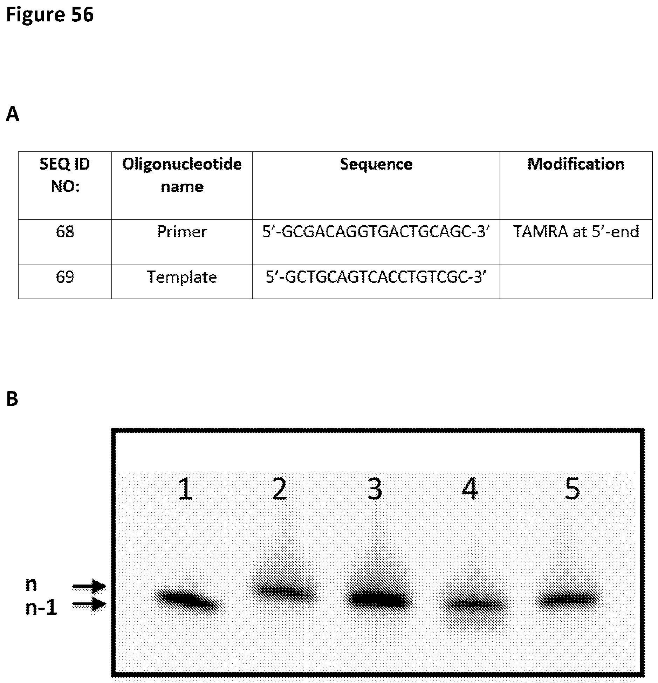

Figure 56a provides the nucleic acid sequences of primer strand (primer strand portion of synthesis strand; SEQ ID NO: 68) and template strand (support strand; SEQ ID NO: 69).

Figure 56b depicts a gel showing the results of incorporation of 3'-*O*-modified-dNTPs by Therminator X DNA polymerase in presence of Mn2+ ions at 37°C.
Lane 1: Starting oligonucleotide.
Lane 2: Incorporation of 3'-*O*-azidomethyl-dTTP (>99% efficiency)
Lane 3: Incorporation of 3'-*O*-azidomethyl-dATP (>99% efficiency).
Lane 4: Incorporation of 3'-*O*-azidomethyl-dCTP (>90% efficiency).
Lane 5: Incorporation of 3'-*O*-azidomethyl-dGTP (>99% efficiency).

Upon addition, the newly added 3'-*O*-modified-dNTP occupies position n in the primer strand portion. The next nucleotide position in the primer strand portion is designated n-1.

### Figure 57.

The figure shows a scheme depicting a DNA synthesis reaction cycle as described in Example 14.

### Figure 58.

The figure shows oligonucleotides used in the experiments described in Example 14.

### Figure 59.

The figure shows a photograph of a gel demonstrating the results of the ligation of a polynucleotide ligation molecule comprising 2-deoxyinosine, used as a universal nucleotide, to a hairpin scaffold polynucleotide as described in Example 14. Lane 1 shows the starting hairpin scaffold polynucleotide and lane 2 shows the hairpin scaffold polynucleotide ligated to the polynucleotide ligation molecule.

### Figures 60 and 61.

The figures show schemes depicting DNA synthesis reaction cycles as described in Example 15.

### Figure 62.

The figure shows oligonucleotides used in the experiments described in Example 15.

### Figure 63.

The figure shows a photograph of a gel demonstrating the results of the ligation of polynucleotide ligation molecules comprising 2-deoxyinosine, used as a universal nucleotide, to hairpin scaffold polynucleotides as described in Example 15. The lanes of the gel are as follows:

| | |
|---|---|
| Lane 1: | Starting hairpin scaffold polynucleotide. |
| Lane 2: | Hairpin scaffold polynucleotide ligated to polynucleotide ligation molecule (1 base T overhang). |
| Lane 3: | Hairpin scaffold polynucleotide ligated to polynucleotide ligation molecule (1 base C overhang). |
| Lane 4: | Starting hairpin scaffold polynucleotide. |
| Lane 5: | Hairpin scaffold polynucleotide ligated to polynucleotide ligation molecule (2 bases overhang). |
| Lane 6: | Hairpin scaffold polynucleotide ligated to polynucleotide ligation molecule (3 bases overhang). |
| Lane 7: | Hairpin scaffold polynucleotide ligated to polynucleotide ligation molecule (4 bases overhang). |

### Interpretation of Figures.

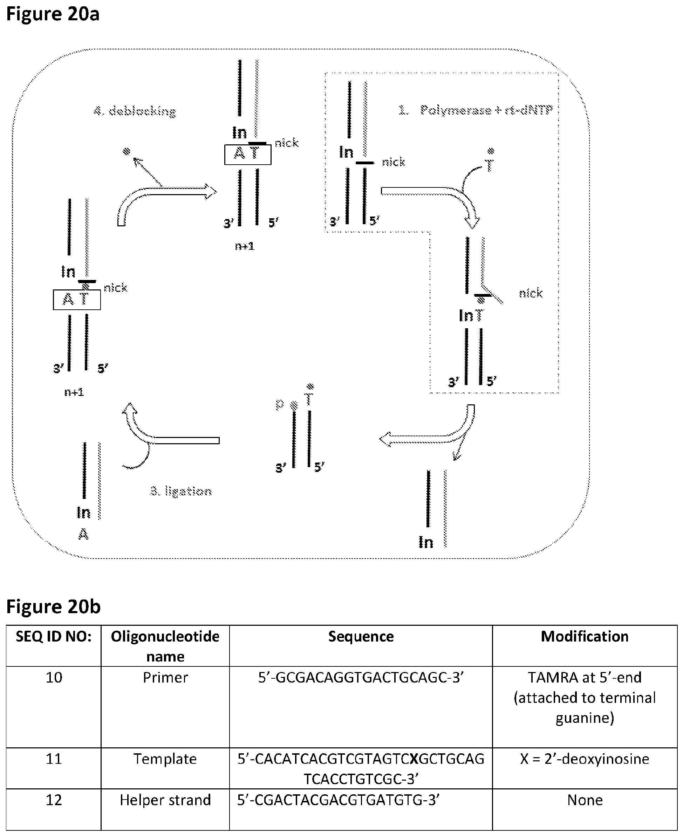

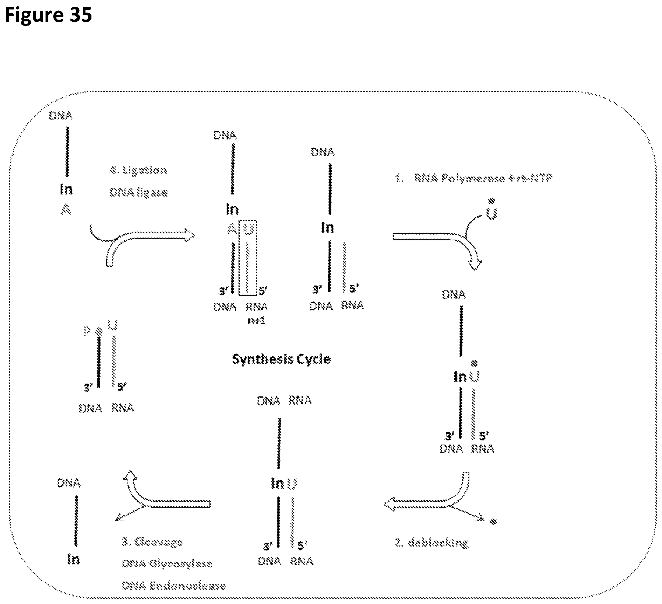

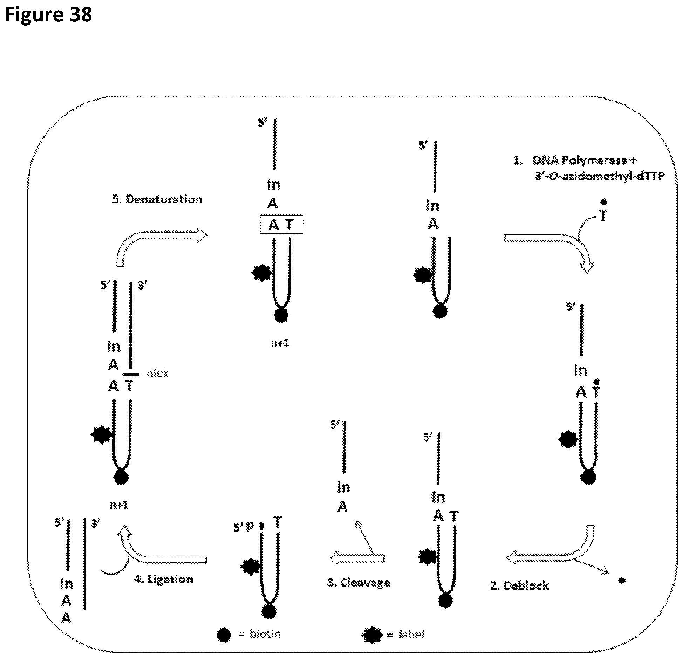

The structures depicted in Figures 16, 17a, 18a, 19a, 20a, 21a, 22a, 23a, 24a, 25a, 26a, 27a, 28a, 29a, 30a, 31a, 32a, 33a, 33b, 34, 35, 36, 37, 38, 39, and 40 are to be interpreted consistently with those depicted in Figures 11, 12, 13, 14 and 15. Thus in these Figures, each left hand strand of a double-stranded scaffold polynucleotide molecule relates to the support strand (corresponding to strand "a" in Figures 11 to 15); each right hand strand of a double-stranded scaffold polynucleotide molecule relates to the synthesis strand (corresponding to strand "b" in Figures 11 to 15); all scaffold polynucleotide molecules comprise a lower synthesis strand which corresponds to a strand comprising a primer strand portion (corresponding to the solid and dotted line of strand "b" in Figures 6 to 10); certain scaffold polynucleotide molecules (*e.g*. in Figures 20a and 28a) are shown, prior to incorporation of the new nucleotide, with an upper synthesis strand which corresponds to a strand comprising a helper strand portion (corresponding to the dashed line of strand "b" in Figures 11 to 15); certain scaffold polynucleotide molecules (*e.g*. in Figures 17a, 18a and 19a) are shown with no helper strand portion (corresponding to an absence of the dashed line of strand "b" in Figures 11 to 15); and certain scaffold polynucleotide molecules (*e.g*. in Figures 38, 39 and 40) are shown, after the ligation step, with an upper synthesis strand which corresponds to a strand comprising a helper strand portion (corresponding to the dashed line of strand "b" in Figures 11 to 15) and wherein the helper strand portion is removed prior to incorporation of the new nucleotide in the next synthesis cycle.

In addition, in these Figures, where relevant, each new nucleotide is shown to be incorporated together with a reversible terminator group, labelled rtNTP and depicted as a small circular structure (corresponding to the small triangular structure in Figures 11 to 15) and terminal phosphate groups are labelled "p" and depicted as a small elliptical structure.

Figures 16c, 16d, 16g, 16h, 27a, 28a, 29a, 30a, 32a, 33a, 33b, and 34 show scaffold polynucleotide molecules wherein strands comprising a helper strand portion and support strands are connected by a hairpin loop. Figures 16b, 27a, 28a, 29a, 30a, 31a, 32a, 33a, 33b, 34, 38, 39, and 40 show scaffold polynucleotide molecules wherein strands comprising a primer strand portion and support strands are connected by a hairpin loop.

Figures such as Figure 32a and 33a show scaffold polynucleotide molecules wherein the strand comprising a helper strand portion (upper right strand) and the support strand (upper left strand) is connected by a hairpin loop and, in the same molecule, the strand comprising the primer strand portion (lower right strand) and the support strand (lower left strand) are connected by a hairpin loop.

### DETAILED DESCRIPTION OF THE INVENTION

The present invention provides methods for the *de novo* synthesis of polynucleotide molecules according to a predefined nucleotide sequence. Synthesised polynucleotides are preferably DNA and are preferably double-stranded polynucleotide molecules. The invention provides advantages compared with existing synthesis methods. For example, all reaction steps may be performed in aqueous conditions at mild pH, extensive protection and deprotection procedures are not required. Furthermore, synthesis is not dependent upon the copying of a pre-existing template strand comprising the predefined nucleotide sequence.

The present inventors have determined that the use of a universal nucleotide, as defined herein, allows for the creation of a polynucleotide cleavage site within a synthesised region which facilitates cleavage and repeat cycles of synthesis. The invention provides versatile methods for synthesising polynucleotides, and for assembling large fragments comprising such synthesised polynucleotides.

Certain embodiments of the synthesis methods of the invention will be described in more general detail herein by reference to exemplary methods including four exemplary method versions of the invention and certain variants thereof (Figures 1 to 10). It is to be understood that all exemplary methods, including the exemplary method versions of the invention and variants thereof, are not intended to be limiting on the invention. The invention provides an *in vitro* method of synthesising a double-stranded polynucleotide molecule having a predefined sequence, the method comprising performing cycles of synthesis wherein in each cycle a first polynucleotide strand is extended by the incorporation of a first nucleotide of the predefined sequence, and then the second polynucleotide strand which is hybridized to the first strand is extended by the incorporation of a second nucleotide of the predefined sequence. Preferably, the methods are for synthesising DNA. Specific methods described herein are provided as embodiments of the invention.

### Reaction conditions

In one aspect the invention provides a method for synthesising a double-stranded polynucleotide having a predefined sequence.

In some embodiments, synthesis is carried out under conditions suitable for hybridization of nucleotides within double-stranded polynucleotides. Polynucleotides are typically contacted with reagents under conditions which permit the hybridization of nucleotides to complementary nucleotides. Conditions that permit hybridization are well-known in the art (for example, Sambrook et al., 2001, Molecular Cloning: a laboratory manual, 3rd edition, Cold Spring Harbour Laboratory Press; and Current Protocols in Molecular Biology, Greene Publishing and Wiley-lnterscience, New York (1995)).

Incorporation of nucleotides into polynucleotides can be carried out under suitable conditions, for example using a polymerase (*e.g*., Therminator IX polymerase) or a terminal deoxynucleotidyl transferase (TdT) enzyme or functional variant thereof to incoprorate modified nucleotides (*e.g*., 3'-*O*-modified-dNTPs) at a suitable temperature *(e.g.,~65°C)* in the presence of a suitable buffered solution. In one embodiment, the buffered solution can comprise 2 mM Tris-HCl, 1 mM (NH₄)₂SO₄, 1 mM KCl, 0.2 mM MgSO₄ and 0.01% Triton^{®} X-100.

Cleavage of polynucleotides can be carried out under suitable conditions, for example using a polynucleotide cleaving enzyme (*e.g*., endonuclease) at a temperature that is compatible with the enzyme (*e.g*., 37°C) in the presence of a suitable buffered solution. In one embodiment, the buffered solution can comprise 5 mM potassium acetate, 2 mM Tris-acetate, 1 mM magnesium acetate and 0.1 mM DTT.

Ligation of polynucleotides can be carried out under suitable conditions, for example using a ligase *(e.g.,* T4 DNA ligase) at a temperature that is compatible with the enzyme (*e.g*., room temperature) in the presence of a suitable buffered solution. In one embodiment, the buffered solution can comprise 4.4 mM Tris-HCl, 7mM MgCl₂, 0.7mM dithiothreitol, 0.7mM ATP, 5% polyethylene glycol (PEG6000).

Deprotection can be carried out under suitable conditions, for example using a reducing agent *(e.g.,* TCEP). For example, deprotection can be performed using TCEP in Tris buffer *(e.g.,* at a final concentration of 300mM).

### Anchor polynucleotides and scaffold polynucleotides

Double-stranded polynucleotides having a predefined sequence are synthesized by methods of the invention by incorporation of pre-defined nucleotides into a pre-existing polynucleotide, referred to herein as a scaffold polynucleotide, which may be attached to or capable of being attached to a surface as described herein. As described in more detail herein a scaffold polynucleotide forms a support structure to accommodate the newly-synthesised polynucleotide and, as will be apparent from the description herein, does not comprise a pre-existing template strand which is copied as in conventional methods of synthesis. A scaffold polynucleotide may be referred to as an anchor polynucleotide if the scaffold polynucleotide is attached to a surface. Surface attachment chemistries for attaching a scaffold polynucleotide to a surface to form an anchor polynucleotide are described in more detail herein.

A scaffold polynucleotide comprises a synthesis strand hybridized to a complementary support strand. The synthesis strand comprises a primer strand portion *(e.g.* see Figures 1 to 10). The synthesis strand may be provided hybridized to the complementary support strand. Alternatively, the support strand and the synthesis strand may be provided separately and then allowed to hybridise.

A scaffold polynucleotide may be provided with each of the support and synthesis strands unconnected at adjacent ends. A scaffold polynucleotide may be provided with both support and synthesis strands connected at adjacent ends, such as via a hairpin loop, at both ends of the scaffold polynucleotide. A scaffold polynucleotide may be provided with both support and synthesis strands connected at adjacent ends, such as via a hairpin loop, at one end of the scaffold polynucleotide or any other suitable linker.

Scaffold polynucleotides with or without hairpins may be immobilized to a solid support or surface as described in more detail herein (see Figure 16).

The terms "hairpin" or "hairpin loop" are commonly used in the current technical field. The term "hairpin loop" is also often referred to as a "stem loop". Such terms refer to a region of secondary structure in a polynucleotide comprising a loop of unpaired nucleobases which form when one strand of a polynucleotide molecule hybridizes with another section of the same strand due to intramolecular base pairing. Thus hairpins can resemble U-shaped structures. Examples of such structures are shown in Figure 16.

In certain methods described herein new synthesis is initiated by incorporating into the scaffold polynucleotide a first nucleotide of the predefined sequence by the action of a ligase enzyme. Thus the first nucleotide of the predefined sequence is ligated to the terminal nucleotide of the support strand of the scaffold polynucleotide as described further herein. The first nucleotide of the predefined sequence is provided by a polynucleotide ligation molecule which comprises a support strand, a helper strand and a complementary ligation end. The first nucleotide of the predefined sequence is provided as the terminal nucleotide of the support strand of the complementary ligation end.

The terminal nucleotide of the helper strand at the complementary ligation end is a non-ligatable nucleotide, and is typically provided lacking a phosphate group. This prevents the terminal nucleotide of the helper strand ligating with the terminal nucleotide of the primer strand portion of the scaffold polynucleotide and creates a single-strand break site between the helper strand and the primer strand portion following ligation. Creation and maintenance of the single-strand break could be effected by other means. For example, the terminal nucleotide of the helper strand may be provided with a suitable blocking group which prevents ligation with the primer strand portion.

In certain methods described herein a second nucleotide of the predefined sequence is incorporated into the scaffold polynucleotide by the action of a polymerase or transferase enzyme. Thus the polymerase enzyme or transferase enzyme will act to extend the terminal nucleotide of the primer strand portion.

Further details of the general method schemes of exemplary methods are provided further herein.

### Nucleotides and universal nucleotides

Nucleotides which can be incorporated into synthetic polynucleotides by any of the methods described herein may be nucleotides, nucleotide analogues and modified nucleotides.

Nucleotides may comprise natural nucleobases or non-natural nucleobases. Nucleotides may contain a natural nucleobase, a sugar and a phosphate group. Natural nucleobases comprise adenosine (A), thymine (T), uracil (U), guanine (G) and cytosine (C). One of the components of the nucleotide may be further modified.

Nucleotide analogues are nucleotides that are modified structurally either in the base, sugar or phosphate or combination therein and that are still acceptable to a polymerase enzyme as a substrate for incorporation into an oligonucleotide strand.

A non-natural nucleobase may be one which will bond, *e.g.* hydrogen bond, to some degree to all of the nucleobases in the target polynucleotide. A non-natural nucleobase is preferably one which will bond, *e.g*. hydrogen bond, to some degree to nucleotides comprising the nucleosides adenosine (A), thymine (T), uracil (U), guanine (G) and cytosine (C).

A non-natural nucleotide may be a peptide nucleic acid (PNA), a locked nucleic acid (LNA) and an unlocked nucleic acid (UNA), a bridged nucleic acid (BNA) or a morpholino, a phosphorothioate or a methylphosphonate.

A non-natural nucleotide may comprise a modified sugar and/or a modified nucleobase. Modified sugars include but are not limited to 2'-*O-*methylribose sugar. Modified nucleobases include but are not limited to methylated nucleobases. Methylation of nucleobases is a recognised form of epigenetic modification which has the capability of altering the expression of genes and other elements such as microRNAs. Methylation of nucleobases occurs at discrete loci which are predominately dinucleotide consisting of a CpG motif, but may also occur at CHH motifs (where H is A, C, or T). Typically, during methylation a methyl group is added to the fifth carbon of cytosine bases to create methylcytosine. Thus modified nucleobases include but are not limited to 5-methylcytosine.

Nucleotides of the predefined sequence may be incorporated opposite partner nucleotides to form a nucleotide pair. A partner nucleotide may be a complementary nucleotide. A complementary nucleotide is a nucleotide which is capable of bonding, *e.g*. hydrogen bonding, to some degree to the nucleotides of the predefined sequence.

Typically, a nucleotide of the predefined sequence is incorporated into a polynucleotide opposite a naturally complementary partner nucleobase. Thus adenosine may be incorprated opposite thymine and vice versa. Guanine may be incorprated opposite cytosine and vice versa. Alternatively, a nucleotide of the predefined sequence may be incoporated opposite a partner nucleobase to which it will bond, *e.g*. hydrogen bond, to some degree.

Alternatively a partner nucleotide may be a non-complementary nucleotide. A non-complementary nucleotide is a nucleotide which is not capable of bonding, *e.g*. hydrogen bonding, to the nucleotide of the predefined sequence. Thus a nucleotide of the predefined sequence may be incorporated opposite a partner nucleotide to form a mismatch, provided that the synthesised polynucleotide overall is double-stranded and wherein the first strand is attached to the second strand by hybridization.

The term "opposite" is to be understood as relating to the normal use of the term in the field of nucleic acid biochemistry, and specifically to conventional Watson-Crick base-pairing. Thus a first nucleic acid molecule of sequence 5'-ACGA-3' may form a duplex with a second nucleic acid molecule of sequence 5'-TCGT-3' wherein the G of the first molecule will be positioned opposite the C of the second molecule and will hydrogen bond therewith. A first nucleic acid molecule of sequence 5'-ATGA-3' may form a duplex with a second nucleic acid molecule of sequence 5'-TCGT-3', wherein the T of the first molecule will mismatch with the G of the second molecule but will still be positioned opposite therewith and will act as a partner nucleotide. This principle applies to any nucleotide partner pair relationship disclosed herein, including partner pairs comprising universal nucleotides.

In all of the methods described herein a position in the support strand, and the opposite position in the synthesis strand, is assigned the position number "n". This position refers to the position of a nucleotide in the support strand of a scaffold polynucleotide which in any given synthesis cycle is opposite the nucleotide position in the synthesis strand which is occupied by or will be occupied by the second or further nucleotide of the predefined sequence upon its addition to the terminal end of the primer strand portion in that cycle or in incorporation steps of subsequent cycles. Position "n" also refers to the position in the support strand of a polynucleotide ligation molecule prior to the ligation step which position is the nucleotide position which will be opposite the second or further nucleotide of the predefined sequence upon ligation of the polynucleotide ligation molecule scaffold polynucleotide and incorporation of the second or further nucleotide of the predefined sequence by the action of the polymerase enzyme or transferase enzyme.

Both the position in the support strand and the opposite position in the synthesis strand may be referred to as positon n.

Further details concerning the definition of position "n" are provided with reference to Figures 1 to 10 and the descriptions thereof in relation to the exemplary synthesis method versions of the invention and variants described in more detail herein.

Nucleotides and nucleotide analogues may preferably be provided as nucleoside triphosphates. Thus in any of the methods of the invention in order to synthesise DNA polynucleotides, nucleotides may be incorporated from 2'-deoxyribonucleoside-5'-O-triphosphates (dNTPs), *e.g.* via the action of a DNA polymerase enzyme or *e.g.* via the action of an enzyme having deoxynucleotidyl terminal transferase activity. In any of the methods of the invention in order to synthesise RNA polynucleotides, nucleotides may be incorporated ribonucleoside-5'-*O*-triphosphates (NTPs), *e.g.* via the action of a RNA polymerase enzyme or *e.g.* via the action of an enzyme having nucleotidyl terminal transferase activity. Triphosphates can be substituted by tetraphosphates or pentaphosphates (generally oligophosphate). These oligophosphates can be substituted by other alkyl or acyl groups:

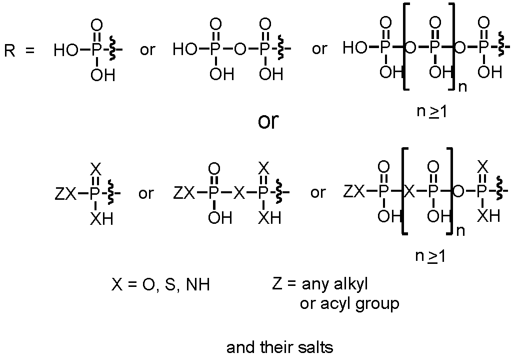

Methods of the invention may use a universal nucleotide. A universal nucleotide may be used as a component of the support strand of a scaffold molecule to facilitate a newly-incorporated nucleotide to be correctly paired with its desired partner nucleotide during each cycle of synthesis. A universal nucleotide may also be incorporated into the synthesis strand as a component of the predefined nucleotide sequence if desired.

A universal nucleotide is one wherein the nucleobase will bond, *e.g*. hydrogen bond, to some degree to the nucleobase of any nucleotide of the predefined sequence. A universal nucleotide is preferably one which will bond, *e.g*. hydrogen bond, to some degree to nucleotides comprising the nucleosides adenosine (A), thymine (T), uracil (U), guanine (G) and cytosine (C). The universal nucleotide may bond more strongly to some nucleotides than to others. For instance, a universal nucleotide (I) comprising the nucleoside, 2'-deoxyinosine, will show a preferential order of pairing of I-C > I-A > I-G approximately = I-T.

Examples of possible universal nucleotides are inosines or nitro-indoles. The universal nucleotide preferably comprises one of the following nucleobases: hypoxanthine, 4-nitroindole, 5-nitroindole, 6-nitroindole, 3-nitropyrrole, nitroimidazole, 4-nitropyrazole, 4-nitrobenzimidazole, 5-nitroindazole, 4-aminobenzimidazole or phenyl (C6-aromatic ring. The universal nucleotide more preferably comprises one of the following nucleosides: 2'-deoxyinosine, inosine, 7-deaza-2'-deoxyinosine, 7-deaza-inosine, 2-aza-deoxyinosine, 2-aza-inosine, 4-nitroindole 2'-deoxyribonucleoside, 4-nitroindole ribonucleoside, 5-nitroindole 2' deoxyribonucleoside, 5-nitroindole ribonucleoside, 6-nitroindole 2' deoxyribonucleoside, 6-nitroindole ribonucleoside, 3-nitropyrrole 2' deoxyribonucleoside, 3-nitropyrrole ribonucleoside, an acyclic sugar analogue of hypoxanthine, nitroimidazole 2' deoxyribonucleoside, nitroimidazole ribonucleoside, 4-nitropyrazole 2' deoxyribonucleoside, 4-nitropyrazole ribonucleoside, 4-nitrobenzimidazole 2' deoxyribonucleoside, 4-nitrobenzimidazole ribonucleoside, 5-nitroindazole 2' deoxyribonucleoside, 5-nitroindazole ribonucleoside, 4-aminobenzimidazole 2' deoxyribonucleoside, 4-aminobenzimidazole ribonucleoside, phenyl C-ribonucleoside or phenyl C-2'-deoxyribosyl nucleoside.

Some examples of universal bases are shown below:

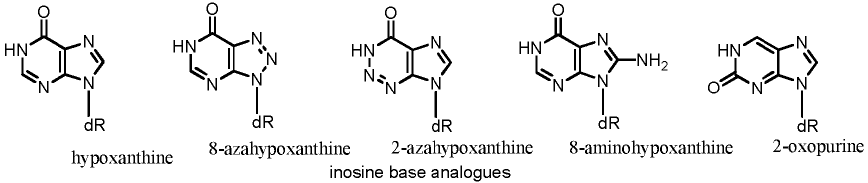

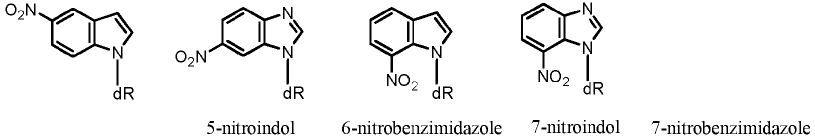

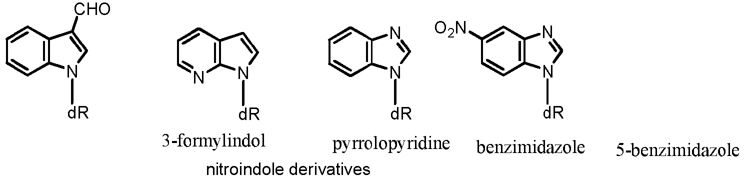

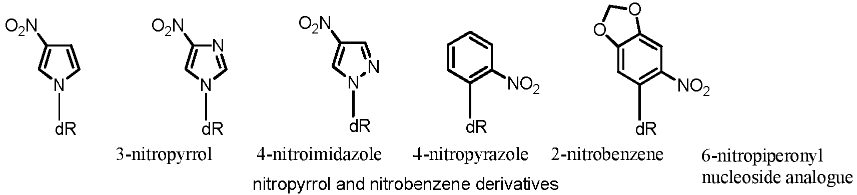

Universal nucleotides incorporating cleavable bases may also be used, including photo- and enzymatically-cleavable bases, some examples of which are shown below.

Photocleavable bases:

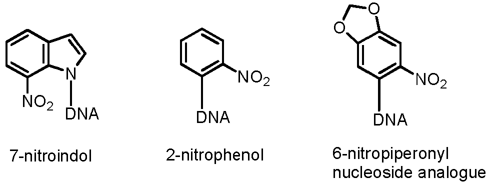

Base analogues cleavable by Endonuclease III:

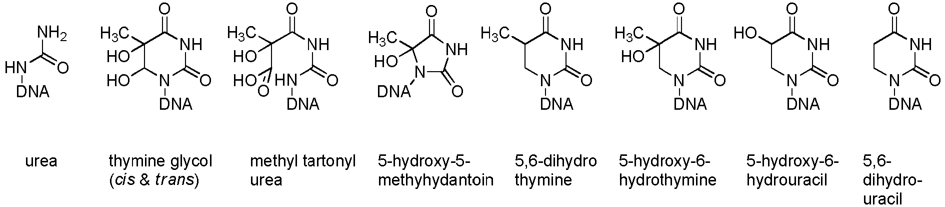

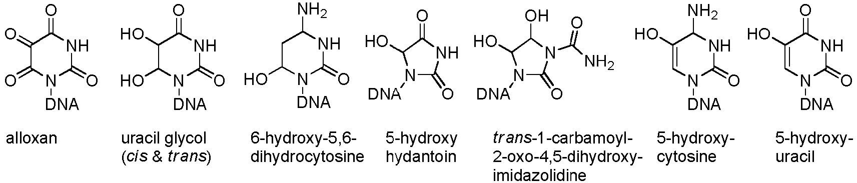

Base analogues cleavable by Formamidopyrimidine DNA glycosylase (Fpg):

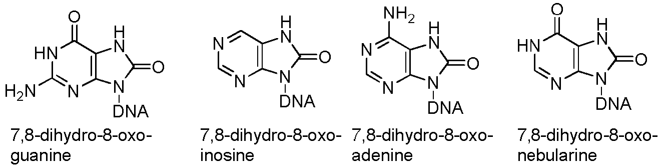

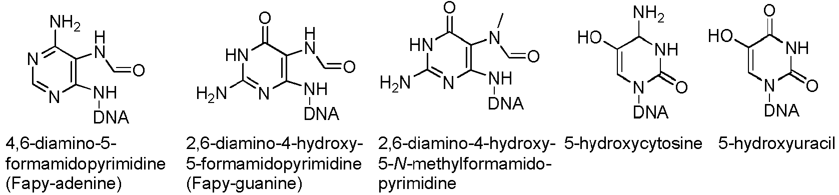

Base analogues cleavable by 8-oxoguanine DNA glycosylase (hOGG1):

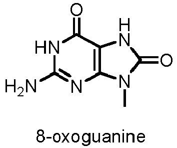

Base analogues cleavable by hNeil1:

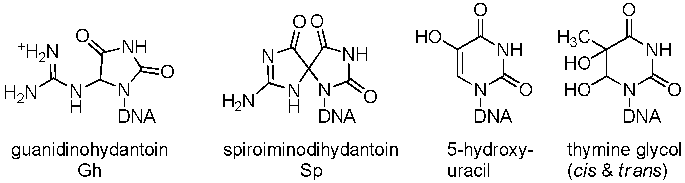

Base analogues cleavable by Thymine DNA glycosylase (TDG):

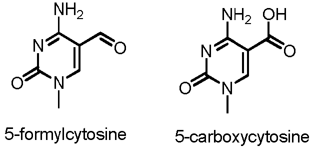

Base analogues cleavable by Human Alkyladenine DNA glycosylase (hAAG):

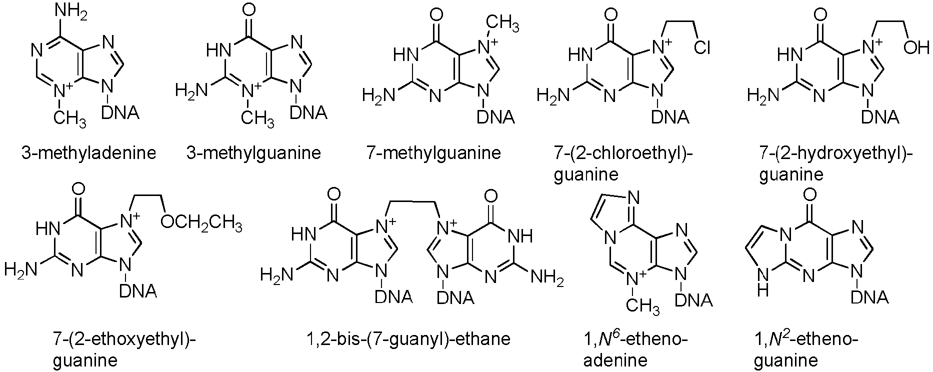

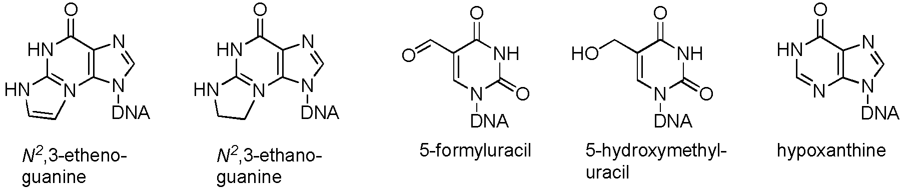

Bases cleavable by uracil DNA glycosylase:

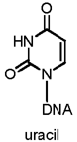

Bases cleavable by Human single-strand-selective monofunctional uracil-DNA Glycosylase (SMUG1):

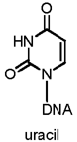

Bases cleavable by 5-methylcytosine DNA glycosylase (ROS1):

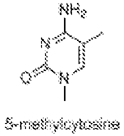

(see S. S. David, S. D. Williams Chemical reviews 1998, 98, 1221-1262 and M. I. Ponferrada-Marin, T. Roldán-Arjona, R. R. Ariza, Nucleic Acids Res 2009 ,37, 4264-4274).

In any of the methods involving scaffold polynucleotides, the universal nucleotide most preferably comprises 2'-deoxyinosine.

Examples of epigenetic bases which may be incorporated using any of the synthesis methods described herein include the following:

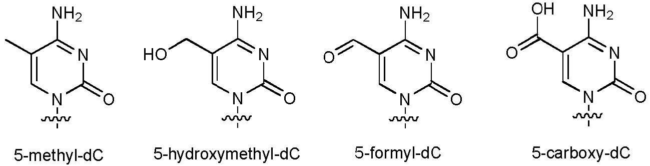

Examples of modified bases which may be incorporated using any of the synthesis methods described herein include the following:

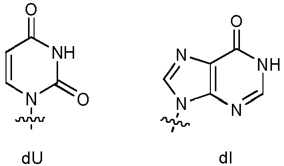

Examples of halogenated bases which may be incorporated using any of the synthesis methods described herein include the following: 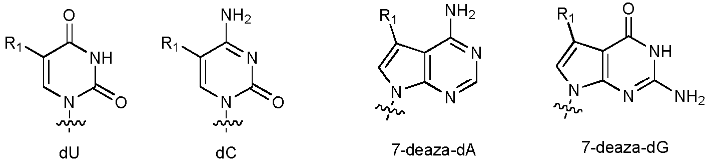 where R1 = F, Cl, Br, I, alkyl, aryl, fluorescent label, aminopropargyl, aminoallyl.

Examples of amino-modified bases, which may be useful in e.g. attachment/linker chemistry, which may be incorporated using any of the synthesis methods described herein include the following: 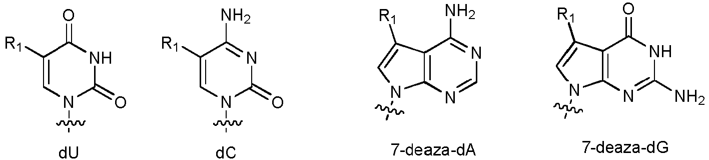 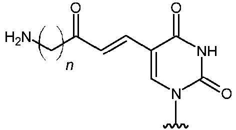 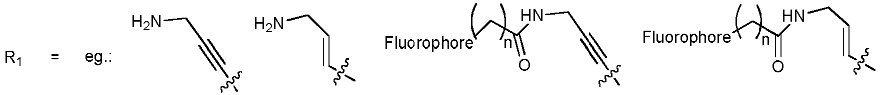 where base = A, T, G or C with alkyne or alkene linker.

Examples of modified bases, which may be useful in *e.g.* click chemistry, which may be incorporated using any of the synthesis methods described herein include the following:

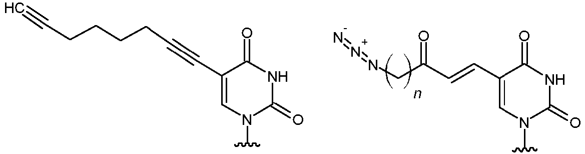

Examples of biotin-modified bases which may be incorporated using any of the synthesis methods described herein include the following: 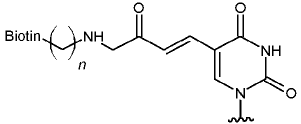 where base = A, T, G or C with alkyne or alkene linker.

Examples of bases bearing fluorophores and quenchers which may be incorporated using any of the synthesis methods described herein include the following:

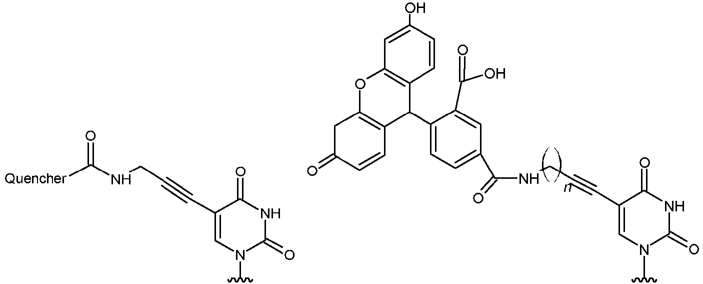

### Nucleotide-incorporating enzymes

Enzymes are available that are capable of extending, by the addition of a nucleotide, a single-stranded polynucleotide portion of a double-stranded polynucleotide molecule and/or that are capable of extending one strand of a blunt-ended double-stranded polynucleotide molecule. This includes enzymes which have template-independent enzyme activity, such as template-independent polymerase or template-independent transferase activity.

Thus in any of the methods described herein the enzyme which is used for the addition of a second nucleotide of the predefined sequence and/or a further nucleotide of the predefined sequence to the terminal end of the primer strand portion of the synthesis strand of a scaffold polynucleotide, has template-independent enzyme activity, such as template-independent polymerase or template-independent transferase activity.

Any suitable enzyme may be employed to add a predefined nucleotide using the methods described herein. Thus in all methods defined and described herein referring to the use of a polymerase or a transferase enzyme, the polymerase or transferase enzyme may be substituted with another enzyme capable of performing the same function as a polymerase or transferase enzyme in the context of the methods of the invention.

A polymerase enzyme may be employed in the methods described herein. Polymerase enzymes may be chosen based on their ability to incorporate modified nucleotides, in particular nucleotides having attached reversible terminator groups, as described herein. In the exemplary methods described herein all polymerases which act on DNA must not have 3' to 5' exonuclease activity. The polymerase may have strand displacement activity.

Thus preferably the polymerase is a modified polymerase having an enhanced ability to incorporate a nucleotide comprising a reversible terminator group compared to an unmodified polymerase. The polymerase is more preferably a genetically engineered variant of the native DNA polymerase from *Thermococcus* species 9°N, preferably species 9°N-7. Examples of modified polymerases are Therminator IX DNA polymerase and Therminator X DNA polymerase available from New England BioLabs. This enzyme has an enhanced ability to incorporate 3'-*O*-modified dNTPs.

Examples of other polymerases that can be used for incorporation of reversible terminator dNTPs in any of the methods of the invention are Deep Vent (exo-), Vent (Exo-), 9°N DNA polymerase, Therminator DNA polymerase, Therminator IX DNA polymerase, Therminator X DNA polymerase, Klenow fragment (Exo-), Bst DNA polymerase, Bsu DNA polymerase, Sulfolobus DNA polymerase I, and Taq Polymerase.

Examples of other polymerases that can be used for incorporation of reversible terminator NTPs in any of the methods of the invention are T3 RNA polymerase, T7 RNA polymerase, SP6 RNA polymerase, pol lambda, pol micro or Φ29 DNA polymerase.

For the extension of such a polynucleotide synthesis molecule comprising DNA, a DNA polymerase may be used. Any suitable DNA polymerase may be used.

The DNA polymerase may be for example Bst DNA polymerase full length, Bst DNA polymerase large fragment, Bsu DNA polymerase large fragment, E. coli DNA polymerase DNA Pol I large (Klenow) fragment, M-MuLV reverse transcriptase, phi29 DNA polymerase, Sulfolobus DNA polymerase IV, Taq DNA polymerase, T4 DNA polymerase, T7 DNA polymerase and enzymes having reverse transcriptase activity, for example M-MuLV reverse transcriptase.

The DNA polymerase may lack 3' to 5' exonuclease activity. Any such suitable polymerase enzyme may be used. Such a DNA polymerase may be, for example, Bst DNA polymerase full length, Bst DNA polymerase large fragment, Bsu DNA polymerase large fragment, DNA Pol I large (Klenow) fragment (3'→5' exo-), M-MuLV reverse transcriptase, Sulfolobus DNA polymerase IV, Taq DNA polymerase.

The DNA polymerase may possess strand displacement activity. Any such suitable polymerase enzyme may be used. Such a DNA polymerase may be, for example, Bst DNA polymerase large fragment, Bsu DNA polymerase large fragment, DNA Pol I large (Klenow) fragment (3'-5' exo-), M-MuLV reverse transcriptase, phi29 DNA polymerase.

The DNA polymerase may lack 3' to 5' exonuclease activity and may posess strand displacement activity. Any such suitable polymerase enzyme may be used. Such a DNA polymerase may be, for example, Bst DNA polymerase large fragment, Bsu DNA polymerase large fragment, E. coli DNA polymerase DNA Pol I large (Klenow) fragment, M-MuLV reverse transcriptase.

The DNA polymerase may lack 5' to 3' exonuclease activity. Any such suitable polymerase enzyme may be used. Such a DNA polymerase may be, for example, Bst DNA polymerase large fragment, Bsu DNA polymerase large fragment, DNA Pol I large (Klenow) fragment, DNA Pol I large (Klenow) fragment (3'→5' exo-), M-MuLV reverse transcriptase, phi29 DNA polymerase, Sulfolobus DNA polymerase IV, T4 DNA polymerase, T7 DNA polymerase.

The DNA polymerase may lack both 3' to 5' and 5' to 3' exonuclease activities and may possess strand displacement activity. Any such suitable polymerase enzyme may be used. Such a DNA polymerase may be, for example, Bst DNA polymerase large fragment, Bsu DNA polymerase large fragment, DNA Pol I large (Klenow) fragment (3'→5' exo-), M-MuLV reverse transcriptase.

The DNA polymerase may also be a genetically engineered variant. For example, the DNA polymerase may be a genetically engineered variant of the native DNA polymerase from *Thermococcus* species 9°N, such as species 9°N-7. One such example of a modified polymerase is Therminator IX DNA polymerase or Therminator X DNA polymerase available from New England BioLabs. Other engineered or variant DNA polymerases include Deep Vent (exo-), Vent (Exo-), 9°N DNA polymerase, Therminator DNA polymerase, Klenow fragment (Exo-), Bst DNA polymerase, Bsu DNA polymerase, Sulfolobus DNA polymerase I, and Taq Polymerase.

For the extension of such a polynucleotide synthesis molecule comprising RNA, any suitable enzyme may be used. For example an RNA polymerase may be used. Any suitable RNA polymerase may be used.

The RNA polymerase may be T3 RNA polymerase, T7 RNA polymerase, SP6 RNA polymerase, E. coli RNA polymerase holoenzyme.

The enzyme may have a terminal transferase activity, *e.g.* the enzyme may be a terminal nucleotidyl transferase, or terminal deoxynucleotidyl transferase, and wherein the polynucleotide synthesis molecule is extended to form a polynucleotide molecule comprising DNA or RNA, preferably DNA. Any of these enzymes may be used in the methods of the invention wherein extension of a polynucleotide synthesis molecule is required.

One such enzyme is a terminal nucleotidyl transferase enzyme, such as terminal deoxynucleotidyl transferase (TdT) (see *e.g.* Motea et al, 2010; Minhaz Ud-Dean, Syst. Synth. Biol., 2008, 2(3-4), 67-73). TdT is capable of catalysing the addition to a polynucleotide synthesis molecule of a nucleotide molecule (nucleoside monophosphate) from a nucleoside triphosphate substrate (NTP or dNTP). TdT is capable of catalysing the addition of natural and non-natural nucleotides. It is also capable of catalysing the addition of nucleotide analogues (Motea et al, 2010). Pol lambda and pol micro enzymes may also be used (Ramadan K, et al., J. Mol. Biol., 2004, 339(2), 395-404), as may Φ29 DNA polymerase.

Techniques for the extension of a single-stranded polynucleotide molecule, both DNA and RNA, in the absence of a template by the action of a terminal transferase enzyme (e.g. terminal deoxynucleotidyl transferase; TdT) to create an artificially-synthesised single-stranded polynucleotide molecule has been extensively discussed in the art. Such techniques are disclosed in, for example, Patent application publications WO2016/034807, WO 2016/128731, WO2016/139477 and WO2017/009663, as well as US2014/0363852, US2016/0046973, US2016/0108382, and US2016/0168611. These documents describe the controlled extension of a single-stranded polynucleotide synthesis molecule by the action of TdT to create an artificially-synthesised single-stranded polynucleotide molecule. Extension by natural and non-natural/artificial nucleotides using such enzymes is described, as is extension by modified nucleotides, for example, nucleotides incorporating blocking groups. Any of the terminal transferase enzymes disclosed in these documents may be applied to methods of the present invention, as well as any enzyme fragment, derivative, analogue or functional equivalent thereof provided that the terminal transferase function is preserved in the enzyme.

Directed evolution techniques, conventional screening, rational or semi-rational engineering/mutagenesis methods or any other suitable methods may be used to alter any such enzyme to provide and/or optimise the required function. Any other enzyme which is capable of extending a single-stranded polynucleotide molecule portion, such as a molecule comprising DNA or RNA, or one strand of a blunt-ended molecule with a nucleotide without the use of a template may be used.

Thus in any of the methods defined herein a single stranded polynucleotide synthesis molecule portion comprising DNA or blunt-ended double-stranded polynucleotide comprising DNA may be extended by an enzyme which has template-independent enzyme activity, such as template-independent polymerase or transferase activity. The enzyme may have nucleotidyl transferase enzyme activity, *e.g*. a deoxynucleotidyl transferase enzyme, such as terminal deoxynucleotidyl transferase (TdT), or an enzyme fragment, derivative, analogue or functional equivalent thereof. A polynucleotide synthesis molecule extended by the action of such an enzyme comprises DNA.

In any of the methods defined herein a single stranded portion of a polynucleotide synthesis molecule comprising RNA, or blunt-ended double-stranded polynucleotide comprising RNA may be extended by an enzyme which has nucleotidyl transferase enzyme (*e.g*. including TdT), or an enzyme fragment, derivative, analogue or functional equivalent thereof. A polynucleotide synthesis molecule extended by the action of such an enzyme may comprise RNA. For the synthesis of a single stranded polynucleotide synthesis molecule comprising RNA, or a single stranded portion of a polynucleotide synthesis molecule comprising RNA, any suitable nucleotidyl transferase enzyme may be used. Nucleotidyl transferase enzymes such as poly (U) polymerase and poly(A) polymerase *(e.g.* from *E. coli)* are capable of template-independent addition of nucleoside monophosphate units to polynucleotide synthesis molecules. Any of these enzymes may be applied to methods of the present invention, as well as any enzyme fragment, derivative, analogue or functional equivalent thereof provided that the nucleotidyl transferase function is preserved in the enzyme. Directed evolution techniques, conventional screening, rational or semi-rational engineering/mutagenesis methods or any other suitable methods may be used to alter any such enzyme to provide and/or optimise the required function.

### Reversible terminator groups

In any of the synthesis methods of the invention defined and described herein, nucleotides which are incorporated into the synthesis strand by the action of a polymerase enzyme or a transferase enzyme are preferably incorporated as nucleotides comprising one or more reversible blocking groups, also referred to as a reversible terminator group as described herein.

Such groups act to prevent further extension by the enzyme in a given synthesis cycle so that only one nucleotide of predefined sequence may controllably be used to extend the synthesis strand, and thus non-specific nucleotide incorporation is prevented. Any functionality which achieves this effect may be used in any of the methods defined and described herein. Reversible blocking groups/reversible terminator groups attached to nucleotides and deblocking steps are preferred means for achieving this effect. However this effect may be achieved by alternative means as appropriate.

Any suitable reversible blocking group may be attached to a nucleotide to prevent further extension by the enzyme following the incorporation of a nucleotide into the synthesis strand in a given cycle and to limit incorporation into the synthesis strand to one nucleotide per step. In any the methods of the invention the reversible blocking group is preferably a reversible terminator group which acts to prevent further extension by a polymerase enzyme. Examples of reversible terminators are provided below.

Propargyl reversible terminators:

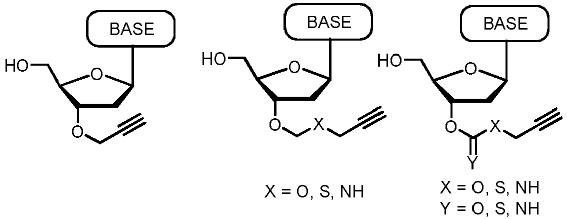

Allyl reversible terminators:

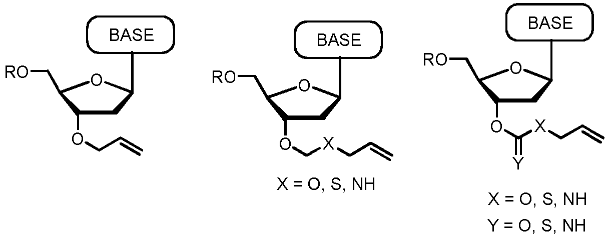

Cyclooctene reversible terminators:

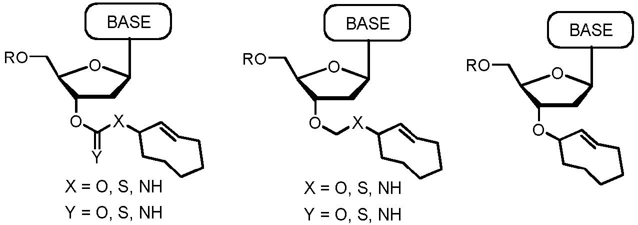

Cyanoethyl reversible terminators:

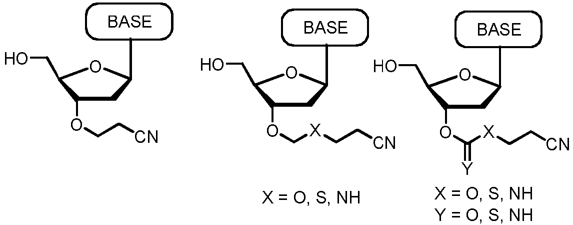

Nitrobenzyl reversible terminators:

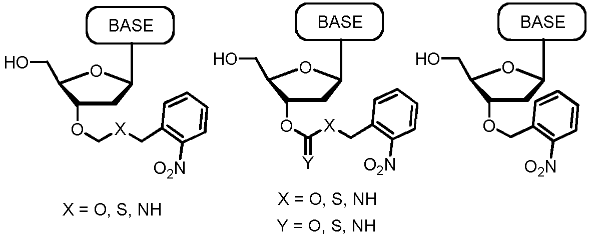

Disulfide reversible terminators:

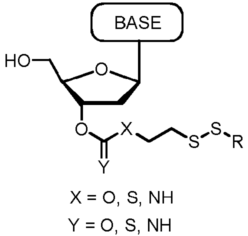

Azidomethyl reversible therminators:

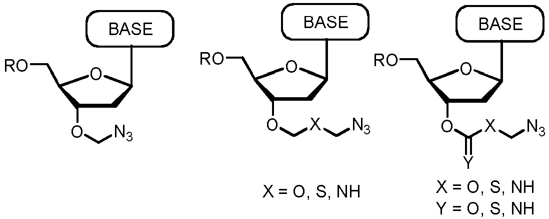

Aminoalkoxy reversible therminators:

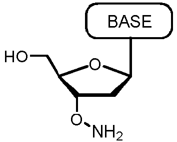

Nucleoside triphosphates with bulky groups attached to the base can serve as substitutes for a reversible terminator group on 3'-hydroxy group and can block further incorporation. This group can be deprotected by TCEP or DTT producing natural nucleotides.

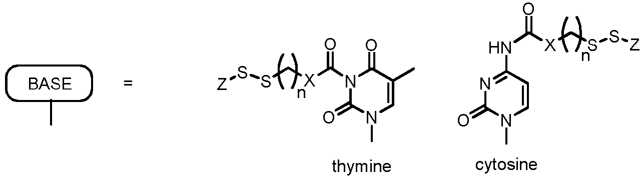

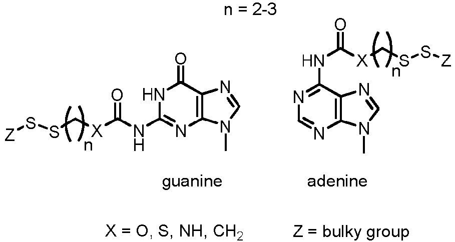

For synthesising DNA polynucleotides according to any of the methods of the invention preferred modified nucleosides are 3'-*O*-modified-2'-deoxyribonucleoside-5'-O-triphosphate. For synthesising RNA polynucleotides according to any of the methods of the invention preferred modified nucleosides are 3'-*O*-modified-ribonucleoside-5'-O-triphosphate.

Preferred modified dNTPs are modified dNTPs which are 3'*-O*-allyl-dNTPs and 3'-O-azidomethyl-dNTPs.

3'*-O*-allyl-dNTPs are shown below.

| | |
|---|---|
| 3'-*O*- allyl -dTTP: | 3'-O- allyl -dCTP: |
| 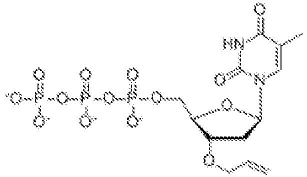 | 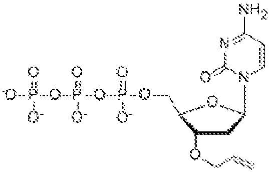 |
| 3'-*O*- allyl -dATP: | 3'-*O*- allyl -dGTP: |
| 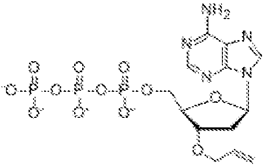 | 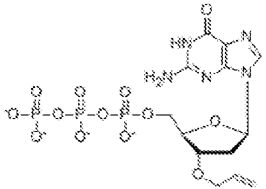 |

3'-*O*-azidomethyl-dNTPs are shown below.

| | |
|---|---|
| 3'-*O*-azidomethyl-dTTP: | 3'*-O*-azidomethyl-dCTP: |
| 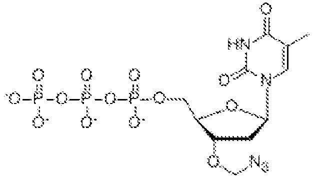 | 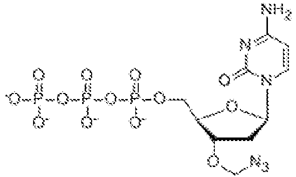 |
| 3'*-O*-azidomethyl-dATP: | 3'-*O*-azidomethyl-dGTP: |
| 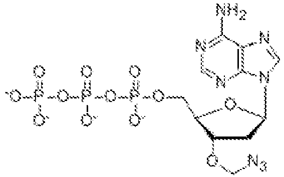 | 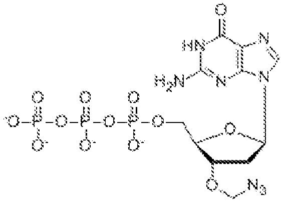 |

Methods of the invention described and defined herein may refer to a deprotection or deblocking step. Such a step involves removal of the reversible blocking group *(e.g.* the reversible terminator group) by any suitable means, or otherwise reversing the functionality of the blocking/terminator group to inhibit further extension by the enzyme/polymerase.

Any suitable reagent may be used to remove the reversible terminator group at the deprotection step.

A preferred deprotecting reagent is tris(carboxyethyl)phosphine (TCEP). TCEP may be used to remove reversible terminator groups from 3'-*O*-allyl-nucleotides (in conjunction with Pd⁰) and 3'-O-azidomethyl- nucleotides following incorporation.

Examples of deprotecting reagents are provided below.

Propargyl reversible terminators:
Treatment by Pd catalysts - Na₂PdCl₄, PdCl_{2.}
Ligands can be used e. g.: Triphenylphosphine-3,3',3"- trisulfonic acid trisodium salt.

Allyl reversible terminators:
Treatment by Pd catalysts - Na₂PdCl₄, PdCl_{2.}
Ligands can be used e. g.: Triphenylphosphine-3,3',3"- trisulfonic acid trisodium salt.

Azidomethyl reversible terminators:
Treatment by thiol (mercaptoethanol or dithiothreitol), or Tris (2-carboxyethyl)phosphine - TCEP.

Cyanoethyl reversible terminators:
Treatment by fluoride - ammonium fluoride, tetrabutylammonium fluoride (TBAF).

Nitrobenzyl reversible terminators:
Exposure to UV light

Disulfide reversible terminators:
Treatment by thiol (mercaptoethanol or dithiothreitol), or Tris (2-carboxyethyl)phosphine - TCEP.

Aminoalkoxy reversible terminators:
Treatment by nitrite (NO₂⁻, HNO₂) pH = 5.5

A reversible blocking group *(e.g.,* a reversible terminator group) can be removed by a step performed immediately after the incorporation step, provided that unwanted reagent from the incorporation step is removed to prevent further incorporation following removal of the reversible terminator group.

### Polynucleotide ligation molecule

In methods requiring the presence of scaffold polynucleotides and steps of ligation, the selection of the configuration and structure of the polynucleotide ligation molecule will also depend upon the particular method employed. The polynucleotide ligation molecule generally comprises a support strand as described herein and a helper strand as described herein. The polynucleotide ligation molecule comprises a complementary ligation end at one end of the molecule. The complementary ligation end of the polynucleotide ligation molecule will be ligated to a terminal end of the scaffold polynucleotide.

The complementary ligation end of the polynucleotide ligation molecule is provided with a non-ligatable terminal nucleotide in the helper strand, typically a non-phosphorylated terminal nucleotide. This prevents ligation of the helper strand portion of the synthesis strand to the primer strand portion of the synthesis strand and thus creates a single-strand break in the synthesis strand following ligation. Alternative means for preventing ligation in the synthesis strands could be employed. For example blocking moieties could be attached to the terminal nucleotide in the helper strand. Moreover, the helper stand may be removed from the scaffold molecule, e.g. by denaturation, prior to cleavage, as described further herein. The complementary ligation end of the polynucleotide ligation molecule is provided with a ligatable terminal nucleotide in the support strand adjacent the non-ligatable terminal nucleotide in the helper strand. The ligatable terminal nucleotide of the support strand is the first nucleotide of the predefined sequence to be incorporated into the scaffold molecule by the action of a ligase enzyme. The complementary ligation end of the polynucleotide ligation molecule is also provided with a universal nucleotide in the support strand. The exact positioning of the universal nucleotide in the support strand relative to the ligatable terminal nucleotide of the support strand will depend upon the specific reaction chemistry employed as will be apparent from the descriptions of the specific method versions and variants thereof.

The appropriate structure of a polynucleotide ligation molecule can readily be ascertained by reference to the exemplary methods described herein and depictions of the same in the Figures.

### Ligation

In methods of the invention which involve a ligation step, ligation may be achieved using any suitable means. Preferably, the ligation step will be performed by a ligase enzyme. The ligase may be a modified ligase with enhanced activity for single-base overhang substrates. The ligase may be a T3 DNA ligase or a T4 DNA ligase. The ligase may a blunt TA ligase. For example a blunt TA ligase is available from New England BioLabs (NEB). This is a ready-to-use master mix solution of T4 DNA Ligase, ligation enhancer, and optimized reaction buffer specifically formulated to improve ligation and transformation of both blunt-end and single-base overhang substrates. Molecules, enzymes, chemicals and methods for ligating (joining) single- and double-stranded polynucleotides are well known to the skilled person.

### Cleavage of scaffold polynucleotide

In methods requiring the presence of scaffold polynucleotides and steps of cleavage, the selection of the reagent to perform the cleavage step will depend upon the particular method employed. The cleavage site is defined by the specific position of the universal nucleotide in the support strand. Configuration of the desired cleavage site and selection of the appropriate cleavage reagent will therefore depend upon the specific chemistry employed in the method, as will readily be apparent by reference to the exemplary methods described herein.

Some examples of DNA cleaving enzymes recognizing modified bases are shown in the Table below.

| **DNA glycosylase/ Endonuclease** | **Main substrate** | **Cleavage site** | **Termini created from the cleavage** | |
|---|---|---|---|---|
| | | | **5'-end** | **3'-end** |
| APE1 | AP site | 1^{st} phosphodiester bond 5' to the lesion | Deoxyribose-5'-phosphate | OH |
| Endonuclease III | AP site, thymine glycol | 1^{st} phosphodiester bond 3' to the lesion | phosphate | 3'-phospho-α, β-unsaturated aldehyde |
| Endonuclease IV | AP site | 1^{st} phosphodiester bond 5' to the lesion | Deoxyribose-5'-phosphate | OH |
| Endonuclease V | Inosine | 2^{nd} phosphodiester bond 3' to the lesion | phosphate | OH |
| Endonuclease VIII | AP site, thymine glycol | 1^{st} phosphodiester bond 5' and 3' to the lesion | phosphate | phosphate |
| FpG | 8-oxoguanine | 1^{st} phosphodiester bond 5' and 3' to the lesion | phosphate | phosphate |
| hOGG1 | 8-oxoguanine | 1^{st} phosphodiester bond 3' to the lesion | phosphate | 3 '-phospho-α, β-unsaturated aldehyde |
| hNeil 1 | Oxidized purines | 1^{st} phosphodiester bond 5' and 3' to the lesion | phosphate | phosphate |
| ROS1 | 5-methylcytosine | 1^{st} phosphodiester bond 5' and 3' to the lesion | phosphate | phosphate |
| Uracil DNA glycosylase | Uracil | *N*-glycosidic bond | AP site (no break) | |
| hSMUG | Uracil | *N*-glycosidic bond | AP site (no break) | |
| hAAG | Inosine | *N*-glycosidic bond | AP site (no break) | |

### Synthesis strand

In methods of synthesising a polynucleotide or oligonucleotide described herein including, but not limited to, synthesis method versions 1 to 4 of the invention and variants thereof as described in Figures 1 to 10 and further herein, the scaffold polynucleotide is provided with a synthesis strand. The synthesis strand comprises a primer strand portion. During cycles of synthesis each new second nucleotide of the predefined sequence is incorporated into the synthesis strand by extension of the primer strand portion, the first nucleotide of the predefined sequence being incorporated into the support strand. An enzyme, such as a polymerase enzyme or enzyme having terminal transferase activity, can be used to catalyse incorporation/addition of each new second nucleotide. Each newly-incorporated second nucleotide of the predefined sequence will act as the terminal nucleotide of the primer strand portion for use in priming incorporation in the next incorporation step. Thus in any given cycle of synthesis the primer strand portion of the synthesis strand will comprise sufficient polynucleotide sequence to allow priming by the appropriate enzyme. In certain embodiments, described further herein, in a given cycle of synthesis a second nucleotide of the predefined sequence is incorporated into the synthesis strand followed by incorporation into the synthesis strand of one or more further nucleotides. In such embodiments the second nucleotide of the predefined sequence and further nucleotides comprise a reversible terminator group and the methods additionally comprise steps of removing the reversible terminator group from the nucleotide following incorporation and prior to incorporation of the next nucleotide.

The terms "incorporation", "extension" and "addition" of a nucleotide are intended to have the same meaning herein.

### Helper strand

A helper strand may be provided in the polynucleotide ligation molecule to facilitate ligation of the polynucleotide ligation molecule to the scaffold polynucleotide at the ligation step. A helper strand may also facilitate binding of cleavage enzyme(s) at the cleavage step. The helper strand may be omitted, provided that alternative means are provided to ensure binding of cleavage enzyme(s) at the cleavage step and to ensure ligation at the ligation step, if necessary. In preferred methods of the invention the polynucleotide ligation molecule is provided with a helper strand.

There are no special requirements for the parameters of length, sequence and structure of the helper strand, provided that the helper strand is suitable to facilitate binding of ligase and cleavage enzyme(s) as necessary.

The helper strand may comprise nucleotides, nucleotide analogues/derivatives and/or non-nucleotides.

Preferably, within the region of sequence of the helper strand mismatches with the support strand should be avoided, GC- and AT-rich regions should be avoided, and in addition regions of secondary structure such as hairpins or bulges should be avoided.

The length of the helper strand may be 10 bases or more. Optionally, the length of the helper strand may be 15 bases or more, preferably 30 bases or more. However, the length of the helper strand may be varied, provided that the helper strand is capable of facilitating cleavage and/or ligation.

The helper strand must be hybridized to the corresponding region of the support strand. It is not essential that the entirety of the helper strand is hybridized to the corresponding region of the support strand, provided that the helper strand can facilitate binding of ligase enzyme at the ligation step and/or binding of cleavage enzyme(s) at the cleavage step. Thus, mismatches between the helper strand and the corresponding region of the support strand can be tolerated. The helper strand may be longer than the corresponding region of the support strand. The support strand may extend beyond the region which corresponds with the helper strand in the direction distal to the primer strand. The helper strand may be connected to the corresponding region of the support strand, *e.g.* via a hairpin.

The helper strand may be hybridized to the support strand such that the terminal nucleotide of the helper strand at the site of the nick occupies the next sequential nucleotide position in the synthesis strand relative to the terminal nucleotide of the primer strand portion at the site of the nick. Thus in this configuration there are no nucleotide position gaps between the helper strand and the primer strand. The helper strand and primer strand will nevertheless be physically separated due to the presence of the single-stranded break or nick.

The nucleotide in the helper strand which pairs with the universal nucleotide may be any suitable nucleotide. Preferably, pairings which are likely to distort the helical structure of the molecule should be avoided. Preferably cytosine acts as a partner for the universal nucleotide. In a particularly preferred embodiment the universal nucleotide is inosine, or an analogue, variant or derivative thereof, and the partner nucleotide for the universal nucleotide in the helper strand is cytosine.

### Removal of helper strand

In any of the synthesis methods of the invention described herein, prior to the step of incorporation of the second nucleotide of the predefined sequence the helper strand provided by the polynucleotide ligation molecule may be removed from the scaffold polynucleotide.

The helper strand portion of the synthesis strand may be removed from the scaffold polynucleotide by any suitable means including, but not limited to: (i) heating the scaffold polynucleotide to a temperature of about 80°C to about 95°C and separating the helper strand portion from the scaffold polynucleotide, (ii) treating the scaffold polynucleotide with urea solution, such as 8M urea and separating the helper strand portion from the scaffold polynucleotide, (iii) treating the scaffold polynucleotide with formamide or formamide solution, such as 100% formamide and separating the helper strand portion from the scaffold polynucleotide, or (iv) contacting the scaffold polynucleotide with a single-stranded polynucleotide molecule which comprises a region of nucleotide sequence which is complementary with the sequence of the helper strand portion, thereby competitively inhibiting the hybridisation of the helper strand portion to the scaffold polynucleotide.

In methods wherein the helper strand portion is removed from the scaffold polynucleotide after the step of ligating the double-stranded polynucleotide ligation molecule to the cleaved scaffold polynucleotide and before the step of cleavage of the scaffold polynucleotide, the cleavage step will comprise cleaving the support strand in the absence of a double-stranded region provided by the helper strand. Any suitable enzyme may be chosen for performing such a cleavage step, such as selected from any suitable enzyme disclosed herein.

### Primer strand

The primer strand portion should be suitable to allow an enzyme, such as a polymerase enzyme or enzyme having terminal transferase activity, to initiate synthesis, i.e. catalyse the addition of a new nucleotide at the terminal end of the primer strand portion.

The primer strand may comprise a region of sequence which can act to prime new polynucleotide synthesis (e.g. as shown by the dotted line in the structures depicted in each of Figures 1 to 10). The primer strand may consist of a region of sequence which can act to prime new polynucleotide synthesis, thus the entirety of the primer strand may be sequence which can act to prime new polynucleotide synthesis as described herein.

There are no special requirements for the parameters of length, sequence and structure of the primer strand, provided that the primer strand is suitable to prime new polynucleotide synthesis.

The primer strand may comprise nucleotides, nucleotide analogues/derivatives and/or non-nucleotides.

The skilled person is readily able to construct a primer strand which will be capable of priming new polynucleotide synthesis. Thus, within the region of sequence of the primer strand which can act to prime new polynucleotide synthesis mismatches with the support strand should be avoided, GC- and AT-rich regions should be avoided, and in addition regions of secondary structure such as hairpins or bulges should be avoided.

The length of the region of sequence of the primer strand which can act to prime new polynucleotide synthesis can be chosen by the skilled person depending on preference and the polymerase enzyme to be used. The length of this region may be 7 bases or more, 8 bases or more, 9 bases or more or 10 bases or more. Optionally the length of this region will be 15 bases or more, preferably 30 bases or more.

The primer strand must be hybridized to the corresponding region of the support strand. It is not essential that the entirety of the primer strand is hybridized to the corresponding region of the support strand, provided that the primer strand is capable of priming new polynucleotide synthesis. Thus, mismatches between the primer strand and the corresponding region of the support strand can be tolerated to a degree. Preferably, the region of sequence of the primer strand which can act to prime new polynucleotide synthesis should comprise nucleobases which are complementary to corresponding nucleobases in the support strand.

The primer strand may be connected to the corresponding region of the support strand, *e.g*. via a hairpin.

### Support strand

In methods of the invention including, but not limited to, synthesis method versions of the invention 1 and 2 and variants thereof as described above, the scaffold polynucleotide is provided with a support strand. The support strand is hybridized to the synthesis strand. There are no special requirements for the parameters of length, sequence and structure of the support strand, provided that the support strand is compatible with the primer strand portion as described herein and, if present, the helper strand portion of the synthesis strand, as described above.

### Synthetic polynucleotide

The polynucleotide having a predefined sequence synthesised according to the methods described herein is double-stranded. The synthesised polynucleotide overall is double-stranded and wherein the first strand is attached to the second strand by hybridization. Mismatches and regions of non-hybridization may be tolerated, provided that overall the first strand is attached to the second strand by hybridization.

Hybridisation may be defined by moderately stringent or stringent hybridisation conditions. A moderately stringent hybridisation condition uses a prewashing solution containing 5x sodium chloride/sodium citrate (SSC), 0.5% SDS, 1.0 mM EDTA (pH 8.0), hybridisation buffer of about 50% formamide, 6xSSC, and a hybridisation temperature of 55° C (or other similar hybridisation solutions, such as one containing about 50% formamide, with a hybridisation temperature of 42° C), and washing conditions of 60° C, in 0.5xSSC, 0.1% SDS. A stringent hybridisation condition hybridises in 6xSSC at 45° C, followed by one or more washes in 0.1xSSC, 0.2% SDS at 68° C.

The double-stranded polynucleotide having a predefined sequence synthesised according to the methods described herein may be retained as a double-stranded polynucleotide. Alternatively the two strands of the double-stranded polynucleotide may be separated to provide a single-stranded polynucleotide having a predefined sequence. Conditions that permit separation of two strands of a double-stranded polynucleotide (melting) are well-known in the art (for example, Sambrook et al., 2001, Molecular Cloning: a laboratory manual, 3rd edition, Cold Spring Harbour Laboratory Press; and Current Protocols in Molecular Biology, Greene Publishing and Wiley-lnterscience, New York (1995)).

The double-stranded polynucleotide having a predefined sequence synthesised according to the methods described herein may be amplified following synthesis. Any region of the double-stranded polynucleotide may be amplified. The whole or any region of the double-stranded polynucleotide may be amplified together with the whole or any region of the scaffold polynucleotide. Conditions that permit amplification of a double-stranded polynucleotide are well-known in the art (for example, Sambrook et al., 2001, Molecular Cloning: a laboratory manual, 3rd edition, Cold Spring Harbour Laboratory Press; and Current Protocols in Molecular Biology, Greene Publishing and Wiley-lnterscience, New York (1995)). Thus any of the synthesis methods described herein may further comprise an amplification step wherein the double-stranded polynucleotide having a predefined sequence, or any region thereof, is amplified as described above. Amplification may be performed by any suitable method, such as polymerase chain reaction (PCR), polymerase spiral reaction (PSR), loop mediated isothermal amplification (LAMP), nucleic acid sequence based amplification (NASBA), self-sustained sequence replication (3SR), rolling circle amplification (RCA), strand displacement amplification (SDA), multiple displacement amplification (MDA), ligase chain reaction (LCR), helicase dependant amplification (HDA), ramification amplification method (RAM) etc. Preferably, amplification is performed by polymerase chain reaction (PCR).

The double-stranded or single-stranded polynucleotide having a predefined sequence synthesised according to the methods described herein can be any length. For example, the polynucleotides can be at least 10, at least 50, at least 100, at least 150, at least 200, at least 250, at least 300, at least 350, at least 400, at least 450 or at least 500 nucleotides or nucleotide pairs in length. For example, the polynucleotides may be from about 10 to about 100 nucleotides or nucleotide pairs, about 10 to about 200 nucleotides or nucleotide pairs, about 10 to about 300 nucleotides or nucleotide pairs, about 10 to about 400 nucleotides or nucleotide pairs and about 10 to about 500 nucleotides or nucleotide pairs in length. The polynucleotides can be up to about 1000 or more nucleotides or nucleotide pairs, up to about 5000 or more nucleotides or nucleotide pairs in length or up to about 100000 or more nucleotides or nucleotide pairs in length.

### RNA synthesis

Methods described for DNA synthesis may be adapted for the synthesis of RNA. In one adaptation the synthesis steps described for synthesis method versions of the invention 1 to 4 and variants thereof may be adapted. Thus in each of synthesis method versions 1 to 4 and variants thereof the support strand of the scaffold polynucleotide is a DNA strand, as described above. The primer strand portion of the synthesis strand of the scaffold polynucleotide is an RNA strand. The helper strand, if present, may be an RNA strand. The helper strand, if present, may be a DNA strand.

Nucleotides may be incorporated from ribonucleoside-5'-*O*-triphosphates (NTPs) which may be modified to comprise a reversible terminator group, as described above. Preferably 3'*-*O-modified-ribonucleoside-5'-*O*-triphosphates are used. Modified nucleotides are incorporated by the action of RNA polymerase.

Thus the descriptions relating to synthesis method versions of the invention 1 to 4 and variants thereof may be applied *mutatis mutandis* for RNA synthesis but adapted as described.

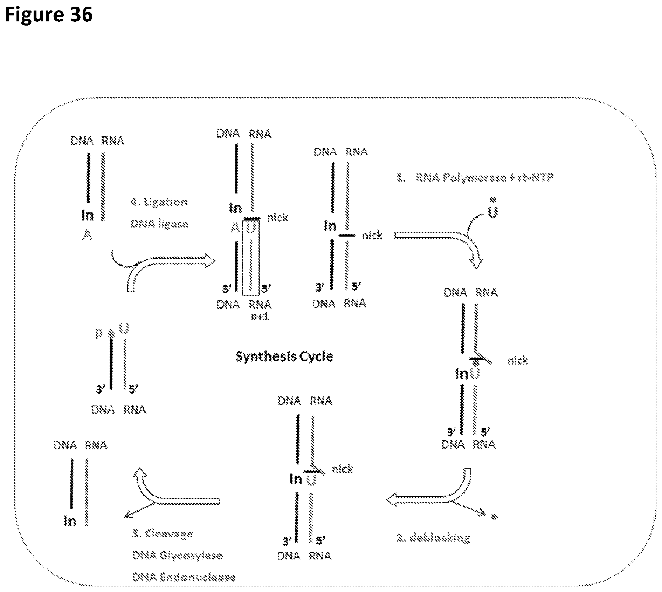

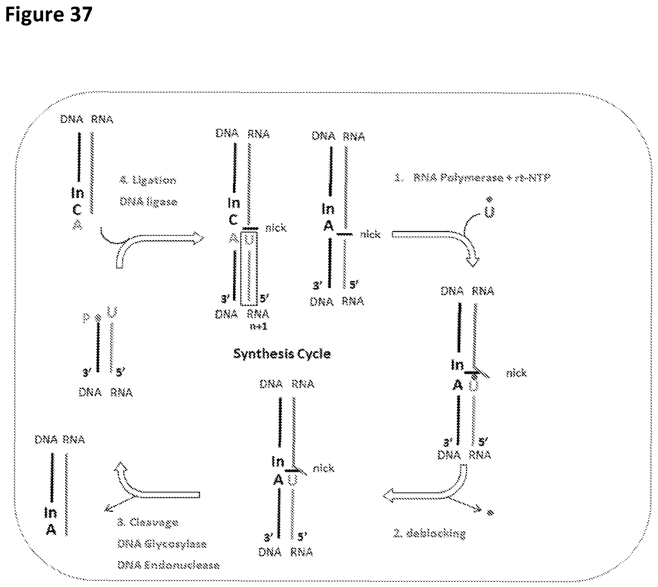

Figures 36 and 37 describe reaction schemes for RNA synthesis which are adaptations of DNA synthesis method versions 1 and 2 of the Examples as depicted in Figures 11 and 12 respectively. Method versions of the invention 1 to 4 and variants thereof, as depicted in Figures 1 to 10 may be adapted in the same way.

In any of the adapted methods for RNA synthesis, the above descriptions of support strand, primer strand, helper strand, polynucleotide ligation molecule and universal nucleotide may be applied *mutatis mutandis* but adapted as described. Cleavage steps and cleavage positions as previously described may be applied *mutatis mutandis* since the support strand which comprises the universal nucleotide is a DNA strand. In a preferred embodiment SplintR DNA ligase is used in the ligation step.

### Solid phase synthesis

Synthetic polynucleotides produced in accordance with the synthesis methods of the invention may preferably be synthesised using solid phase or reversible solid phase techniques. A variety of such techniques is known in the art and may be used. Before initiating synthesis of a new double-stranded polynucleotide of predefined sequence, scaffold polynucleotides may be immobilized to a surface *e.g.* a planar surface such as glass, a gel-based material, or the surface of a microparticle such as a bead or functionalised quantum dot. The material comprising the surface may itself be bound to a substrate. For example, scaffold polynucleotides may be immobilized to a gel-based material such as e.g. polyacrylamide, and wherein the a gel-based material is bound to a supporting substrate such as glass.

Polynucleotides may be immobilized or tethered to surfaces directly or indirectly. For example they may be attached directly to surfaces by chemical bonding. They may be indirectly tethered to surfaces via an intermediate surface, such as the surface of a microparticle or bead *e.g.* as in SPRI or as in electrowetting systems, as described below. Cycles of synthesis may then be initiated and completed whilst the scaffold polynucleotide incorporating the newly-synthesised polynucleotide is immobilized.

In such methods a double-stranded scaffold polynucleotide may be immobilized to a surface prior to the incorporation of the first nucleotide of the predefined sequence. Such an immobilized double-stranded scaffold polynucleotide may therefore act as an anchor to tether the double-stranded polynucleotide of the predefined sequence to the surface during and after synthesis.

Only one strand of such a double-stranded anchor/scaffold polynucleotide may be immobilized to the surface at the same end of the molecule. Alternatively both strands of a double-stranded anchor/scaffold polynucleotide may each be immobilized to the surface at the same end of the molecule. A double-stranded anchor/scaffold polynucleotide may be provided with each strand connected at adjacent ends, such as via a hairpin loop at the opposite end to the site of initiation of new synthesis, and connected ends may be immobilized on a surface (for example as depicted schematically in Figure 16).

**In** methods involving a scaffold polynucleotide, as described herein, the scaffold polynucleotide may be attached to a surface prior to the incorporation of the first nucleotide in the predefined sequence. Thus the synthesis strand comprising the primer strand portion and the portion of the support strand hybridized thereto may both be separately attached to a surface, as depicted in Figure 16(a) and (c). The synthesis strand comprising the primer strand portion and the portion of the support strand hybridized thereto may be connected at adjacent ends, such as via a hairpin loop, *e.g.* at the opposite end to the site of initiation of new synthesis, and connected ends may be tethered to a surface, as depicted in Figure 16(b) and (d). One or other of the synthesis strand comprising the primer strand portion and the portion of the support strand hybridized thereto may be attached separately to a surface, as depicted in Figure 16(e) to (h). Preferably the synthesis strand comprising the primer strand portion and the portion of the support strand hybridized thereto is attached to a surface.

### Solid phase synthesis on planar surfaces

Before initiating synthesis of a new double-stranded polynucleotide of predefined sequence synthetic anchor/scaffold polynucleotides can be synthesised by methods known in the art, including those described herein, and tethered to a surface.

Pre-formed polynucleotides can be tethered to surfaces by methods commonly employed to create nucleic acid microarrays attached to planar surfaces. For example, anchor/scaffold polynucleotides may be created and then spotted or printed onto a planar surface. Anchor/scaffold polynucleotides may be deposited onto surfaces using contact printing techniques. For example, solid or hollow tips or pins may be dipped into solutions comprising pre-formed scaffold polynucleotides and contacted with the planar surface. Alternatively, oligonucleotides may be adsorbed onto micro-stamps and then transferred to a planar surface by physical contact. Non-contact printing techniques include thermic printing or piezoelectric printing wherein sub-nanolitre size microdroplets comprising pre-formed scaffold polynucleotides may be ejected from a printing tip using methods similar to those used in inkjet and bubblejet printing.

Single-stranded oligonucleotides may be synthesised directly on planar surfaces such as using so-called "on-chip" methods employed to create microarrays. Such single-stranded oligonucleotides may then act as attachment sites to immobilize pre-formed anchor/scaffold polynucleotides.

On-chip techniques for generating single-stranded oligonucleotides include photolithography which involves the use of UV light directed through a photolithographic mask to selectively activate a protected nucleotide allowing for the subsequent incorporation of a new protected nucleotide. Cycles of UV-mediated deprotection and coupling of pre-determined nucleotides allows the in situ generation of an oligonucleotide having a desired sequence. As an alternative to the use of a photolithographic mask, oligonucleotides may be created on planar surfaces by the sequential deposition of nucleobases using inkjet printing technology and the use of cycles of coupling, oxidation and deprotection to generate an oligonucleotide having a desired sequence (for a review see Kosuri and Church, Nature Methods, 2014, 11, 499-507).

In any of the synthesis methods described herein, including methods involving reversible immobilisation as described below, surfaces can be made of any suitable material. Typically a surface may comprise silicon, glass or polymeric material. A surface may comprise a gel surface, such as a polyacrylamide surface, such as about 2% polyacrylamide, optionally a polyacrylamide surface derived using N- (5-bromoacetamidylpentyl) acrylamide (BRAPA), preferably the polyacrylamide surface is coupled to a solid support, such as glass.

### Reversible immobilization

Synthetic polynucleotides having a predefined sequence can be synthesised in accordance with the invention using binding surfaces and structures, such as microparticles and beads, which facilitate reversible immobilization. Solid phase reversible immobilization (SPRI) methods or modified methods are known in the art and may be employed (e.g. see DeAngelis M. M. et al. (1995) Solid-Phase Reversible Immobilization for the Isolation of PCR Products, Nucleic Acids Research, 23(22): 4742-4743.).

Surfaces can be provided in the form of microparticles, such as paramagnetic beads. Paramagnetic beads can agglomerate under the influence of a magnetic field. For example, paramagnetic surfaces can be provided with chemical groups, e.g. carboxyl groups, which in appropriate attachment conditions will act as binding moieties for nucleic acids, as described in more detail below. Nucleic acids can be eluted from such surfaces in appropriate elution conditions. Surfaces of microparticles and beads can be provided with UV-sensitive polycarbonate. Nucleic acids can be bound to the activated surface in the presence of a suitable immobilization buffer.

Microparticles and beads may be allowed to move freely within a reaction solution and then reversibly immobilized, e.g. by holding the bead within a microwell or pit etched into a surface. A bead can be localised as part of an array e.g. by the use of a unique nucleic acid "barcode" attached to the bead or by the use of colour-coding.

Thus before initiating synthesis of a new double-stranded polynucleotide of predefined sequence, anchor/scaffold polynucleotides in accordance with the invention can be synthesised and then reversibly immobilized to such binding surfaces. Polynucleotides synthesised by methods of the invention can be synthesised whilst reversibly immobilized to such binding surfaces.

### Microfluidic techniques and systems

The surface may be part of an electrowetting-on-dielectric system (EWOD). EWOD systems provide a dielectric-coated surface which facilitates microfluidic manipulation of very small liquid volumes in the form of microdroplets (e.g. see Chou, W-L., et al. (2015) Recent Advances in Applications of Droplet Microfluidics, Micromachines, 6: 1249-1271.). Droplet volumes can programmably be created, moved, partitioned and combined on-chip by electrowetting techniques. Thus electrowetting systems provide alternative means to reversibly immobilize polynucleotides during and after synthesis.

Polynucleotides having a predefined sequence may be synthesised in solid phase by methods described herein, wherein polynucleotides are immobilized on an EWOD surface and required steps in each cycle facilitated by electrowetting techniques. For example, in methods involving scaffold polynucleotides and requiring incorporation, cleavage, ligation and deprotection steps, reagents required for each step, as well as for any required washing steps to remove used and unwanted reagent, can be provided in the form of microdroplets transported under the influence of an electric field via electrowetting techniques.

Other microfluidic platforms are available which may be used in the synthesis methods of the invention. For example, the emulsion-based microdroplet techniques which are commonly employed for nucleic acid manipulation can be used. In such systems microdroplets are formed in an emulsion created by the mixing of two immiscible fluids, typically water and an oil. Emulsion microdroplets can be programmably be created, moved, partitioned and combined in microfluidic networks. Hydrogel systems are also available. In any of the synthesis methods described herein microdroplets may be manipulated in any suitable compatible system, such as EWOD systems described above and other microfluidic systems, e.g. microfluidic systems comprising architectures based on components comprising elastomeric materials.

Microdroplets may be of any suitable size, provided that they are compatible with the synthesis methods herein. Microdroplet sizes will vary depending upon the particular system employed and the relevant architecture of the system. Sizes may thus be adapted as appropriate. In any of the synthesis methods described herein droplet diameters may be in the range from about 150nm to about 5mm. Droplet diameters below 1µm may be verified by means known in the art, such as by techniques involving capillary jet methods, e.g. as described in Gañán-Calvo et al. (Nature Physics, 2007, 3, pp737-742)

### Sequencing of intermediate or final synthesis products.

The intermediate products of synthesis or assembly, or the final polynucleotide synthesis products may be sequenced as a quality control check to determine whether the desired polynucleotide or polynucleotides have been correctly synthesised or assembled. The polynucleotide or polynucleotides of interest can be removed from the solid phase synthesis platform and sequenced by any one of a number of known commercially available sequencing techniques such as nanopore sequencing using a MinION^{™} device sold by Oxford Nanopore Technologies Ltd. In a particular example, the sequencing may be carried out on the solid phase platform itself, removing the need to transfer the polynucleotide to a separate synthesis device. Sequencing may be conveniently carried out on the same electrowetting device, such as an EWOD device as used for synthesis whereby the synthesis device comprises one or more measurement electrode pairs. A droplet comprising the polynucleotide of interest can be contacted with one of the electrodes of the electrode pair, the droplet forming a droplet interface bilayer with a second droplet in contact with the second electrode of the electrode pair wherein the droplet bilayer interface comprises a nanopore in an amphipathic membrane. The polynucleotide can be caused to translocate the nanopore for example under enzyme control and ion current flow through the nanopore can be measured under a potential difference between the electrode pair during passage of the polynucleotide through the nanopore. The ion current measurements over time can be recorded and used to determine the polynucleotide sequence. Prior to sequencing, the polynucleotide may be subjected to one or more sample preparation steps in order to optimise it for sequencing such as disclosed in patent application no. PCT/GB2015/050140. Examples of enzymes, amphipathic membranes and nanopores which may be suitably employed are disclosed in patent application nos. PCT/GB2013/052767 and PCT/GB2014/052736. The necessary reagents for sample preparation of the polynucleotide, nanopores, amphipathic membranes and so on may be supplied to the EWOD device via sample inlet ports. The sample inlet ports may be connected to reagent chambers.

### Surface attachment chemistries

Although oligonucleotides will typically be attached chemically, they may also be attached to surfaces by indirect means such as via affinity interactions. For example, oligonucleotides may be functionalised with biotin and bound to surfaces coated with avidin or streptavidin.

For the immobilization of polynucleotides to surfaces (e.g. planar surfaces), microparticles and beads etc., a variety of surface attachment methods and chemistries are available. Surfaces may be functionalised or derivatized to facilitate attachment. Such functionalisations are known in the art. For example, a surface may be functionalised with a polyhistidine-tag (hexa histidine-tag, 6xHis-tag, His6 tag or His-tag^{®}), Ni-NTA, streptavidin, biotin, an oligonucleotide, a polynucleotide (such as DNA, RNA, PNA, GNA, TNA or LNA), carboxyl groups, quaternary amine groups, thiol groups, azide groups, alkyne groups, DIBO, lipid, FLAG-tag (FLAG octapeptide), polynucleotide binding proteins, peptides, proteins, antibodies or antibody fragments. The surface may be functionalised with a molecule or group which specifically binds to the anchor/scaffold polynucleotide.

Some examples of chemistries suitable for attaching polynucleotides to surfaces are shown in Figure 16i and Figure 16j.

In any of the methods described herein the scaffold polynucleotide comprising the synthesis strand comprising the primer strand portion and the portion of the support strand hybridized thereto may be tethered to a common surface via one or more covalent bonds. The one or more covalent bonds may be formed between a functional group on the common surface and a functional group on the scaffold molecule. The functional group on the scaffold molecule may be *e.g.* an amine group, a thiol group, a thiophosphate group or a thioamide group. The functional group on the common surface may be a bromoacetyl group, optionally wherein the bromoacetyl group is provided on a polyacrylamide surface derived using N- (5- bromoacetamidylpentyl) acrylamide (BRAPA).

In any of the methods of the invention a scaffold polynucleotide may be attached to a surface, either directly or indirectly, via a linker. Any suitable linker which is biocompatible and hydrophilic in nature may be used.

A linker may be a linear linker or a branched linker.

A linker may comprise a hydrocarbon chain. A hydrocarbon chain may comprise from 2 to about 2000 or more carbon atoms. The hydrocarbon chain may comprise an alkylene group, *e.g.* C2 to about 2000 or more alkylene groups. The hydrocarbon chain may have a general formula of -(CH₂)ₙ- wherein n is from 2 to about 2000 or more. The hydrocarbon chain may be optionally interrupted by one or more ester groups (i.e. -C(O)-O-) or one or more amide groups (i.e. -C(O)-N(H)-).

Any linker may be used selected from the group comprising PEG, polyacrylamide, poly(2-hydroxyethyl methacrylate), Poly-2-methyl-2-oxazoline (PMOXA), zwitterionic polymers, e.g. poly(carboxybetaine methacrylate) (PCBMA), poly[ N -(3-sulfopropyl)- N - methacryloxyethyl- N , N dimethyl ammonium betaine] (PSBMA), glycopolymers, and polypeptides.

A linker may comprise a polyethylene glycol (PEG) having a general formula of -(CH₂-CH₂-O)n-, wherein n is from 1 to about 600 or more.

A linker may comprise oligoethylene glycol-phosphate units having a general formula of -[(CH₂-CH₂-O)ₙ-PO₂⁻-O]ₘ- where n is from 1 to about 600 or more and m could be 1-200 or more.

Any of the above-described linkers may be attached at one end of the linker to a scaffold molecule as described herein, and at the other end of the linker to a first functional group wherein the first functional group may provide a covalent attachment to a surface. The first functional group may be *e.g.* an amine group, a thiol group, a thiophosphate group or a thioamide group as further described herein. The surface may be functionalised with a further functional group to provide a covalent bond with the first functional group. The further functional group may be *e.g.* a 2-bromoacetamido group as further described herein. Optionally a bromoacetyl group is provided on a polyacrylamide surface derived using N- (5- bromoacetamidylpentyl) acrylamide (BRAPA). The further functional group on the surface may be a bromoacetyl group, optionally wherein the bromoacetyl group is provided on a polyacrylamide surface derived using N- (5- bromoacetamidylpentyl) acrylamide (BRAPA) and the first functional group may be *e.g.* an amine group, a thiol group, a thiophosphate group or a thioamide group as appropriate. The surface to which polynucleotides are attached may comprise a gel. The surface comprises a polyacrylamide surface, such as about 2% polyacrylamide, preferably the polyacrylamide surface is coupled to a solid support such as glass.

In any of the methods of the invention a scaffold polynucleotide may optionally be attached to a linker via a branching nucleotide incorporated into the scaffold polynucleotide. Any suitable branching nucleotide may be used with any suitable compatible linker.

Prior to initiating synthesis cycles of the invention, scaffold polynucleotides may be synthesised with one or more branching nucleotides incorporated into the scaffold polynucleotide. The exact position at which the one or more branching nucleotides are incorporated into the scaffold polynucleotide, and thus where a linker may be attached, may vary and may be chosen as desired. The position may *e.g.* be at the terminal end of a support strand and/or a synthesis strand or *e.g.* in the loop region which connects the support strand to the synthesis strand in embodiments which comprise a hairpin loop.

During synthesis of the scaffold polynucleotide the one or more branching nucleotides may be incorporated into the scaffold polynucleotide with a blocking group which blocks a reactive group of the branching moiety. The blocking group may then be removed (deblocked) prior to the coupling to the branching moiety of the linker, or a first unit (molecule) of the linker if a linker comprises multiple units.

During synthesis of the scaffold polynucleotide the one or more branching nucleotides may be incorporated into the scaffold polynucleotide with a group suitable for use in a subsequent "click chemistry" reaction to couple to the branching moiety the linker, or a first unit of the linker if a linker comprises multiple units. An example of such a group is an acetylene group.

Some non-limiting exemplary branching nucleotides are shown below.

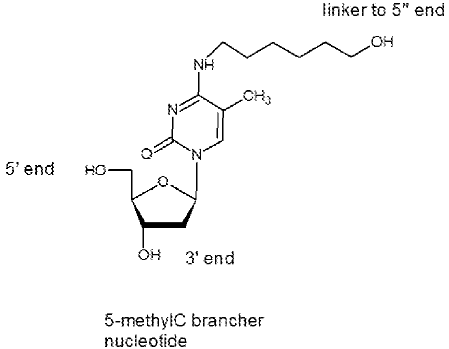

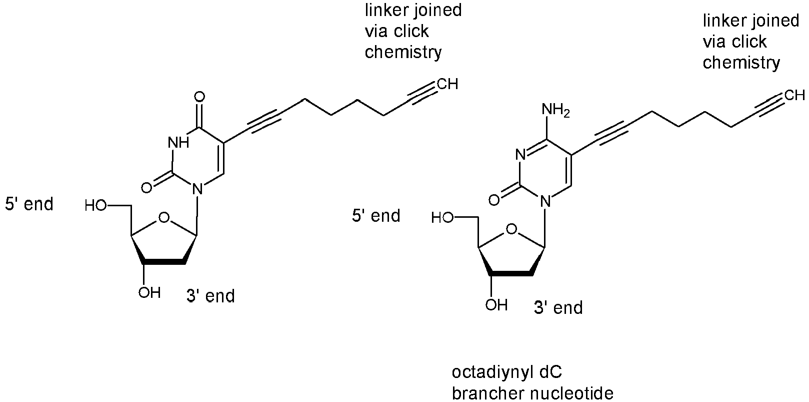

A linker may optionally comprise one or more spacer molecules (units), such as *e.g.* an Sp9 spacer, wherein the first spacer unit is attached to the branching nucleotide.

The linker may comprise one or more further spacer groups attached to the first spacer group. For example, the linker may comprise multiple *e.g.* Sp9 spacer groups. A first spacer group is attached to the branching moiety and then one or more further spacer groups are sequentially added to extend a spacer chain comprising multiple spacer units connected with phosphate groups therebetween.

Shown below are some non-limiting examples of spacer units (Sp3, Sp9 and Sp13) which could comprise the first spacer unit attached to a branching nucleotide, or a further spacer unit to be attached to an existing spacer unit already attached to the branching nucleotide.

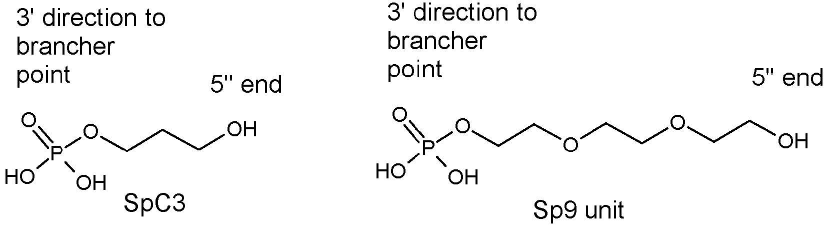

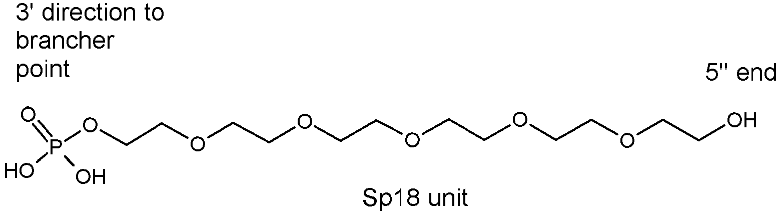

A linker may comprise one or more ethylene glycol units.

A linker may comprise an oligonucleotide, wherein multiple units are nucleotides.

In the structures depicted above the term 5" is used to differentiate from the 5' end of the nucleotide to which the branching moiety is attached, wherein 5' has its ordinary meaning in the art. By 5" it is intended to mean a position on the nucleotide from which a linker can be extended. The 5" position may vary. The 5" position is typically a position in the nucleobase of the nucleotide. The 5" position in the nucleobase may vary depending on the nature of the desired branching moiety, as depicted in the structures above.

### Microarrays

Any of the polynucleotide synthesis methods described herein may be used to manufacture a polynucleotide microarray (Trevino, V. et al., Mol. Med. 2007 13, pp527-541). Thus anchor or scaffold polynucleotides may be tethered to a plurality of individually addressable reaction sites on a surface and polynucleotides having a predefined sequence may be synthesised in situ on the microarray.

Following synthesis, at each reaction area the polynucleotide of predefined sequence may be provided with a unique sequence. The anchor or scaffold polynucleotides may be provided with barcode sequences to facilitate identification.

Other than the method of synthesising the polynucleotides of predefined sequence, microarray manufacture may be performed using techniques commonly used in this technical field, including techniques described herein. For example, anchor or scaffold polynucleotides may be tethered to surfaces using known surface attachment methods and chemistries, including those described herein.

Following synthesis of the polynucleotides of predefined sequence, there may be provided a final cleavage step to remove any unwanted polynucleotide sequence from untethered ends.

Polynucleotides of predefined sequence may be provided at reaction sites in double-stranded form. Alternatively, following synthesis double-stranded polynucleotides may be separated and one strand removed, leaving single-stranded polynucleotides at reaction sites. Selective tethering of strands may be provided to facilitate this process. For example, in methods involving a scaffold polynucleotide the synthesis strand may be tethered to a surface and the support strand may be untethered, or vice versa. The synthesis strand may be provided with a non-cleavable linker and the support strand may be provided with a cleavable linker, or vice versa. Separation of strands may be performed by conventional methods, such as heat treatment.

### Assembly of synthetic polynucleotides

A polynucleotide having a predefined sequence synthesised by methods described herein, and optionally amplified by methods described herein, may be joined to one or more other such polynucleotides to create larger synthetic polynucleotides.

Joining of multiple polynucleotides can be achieved by techniques commonly known in the art. A first polynucleotide and one or more additional polynucleotides synthesised by methods described herein may be cleaved to create compatible termini and then polynucleotides joined together by ligation. Cleavage can be achieved by any suitable means. Typically, restriction enzyme cleavage sites may be created in polynucleotides and then restriction enzymes used to perform the cleavage step, thus releasing the synthesised polynucleotides from any anchor/scaffold polynucleotide. Cleavage sites could be designed as part of the anchor/scaffold polynucleotides. Alternatively, cleavage sites could be created within the newly-synthesised polynucleotide as part of the predefined nucleotide sequence.

Assembly of polynucleotides is preferably performed using solid phase methods. For example, following synthesis a first polynucleotide may be subject to a single cleavage at a suitable position distal to the site of surface immobilization. The first polynucleotide will thus remain immobilized to the surface, and the single cleavage will generate a terminus compatible for j oining to another polynucleotide. An additional polynucleotide may be subject to cleavage at two suitable positions to generate at each terminus a compatible end for joining to other polynucleotides, and at the same time releasing the additional polynucleotide from surface immobilization. The additional polynucleotide may be compatibly joined with the first polynucleotide thus creating a larger immobilized polynucleotide having a predefined sequence and having a terminus compatible for j oining to yet another additional polynucleotide. Thus iterative cycles of j oining of preselected cleaved synthetic polynucleotides may create much longer synthetic polynucleotide molecules. The order of joining of the additional polynucleotides will be determined by the required predefined sequence.

Thus the assembly methods of the invention may allow the creation of synthetic polynucleotide molecules having lengths in the order of one or more Mb.

The assembly and/or synthesis methods of the invention may be performed using apparatuses known in the art. Techniques and apparatuses are available which allow very small volumes of reagents to be selectively moved, partitioned and combined with other volumes in different locations of an array, typically in the form of droplets Electrowetting techniques, such as electrowetting-on-dielectric (EWOD), may be employed, as described above. Suitable electrowetting techniques and systems that may be employed in the invention that are able to manipulate droplets are disclosed for example in US8653832, US8828336, US20140197028 and US20140202863.

Cleavage from the solid phase may be achieved by providing cleavable linkers in one or both the primer strand portion and the portion of the support strand hybridized thereto. The cleavable linker may be *e.g.* a UV cleavable linker.

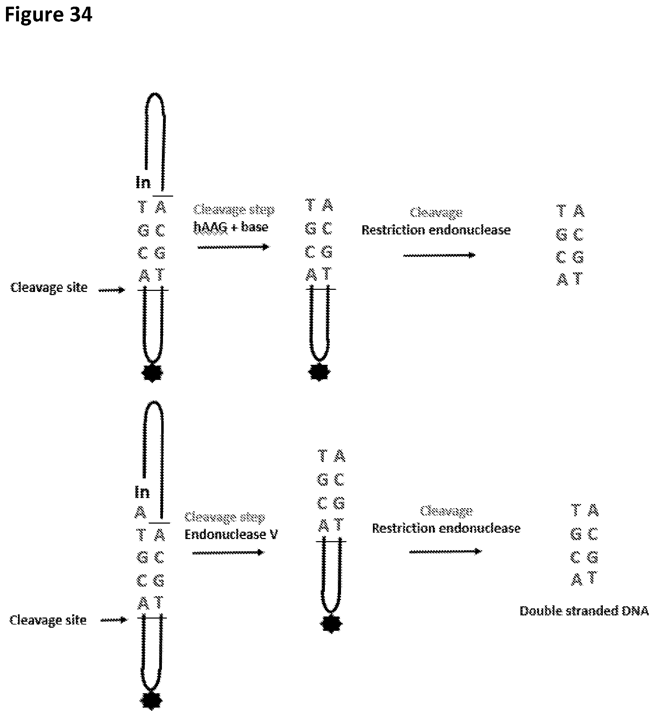

Examples of cleavage methods involving enzymatic cleavage are shown in Figure 34. The schematic shows a scaffold polynucleotide attached to a surface (via black diamond structures) and comprising a polynucleotide of predefined sequence. The scaffold polynucleotide comprises top and bottom hairpins. In each case the top hairpin can be cleaved using a cleavage step utilizing the universal nucleotide to define a cleavage site. The bottom hairpin can be removed by a restriction endonuclease via a site that is engineered into the scaffold polynucleotide or engineered into the newly-synthesised polynucleotide of predefined sequence.

Thus polynucleotides having a predefined sequence may be synthesised whilst immobilized to an electrowetting surface, as described above. Synthesised polynucleotides may be cleaved from the electrowetting surface and moved under the influence of an electric field in the form of a droplet. Droplets may be combined at specific reaction sites on the surface where they may deliver cleaved synthesised polynucleotides for ligation with other cleaved synthesised polynucleotides. Polynucleotides can then be joined, for example by ligation. Using such techniques populations of different polynucleotides may be synthesised and attached in order according to the predefined sequence desired. Using such systems a fully automated polynucleotide synthesis and assembly system may be designed. The system may be programmed to receive a desired sequence, supply reagents, perform synthesis cycles and subsequently assemble desired polynucleotides according to the predefined sequence desired.

### Systems and kits

The disclosure also provides polynucleotide synthesis systems for carrying out any of the synthesis methods described and defined herein, as well as any of the subsequent amplification and assembly steps described and defined herein.

Typically, synthesis cycle reactions will be carried out by incorporating nucleotides of predefined sequence into scaffold polynucleotide molecules which are tethered to a surface by means described and defined herein. The surface may be any suitable surface as described and defined herein.

In one aspect, reactions to incorporate nucleotides of predefined sequence into a scaffold polynucleotide molecule involve performing any of the synthesis methods on a scaffold polynucleotide within a reaction area.

A reaction area is any area of a suitable substrate to which a scaffold polynucleotide molecule is attached and wherein reagents for performing the synthesis methods may be delivered.

In one aspect a reaction area may be a single area of a surface comprising a single scaffold polynucleotide molecule wherein the single scaffold polynucleotide molecule can be addressed with reagents.

In another aspect a reaction area may be a single area of a surface comprising multiple scaffold polynucleotide molecules, wherein the scaffold polynucleotide molecules cannot be individually addressed with reagent in isolation from each other. Thus in such an embodiment the multiple scaffold polynucleotide molecules in the reaction area are exposed to the same reagents and conditions and may thus give rise to synthetic polynucleotide molecules having the same or substantially the same nucleotide sequence.

In one aspect a synthesis system for carrying out any of the synthesis methods described and defined herein may comprise multiple reaction areas, wherein each reaction area comprises one or more attached scaffold polynucleotide molecules and wherein each reaction area may be individually addressed with reagent in isolation from each of the other reaction areas. Such a system may be configured *e.g.* in the form of an array, e.g. wherein reaction areas are formed upon a substrate, typically a planar substrate.

A system having a substrate comprising a single reaction area or comprising multiple reaction areas may be comprised within e.g. an EWOD system or a microfluidic system and the systems configured to deliver reagents to the reaction site. EWOD and microfluidic systems are described in more detail herein. For example an EWOD system may be configured to deliver reagents to the reaction site(s) under electrical control. A microfluidic system, such as comprising microfabricated architecture e.g. as formed from elastomeric or similar material, may be configured to deliver reagents to the reaction site(s) under fluidic pressure and/or suction control or by mechanical means. Reagents may be delivered by any suitable means, for example via carbon nanotubes acting as conduits for reagent delivery. Any suitable system may be envisaged.

EWOD, microfluidic and other systems may be configured to deliver any other desired reagents to reaction sites, such as enzymes for cleaving a synthesised double-stranded polynucleotide from the scaffold polynucleotide following synthesis, and/or reagents for cleaving a linker to release an entire scaffold polynucleotide from the substrate and/or reagents for amplifying a polynucleotide molecule following synthesis or any region or portion thereof, and/or reagents for assembling larger polynucleotide molecules from smaller polynucleotide molecules which have been synthesised according to the synthesis methods of the invention.

The disclosure also provides kits for carrying out any of the synthesis methods described and defined herein. A kit may contain any desired combination of reagents for performing any of the synthesis and/or assembly methods of the invention described and defined herein. For example, a kit may comprise any one or more volume(s) of reaction reagents comprising scaffold polynucleotides, volume(s) of reaction reagents corresponding to any one or more steps of the synthesis cycles described and defined herein, volume(s) of reaction reagents comprising nucleotides comprising reversible blocking groups or reversible terminator groups, volume(s) of reaction reagents for amplifying one or more polynucleotide molecules following synthesis or any region or portion thereof, volume(s) of reaction reagents for assembling larger polynucleotide molecules from smaller polynucleotide molecules which have been synthesised according to the synthesis methods of the invention, volume(s) of reaction reagents for cleaving a synthesised double-stranded polynucleotide from the scaffold polynucleotide following synthesis, and volume(s) of reaction reagents for cleaving one or more linkers to release entire scaffold polynucleotides from a substrate.

### Exemplary methods

Exemplary non-limiting methods of synthesising a polynucleotide according to the invention defined in the appended claims.

In the following four exemplary non-limiting methods of synthesising a polynucleotide or an oligonucleotide molecule according to the invention and variants thereof, references to synthesis method versions 1, 2, 3 and 4 are to be interpreted according to the reaction schematics set out respectively in Figures 1 to 10, and not according to the reaction schematics set out in any of Figures 11 to 15 or descriptions of the same in the Examples section. The reaction schematics set out in any of Figures 11 to 15 and descriptions of the same in the Examples section below provide illustrative support for the methods of the invention based on reaction schemes which are modified in comparison with the methods of the present invention.

In each exemplary method described below, the structures described in each step may be referred to by reference to specific figures with the aid of reference signs as appropriate. The reference signs in the text below correspond with those in Figures 1 to 10. Such references signs are not intended to be limited to the specific structures shown in the figures, and the description of the relevant structures correspond to the description thereof as provided herein in its entirety, including but not limited to those specifically illustrated.

Four non-limiting exemplary methods of the invention, referred to herein as method versions of the invention 1, 2, 3 and 4, are described below (see *e.g.* Figures 1, 2, 4 and 5 respectively).

In step (1) of each of these exemplary methods a scaffold polynucleotide (see structure depicted in step 1 of each of Figures 1, 2, 4 and 5) is provided (101, 201, 301, 401) comprising a synthesis strand (see strand labelled "b" in the structure depicted in step 1 of each of Figures 1, 2, 4 and 5) hybridized to a complementary support strand (see strand labelled "a" in the structure depicted in step 1 of each of Figures 1, 2, 4 and 5).

The scaffold polynucleotide is double-stranded and provides a support structure to accommodate the region of synthetic polynucleotide as it is synthesised *de novo.* The scaffold polynucleotide comprises a synthesis strand comprising a primer strand portion (see dotted portion of the strand labelled "b" in the structure depicted in step 1 of each of Figures 1, 2, 4 and 5) and a helper strand portion (see dashed portion of the strand labelled "b" in the structure depicted in step 1 of each of Figures 1 to 4). Both the primer strand portion and the helper strand portion of the synthesis strand are provided hybridised to a complementary support strand.

In method versions 1 and 2 the terminal end of the scaffold polynucleotide comprising the primer strand portion comprises a blunt end, *i.e.* with no overhanging nucleotides in either strand. In method versions 3 and 4 the terminal end of the support strand of the scaffold polynucleotide comprises an overhang, with the terminal end of the scaffold polynucleotide overhanging the primer strand portion of the synthesis strand.

In step (2) of the methods a ligation step is performed (102, 202, 402, 502) wherein a polynucleotide ligation molecule is ligated to the double-stranded scaffold polynucleotide. The polynucleotide ligation molecule comprises a first nucleotide of the predefined nucleotide sequence. The polynucleotide ligation molecule comprises a support strand and a helper strand hybridized to the support strand. In method versions 1 and 2 the polynucleotide ligation molecule comprises a blunt-ended complementary ligation end, *i.e.* with no overhanging nucleotides in either strand. In method versions 3 and 4 the polynucleotide ligation molecule comprises an overhang in the complementary ligation end, wherein the terminal end of the helper strand overhangs the terminal end of the support strand. The blunt-ended or overhanging-ended complementary ligation end is complementary with the blunt end or the overhanging end of the double-stranded scaffold polynucleotide. The support strand of the polynucleotide ligation molecule comprises a first nucleotide of the predefined nucleotide sequence at the complementary ligation end. The first nucleotide of the predefined nucleotide sequence is the terminal nucleotide of the support strand of the polynucleotide ligation molecule at the complementary ligation end. The first nucleotide of the predefined nucleotide sequence is a ligatable nucleotide and is ligated to the terminal nucleotide of the support strand of the scaffold polynucleotide. Upon ligation the first nucleotide of the predefined nucleotide sequence is incorporated into the double-stranded scaffold polynucleotide by attachment to the support strand of the double-stranded scaffold polynucleotide at the blunt end of the double-stranded scaffold polynucleotide.

In each of the four method versions and variants thereof the support strand of the polynucleotide ligation molecule also comprises a universal nucleotide (labelled "Un" in the structures depicted in each of Figures 1, 2, 4 and 5) at the complementary ligation end which will facilitate cleavage in the cleavage step. The role of the universal nucleotide will be apparent from the detailed description of each method below.

The terminal nucleotide of the helper strand of the polynucleotide ligation molecule at the complementary ligation end is provided such that the helper strand cannot be ligated to the primer strand portion of the synthesis strand, *i.e.* it is provided as a non-ligatable nucleotide. This is typically achieved by providing the terminal nucleotide of the helper strand without a phosphate group, *i.e.* it is provided as a nucleoside. Alternatively a 5'-protected nucleoside, a nucleoside with a non-ligatable group at the 5' position, such as 5'-deoxynucleoside or a 5'-aminonucleoside, or any other suitable non-ligatable nucleotide or nucleoside may be used.

Thus upon ligation of the support strand of the polynucleotide ligation molecule to the support strand of the double-stranded scaffold polynucleotide, a single-strand break or "nick" is provided in the synthesis strand between the primer strand portion of the synthesis strand and the helper strand.

Upon ligation of the polynucleotide ligation molecule to the double-stranded scaffold polynucleotide a double-stranded scaffold polynucleotide is formed comprising the newly incorporated first nucleotide, a universal nucleotide for use in facilitating cleavage in the cleavage step and a "nick".

As the first nucleotide of the predefined sequence of that cycle is ligated to the terminal nucleotide of the support strand of the double-stranded scaffold polynucleotide, the terminal nucleotide of the support strand of the double-stranded scaffold polynucleotide must be provided, prior to the ligation step, with an attached phosphate group or other ligatable group so as to allow the terminal nucleotide of the support strand of the double-stranded scaffold polynucleotide to act as a substrate for the ligase enzyme.

As described in more detail herein, in certain of the exemplary methods relating to versions 1 to 4 and variants thereof described herein, the helper strand may be removed prior to the steps of cleavage and incorporating a second nucleotide of the predefined sequence in that cycle of synthesis, e.g. by denaturation and release from the support strand to which it was previously hybridised.

In the context of the first cycle of synthesis the term "a first nucleotide of the predefined sequence" is not necessarily to be understood as meaning the very first nucleotide of the predefined sequence. The methods described herein relate to the synthesis of a double-stranded polynucleotide having a predefined sequence and a portion of the predefined sequence may be provided pre-synthesised in the scaffold polynucleotide before initiation of the first cycle of synthesis. In this context the term "a" first nucleotide of the predefined sequence can mean "any" nucleotide of the predefined sequence.

Following the ligation step (step 2), the scaffold polynucleotide is then cleaved (step 3, 103, 203, 403, 503). Cleavage results in release of the polynucleotide ligation molecule from the scaffold polynucleotide and retention of the first nucleotide of that cycle attached to the support strand of the cleaved scaffold polynucleotide and paired with the second nucleotide of that cycle which is attached to the synthesis strand of the cleaved scaffold polynucleotide. Cleavage results in release of the helper strand, if present and hybridised to the support strand immediately prior to cleavage, and release of the support strand comprising the universal nucleotide. Cleavage thus leaves in place a cleaved double-stranded scaffold polynucleotide comprising, at the site of cleavage, a cleaved terminal end of the support strand comprising the first nucleotide of that cycle and a terminal end of the primer strand portion of the synthesis strand comprising the second nucleotide of that cycle.

The terminal end of the primer strand portion of the synthesis strand provides a primer site which is a site for attachment of a second nucleotide of the predefined sequence which is attached to/incorporated into the synthesis strand by an enzyme which possesses the capability of extending an oligonucleotide or a polynucleotide molecule with a single nucleotide. Such an enzyme is typically a nucleotide transferase enzyme or a polymerase enzyme. Any suitable enzyme as defined further herein and/or known to a skilled person may be used. Thus the enzyme will act to extend the terminal nucleotide of the primer strand portion. This terminal nucleotide will therefore typically define a 3' terminus of the primer strand portion, *e.g.* to allow extension by polymerase, transferase or any other enzyme which catalyses extension in a 5' to 3' direction. The opposite terminus of the synthesis strand comprising the primer strand portion will consequently typically define a 5' terminus of the synthesis strand, and the terminal nucleotide of the support strand adjacent the 5' terminus of the synthesis strand will consequently typically define the 3' terminus of the support strand.

The terminal nucleotide of the helper strand portion of the synthesis strand, which is positioned at the site of the single-strand break, will typically define a 5' terminus of the helper strand portion and consequently the opposite terminus of the helper strand portion of the synthesis strand will typically define the 3' terminus of the synthesis strand.

In the step of incorporating the second nucleotide (step 4; 104, 204, 404, 504), the second nucleotide is provided with a reversible terminator group (depicted as the small triangle of the incorporated nucleotide in step 4 of each of Figures 1, 2, 4 and 5) which prevents further extension by the enzyme. Thus in step (4) only a single nucleotide is incorporated.

Nucleotides comprising any suitable reversible terminator group could be used. Preferred nucleotides with reversible terminator groups are 3'*-O*-allyl-dNTPs and/or 3'-O-azidomethyl-dNTPs or other groups as described further herein.

As will be apparent from the description of the various methods defined herein, the term "a second nucleotide of the predefined sequence" is not to be understood as meaning the next nucleotide following on from the first nucleotide in a linear sequence in one strand comprising the predefined sequence, but merely "a" further nucleotide of the predefined sequence in the context of the synthesised double-stranded polynucleotide as a whole. In the case of the specific and non-limiting method versions 1 to 4 of the invention defined herein and certain variants thereof, each "first nucleotide" of one cycle will be sequentially ligated to the "first nucleotide" of the previous cycle in the same nucleic acid strand, thereby extending the first strand sequentially by one nucleotide per cycle. In each cycle, each "second nucleotide" of one cycle will be sequentially incorporated next to the "second nucleotide" of the previous cycle in the same nucleic acid strand, thereby extending the second strand sequentially by one nucleotide per cycle. Thus, when synthesis cycles are completed the synthesised double-stranded polynucleotide molecule will comprise a predefined sequence of one strand defined by the ligated first nucleotides of each cycle, and a predefined sequence of the opposite strand defined by the incorporated second nucleotides of each cycle. The sequences of both strands are necessarily predefined, and are determined by the identity of first and second nucleotides chosen by the user in each cycle of synthesis. In method versions 1 and 2 described herein, given that first and second nucleotides of each cycle form a nucleotide pair with respective partner nucleotides, if the first and second nucleotides of each cycle is chosen by the user to be naturally complementary to their respective partner nucleotides of each cycle, then the final synthesised strands will be perfectly complementary. If first and second nucleotides of certain cycles are chosen by the user to be non-complementary to their respective partner nucleotides of those cycles, then the final synthesised strands will not be perfectly complementary. Nevertheless, in either situation the final synthesised strands both comprise sequence which in the context of the synthesised double-stranded polynucleotide as a whole is predefined.

In step (5) of the methods a deprotection step is performed to remove the reversible terminator group from the incorporated second nucleotide of the predefined nucleotide sequence (step 5 of Figures 1, 2, 4 and 5; 105, 205, 405, 505).

Iterative cycles of synthesis comprising the same steps as described above are performed in order to generate the synthetic polynucleotide.

Specific methods are described in more detail below.

### Synthesis Method Version 1

With reference to Figure 1, in a first specific non-limiting exemplary version of the synthesis methods of the invention a double-stranded scaffold polynucleotide is provided (step 1 of Figure 1; 101). The double-stranded scaffold polynucleotide comprises a support strand and a synthesis strand hybridised thereto. The synthesis strand comprises a primer strand portion. The double-stranded scaffold polynucleotide is provided with at least one blunt end, wherein the at least one blunt end comprises the terminal end of the primer strand portion and the terminal end of the support strand hybridized thereto. The terminal nucleotide of the support strand is capable of acting as a substrate for a ligase enzyme, and preferably comprises a phosphate group, or other ligatable group.

In step (2) of the methods a polynucleotide ligation molecule (see structure depicted at the top right of the upper part of Figure 1) is ligated to the scaffold polynucleotide. Ligation incorporates a first nucleotide of the predefined sequence into the support strand of the scaffold polynucleotide (step 2 of Figure 1; 102).

In each cycle of synthesis the scaffold polynucleotide is then cleaved at a cleavage site defined by a sequence comprising the universal nucleotide in the support strand (step 3; 103). In method version 1 cleavage comprises cleaving the support strand immediately after the universal nucleotide in the direction proximal to the primer strand portion/distal to the helper strand, *i.e.* the support strand is cleaved between the position occupied by the universal nucleotide and the next nucleotide position in the support strand in the direction proximal to the primer strand portion/distal to the helper strand. Cleavage of the scaffold polynucleotide (step 3) results in release of the polynucleotide ligation molecule from the scaffold polynucleotide and retention of the first nucleotide of that cycle unpaired and attached to the first strand of the cleaved scaffold polynucleotide. Cleavage of the scaffold polynucleotide (step 3) results in loss of the helper strand from the scaffold polynucleotide, if present and hybridised to the support strand immediately prior to cleavage, and loss of the universal nucleotide from the scaffold polynucleotide. Cleavage leaves in place a cleaved double-stranded scaffold polynucleotide comprising, at the site of cleavage, a cleaved terminal end of the support strand and the terminal end of the primer strand portion of the synthesis strand which comprised the nick site prior to cleavage. Cleavage results in a single-nucleotide overhang at the cleaved end of the ligated scaffold polynucleotide, wherein the first nucleotide of that cycle is the terminal nucleotide of the support strand and overhangs the terminal nucleotide of the primer strand portion of the synthesis strand.

In step (4) of the methods a second nucleotide of the predefined nucleotide sequence is added to the terminal end of the primer strand portion of the synthesis strand by the action of an enzyme having the capability of extending an oligonucleotide or a polynucleotide molecule with a single nucleotide. Such an enzyme is typically a nucleotide transferase enzyme or a polymerase enzyme (step 4 of Figure 1; 104). The second nucleotide is provided with a reversible terminator group which prevents further extension by the enzyme. Thus in step (4) only a single nucleotide is incorporated.

In step (5) of the methods a deprotection step is performed to remove the terminator group from the newly-incorporated nucleotide (step 5 of Figure 1).

### Step 1 - provision of a scaffold polynucleotide

In exemplary version 1 of the synthesis methods of the invention a double-stranded scaffold polynucleotide is provided in step (1) (101). The double-stranded scaffold polynucleotide is provided comprising a synthesis strand and a support strand hybridised thereto, wherein the synthesis strand comprises a primer strand portion. The terminal nucleotide of the support strand is paired with the terminal nucleotide of the primer strand portion thus forming a blunt end. The terminal nucleotide of the support strand is capable of acting as a substrate for a ligase enzyme, and comprises a ligatable group, preferably a phosphate group.

### Step 2 - ligation of a polynucleotide ligation molecule to the scaffold polynucleotide and incorporation of a first nucleotide of the predefined sequence

In step (2) of the method a double-stranded polynucleotide ligation molecule is ligated (102) to the scaffold polynucleotide by the action of a ligase enzyme in a blunt-ended ligation reaction.

The polynucleotide ligation molecule comprises a support strand and a helper strand hybridised thereto. The polynucleotide ligation molecule further comprises a complementary ligation end comprising in the support strand a universal nucleotide and a first nucleotide of the predefined sequence.

The complementary ligation end of the polynucleotide ligation molecule is structured such that the terminal nucleotide of the support strand is the first nucleotide of the predefined sequence to be incorporated into the scaffold polynucleotide in any given cycle of synthesis. The terminal nucleotide of the support strand is paired with the terminal nucleotide of the helper strand. The terminal nucleotide of the support strand, i.e. the first nucleotide of the predefined sequence of that cycle, occupies nucleotide position n in the support strand. By position n it is meant the nucleotide position which will be opposite the second nucleotide of the predefined sequence of that cycle upon incorporation of the second nucleotide, the first and second nucleotides thereby forming a nucleotide pair. In Figure 1 in step (2) the first nucleotide of the predefined sequence is depicted as adenosine, the second nucleotide of the predefined sequence is depicted as thymine and the terminal nucleotide of the helper strand is depicted as thymine. Adenosine and thymine are depicted purely for illustration. The first and second nucleotides and the terminal nucleotide of the helper strand can be any suitable nucleotides as chosen by the user. Nucleotides depicted as X may also be any suitable nucleotides as chosen by the user.

Typically, the terminal nucleotide of the support strand at the complementary ligation end will define the 3' terminus of the support strand of the complementary ligation end of the polynucleotide ligation molecule.

The terminal nucleotide of the helper strand of the polynucleotide ligation molecule is configured such that it cannot be ligated to another polynucleotide in a polynucleotide strand (position labelled "T" in the structure depicted at the top right of the upper part of Figure 1). This nucleotide is referred to as a non-ligatable terminal nucleotide. Typically this terminal nucleotide will lack a phosphate group, *i.e.* it will be a nucleoside. Typically, this terminal nucleotide of the helper strand will define the 5' terminus of the helper strand.

In the complementary ligation end of the polynucleotide ligation molecule the universal nucleotide in the support strand is positioned such that it is the penultimate nucleotide of the support strand, is paired with the penultimate nucleotide of the helper strand, and occupies nucleotide position n+1 in the support strand. By position n+1 it is meant the next nucleotide position in the support strand relative to position n in the direction distal to the complementary ligation end.

The complementary ligation end is configured so that it will compatibly join with the blunt end of the scaffold polynucleotide when subjected to suitable ligation conditions. Upon ligation of the support strands of the polynucleotide ligation molecule and the scaffold polynucletide, the first nucleotide becomes incorporated into the scaffold polynucletide. Because the terminal nucleotide of the helper strand of the polynucleotide ligation molecule is a non-ligatable nucleotide, the ligase enzyme will be prevented from ligating the helper strand and the primer strand portion of the synthesis strand, thus creating a single-strand break or "nick" between the helper strand and the primer strand portion of the synthesis strand.

Ligation of the polynucleotide ligation molecule to the scaffold polynucleotide extends the length of the support strand of the double-stranded scaffold polynucleotide of step (1) and wherein the first nucleotide of the predefined nucleotide sequence is incorporated into the support strand of the scaffold polynucleotide.

Ligation of the support strands may be performed by any suitable means. Ligation may typically and preferably be performed by enzymes having ligase activity. For example, ligation may be performed with T3 DNA ligase or T4 DNA ligase or functional variants or equivalents thereof or other enzymes described further herein. The use of such enzymes will result in the maintenance of the single-strand break in the synthesis strand, since the terminal nucleotide of the helper strand is provided such that it cannot act as a substrate for ligase, *e.g.* due to the absence of a terminal phosphate group or presence of a non-ligatable blocking group.

Following ligation of the polynucleotide ligation molecule to the scaffold polynucleotide the first nucleotide of the predefined nucleotide sequence is referred to as occupying nucleotide position n, the universal nucleotide is referred to as occupying nucleotide position n+1 and the nucleotide which prior to ligation was the terminal nucleotide of the support strand of the scaffold polynucleotide proximal to the primer strand portion is referred to as occupying nucleotide position n-1. By nucleotide position n-1 it is meant the next nucleotide position in the support strand relative to position n in the direction proximal to the primer strand portion/distal to the helper strand.

### Step 3 - cleavage

In step (3) of the method the ligated scaffold polynucleotide is cleaved (103) at a cleavage site. The cleavage site is defined by a sequence comprising the universal nucleotide in the support strand. Cleavage results in a double-strand break in the scaffold polynucleotide. Cleavage of the scaffold polynucleotide (step 3) results in loss of the helper strand, if present and hybridised to the support strand immediately prior to cleavage, and loss of the support strand comprising the universal nucleotide.

Cleavage of the scaffold polynucleotide thereby releases the polynucleotide ligation molecule from the scaffold polynucleotide but leads to the retention of the first nucleotide of that cycle attached to the first strand of the cleaved scaffold polynucleotide and paired with the second nucleotide of that cycle. Cleavage of the scaffold polynucleotide leaves in place a cleaved overhanging-ended double-stranded scaffold polynucleotide comprising the first nucleotide of the predefined sequence as the terminal nucleotide of the support strand and overhanging the terminal nucleotide of the primer strand portion of the synthesis strand.

The synthesis strand is already provided with a single-strand break or "nick" in this exemplary method, thus only cleavage of the support strand is necessary to provide a double-strand break in the scaffold polynucleotide. Furthermore, as noted previously in this exemplary method version cleavage generates an overhanging-ended cleaved double-stranded scaffold polynucleotide, wherein the universal nucleotide occupies position n+1 in the support strand prior to the cleavage step. To obtain such an overhanging-ended cleaved double-stranded scaffold polynucleotide when the universal nucleotide occupies position n+1 in the support strand, the support strand is cleaved at a specific position relative to the universal nucleotide. When the support strand of the scaffold polynucleotide is cleaved between nucleotide positions n+1 and n the polynucleotide ligation molecule is released from the scaffold polynucleotide (see structure depicted at the top left of the upper part of Figure 1) except that the first nucleotide of that cycle is retained in the scaffold polynucleotide attached to the support strand of the cleaved scaffold polynucleotide.

A phosphate group should continue to be attached to the terminal nucleotide of the support strand of the cleaved scaffold polynucleotide at the cleavage site. This ensures that the support strand of the cleaved scaffold polynucleotide can be ligated to the support strand of the polynucleotide ligation molecule in the ligation step of the next cycle of synthesis. Cleavage is performed so that the terminal nucleotide of the support strand of the cleaved scaffold polynucleotide retains ligatable group, preferably a terminal phosphate group and such that a phosphorylation step need not therefore be performed.

Thus in method version 1 the universal nucleotide occupies position n+1 in the support strand at steps (2) and (3), and the support strand is cleaved between nucleotide positions n+1 and n at step (3).

Preferably, the support strand is cleaved by cleavage of the phosphodiester bond between nucleotide positions n+1 and n (the first phosphodiester bond of the support strand relative to the position of the universal nucleotide, in the direction distal to the helper strand/proximal to the primer strand).

The support strand may be cleaved by cleavage of one ester bond of the phosphodiester bond between nucleotide positions n+1 and n.

Preferably the support strand is cleaved by cleavage of the first ester bond relative to nucleotide position n+1. This will have the effect of retaining a terminal phosphate group on the support strand of the cleaved scaffold polynucleotide at the cleavage position.

Any suitable mechanism may be employed to effect cleavage of the support strand between nucleotide positions n+1 and n when the universal nucleotide occupies position n+1.

Cleavage of the support strand between nucleotide positions n+1 and n as described above may be performed by the action of an enzyme.

Cleavage of the support strand between nucleotide positions n+1 and n as described above may be performed as a two-step cleavage process.

The first cleavage step of a two-step cleavage process may comprise removing the universal nucleotide from the support strand thus forming an abasic site at position n+1, and the second cleavage step may comprise cleaving the support strand at the abasic site, between positions n+1 and n.

One mechanism of cleaving the support strand at a cleavage site defined by a sequence comprising a universal nucleotide in the manner outlined above is described in Example 2. The cleavage mechanism described in Example 2 is exemplary and other mechanisms could be employed, provided that a cleaved double-stranded scaffold polynucleotide comprising a single base overhang as described above is achieved.

In the first cleavage step of a two-step cleavage process the universal nucleotide is removed from the support strand whilst leaving the sugar-phosphate backbone intact. This can be achieved by the action of an enzyme which can specifically excise a single universal nucleotide from a double-stranded polynucleotide. In the exemplified cleavage methods the universal nucleotide is inosine and inosine is excised from the support strand by the action of an enzyme, thus forming an abasic site. In the exemplified cleavage method the enzyme is a 3-methyladenine DNA glycosylase enzyme, specifically human alkyladenine DNA glycosylase (hAAG). Other enzymes, molecules or chemicals could be used provided that an abasic site is formed. The nucleotide-excising enzyme may be an enzyme which catalyses the release of uracil from polynucleotides, such as Uracil-DNA Glycosylase (UDG).

In the second cleavage step of a two-step cleavage process the support strand is cleaved at the abasic site by making a single-strand break. In the exemplified methods the support strand is cleaved by the action of a chemical which is a base, such as NaOH. Alternatively, an organic chemical such as N,N'-dimethylethylenediamine may be used. Alternatively, enzymes having abasic site lyase activity, such as AP Endonuclease 1, Endonuclease III (Nth), or Endonuclease VIII, may be used. Other enzymes, molecules or chemicals could be used provided that the support strand is cleaved at the abasic site as described.

Thus in embodiments wherein the universal nucleotide is at position n+1 of the support strand at steps (1) and (2) and the support strand is cleaved between positions n+1 and n, a first cleavage step may be performed with a nucleotide-excising enzyme. An example of such an enzyme is a 3-methyladenine DNA glycosylase enzyme, such as human alkyladenine DNA glycosylase (hAAG). The second cleavage step may be performed with a chemical which is a base, such as NaOH. The second step may be performed with an organic chemical having abasic site cleavage activity such as N,N'-dimethylethylenediamine. The second step may be performed with an enzyme having abasic site lyase activity such as Endonuclease VIII or Endonuclease **III.**

Cleavage of the support strand between nucleotide positions n+1 and n as described above may also be performed as a one-step cleavage process. Examples of enzymes which may be used in any such process include Endonuclease **III,** Endonuclease VIII. Other enzymes which may be used in any such process include enzymes which cleave 8-oxoguanosine, such as formamidopyrimidine DNA glycosylase (Fpg) and 8-oxoguanine DNA glycosylase (hOGG1).

### Step 4 - incorporation of a second nucleotide of the predefined sequence

In step (4) of the method, following ligation of the polynucleotide ligation molecule to the scaffold polynucleotide and cleavage of the scaffold polynucleotide, a second nucleotide of the predefined sequence is then incorporated into the synthesis strand by extension of the primer strand portion.

Extension of the primer strand portion may be achieved by the action of any suitable enzyme which possesses the capability of extending an oligonucleotide or a polynucleotide molecule with a single nucleotide. Such an enzyme is typically a nucleotide transferase enzyme or a polymerase enzyme. Any suitable enzyme as defined further herein or known to the skilled person may be used.

The second nucleotide of the predefined sequence which is incorporated during step (4) comprises a reversible terminator group which prevents further extension by the enzyme, or comprises any other analogous functionality which prevents further extension by the enzyme. Any suitable reversible terminator group or functionality as defined further herein or known to the skilled person may be used.

Upon its incorporation into the primer strand portion of the scaffold polynucleotide the second nucleotide of the predefined sequence of that cycle becomes paired with the first nucleotide of the predefined sequence of that cycle to form a nucleotide pair of that cycle. A nucleotide pair may be any suitable nucleotide pair as defined further herein.

### Step 5 - deprotection

In this exemplary method version of the invention, as well as with all versions, to allow the next nucleotide to be incorporated in the next synthesis cycle, the reversible terminator group must be removed from the first nucleotide (deprotection step; 105). This is performed as step (5) of the method, after incorporation of the second nucleotide at step (4), as shown in Figure 1 (105). Unincorporated second nucleotides should first be removed following step (4) in order to prevent multiple incorporation of second nucleotides in the same cycle of synthesis.

Removal of the reversible terminator group from the first nucleotide can be performed by any suitable means known to the skilled person. For example, removal can be performed by the use of a chemical, such as tris(carboxyethyl)phosphine (TCEP).

### Further cycles

Following completion of the first cycle of synthesis, second and further cycles of synthesis may be performed using the same method steps.

The product of cleavage, incorporation and deprotection steps (3), (4) and (5) of the previous cycle is provided (in step 6) as the double-stranded scaffold polynucleotide for the next cycle of synthesis.

**In** step (6) of the next and each further cycle of synthesis a further double-stranded polynucleotide ligation molecule is ligated to the cleavage product of steps (3), (4) and (5) of the previous cycle. The polynucleotide ligation molecule may be structured in the same way as described above for step (2) of the previous cycle, except that the polynucleotide ligation molecule comprises a first nucleotide of the further cycle of synthesis. The polynucleotide ligation molecule may be ligated to the cleavage product of steps (3), (4) and (5) of the previous cycle in the same way as described above for step (2).

**In** step (7) of the next and each further cycle of synthesis the ligated scaffold polynucleotide is cleaved at the cleavage site. Cleavage results in a double-strand break in the scaffold polynucleotide. Cleavage of the scaffold polynucleotide (step 7) results in loss of the helper strand, if present and hybridised to the support strand immediately prior to cleavage, and loss of the support strand comprising the universal nucleotide. Cleavage of the scaffold polynucleotide thereby releases the further polynucleotide ligation molecule from the scaffold polynucleotide but leads to the retention of the first nucleotide of that further cycle unpaired and attached to the support strand of the cleaved scaffold polynucleotide. Cleavage of the scaffold polynucleotide leaves in place a cleaved overhanging-ended double-stranded scaffold polynucleotide comprising the first nucleotide of the further cycle as the terminal nucleotide of the support strand of the scaffold polynucleotide overhanging the terminal nucleotide of the primer strand portion of the synthesis strand of the scaffold polynucleotide. Cleavage at step (7) may be performed in the same way as described above for step (3).

In step (8) of the next and each further cycle of synthesis the terminal end of the primer strand portion of the synthesis strand of the double-stranded scaffold polynucleotide is further extended by the incorporation of a second nucleotide of the further cycle of synthesis by the action of the nucleotide transferase enzyme, polymerase enzyme or other enzyme. The second nucleotide of the further cycle of synthesis may be incorporated in the same way as described above for step (4).

In step (9) the reversible terminator group is removed from the second nucleotide of the further cycle (deprotection step; 109). Deprotection by removal of the reversible terminator group in the next cycle and further cycles may be performed as described above with respect to the first synthesis cycle.

Synthesis cycles are repeated as described above for as many times as necessary to synthesise the double-stranded polynucleotide having the predefined nucleotide sequence.

### Synthesis Method Version 2

With reference to Figure 2, in a second specific non-limiting exemplary version of the synthesis methods of the invention a double-stranded scaffold polynucleotide is provided (step 1 of Figure 2; 201). The double-stranded scaffold polynucleotide comprises a support strand and a synthesis strand hybridised thereto. The synthesis strand comprises a primer strand portion. The double-stranded scaffold polynucleotide is provided with at least one blunt end, wherein the at least one blunt end comprises the terminal end of the primer strand portion and the terminal end of the support strand hybridized thereto. The terminal nucleotide of the support strand is capable of acting as a substrate for a ligase enzyme, and preferably comprises a phosphate group or other ligatable group.

In step (2) of the methods a polynucleotide ligation molecule (see structure depicted at the top right of the upper part of Figure 2) is ligated to the scaffold polynucleotide. Ligation incorporates a first nucleotide of the predefined sequence into the support strand of the scaffold polynucleotide (step 2 of Figure 2; 202).

In each cycle of synthesis the scaffold polynucleotide is then cleaved at a cleavage site defined by a sequence comprising the universal nucleotide in the support strand (step 3; 203). In method version 2 cleavage comprises cleaving the support strand immediately after the nucleotide which is in the nucleotide position next to the universal nucleotide in the direction proximal to the primer strand portion/distal to the helper strand. Cleavage of the scaffold polynucleotide (step 3) results in release of the polynucleotide ligation molecule from the scaffold polynucleotide and retention of the first nucleotide of that cycle unpaired and attached to the first strand of the cleaved scaffold polynucleotide. Cleavage of the scaffold polynucleotide (step 3) results in loss of the helper strand from the scaffold polynucleotide, if present and hybridised to the support strand immediately prior to cleavage, and loss of the universal nucleotide from the scaffold polynucleotide. Cleavage leaves in place a cleaved double-stranded scaffold polynucleotide comprising, at the site of cleavage, a cleaved terminal end of the support strand and the terminal end of the primer strand portion of the synthesis strand which comprised the nick site prior to cleavage. Cleavage results in a single-nucleotide overhang at the cleaved end of the ligated scaffold polynucleotide, wherein the first nucleotide of that cycle is the terminal nucleotide of the support strand and overhangs the terminal nucleotide of the primer strand portion of the synthesis strand.

In step (4) of the methods a second nucleotide of the predefined nucleotide sequence is added to the terminal end of the primer strand portion of the synthesis strand by the action of an enzyme having the capability of extending an oligonucleotide or a polynucleotide molecule with a single nucleotide. Such an enzyme is typically a nucleotide transferase enzyme or a polymerase enzyme (step 4 of Figure 2; 204). The second nucleotide is provided with a reversible terminator group which prevents further extension by the enzyme. Thus in step (4) only a single nucleotide is incorporated.

In step (5) of the methods a deprotection step is performed to remove the terminator group from the newly-incorporated nucleotide.

### Step 1 - provision of a scaffold polynucleotide

In exemplary version 2 of the synthesis methods of the invention a double-stranded scaffold polynucleotide is provided in step (1) (201). The double-stranded scaffold polynucleotide is provided comprising a synthesis strand and a support strand hybridised thereto, wherein the synthesis strand comprises a primer strand portion. The terminal nucleotide of the support strand is paired with the terminal nucleotide of the primer strand portion thus forming a blunt end. The terminal nucleotide of the support strand is capable of acting as a substrate for a ligase enzyme, and comprises a ligatable group, preferably a phosphate group.

### Step 2 - ligation of a polynucleotide ligation molecule to the scaffold polynucleotide and incorporation of a first nucleotide of the predefined sequence

In step (2) of the method a double-stranded polynucleotide ligation molecule is ligated (202) to the scaffold polynucleotide by the action of a ligase enzyme in a blunt-ended ligation reaction.

The polynucleotide ligation molecule comprises a support strand and a helper strand hybridised thereto. The polynucleotide ligation molecule further comprises a complementary ligation end comprising in the support strand a universal nucleotide and a first nucleotide of the predefined sequence.

The complementary ligation end of the polynucleotide ligation molecule is structured such that the terminal nucleotide of the support strand is the first nucleotide of the predefined sequence to be incorporated into the scaffold polynucleotide in any given cycle of synthesis. The terminal nucleotide of the support strand is paired with the terminal nucleotide of the helper strand. The terminal nucleotide of the support strand, i.e. the first nucleotide of the predefined sequence of that cycle, occupies nucleotide position n in the support strand. By position n it is meant the nucleotide position which will be opposite the second nucleotide of the predefined sequence of that cycle upon incorporation of the second nucleotide, the first and second nucleotides thereby forming a nucleotide pair. In Figure 2 in step (2) the first nucleotide of the predefined sequence is depicted as adenosine, the second nucleotide of the predefined sequence is depicted as thymine and the terminal nucleotide of the helper strand is depicted as thymine. Adenosine and thymine are depicted purely for illustration. The first and second nucleotides and the terminal nucleotide of the helper strand can be any suitable nucleotides. Nucleotides depicted as X may also be any suitable nucleotides as chosen by the user.

Typically, the terminal nucleotide of the support strand at the complementary ligation end will define the 3' terminus of the support strand of the complementary ligation end of the polynucleotide ligation molecule.

The terminal nucleotide of the helper strand of the polynucleotide ligation molecule is configured such that it cannot be ligated to another polynucleotide in a polynucleotide strand (position labelled "T" in the structure depicted at the top right of the upper part of Figure 2). This nucleotide is referred to as a non-ligatable terminal nucleotide. Typically this terminal nucleotide will lack a phosphate group, *i.e.* it will be a nucleoside. Typically, this terminal nucleotide of the helper strand will define the 5' terminus of the helper strand.

In the complementary ligation end of the polynucleotide ligation molecule the universal nucleotide in the support strand is positioned such that it occupies nucleotide position n+2 in the support strand and is paired with a partner nucleotide in the helper strand. By position n+2 it is meant the second nucleotide position in the support strand relative to position n in the direction distal to the complementary ligation end.

The complementary ligation end is configured so that it will compatibly join with the blunt end of the scaffold polynucleotide when subjected to suitable ligation conditions. Upon ligation of the support strands of the polynucleotide ligation molecule and the scaffold polynucletide, the first nucleotide becomes incorporated into the scaffold polynucletide. Because the terminal nucleotide of the helper strand of the polynucleotide ligation molecule is a non-ligatable nucleotide, the ligase enzyme will be prevented from ligating the helper strand and the primer strand portion of the synthesis strand, thus creating a single-strand break or "nick" between the helper strand and the primer strand portion of the synthesis strand.

Ligation of the polynucleotide ligation molecule to the scaffold polynucleotide extends the length of the support strand of the double-stranded scaffold polynucleotide of step (1) and wherein the first nucleotide of the predefined nucleotide sequence is incorporated into the support strand of the scaffold polynucleotide.

Ligation of the support strands may be performed by any suitable means. Ligation may typically and preferably be performed by enzymes having ligase activity. For example, ligation may be performed with T3 DNA ligase or T4 DNA ligase or functional variants or equivalents thereof or other enzymes described further herein. The use of such enzymes will result in the maintenance of the single-strand break in the synthesis strand, since the terminal nucleotide of the helper strand is provided such that it cannot act as a substrate for ligase, *e.g.* due to the absence of a terminal phosphate group or presence of a non-ligatable blocking group.

Following ligation of the polynucleotide ligation molecule to the scaffold polynucleotide the first nucleotide of the predefined nucleotide sequence is referred to as occupying nucleotide position n, the universal nucleotide is referred to as occupying nucleotide position n+2 and the nucleotide which prior to ligation was the terminal nucleotide of the support strand of the scaffold polynucleotide proximal to the primer strand portion is referred to as occupying nucleotide position n-1. By nucleotide position n-1 it is meant the next nucleotide position in the support strand relative to position n in the direction proximal to the primer strand portion/distal to the helper strand.

### Step 3 - cleavage

In step (3) of the method the ligated scaffold polynucleotide is cleaved (203) at a cleavage site. The cleavage site is defined by a sequence comprising the universal nucleotide in the support strand. Cleavage results in a double-strand break in the scaffold polynucleotide. Cleavage of the scaffold polynucleotide (step 3) results in loss of the helper strand, if present and hybridised to the support strand immediately prior to cleavage, and loss of the support strand comprising the universal nucleotide.

Cleavage of the scaffold polynucleotide thereby releases the polynucleotide ligation molecule from the scaffold polynucleotide but leads to the retention of the first nucleotide of that cycle attached to the first strand of the cleaved scaffold polynucleotide and paired with the second nucleotide of that cycle. Cleavage of the scaffold polynucleotide leaves in place a cleaved overhanging-ended double-stranded scaffold polynucleotide comprising the first nucleotide of the predefined sequence as the terminal nucleotide of the support strand and overhanging the terminal nucleotide of the primer strand portion of the synthesis strand.

The synthesis strand is already provided with a single-strand break or "nick" in this exemplary method, thus only cleavage of the support strand is necessary to provide a double-strand break in the scaffold polynucleotide. Furthermore, as noted previously in this exemplary method version cleavage generates an overhanging-ended cleaved double-stranded scaffold polynucleotide, wherein the universal nucleotide occupies position n+2 in the support strand prior to the cleavage step. To obtain such an overhanging-ended cleaved double-stranded scaffold polynucleotide when the universal nucleotide occupies position n+2 in the support strand, the support strand is cleaved at a specific position relative to the universal nucleotide. When the support strand of the scaffold polynucleotide is cleaved between nucleotide positions n+1 and n the polynucleotide ligation molecule is released from the scaffold polynucleotide (see structure depicted at the top left of the upper part of Figure 2) except that the first nucleotide of that cycle is retained in the scaffold polynucleotide attached to the support strand of the cleaved scaffold polynucleotide.

A phosphate group should continue to be attached to the terminal nucleotide of the support strand of the cleaved scaffold polynucleotide at the cleavage site. This ensures that the support strand of the cleaved scaffold polynucleotide can be ligated to the support strand of the polynucleotide ligation molecule in the ligation step of the next cycle of synthesis. Cleavage is performed so that the terminal nucleotide of the support strand of the cleaved scaffold polynucleotide retains a ligatable group, preferably a terminal phosphate group and such that a phosphorylation step need not therefore be performed.

Thus in method version 2 the universal nucleotide occupies position n+2 in the support strand at steps (2) and (3) and the support strand is cleaved between nucleotide positions n+1 and n at step (3).

Preferably, the support strand is cleaved by cleavage of the phosphodiester bond between nucleotide positions n+1 and n (the first phosphodiester bond of the support strand relative to the position of the universal nucleotide, in the direction distal to the helper strand/proximal to the primer strand).

The support strand may be cleaved by cleavage of one ester bond of the phosphodiester bond between nucleotide positions n+1 and n.

Preferably the support strand is cleaved by cleavage of the first ester bond relative to nucleotide position n+1. This will have the effect of retaining a terminal phosphate group on the support strand of the cleaved scaffold polynucleotide at the cleavage position.

Any suitable mechanism may be employed to effect cleavage of the support strand between nucleotide positions n+1 and n when the universal nucleotide occupies position n+2.

Cleavage of the support strand between nucleotide positions n+1 and n as described above may be performed by the action of an enzyme.

Cleavage of the support strand between nucleotide positions n+1 and n when the universal nucleotide occupies position n+2 in the support strand, as described above, may be performed by the action of an enzyme such as Endonuclease V.

One mechanism of cleaving the support strand between nucleotide positions n+1 and n at a cleavage site defined by a sequence comprising a universal nucleotide which is occupying position n+2 in the support strand is described in analogous fashion in Example 3. The mechanism described is exemplary and other mechanisms could be employed, provided that the cleavage arrangement described above is achieved.

In this exemplary mechanism an endonuclease enzyme is employed. In the exemplified method the enzyme is Endonuclease V. Other enzymes, molecules or chemicals could be used provided that the support strand is cleaved between nucleotide positions n+1 and n when the universal nucleotide occupies position n+2 in the support strand.

### Step 4 - incorporation of a second nucleotide of the predefined sequence

In step (4) of the method, following ligation of the polynucleotide ligation molecule to the scaffold polynucleotide a second nucleotide of the predefined sequence is then incorporated into the synthesis strand by extension of the primer strand portion.

Extension of the primer strand portion may be achieved by the action of any suitable enzyme which possesses the capability of extending an oligonucleotide or a polynucleotide molecule with a single nucleotide. Such an enzyme is typically a nucleotide transferase enzyme or a polymerase enzyme. Any suitable enzyme as defined further herein or known to the skilled person may be used.

The second nucleotide of the predefined sequence which is incorporated during step (4) comprises a reversible terminator group which prevents further extension by the enzyme, or comprises any other analogous functionality which prevents further extension by the enzyme. Any suitable reversible terminator group of functionality as defined further herein or known to the skilled person may be used.

Upon its incorporation into the primer strand portion of the scaffold polynucleotide the second nucleotide of the predefined sequence of that cycle becomes paired with the first nucleotide of the predefined sequence of that cycle to form a nucleotide pair of that cycle. A nucleotide pair may be any suitable nucleotide pair as defined further herein.

### Step 5 - deprotection

In this exemplary method version of the invention, as well as with all versions, to allow the next nucleotide to be incorporated in the next synthesis cycle, the reversible terminator group must be removed from the first nucleotide (deprotection step; 205). This is performed as step (5) of the method, after incorporation of the second nucleotide at step (4), as shown in Figure 2 (205). Unincorporated second nucleotides should first be removed following step (4) in order to prevent multiple incorporation of second nucleotides in the same cycle of synthesis.

Removal of the reversible terminator group from the first nucleotide can be performed by any suitable means known to the skilled person. For example, removal can be performed by the use of a chemical, such as tris(carboxyethyl)phosphine (TCEP).

### Further cycles

Following completion of the first cycle of synthesis, second and further cycles of synthesis may be performed using the same method steps.

The product of cleavage, incorporation and deprotection steps (3), (4) and (5) of the previous cycle is provided (in step 6) as the double-stranded scaffold polynucleotide for the next cycle of synthesis.

In step (6) of the next and further cycle of synthesis a further double-stranded polynucleotide ligation molecule is ligated to the cleavage product of steps (3), (4) and (5) of the previous cycle. The polynucleotide ligation molecule may be structured in the same way as described above for step (2) of the previous cycle, except that the polynucleotide ligation molecule comprises a first nucleotide of the further cycle of synthesis. The polynucleotide ligation molecule may be ligated to the cleavage product of steps (3), (4) and (5) of the previous cycle in the same way as described above for step (2).

In step (7) of the next and each further cycle of synthesis the ligated scaffold polynucleotide is cleaved at the cleavage site. Cleavage results in a double-strand break in the scaffold polynucleotide. Cleavage of the scaffold polynucleotide (step 7) results in loss of the helper strand, if present and hybridised to the support strand immediately prior to cleavage, and loss of the support strand comprising the universal nucleotide. Cleavage of the scaffold polynucleotide thereby releases the further polynucleotide ligation molecule from the scaffold polynucleotide but leads to the retention of the first nucleotide of that further cycle unpaired and attached to the support strand of the cleaved scaffold polynucleotide. Cleavage of the scaffold polynucleotide leaves in place a cleaved overhanging-ended double-stranded scaffold polynucleotide comprising the first nucleotide of the further cycle as the terminal nucleotide of the support strand of the scaffold polynucleotide overhanging the terminal nucleotide of the primer strand portion of the synthesis strand of the scaffold polynucleotide. Cleavage at step (7) may be performed in the same way as described above for step (3).

In step (8) of the next and each further cycle of synthesis the terminal end of the primer strand portion of the synthesis strand of the double-stranded scaffold polynucleotide is further extended by the incorporation of a second nucleotide of the further cycle of synthesis by the action of the nucleotide transferase enzyme, polymerase enzyme or other enzyme. The second nucleotide of the further cycle of synthesis may be incorporated in the same way as described above for step (4).

In step (9) the reversible terminator group is removed from the second nucleotide of the further cycle (deprotection step; 209). Deprotection by removal of the reversible terminator group in the next cycle and further cycles may be performed as described above with respect to the first synthesis cycle.

Synthesis cycles are repeated as described above for as many times as necessary to synthesise the double-stranded polynucleotide having the predefined nucleotide sequence.

### Variants of Synthesis Method Version 2

In addition to the above-described methods, variants of synthesis method version 2 are provided wherein the method is performed in the same way as synthesis method version 2 described above except for the following variations.

In the ligation step of the first cycle (step 2) the complementary ligation end of the polynucleotide ligation molecule is structured such that the universal nucleotide instead occupies nucleotide position n+2+x in the support strand and is paired with a partner nucleotide in the helper strand which is 2+x positions removed from the terminal nucleotide of the helper strand at the complementary ligation end; wherein nucleotide position n+2 is the second nucleotide position in the support strand relative to nucleotide position n in the direction distal to the complementary ligation end.

In the cleavage step of the first cycle (step 3) the universal nucleotide instead occupies nucleotide position n+2+x in the support strand of the scaffold polynucleotide, wherein nucleotide position n+2 is the second nucleotide position in the support strand relative to nucleotide position n in the direction proximal to the helper strand/distal to the primer strand portion; and the support strand of the scaffold polynucleotide is cleaved between positions n+1 and n.

In the ligation step of the second cycle (step 6) and in ligation steps of all further cycles the complementary ligation end of the polynucleotide ligation molecule is structured such that the universal nucleotide occupies nucleotide position n+2+x in the support strand and is paired with a partner nucleotide which is 2+x positions removed from the terminal nucleotide of the helper strand at the complementary ligation end; wherein nucleotide position n+2 is the second nucleotide position in the support strand relative to nucleotide position n in the direction distal to the complementary ligation end.

Finally, in the cleavage step of the second cycle (step 7) and in cleavage steps of all further cycles the universal nucleotide instead occupies nucleotide position n+2+x in the support strand of the scaffold polynucleotide, wherein nucleotide position n+2 is the second nucleotide position in the support strand relative to nucleotide position n in the direction proximal to the helper strand/distal to the primer strand portion; and the support strand of the scaffold polynucleotide is cleaved between positions n+1 and n.

In all of these variant methods, x is a whole number from 1 to 10 or more, and wherein x is the same whole number in steps (2), (3), (6) and (7).

As with synthesis method version 2, with variant methods based on version 2 it will be noted that in any given cycle the nucleotide position occupied by the first nucleotide of that cycle in the support strand following ligation and during the incorporation and cleavage steps is defined as nucleotide position n, and upon completion of the given cycle of synthesis the position occupied by that first nucleotide of that cycle in the support strand of the cleaved scaffold polynucleotide is defined as nucleotide position n-1 in the next cycle of synthesis.

Thus in these particular variants of synthesis method version 2 the position of the cleavage site relative to nucleotide position n is held constant, and the position of the universal nucleotide relative to nucleotide position n is increased by moving the position of the universal nucleotide in the direction proximal to the helper strand/distal to the primer strand portion by a number of nucleotide positions determined by the number selected for x.

A diagrammatic representation of these variant methods is provided in Figure 3.

### Synthesis Method Version 3

With reference to Figure 4, in a third specific non-limiting exemplary version of the synthesis methods of the invention a double-stranded scaffold polynucleotide is provided (step 1 of Figure 4; 401). The double-stranded scaffold polynucleotide comprises a support strand and a synthesis strand hybridised thereto. The synthesis strand comprises a primer strand portion. The double-stranded scaffold polynucleotide is provided with at least one overhanging end, wherein the at least one overhanging end comprises the terminal end of the support strand overhanging the terminal end of the primer strand portion. The terminal nucleotide of the support strand is capable of acting as a substrate for a ligase enzyme, and preferably comprises a phosphate group or other ligatable group.

In step (2) of the methods a polynucleotide ligation molecule (see structure depicted at the top right of the upper part of Figure 4) is ligated to the scaffold polynucleotide. Ligation incorporates a first nucleotide of the predefined sequence into the support strand of the scaffold polynucleotide (step 2 of Figure 4; 402).

In each cycle of synthesis the scaffold polynucleotide is then cleaved at a cleavage site defined by a sequence comprising the universal nucleotide in the support strand (step 3; 403). In method version 3 cleavage comprises cleaving the support strand immediately after the universal nucleotide in the direction proximal to the primer strand portion/distal to the helper strand, *i.e.* the support strand is cleaved between the position occupied by the universal nucleotide and the next nucleotide position in the support strand in the direction proximal to the primer strand portion/distal to the helper strand. Cleavage of the scaffold polynucleotide (step 3) results in release of the polynucleotide ligation molecule from the scaffold polynucleotide and retention of the first nucleotide of that cycle unpaired and attached to the first strand of the cleaved scaffold polynucleotide. Cleavage of the scaffold polynucleotide (step 3) results in loss of the helper strand from the scaffold polynucleotide, if present and hybridised to the support strand immediately prior to cleavage, and loss of the universal nucleotide from the scaffold polynucleotide. Cleavage leaves in place a cleaved double-stranded scaffold polynucleotide comprising, at the site of cleavage, a cleaved terminal end of the support strand and the terminal end of the primer strand portion of the synthesis strand which comprised the nick site prior to cleavage, and wherein the cleaved double-stranded scaffold polynucleotide comprises the first nucleotide of that cycle as the terminal nucleotide of the cleaved end of the support strand overhanging the terminal nucleotide of the primer strand portion of the synthesis strand.

In step (4) of the methods a second nucleotide of the predefined nucleotide sequence is added to the terminal end of the primer strand portion of the synthesis strand by the action of an enzyme having the capability of extending an oligonucleotide or a polynucleotide molecule with a single nucleotide. Such an enzyme is typically a nucleotide transferase enzyme or a polymerase enzyme (step 4 of Figure 4; 404). The second nucleotide is provided with a reversible terminator group which prevents further extension by the enzyme. Thus in step (3) only a single nucleotide is incorporated.

In step (5) of the methods a deprotection step is performed to remove the terminator group from the newly-incorporated nucleotide (step 5 of Figure 4; 405).

### Step 1 - provision of a scaffold polynucleotide

In exemplary version 3 of the synthesis methods of the invention a double-stranded scaffold polynucleotide is provided in step (1) (401). The double-stranded scaffold polynucleotide is provided comprising a synthesis strand and a support strand hybridised thereto, wherein the synthesis strand comprises a primer strand portion. The terminal nucleotide of the support strand comprises a single-nucleotide overhang wherein the terminal nucleotide of the support strand is unpaired and overhangs the terminal nucleotide of the primer strand portion of the synthesis strand. The terminal nucleotide of the support strand is capable of acting as a substrate for a ligase enzyme, and comprises a ligatable group, preferably a phosphate group.

### Step 2 - ligation of a polynucleotide ligation molecule to the scaffold polynucleotide and incorporation of a first nucleotide of the predefined sequence

In step (2) of the method a double-stranded polynucleotide ligation molecule is ligated (402) to the scaffold polynucleotide by the action of a ligase enzyme in a sticky-ended ligation reaction.

The polynucleotide ligation molecule comprises a support strand and a helper strand hybridised thereto. The polynucleotide ligation molecule further comprises a complementary ligation end comprising in the support strand a universal nucleotide and a first nucleotide of the predefined sequence.

The complementary ligation end of the polynucleotide ligation molecule is structured such that the terminal nucleotide of the support strand is the first nucleotide of the predefined sequence to be incorporated into the scaffold polynucleotide in any given cycle of synthesis. The terminal nucleotide of the support strand is paired with the penultimate nucleotide of the helper strand. The complementary ligation end comprises a single-nucleotide overhang, wherein the terminal nucleotide of the helper strand overhangs the terminal nucleotide of the support strand. The terminal nucleotide of the support strand, *i.e.* the first nucleotide of the predefined sequence of that cycle, occupies nucleotide position n+1 in the support strand. By position n it is meant the nucleotide position which will be opposite the second nucleotide of the predefined sequence of that cycle upon incorporation of the second nucleotide. Position n+1 is the next nucleotide position relative to position n in the direction distal to the complementary ligation end. In Figure 4 in step (2) the first nucleotide of the predefined sequence is depicted as guanine, the second nucleotide of the predefined sequence is depicted as thymine, the terminal nucleotide of the helper strand is depicted as thymine and the penultimate nucleotide of the helper strand is depicted as cytosine. Adenosine and thymine are depicted purely for illustration. These nucleotides can be any suitable nucleotides. Nucleotides depicted as X may also be any suitable nucleotides as chosen by the user.

Typically, the terminal nucleotide of the support strand at the complementary ligation end will define the 3' terminus of the support strand of the complementary ligation end of the polynucleotide ligation molecule.

The terminal nucleotide of the helper strand of the polynucleotide ligation molecule is configured such that it cannot be ligated to another polynucleotide in a polynucleotide strand (position labelled "T" in the structure depicted at the top right of the upper part of Figure 4). This nucleotide is referred to as a non-ligatable terminal nucleotide. Typically this terminal nucleotide will lack a phosphate group, *i.e.* it will be a nucleoside. Typically, this terminal nucleotide of the helper strand will define the 5' terminus of the helper strand.

In the complementary ligation end of the polynucleotide ligation molecule the universal nucleotide in the support strand is positioned such that it is the penultimate nucleotide of the support strand, is paired with a partner nucleotide in the helper strand, and occupies nucleotide position n+2 in the support strand. By position n+2 it is meant the second nucleotide position in the support strand relative to position n in the direction distal to the complementary ligation end.

The overhanging complementary ligation end is configured so that it will compatibly join with the overhanging end of the scaffold polynucleotide when subjected to suitable ligation conditions. Upon ligation of the support strands of the polynucleotide ligation molecule and the scaffold polynucletide, the first nucleotide becomes incorporated into the scaffold polynucletide. Because the terminal nucleotide of the helper strand of the polynucleotide ligation molecule is a non-ligatable nucleotide, the ligase enzyme will be prevented from ligating the helper strand and the primer strand portion of the synthesis strand, thus creating a single-strand break or "nick" between the helper strand and the primer strand portion of the synthesis strand.

Ligation of the polynucleotide ligation molecule to the scaffold polynucleotide extends the length of the support strand of the double-stranded scaffold polynucleotide of step (1) and wherein the first nucleotide of the predefined nucleotide sequence is incorporated into the support strand of the scaffold polynucleotide.

Ligation of the support strands may be performed by any suitable means. Ligation may typically and preferably be performed by enzymes having ligase activity. For example, ligation may be performed with T3 DNA ligase or T4 DNA ligase or functional variants or equivalents thereof or other enzymes described further herein. The use of such enzymes will result in the maintenance of the single-strand break in the synthesis strand, since the terminal nucleotide of the helper strand is provided such that it cannot act as a substrate for ligase, *e.g.* due to the absence of a terminal phosphate group or presence of a non-ligatable blocking group.

Following ligation of the polynucleotide ligation molecule to the scaffold polynucleotide the first nucleotide of the predefined nucleotide sequence is referred to as occupying nucleotide position n+1, the universal nucleotide is referred to as occupying nucleotide position n+2 and the nucleotide which prior to ligation was the terminal nucleotide of the support strand of the scaffold polynucleotide proximal to the primer strand portion is referred to as occupying nucleotide position n.

### Step 3 - cleavage

In step (3) of the method the ligated scaffold polynucleotide is cleaved (403) at a cleavage site. The cleavage site is defined by a sequence comprising the universal nucleotide in the support strand. Cleavage results in a double-strand break in the scaffold polynucleotide. Cleavage of the scaffold polynucleotide (step 3) results in loss of the helper strand, if present and hybridised to the support strand immediately prior to cleavage, and loss of the support strand comprising the universal nucleotide.

Cleavage of the scaffold polynucleotide thereby releases the polynucleotide ligation molecule from the scaffold polynucleotide but leads to the retention of the first nucleotide of that cycle unpaired and attached to the support strand of the cleaved scaffold polynucleotide. Cleavage of the scaffold polynucleotide leaves in place a cleaved double-stranded scaffold polynucleotide comprising the first nucleotide of the predefined sequence at the terminal end of the support strand. The terminal end of the support strand comprises a double-nucleotide overhang, with two nucleotides overhanging the terminal nucleotide of the primer strand portion of the synthesis strand. Thus the first nucleotide of that cycle which is the terminal nucleotide of the cleaved end of the support strand overhangs the terminal nucleotide of the primer strand portion of the synthesis strand.

The synthesis strand is already provided with a single-strand break or "nick" in this exemplary method, thus only cleavage of the support strand is necessary to provide a double-strand break in the scaffold polynucleotide. To obtain such an overhanging cleaved double-stranded scaffold polynucleotide when the universal nucleotide occupies position n+2 in the support strand, the support strand is cleaved at a specific position relative to the universal nucleotide. When the support strand of the scaffold polynucleotide is cleaved between nucleotide positions n+2 and n+1 the polynucleotide ligation molecule is released from the scaffold polynucleotide (see structure depicted at the top left of the upper part of Figure 4) except that the first nucleotide of that cycle is retained in the scaffold polynucleotide attached to the support strand of the cleaved scaffold polynucleotide.

A phosphate group should continue to be attached to the terminal nucleotide of the support strand of the cleaved scaffold polynucleotide at the cleavage site. This ensures that the support strand of the cleaved scaffold polynucleotide can be ligated to the support strand of the polynucleotide ligation molecule in the ligation step of the next cycle of synthesis. Cleavage is performed so that the terminal nucleotide of the support strand of the cleaved scaffold polynucleotide retains a ligatable group, preferably a terminal phosphate group and such that a phosphorylation step need not therefore be performed.

Thus in method version 3 the universal nucleotide occupies position n+2 in the support strand at steps (2) and (3) and the support strand is cleaved between nucleotide positions n+2 and n+1 at step (3).

Preferably, the support strand is cleaved by cleavage of the phosphodiester bond between nucleotide positions n+2 and n+1 (the first phosphodiester bond of the support strand relative to the position of the universal nucleotide, in the direction distal to the helper strand/proximal to the primer strand).

The support strand may be cleaved by cleavage of one ester bond of the phosphodiester bond between nucleotide positions n+2 and n+1.

Preferably the support strand is cleaved by cleavage of the first ester bond relative to nucleotide position n+2. This will have the effect of retaining a terminal phosphate group on the support strand of the cleaved scaffold polynucleotide at the cleavage position.

Any suitable mechanism may be employed to effect cleavage of the support strand between nucleotide positions n+2 and n+1 when the universal nucleotide occupies position n+2.

Cleavage of the support strand between nucleotide positions n+2 and n+1 as described above may be performed by the action of an enzyme.

Cleavage of the support strand between nucleotide positions n+2 and n+1 as described above may be performed as a two-step cleavage process.

The first cleavage step of a two-step cleavage process may comprise removing the universal nucleotide from the support strand thus forming an abasic site at position n+2, and the second cleavage step may comprise cleaving the support strand at the abasic site, between positions n+2 and n+1.

One mechanism of cleaving the support strand at a cleavage site defined by a sequence comprising a universal nucleotide in the manner outlined above is described in Example 2. The cleavage mechanism described in Example 2 is exemplary and other mechanisms could be employed, provided that the blunt-ended cleaved double-stranded scaffold polynucleotide described above is achieved.

In the first cleavage step of a two-step cleavage process the universal nucleotide is removed from the support strand whilst leaving the sugar-phosphate backbone intact. This can be achieved by the action of an enzyme which can specifically excise a single universal nucleotide from a double-stranded polynucleotide. In the exemplified cleavage methods the universal nucleotide is inosine and inosine is excised from the support strand by the action of an enzyme, thus forming an abasic site. In the exemplified cleavage method the enzyme is a 3-methyladenine DNA glycosylase enzyme, specifically human alkyladenine DNA glycosylase (hAAG). Other enzymes, molecules or chemicals could be used provided that an abasic site is formed. The nucleotide-excising enzyme may be an enzyme which catalyses the release of uracil from polynucleotides, such as Uracil-DNA Glycosylase (UDG).

In the second cleavage step of a two-step cleavage process the support strand is cleaved at the abasic site by making a single-strand break. In the exemplified methods the support strand is cleaved by the action of a chemical which is a base, such as NaOH. Alternatively, an organic chemical such as N,N'-dimethylethylenediamine may be used. Alternatively, enzymes having abasic site lyase activity, such as AP Endonuclease 1, Endonuclease III (Nth), or Endonuclease VIII, may be used. Other enzymes, molecules or chemicals could be used provided that the support strand is cleaved at the abasic site as described.

Thus in embodiments wherein the universal nucleotide is at position n+2 of the support strand at steps (1) and (2) and the support strand is cleaved between positions n+2 and n+1, a first cleavage step may be performed with a nucleotide-excising enzyme. An example of such an enzyme is a 3-methyladenine DNA glycosylase enzyme, such as human alkyladenine DNA glycosylase (hAAG). The second cleavage step may be performed with a chemical which is a base, such as NaOH. The second step may be performed with an organic chemical having abasic site cleavage activity such as N,N'-dimethylethylenediamine. The second step may performed with an enzyme having abasic site lyase activity such as Endonuclease VIII or Endonuclease III.

Cleavage of the support strand between nucleotide positions n+2 and n+1 as described above may also be performed as a one-step cleavage process. Examples of enzymes which may be used in any such process include Endonuclease III, Endonuclease VIII. Other enzymes which may be used in any such process include enzymes which cleave 8-oxoguanosine, such as formamidopyrimidine DNA glycosylase (Fpg) and 8-oxoguanine DNA glycosylase (hOGG1).

### Step 4 - incorporation of a second nucleotide of the predefined sequence

In step (4) of the method, following ligation of the polynucleotide ligation molecule to the scaffold polynucleotide and cleavage of the scaffold polynucleotide, a second nucleotide of the predefined sequence is then incorporated into the synthesis strand by extension of the primer strand portion.

Extension of the primer strand portion may be achieved by the action of any suitable enzyme which possesses the capability of extending an oligonucleotide or a polynucleotide molecule with a single nucleotide. Such an enzyme is typically a nucleotide transferase enzyme or a polymerase enzyme. Any suitable enzyme as defined further herein or known to the skilled person may be used.

The second nucleotide of the predefined sequence which is incorporated during step (4) comprises a reversible terminator group which prevents further extension by the enzyme, or comprises any other analogous functionality which prevents further extension by the enzyme. Any suitable reversible terminator group or functionality as defined further herein or known to the skilled person may be used.

Upon its incorporation into the primer strand portion of the scaffold polynucleotide the second nucleotide of the predefined sequence of that cycle becomes paired with a partner nucleotide to form a nucleotide pair of that cycle. A nucleotide pair may be any suitable nucleotide pair as defined further herein.

### Step 5 - deprotection

In this exemplary method version of the invention, as well as with all versions, to allow the next nucleotide to be incorporated in the next synthesis cycle, the reversible terminator group must be removed from the first nucleotide (deprotection step; 405). Enzyme and residual unincorporated second nucleotides should first be removed following step (5) in order to prevent multiple incorporation of second nucleotides in the same cycle of synthesis.

Removal of the reversible terminator group from the first nucleotide can be performed by any suitable means known to the skilled person. For example, removal can be performed by the use of a chemical, such as tris(carboxyethyl)phosphine (TCEP).

### Further cycles

Following completion of the first cycle of synthesis, second and further cycles of synthesis may be performed using the same method steps.

The cleavage product of steps (3), (4) and (5) of the previous cycle is provided (in step 6) as the double-stranded scaffold polynucleotide for the next cycle of synthesis.

In step (6) of the next and each further cycle of synthesis a further double-stranded polynucleotide ligation molecule is ligated to the cleavage product of steps (3), (4) and (5) of the previous cycle. The polynucleotide ligation molecule may be structured in the same way as described above for step (2) of the previous cycle, except that the polynucleotide ligation molecule comprises a first nucleotide of the further cycle of synthesis. The polynucleotide ligation molecule may be ligated to the cleavage product of steps (3), (4) and (5) of the previous cycle in the same way as described above for step (2).

In step (7) of the next and each further cycle of synthesis the ligated scaffold polynucleotide is cleaved at the cleavage site. Cleavage results in a double-strand break in the scaffold polynucleotide. Cleavage of the scaffold polynucleotide (step 7) results in loss of the helper strand, if present and hybridised to the support strand immediately prior to cleavage, and loss of the support strand comprising the universal nucleotide. Cleavage of the scaffold polynucleotide thereby releases the further polynucleotide ligation molecule from the scaffold polynucleotide but leads to the retention of the first nucleotide of that further cycle unpaired and attached to the support strand of the cleaved scaffold polynucleotide. Cleavage of the scaffold polynucleotide leaves in place a cleaved double-stranded scaffold polynucleotide comprising the first nucleotide of the further cycle at the terminal end of the support strand of the scaffold polynucleotide. The terminal end of the support strand comprises a double-nucleotide overhang, with two nucleotides overhanging the terminal nucleotide of the primer strand portion of the synthesis strand. Thus the first nucleotide of that further cycle which is the terminal nucleotide of the cleaved end of the support strand overhangs the terminal nucleotide of the primer strand portion of the synthesis strand. Cleavage at step (7) may be performed in the same way as described above for step (3).

In step (8) of the next and each further cycle of synthesis the terminal end of the primer strand portion of the synthesis strand of the double-stranded scaffold polynucleotide is further extended by the incorporation of a second nucleotide of the further cycle of synthesis by the action of the nucleotide transferase enzyme, polymerase enzyme or other enzyme. The second nucleotide of the further cycle of synthesis may be incorporated in the same way as described above for step (4).

In step (9) the reversible terminator group is removed from the second nucleotide of the further cycle (deprotection step; 409). Deprotection by removal of the reversible terminator group in the next cycle and further cycles may be performed as described above with respect to the first synthesis cycle.

Synthesis cycles are repeated as described above for as many times as necessary to synthesise the double-stranded polynucleotide having the predefined nucleotide sequence.

### Synthesis Method Version 4

With reference to Figure 5, in a fourth specific non-limiting exemplary version of the synthesis methods of the invention a double-stranded scaffold polynucleotide is provided (step 1 of Figure 5; 501). The double-stranded scaffold polynucleotide comprises a support strand and a synthesis strand hybridised thereto. The synthesis strand comprises a primer strand portion. The double-stranded scaffold polynucleotide is provided with at least one overhanging end, wherein the at least one overhanging end comprises the terminal end of the support strand overhanging the terminal end of the primer strand portion. The terminal nucleotide of the support strand is capable of acting as a substrate for a ligase enzyme, and preferably comprises a phosphate group or other ligatable group.

In step (2) of the methods a polynucleotide ligation molecule (see structure depicted at the top right of the upper part of Figure 5) is ligated to the scaffold polynucleotide. Ligation incorporates a first nucleotide of the predefined sequence into the support strand of the scaffold polynucleotide (step 2 of Figure 5; 502).

In each cycle of synthesis the scaffold polynucleotide is then cleaved at a cleavage site defined by a sequence comprising the universal nucleotide in the support strand (step 3; 504). In method version 4 cleavage comprises cleaving the support strand immediately after the nucleotide which is in the nucleotide position next to the universal nucleotide in the direction proximal to the primer strand portion/distal to the helper strand. Cleavage of the scaffold polynucleotide (step 4) results in release of the polynucleotide ligation molecule from the scaffold polynucleotide and retention of the first nucleotide of that cycle unpaired and attached to the first strand of the cleaved scaffold polynucleotide. Cleavage of the scaffold polynucleotide (step 3) results in loss of the helper strand from the scaffold polynucleotide, if present and hybridised to the support strand immediately prior to cleavage, and loss of the universal nucleotide from the scaffold polynucleotide. Cleavage leaves in place a cleaved double-stranded scaffold polynucleotide comprising, at the site of cleavage, a cleaved terminal end of the support strand and the terminal end of the primer strand portion of the synthesis strand which comprised the nick site prior to cleavage, and wherein the cleaved double-stranded scaffold polynucleotide comprises the first nucleotide of that cycle as the terminal nucleotide of the cleaved end of the support strand overhanging the terminal nucleotide of the primer strand portion of the synthesis strand.

In step (4) of the methods a second nucleotide of the predefined nucleotide sequence is added to the terminal end of the primer strand portion of the synthesis strand by the action of an enzyme having the capability of extending an oligonucleotide or a polynucleotide molecule with a single nucleotide. Such an enzyme is typically a nucleotide transferase enzyme or a polymerase enzyme (step 4 of Figure 5; 503). The second nucleotide is provided with a reversible terminator group which prevents further extension by the enzyme. Thus in step (3) only a single nucleotide is incorporated.

In step (5) of the methods a deprotection step is performed to remove the terminator group from the newly-incorporated nucleotide (step 5 of Figure 5; 505).

### Step 1 - provision of a scaffold polynucleotide

In exemplary version 4 of the synthesis methods of the invention a double-stranded scaffold polynucleotide is provided in step (1) (501). The double-stranded scaffold polynucleotide is provided comprising a synthesis strand and a support strand hybridised thereto, wherein the synthesis strand comprises a primer strand portion. The terminal nucleotide of the support strand comprises a single-nucleotide overhang wherein the terminal nucleotide of the support strand is unpaired and overhangs the terminal nucleotide of the primer strand portion of the synthesis strand. The terminal nucleotide of the support strand is capable of acting as a substrate for a ligase enzyme, and comprises a ligatable group, preferably a phosphate group.

### Step 2 - ligation of a polynucleotide ligation molecule to the scaffold polynucleotide and incorporation of a first nucleotide of the predefined sequence

In step (2) of the method a double-stranded polynucleotide ligation molecule is ligated (502) to the scaffold polynucleotide by the action of a ligase enzyme in a sticky-ended ligation reaction.

The polynucleotide ligation molecule comprises a support strand and a helper strand hybridised thereto. The polynucleotide ligation molecule further comprises a complementary ligation end comprising in the support strand a universal nucleotide and a first nucleotide of the predefined sequence.

The complementary ligation end of the polynucleotide ligation molecule is structured such that the terminal nucleotide of the support strand is the first nucleotide of the predefined sequence to be incorporated into the scaffold polynucleotide in any given cycle of synthesis. The terminal nucleotide of the support strand is paired with the penultimate nucleotide of the helper strand. The complementary ligation end comprises a single-nucleotide overhang, wherein the terminal nucleotide of the helper strand overhangs the terminal nucleotide of the support strand. The terminal nucleotide of the support strand, *i.e.* the first nucleotide of the predefined sequence of that cycle, occupies nucleotide position n+1 in the support strand. By position n it is meant the nucleotide position which will be opposite the second nucleotide of the predefined sequence of that cycle upon incorporation of the second nucleotide. Position n+1 is the next nucleotide position relative to position n in the direction distal to the complementary ligation end. In Figure 5 in step (2) the first nucleotide of the predefined sequence is depicted as guanine, the second nucleotide of the predefined sequence is depicted as thymine, the terminal nucleotide of the helper strand is depicted as thymine and the penultimate nucleotide of the helper strand is depicted as cytosine. These nucleotides are depicted purely for illustration. These nucleotides can be any suitable nucleotides. Nucleotides depicted as X may also be any suitable nucleotides as chosen by the user.

Typically, the terminal nucleotide of the support strand at the complementary ligation end will define the 3' terminus of the support strand of the complementary ligation end of the polynucleotide ligation molecule.

The terminal nucleotide of the helper strand of the polynucleotide ligation molecule is configured such that it cannot be ligated to another polynucleotide in a polynucleotide strand (position labelled "T" in the structure depicted at the top right of the upper part of Figure 5). This nucleotide is referred to as a non-ligatable terminal nucleotide. Typically this terminal nucleotide will lack a phosphate group, *i.e.* it will be a nucleoside. Typically, this terminal nucleotide of the helper strand will define the 5' terminus of the helper strand.

In the complementary ligation end of the polynucleotide ligation molecule the universal nucleotide in the support strand is positioned such that it is paired with a partner nucleotide in the helper strand, and occupies nucleotide position n+3 in the support strand. By position n+3 it is meant the third nucleotide position in the support strand relative to position n in the direction distal to the complementary ligation end.

The overhanging complementary ligation end is configured so that it will compatibly join with the overhanging end of the scaffold polynucleotide when subjected to suitable ligation conditions. Upon ligation of the support strands of the polynucleotide ligation molecule and the scaffold polynucletide, the first nucleotide becomes incorporated into the scaffold polynucletide. Because the terminal nucleotide of the helper strand of the polynucleotide ligation molecule is a non-ligatable nucleotide, the ligase enzyme will be prevented from ligating the helper strand and the primer strand portion of the synthesis strand, thus creating a single-strand break or "nick" between the helper strand and the primer strand portion of the synthesis strand.

Ligation of the polynucleotide ligation molecule to the scaffold polynucleotide extends the length of the support strand of the double-stranded scaffold polynucleotide of step (1) and wherein the first nucleotide of the predefined nucleotide sequence is incorporated into the support strand of the scaffold polynucleotide.

Ligation of the support strands may be performed by any suitable means. Ligation may typically and preferably be performed by enzymes having ligase activity. For example, ligation may be performed with T3 DNA ligase or T4 DNA ligase or functional variants or equivalents thereof or other enzymes described further herein. The use of such enzymes will result in the maintenance of the single-strand break in the synthesis strand, since the terminal nucleotide of the helper strand is provided such that it cannot act as a substrate for ligase, *e.g.* due to the absence of a terminal phosphate group or presence of a non-ligatable blocking group.

Following ligation of the polynucleotide ligation molecule to the scaffold polynucleotide the first nucleotide of the predefined nucleotide sequence is referred to as occupying nucleotide position n+1, the universal nucleotide is referred to as occupying nucleotide position n+3 and the nucleotide which prior to ligation was the terminal nucleotide of the support strand of the scaffold polynucleotide proximal to the primer strand portion is referred to as occupying nucleotide position n.

### Step 3 - cleavage

In step (3) of the method the ligated scaffold polynucleotide is cleaved (504) at a cleavage site. The cleavage site is defined by a sequence comprising the universal nucleotide in the support strand. Cleavage results in a double-strand break in the scaffold polynucleotide. Cleavage of the scaffold polynucleotide (step 3) results in loss of the helper strand, if present and hybridised to the support strand immediately prior to cleavage, and loss of the support strand comprising the universal nucleotide.

Cleavage of the scaffold polynucleotide thereby releases the polynucleotide ligation molecule from the scaffold polynucleotide but leads to the retention of the first nucleotide of that cycle unpaired and attached to the support strand of the cleaved scaffold polynucleotide. Cleavage of the scaffold polynucleotide leaves in place a cleaved double-stranded scaffold polynucleotide comprising the first nucleotide of the predefined sequence at the terminal end of the support strand. The terminal end of the support strand comprises a double-nucleotide overhang, with two nucleotides overhanging the terminal nucleotide of the primer strand portion of the synthesis strand. Thus the first nucleotide of that cycle which is the terminal nucleotide of the cleaved end of the support strand overhangs the terminal nucleotide of the primer strand portion of the synthesis strand.

The synthesis strand is already provided with a single-strand break or "nick" in this exemplary method, thus only cleavage of the support strand is necessary to provide a double-strand break in the scaffold polynucleotide. To obtain such an overhanging cleaved double-stranded scaffold polynucleotide when the universal nucleotide occupies position n+3 in the support strand, the support strand is cleaved at a specific position relative to the universal nucleotide. When the support strand of the scaffold polynucleotide is cleaved between nucleotide positions n+2 and n+1 the polynucleotide ligation molecule is released from the scaffold polynucleotide (see structure depicted at the top left of the upper part of Figure 5) except that the first nucleotide of that cycle is retained in the scaffold polynucleotide attached to the support strand of the cleaved scaffold polynucleotide.

A phosphate group should continue to be attached to the terminal nucleotide of the support strand of the cleaved scaffold polynucleotide at the cleavage site. This ensures that the support strand of the cleaved scaffold polynucleotide can be ligated to the support strand of the polynucleotide ligation molecule in the ligation step of the next cycle of synthesis. Cleavage is performed so that the terminal nucleotide of the support strand of the cleaved scaffold polynucleotide retains a ligatable group, preferably a terminal phosphate group and such that a phosphorylation step need not therefore be performed.

Thus in method version 4 the universal nucleotide occupies position n+3 in the support strand at steps (2) and (3) and the support strand is cleaved between nucleotide positions n+2 and n+1 at step (3).

Preferably, the support strand is cleaved by cleavage of the phosphodiester bond between nucleotide positions n+2 and n+1 (the first phosphodiester bond of the support strand relative to the position of the universal nucleotide, in the direction distal to the helper strand/proximal to the primer strand).

The support strand may be cleaved by cleavage of one ester bond of the phosphodiester bond between nucleotide positions n+2 and n+1.

Preferably the support strand is cleaved by cleavage of the first ester bond relative to nucleotide position n+2. This will have the effect of retaining a terminal phosphate group on the support strand of the cleaved scaffold polynucleotide at the cleavage position.

Any suitable mechanism may be employed to effect cleavage of the support strand between nucleotide positions n+2 and n+1 when the universal nucleotide occupies position n+3.

Cleavage of the support strand between nucleotide positions n+2 and n+1 as described above may be performed by the action of an enzyme.

Cleavage of the support strand between nucleotide positions n+2 and n+1 when the universal nucleotide occupies position n+3 in the support strand, as described above, may be performed by the action of an enzyme such as Endonuclease V.

One mechanism of cleaving the support strand between nucleotide positions n+2 and n+1 at a cleavage site defined by a sequence comprising a universal nucleotide which is occupying position n+3 in the support strand is described in analogous fashion in Example 3. The mechanism described is exemplary and other mechanisms could be employed, provided that the cleavage arrangement described above is achieved.

In this exemplary mechanism an endonuclease enzyme is employed. In the exemplified method the enzyme is Endonuclease V. Other enzymes, molecules or chemicals could be used provided that the support strand is cleaved between nucleotide positions n+2 and n+1 when the universal nucleotide occupies position n+3 in the support strand.

### Step 4 - incorporation of a second nucleotide of the predefined sequence

In step (4) of the method, following ligation of the polynucleotide ligation molecule to the scaffold polynucleotide a second nucleotide of the predefined sequence is then incorporated into the synthesis strand by extension of the primer strand portion.

Extension of the primer strand portion may be achieved by the action of any suitable enzyme which possesses the capability of extending an oligonucleotide or a polynucleotide molecule with a single nucleotide. Such an enzyme is typically a nucleotide transferase enzyme or a polymerase enzyme. Any suitable enzyme as defined further herein or known to the skilled person may be used.

The second nucleotide of the predefined sequence which is incorporated during step (4) comprises a reversible terminator group which prevents further extension by the enzyme, or comprises any other analogous functionality which prevents further extension by the enzyme. Any suitable reversible terminator group or functionality as defined further herein or known to the skilled person may be used.

Upon its incorporation into the primer strand portion of the scaffold polynucleotide the second nucleotide of the predefined sequence of that cycle becomes paired with a partner nucleotide to form a nucleotide pair of that cycle. A nucleotide pair may be any suitable nucleotide pair as defined further herein.

### Step 5 - deprotection

**In** this exemplary method version of the invention, as well as with all versions, to allow the next nucleotide to be incorporated in the next synthesis cycle, the reversible terminator group must be removed from the first nucleotide (deprotection step; 505). Enzyme and residual unincorporated second nucleotides should first be removed following step (5) in order to prevent multiple incorporation of second nucleotides in the same cycle of synthesis.

Removal of the reversible terminator group from the first nucleotide can be performed by any suitable means known to the skilled person. For example, removal can be performed by the use of a chemical, such as tris(carboxyethyl)phosphine (TCEP).

### Further cycles

Following completion of the first cycle of synthesis, second and further cycles of synthesis may be performed using the same method steps.

The cleavage product of steps (3), (4) and (5) of the previous cycle is provided (in step 6) as the double-stranded scaffold polynucleotide for the next cycle of synthesis.

In step (6) of the next and each further cycle of synthesis a further double-stranded polynucleotide ligation molecule is ligated to the cleavage product of steps (3), (4) and (5) of the previous cycle. The polynucleotide ligation molecule may be structured in the same way as described above for step (2) of the previous cycle, except that the polynucleotide ligation molecule comprises a first nucleotide of the further cycle of synthesis. The polynucleotide ligation molecule may be ligated to the cleavage product of steps (3), (4) and (5) of the previous cycle in the same way as described above for step (2).

In step (7) of the next and each further cycle of synthesis the ligated scaffold polynucleotide is cleaved at the cleavage site. Cleavage results in a double-strand break in the scaffold polynucleotide. Cleavage of the scaffold polynucleotide (step 7) results in loss of the helper strand, if present and hybridised to the support strand immediately prior to cleavage, and loss of the support strand comprising the universal nucleotide. Cleavage of the scaffold polynucleotide thereby releases the further polynucleotide ligation molecule from the scaffold polynucleotide but leads to the retention of the first nucleotide of that further cycle unpaired and attached to the support strand of the cleaved scaffold polynucleotide. Cleavage of the scaffold polynucleotide leaves in place a cleaved double-stranded scaffold polynucleotide comprising the first nucleotide of the further cycle at the terminal end of the support strand of the scaffold polynucleotide. The terminal end of the support strand comprises a double-nucleotide overhang, with two nucleotides overhanging the terminal nucleotide of the primer strand portion of the synthesis strand. Thus the first nucleotide of that further cycle which is the terminal nucleotide of the cleaved end of the support strand overhangs the terminal nucleotide of the primer strand portion of the synthesis strand. Cleavage at step (7) may be performed in the same way as described above for step (3).

In step (8) of the next and each further cycle of synthesis the terminal end of the primer strand portion of the synthesis strand of the double-stranded scaffold polynucleotide is further extended by the incorporation of a second nucleotide of the further cycle of synthesis by the action of the nucleotide transferase enzyme, polymerase enzyme or other enzyme. The second nucleotide of the further cycle of synthesis may be incorporated in the same way as described above for step (4).

In step (9) the reversible terminator group is removed from the second nucleotide of the further cycle (deprotection step; 509). Deprotection by removal of the reversible terminator group in the next cycle and further cycles may be performed as described above with respect to the first synthesis cycle.

Synthesis cycles are repeated as described above for as many times as necessary to synthesise the double-stranded polynucleotide having the predefined nucleotide sequence.

### Variants of Synthesis Method Version 4

In addition to the above-described methods, variants of synthesis method version 4 are provided wherein the method is performed in the same way as synthesis method version 4 described above except for the following variations.

In the ligation step of the first cycle (step 2) the complementary ligation end of the polynucleotide ligation molecule is structured such that the universal nucleotide instead occupies nucleotide position n+3+x in the support strand and is paired with a partner nucleotide in the helper strand which is 3+x positions removed from the terminal nucleotide of the helper strand at the complementary ligation end; wherein nucleotide position n+3 is the third nucleotide position in the support strand relative to nucleotide position n in the direction distal to the complementary ligation end.

In the cleavage step of the first cycle (step 3) the universal nucleotide instead occupies nucleotide position n+3+x in the support strand of the scaffold polynucleotide, wherein nucleotide position n+3 is the third nucleotide position in the support strand relative to nucleotide position n in the direction proximal to the helper strand/distal to the primer strand portion; and the support strand of the scaffold polynucleotide is cleaved between positions n+2 and n+1.

In the ligation step of the second cycle (step 6) and in ligation steps of all further cycles the complementary ligation end of the polynucleotide ligation molecule is structured such that the universal nucleotide occupies nucleotide position n+3+x in the support strand and is paired with a partner nucleotide which is 3+x positions removed from the terminal nucleotide of the helper strand at the complementary ligation end; wherein nucleotide position n+3 is the third nucleotide position in the support strand relative to nucleotide position n in the direction distal to the complementary ligation end.

Finally, in the cleavage step of the second cycle (step 7) and in cleavage steps of all further cycles the universal nucleotide instead occupies nucleotide position n+3+x in the support strand of the scaffold polynucleotide, wherein nucleotide position n+3 is the third nucleotide position in the support strand relative to nucleotide position n in the direction proximal to the helper strand/distal to the primer strand portion; and the support strand of the scaffold polynucleotide is cleaved between positions n+2 and n+1.

In all of these variant methods, x is a whole number from 1 to 10 or more, and wherein x is the same whole number in steps (2), (3), (6) and (7) of a given series of cycles.

As with synthesis method version 4, with variant methods based on version 4 it will be noted that in any given cycle the nucleotide position occupied by the first nucleotide of that cycle in the support strand following ligation and during the incorporation and cleavage steps is defined as nucleotide position n+1, and upon completion of the given cycle of synthesis the position occupied by the first nucleotide of that cycle in the support strand of the cleaved scaffold polynucleotide is defined as nucleotide position n in the next cycle of synthesis.

Thus in these particular variants of synthesis method version 4 the position of the cleavage site relative to nucleotide position n is held constant, and the position of the universal nucleotide relative to nucleotide position n is increased by moving the position of the universal nucleotide in the direction proximal to the helper strand/distal to the primer strand portion by a number of nucleotide positions determined by the number selected for x.

A diagrammatic representation of these variant methods is provided in Figure 6.

### Further Variants of Synthesis Method Versions 3 and 4.

In addition to the above-described variant, methods which are further variants of synthesis method versions 3 and 4 are provided. These methods are performed in the same way as synthesis method versions 3 and 4 described above except for the variations described below. These further variants of synthesis method versions 3 and 4 are performed according to the same generic format which is described immediately below. The difference between the further variants of synthesis method version 3 and the further variants of synthesis method version 4 is in the positioning of the universal nucleotide relative to nucleotide position n.

### General Format for Further Variants of Synthesis Method Versions 3 and 4.

In step (1) of a first cycle the scaffold polynucleotide is provided such that the terminal end of the support strand of the scaffold polynucleotide proximal to the primer strand portion comprises a multiple-nucleotide overhang comprising 1+y nucleotides. The number selected for y is a whole number which is one or more. Thus the overhang comprises two or more nucleotides. The 1+y nucleotides of the support strand overhang the terminal nucleotide of the primer strand portion.

The nucleotide which is referred to as the first nucleotide of the overhang occupies a position in the overhang distal to the terminal end of the overhang. In other words, it is the unpaired nucleotide occupying the nucleotide position in the support strand next to the nucleotide which is the partner nucleotide for the terminal nucleotide of the primer strand portion. The second nucleotide of the overhang is the unpaired nucleotide occupying the nucleotide position next to the first nucleotide of the overhang in the direction proximal to the terminal end of the overhang. Thus in the case of the minimum overhang length of two nucleotides, the second nucleotide of the overhang is the terminal nucleotide of the overhanging support strand. The first nucleotide of the overhang occupies nucleotide position n and is the partner nucleotide for the second nucleotide of that first cycle incorporated in step (3). The second nucleotide of the overhang occupies nucleotide position n+1 and is the partner nucleotide for the second nucleotide of the next/second cycle of synthesis incorporated in step (7).

In step (2) of the first cycle the complementary ligation end of the polynucleotide ligation molecule is provided such that the terminal end of the helper strand also comprises a multiple-nucleotide overhang comprising 1+y nucleotides, wherein y is a whole number which is one or more. Thus the overhang in the complementary ligation end also comprises two or more nucleotides. The 1+y nucleotides of the helper strand of the complementary ligation end overhang the terminal nucleotide of the support strand and are partner nucleotides for the 1+y overhanging nucleotides of the support strand of the scaffold polynucleotide.

In any given cycle of synthesis the number selected for y is preferably the same number in respect of the overhangs of both the scaffold polynucleotide and the complementary ligation end. If so, when the polynucleotide ligation molecule is ligated to the scaffold polynucleotide all previously overhanging nucleotides will form pairs with corresponding partner nucleotides. If the number selected for y is a different number in respect of the overhangs of both the scaffold polynucleotide and the complementary ligation end, the number selected for y in the complementary ligation end of the polynucleotide ligation molecule is preferably less than the number selected for y in the scaffold polynucleotide. If so, when the polynucleotide ligation molecule is ligated to the scaffold polynucleotide a portion of overhanging nucleotides will form pairs with corresponding partner nucleotides. This will leave one or more of the nucleotides of the support strand of the scaffold polynucleotide adjacent the nick site unpaired. This may facilitate the transferase, polymerase or other enzyme gaining access to the terminal nucleotide of the primer strand portion during the incorporation/extention step.

The terminal nucleotide of the support strand of the complementary ligation end is the first nucleotide of that cycle, occupies nucleotide position n+2+x and is a partner nucleotide in a different nucleotide pair formed in a third cycle of synthesis. The number selected for x is a whole number which is zero or more.

The number selected for x is the number selected for y in respect of the scaffold polynucleotide minus one. Thus where y=1 in respect of the overhang in the scaffold polynucleotide, the length of the overhang in the scaffold polynucleotide is 2. The terminal nucleotide of the scaffold polynucleotide occupies position n+1. If y=1 then x=0. Thus the terminal nucleotide of the support strand of the complementary ligation end, *i.e.* the first nucleotide of the predefined sequence of that cycle, occupies nucleotide position n+2. Where y=2 in respect of the overhang in the scaffold polynucleotide, the length of the overhang in the scaffold polynucleotide is 3. The terminal nucleotide of the scaffold polynucleotide occupies position n+2. If y=2 then x=1. Thus the terminal nucleotide of the support strand of the complementary ligation end, *i.e.* the first nucleotide of the predefined sequence of that cycle, occupies nucleotide position n+3, and so on and so forth.

As with step (2), in step (6) the scaffold polynucleotide is provided such that the terminal end of the support strand of the scaffold polynucleotide proximal to the primer strand portion comprises a multiple-nucleotide overhang comprising 1+y nucleotides, wherein y is a whole number which is one or more. Thus the overhang comprises two or more nucleotides. The 1+y nucleotides of the support strand overhang the terminal nucleotide of the primer strand portion.

The nucleotide which is referred to as the first nucleotide of the overhang occupies a position in the overhang distal to the terminal end of the overhang. The second nucleotide of the overhang is the unpaired nucleotide occupying the nucleotide position next to the first nucleotide of the overhang in the direction proximal to the terminal end of the overhang. Thus in the case of the minimum overhang length of two nucleotides, the second nucleotide of the overhang is the terminal nucleotide of the overhanging support strand. The first nucleotide of the overhang occupies nucleotide position n and is the partner nucleotide for the second nucleotide of the current/second cycle incorporated in step (8). The second nucleotide of the overhang occupies nucleotide position n+1 and is the partner nucleotide for the second nucleotide of the next/third cycle of synthesis.

In step (6) the complementary ligation end of the polynucleotide ligation molecule is provided such that the terminal end of the helper strand also comprises a multiple-nucleotide overhang comprising 1+y nucleotides, wherein y is a whole number which is one or more. Thus the overhang in the complementary ligation end also comprises two or more nucleotides. The 1+y nucleotides of the helper strand of the complementary ligation end overhang the terminal nucleotide of the support strand and are partner nucleotides for the 1+y overhanging nucleotides of the support strand of the scaffold polynucleotide.

As with step (2), in any given further cycle of synthesis the number selected for y in step (6) is preferably the same number in respect of the overhangs of both the scaffold polynucleotide and the complementary ligation end. If the number selected for y is a different number in respect of the overhangs of both the scaffold polynucleotide and the complementary ligation end, the number selected for y in the complementary ligation end of the polynucleotide ligation molecule is preferably less than the number selected for y in the scaffold polynucleotide.

The terminal nucleotide of the support strand of the complementary ligation end is the first nucleotide of that cycle, occupies nucleotide position n+2+x and is a partner nucleotide in a different nucleotide pair formed in a fourth or further cycle of synthesis. The number selected for x is a whole number which is zero or more.

As indicated previously, in any given cycle or series of further cycles the number selected for x is the number selected for y in respect of the scaffold polynucleotide minus one. Thus where y=1 in respect of the overhang in the scaffold polynucleotide, the length of the overhang in the scaffold polynucleotide is 2. The terminal nucleotide of the scaffold polynucleotide occupies position n+1. If y=1 then x=0. Thus the terminal nucleotide of the support strand of the complementary ligation end, *i.e.* the first nucleotide of the predefined sequence of that cycle, occupies nucleotide position n+2. Where y=2 in respect of the overhang in the scaffold polynucleotide, the length of the overhang in the scaffold polynucleotide is 3. The terminal nucleotide of the scaffold polynucleotide occupies position n+2. If y=2 then x=1. Thus the terminal nucleotide of the support strand of the complementary ligation end, *i.e.* the first nucleotide of the predefined sequence of that cycle, occupies nucleotide position n+3, and so on and so forth.

It will be noted that in any given cycle the nucleotide position occupied by the first nucleotide of that cycle in the support strand following ligation and during the incorporation and cleavage steps is defined as nucleotide position n+2+x, and upon completion of the given cycle of synthesis the position occupied by that first nucleotide of that cycle in the support strand of the cleaved scaffold polynucleotide is defined as nucleotide position n+1 in the next cycle of synthesis in the case of a double-nucleotide overhang in the support strand of the scaffold polynucleotide, or is defined as nucleotide position n+2 in the next cycle of synthesis in the case of a triple-nucleotide overhang in the support strand of the scaffold polynucleotide and so on and so forth.

Thus in these particular variants of synthesis method versions 3 and 4, the position of the universal nucleotide and the position of the cleavage site relative to nucleotide position n is increased by moving the position of the universal nucleotide and the position of the cleavage site in the direction proximal to the helper strand/distal to the primer strand portion by a number of nucleotide positions determined by the number selected for x.

### Specific Format for Further Variants of Synthesis Method Version 3.

The invention additionally provides a specific further variant of synthesis method version 3. This variant method is performed in accordance with the general format for further variants of synthesis method versions 3 and 4 described above, together with the following additional feature.

In both first and further cycles, in both the polynucleotide ligation molecule and in the ligated scaffold polynucleotide the universal nucleotide occupies position n+3+x and the scaffold polynucleotide is cleaved between positions n+3+x and n+2+x.

A diagrammatic representation of these variant methods is provided in Figure 7. In this Figure the presence of the vertical double-lined structures represent the possibility of the presence of one or more futher nucleotides in both the overhanging support strand of the scaffold polynucleotide and the overhanging support strand of the complementary ligation end of the polynucleotide ligation molecule. The terminal nucleotide of the overhanging support strand of the scaffold polynucleotide possesses a terminal ligatable group, such as a terminal phosphate group.

### Specific Format for Further Variants of Synthesis Method Version 4.

The invention additionally provides a specific further variant of synthesis method version 4. This variant method is performed in accordance with the general format for further variants of synthesis method versions 3 and 4 described above, together with the following additional feature.

In both first and further cycles, in both the polynucleotide ligation molecule and in the ligated scaffold polynucleotide the universal nucleotide occupies position n+4+x and the scaffold polynucleotide is cleaved between positions n+3+x and n+2+x.

A diagrammatic representation of these variant methods is provided in Figure 8. In this Figure the presence of the vertical double-lined structures represent the possibility of the presence of one or more futher nucleotides in both the overhanging support strand of the scaffold polynucleotide and the overhanging support strand of the complementary ligation end of the polynucleotide ligation molecule. The terminal nucleotide of the overhanging support strand of the scaffold polynucleotide possesses a terminal ligatable group, such as a terminal phosphate group.

### Variants of Synthesis Method Versions 1, 2, 3 and 4 involving incorporation of more than two nucleotides per cycle

The invention further provides yet further additional variant methods wherein more than two nucleotides are incorporated per cycle, including yet futher additional variant methods based on specific versions 1, 2, 3 and 4 and the variants thereof described above. Any of these yet further variant methods may be performed as described above except that the following modifications may be made.

In step (2) the polynucleotide ligation molecule is provided with a complementary ligation end comprising a first nucleotide of the predefined sequence of the first cycle and further comprising one or more further nucleotides of the predefined sequence of the first cycle. The polynucleotide ligation molecule is then ligated to the scaffold polynucleotide. The first nucleotide of the predefined sequence of the first cycle is the terminal nucleotide of the support strand of the complementary ligation end. The complementary ligation end is preferably structured such that the first and further nucleotides of the predefined sequence of the first cycle comprise a linear sequence of nucleotides wherein each nucleotide in the sequence occupies the next nucleotide position in the support strand in the direction distal to the complementary ligation end.

The complementary ligation end of the polynucleotide ligation molecule is structured such that in step (3) prior to cleavage the universal nucleotide occupies a position in the support strand which is after the nucleotide positions of the first and further nucleotides in the direction distal to the complementary ligation end.

In step (3) following cleavage the first and further nucleotides of the predefined sequence of the first cycle are retained in the cleaved scaffold polynucleotide.

In step (4) the terminal end of the primer strand portion of the synthesis strand of the double-stranded scaffold polynucleotide is extended by the incorporation of a second nucleotide of the predefined sequence of the first cycle by the action of the nucleotide transferase or polymerase enzyme, and wherein the terminal end of the primer strand portion is further extended by the incorporation of one or more further nucleotides of the predefined sequence of the first cycle by the action of the nucleotide transferase or polymerase enzyme, wherein each one of the second and further nucleotides of the first cycle comprises a reversible terminator group which prevents further extension by the enzyme, and wherein following each further extension the reversible terminator group is removed from a nucleotide in a deprotection step (step 5) before the incorporation of the next nucleotide.

In step (6) the polynucleotide ligation molecule is provided with a complementary ligation end comprising a first nucleotide of the predefined sequence of the further cycle and further comprising one or more further nucleotides of the predefined sequence of the further cycle. As in step (2) the polynucleotide ligation molecule is then ligated to the scaffold polynucleotide. As in step (2) the first nucleotide of the predefined sequence of the further cycle is the terminal nucleotide of the support strand of the complementary ligation end. The complementary ligation end is preferably structured such that the first and further nucleotides of the predefined sequence of the further cycle comprise a linear sequence of nucleotides wherein each nucleotide in the sequence occupies the next nucleotide position in the support strand in the direction distal to the complementary ligation end.

In step (6) the terminal end of the primer strand portion of the synthesis strand of the double-stranded scaffold polynucleotide is extended by the incorporation of a second nucleotide of the predefined sequence of the further cycle by the action of the nucleotide transferase or polymerase enzyme, and wherein the terminal end of the primer strand portion is further extended by the incorporation of one or more further nucleotides of the predefined sequence of the further cycle by the action of the nucleotide transferase or polymerase enzyme, wherein each one of the second and further nucleotides of the further cycle comprises a reversible terminator group which prevents further extension by the enzyme, and wherein following each further extension the reversible terminator group is removed from a nucleotide before the incorporation of the next nucleotide.

In step (7) following cleavage the first and further nucleotides of the predefined sequence of the further cycle are retained in the cleaved scaffold polynucleotide.

In step (8) the terminal end of the primer strand portion of the synthesis strand of the double-stranded scaffold polynucleotide is extended by the incorporation of a second nucleotide of the predefined sequence of the further cycle by the action of the nucleotide transferase or polymerase enzyme, and wherein the terminal end of the primer strand portion is further extended by the incorporation of one or more further nucleotides of the predefined sequence of the further cycle by the action of the nucleotide transferase or polymerase enzyme, wherein each one of the second and further nucleotides of the further cycle comprises a reversible terminator group which prevents further extension by the enzyme, and wherein following each further extension the reversible terminator group is removed from a nucleotide before the incorporation of the next nucleotide.

In any of these variant methods the complementary ligation end of the polynucleotide ligation molecule is structured such that in steps (3) and (7) prior to cleavage the universal nucleotide occupies a position in the support strand which is the next nucleotide position in the support strand after the nucleotide positions of the first and further nucleotides in the direction distal to the complementary ligation end, and the support strand is cleaved between the position occupied by the last further nucleotide and the position occupied by the universal nucleotide.

In these variant methods in any given cycle of synthesis, prior to cleavage, in the support strand the position occupied by the first nucleotide of the predefined sequence may be referred to as position n, the position occupied by the first further nucleotide of the predefined sequence in the support strand may be referred to as position n+1, and the position occupied by the universal nucleotide may be referred to as position n+1+x wherein x is a whole number from zero to 10 or more, wherein x is zero if the support strand comprises only one further nucleotide and wherein x is one if the support strand comprises only two further nucleotides, and so on and so forth.

A diagrammatic representation of these variant methods is provided in Figure 9 and is based on an adaptation of synthesis method of the invention version 1 as described above. Thus the depicted method comprises a blunt-ended ligation reaction.

A further diagrammatic representation of these variant methods is provided in Figure 10 and is based on an adaptation of synthesis method of the invention version 3 as described above. Thus the depicted method comprises a sticky-ended ligation reaction.

In any of these variant methods the complementary ligation end of the polynucleotide ligation molecule is alternatively structured such that in steps (3) and (7) prior to cleavage the universal nucleotide occupies a position in the support strand which is the next+1 nucleotide position in the support strand after the nucleotide positions of the first and further nucleotides in the direction distal to the complementary ligation end, and the support strand is alternatively cleaved between the position occupied by the last further nucleotide and the position occupied by the next nucleotide in the support strand (*i.e.* distal to the universal nucleotide).

In these variant methods in any given cycle of synthesis, prior to cleavage, in the support strand the position occupied by the first nucleotide of the predefined sequence may be referred to as position n, the position occupied by the first further nucleotide of the predefined sequence in the support strand may be referred to as position n+1, and the position occupied by the universal nucleotide may be referred to as position n+1+x wherein x is a whole number from zero to 10 or more, wherein x is zero if the support strand comprises only one further nucleotide and wherein x is one if the support strand comprises only two further nucleotides, and so on and so forth.

The general variant scheme depicted in Figure 9 based upon an adaptation of synthesis method of the invention version 1 as described above may equally be adapted such that the support strand is alternatively cleaved between the position occupied by the last further nucleotide in the support strand and the position occupied by the next nucleotide in the support strand (*i.e.* distal to the universal nucleotide). Such an adaptation would therefore be based upon synthesis method of the invention version 2 as described above.

The general variant scheme depicted in Figure 10 based upon an adaptation of synthesis method of the invention version 3 as described above may equally be adapted such that the support strand is alternatively cleaved between the position occupied by the last further nucleotide in the support strand and the position occupied by the next nucleotide in the support strand distal to the universal nucleotide. Such an adaptation would therefore be based upon synthesis method of the invention version 4 as described above.

It will be appreciated that in order to accommodate the incorporation of further nucleotides during the first and further cycles of synthesis, in addition to each first and second nucleotides, routine adaptations may need to be made to the configuration of the complementary ligation end of the polynucleotide ligation molecule in terms of the placement of the universal nucleotide relative to nucleotide position n. In all methods of the invention described and defined herein nucleotide position n is invariably the nucleotide position in the support strand which is or will be opposite the second nucleotide of the predefined sequence of any given cycle prior to or upon its incorporation. Thus the skilled person is readily able to make routine modifications to the positioning of the universal nucleotide and the selection of the cleavage site relative to nucleotide position n in order to adapt any of the method versions and variants thereof to accommodate the incorporation of further nucleotides of the first and further cycles of synthesis.

### EXAMPLES

The following Examples provide support for the methods for synthesising a polynucleotide or oligonucleotide according to the invention, as well as exemplary constructs used in the methods. The Examples do not limit the invention.

Other than Example 13, the following Examples describe synthesis methods according to reaction schemes which are related to but which are not within the scope of the synthesis methods according to the invention.

The Examples demonstrate the ability to perform synthesis reactions which involve steps of addition of a nucleotide of a predefined sequence to the synthesis strand of a scaffold polynucleotide, cleavage of the scaffold polynucleotide at a cleavage site defined by a universal nucleotide and ligation of a polynucleotide ligation molecule which comprises a partner nucleotide for the added nucleotide of the predefined sequence as well as a new universal nucleotide for use in creating a cleavage site for use in the next cycle of synthesis. The methods of the present invention incorporate these steps in a modified manner. Thus other than Example 13 the following Examples provide illustrative support for the methods of the invention defined herein. Example 13 provides data relating to incorporation of 3'-*O*-modified-dNTPs by Therminator X DNA polymerase using an incorporation step according to methods of the invention, *e.g*. synthesis method versions of the invention 1 to 4 and variants thereof (Figures 1 to 10).

In the following Examples, and in corresponding Figures 17 to 55, references to synthesis method "versions 1, 2 and 3" or "version 1, 2 or 3 chemistry" etc. are to be interpreted according to the reaction schematics set out respectively in Figures 11, 12 and 13 and not according to the reaction schematics set out in any of Figures 1 to 10 or descriptions of the same herein. Example 13 and Figure 56 are to be interpreted according to synthesis methods the invention. In particular according to synthesis methods of the invention 1, 2, 3 and 4 and variants thereof and associated reaction schematics set out in Figures 1 to 10, and more particularly incorporation step 4 of such methods.

### Example 1. Synthesis in the Absence of a Helper Strand.

This example describes the synthesis of polynucleotides using 4 steps: incorporation of 3'-O-modified dNTPs on partial double-stranded DNA, cleavage, ligation and deprotection, with the first step taking place opposite a universal nucleotide, in this particular case inosine.

### Step1: Incorporation

The first step describes controlled addition of a 3'-O-protected single nucleotide to an oligonucleotide by enzymatic incorporation by DNA polymerase (Figure 17a).

### Materials and Methods

### Materials

1. 3'-O-modified dNTPs were synthesised in-house according to the protocol described in PhD thesis Jian Wu: Molecular Engineering of Novel Nucleotide Analogues for DNA Sequencing by Synthesis, Columbia University, 2008. The protocol for synthesis is also described in the patent application publication: J. William Efcavitch, Juliesta E. Sylvester, Modified Template-Independent Enzymes for Polydeoxynucleotide Synthesis, Molecular Assemblies US2016/0108382A1.
2. Oligonucleotides were designed in house and obtained from Sigma-Aldrich (Figure 17h). The stock solutions were prepared at a concentration of 100 µM.
3. Therminator IX DNA polymerase was used that has been engineered by New England BioLabs with enhanced ability to incorporate 3-O-modified dNTPs. However, any DNA polymerase that could incorporate modified dNTPs could be used.

Two types of reversible terminators were tested:

| | |
|---|---|
| 3'-*O*-azidomethyl-dTTP: | 3'-*O*-allyl-dTTP: |
| 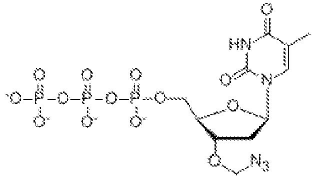 | 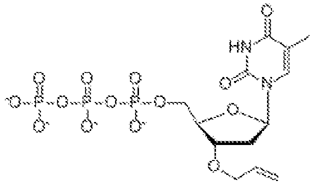 |

### Methods

1. 2 µl of 10x Thermopol^{®} buffer (20 mM Tris-HCl, 10 mM (NH₄)₂SO₄, 10 mM KCl, 2 mM MgSO₄, 0.1% Triton^{®} X-100, pH 8.8, New England BioLabs) was mixed with 12.25 µl of sterile deionized water (ELGA VEOLIA) in 1.5ml Eppendorf tube.
2. 0.5 µl of 10 µM primer (synthesised strand) (5 pmol, 1 equiv) (SEQ ID NO: 1, Figure 17h) and 0.75 µl of 10 µM template (support strand) (6 pmol, 1.5equiv) (SEQ ID NO: 2, Figure 17h) were added to the reaction mixture.
3. 3'-O-modified-dTTP (2 µl of 100 µM) and MnCl₂ (1 µl of 40 mM) were added.
4. 1.5 µl of Therminator IX DNA polymerase (15 U, New England BioLabs) was then added. However, any DNA polymerase that could incorporate modified dNTPs could be used.
5. The reaction was incubated for 20 minutes at 65°C.
6. The reaction was stopped by addition of TBE-Urea sample buffer (Novex).
7. The reaction was separated on polyacrylamide gel (15%) with TBE buffer and visualized by ChemiDoc MP imaging system (BioRad).

Gel Electrophoresis and DNA Visualization:
1. 5 µl of reaction mixture was added to 5 µl of TBE-Urea sample buffer (Novex) in a sterile 1.5ml Eppendorf tube and heated to 95°C for 5 minutes using a heat ThermoMixer (Eppendorf).
2. 5 µl of the sample was then loaded into the wells of a 15% TBE-Urea gel 1.0 mm x 10 well (Invitrogen) which contained preheated 1X TBE buffer Thermo Scientific (89mM Tris, 89mM boric acid and 2mM EDTA).
3. X-cell sure lock module (Novex) was fastened in place and electrophoresis performed at the following conditions; 260V, 90 Amps for 40 minutes at room temperature.
4. The gel was visualized by ChemiDoc MP (BioRad) using Cy3 LEDS. Visualization and analysis was carried out on the Image lab 2.0 platform.

### Results

Customised engineered Therminator IX DNA polymerase from New England BioLabs is an efficient DNA polymerase able to incorporate 3'-*O*-modified-dNTPs opposite a universal nucleotide *e.g*. inosine (Figure 17b-c).

Efficient incorporation opposite inosine occurred at a temperature of 65°C (Figure 17d-e). Incorporation of 3'-*O*-modified-dTTPs opposite inosine requires the presence of Mn²⁺ ions (Figure 17f-g). Successful conversion is marked in bold in Figures 17 c, e, g and h.

### Conclusion

Incorporation of 3-*O*-modified-dTTPs opposite inosine can be achieved with particularly high efficiency using customized engineered Therminator IX DNA polymerase from New England BioLabs, in the presence of Mn²⁺ ions and at a temperature at 65°C.

### Step2: Cleavage

The second step describes a two-step cleavage of polynucleotides with either hAAG/Endo VIII or hAAG/chemical base (Figure 18a).

### Materials and Methods

### Materials

1. Oligonucleotides utilized in Example 1 were designed in-house and synthesised by Sigma Aldrich (see table in Figure 18(e) for sequences).
2. The oligonucleotides were diluted to a stock concentration of 100uM using sterile distilled water (ELGA VEOLIA).

### Methods

A cleavage reaction on oligonucleotides was carried out using the procedure below:
1. A pipette (Gilson) was used to transfer 41µl sterile distilled water (ELGA VEOLIA) into a 1.5ml Eppendorf tube.
2. 5µl of 10X ThermoPol^{®} reaction buffer NEB (20 mM Tris-HCl, 10 mM (NH₄)₂SO₄, 10 mM KCl, 2 mM MgSO₄, 0.1% Triton^{®} X-100, pH 8.8) were then added into the same Eppendorf tube.
3. 1µl each of oligonucleotides (Figure 18e); template (SEQ ID NO: 3) or any fluorescently tagged long oligo strand, primer with T (SEQ ID NO: 4) and control (SEQ ID NO: 5) all at 5pmols were added into the same tube.
4. 1µl of Human Alkyladenine DNA Glycosylase (hAAG) NEB (10units/µl) was added into the same tube.
5. Reaction mixture was then gently mixed by resuspension with a pipette, centrifuged at 13,000rpm for 5 seconds and incubated at 37°C for 1 hour.
6. Typically after incubation time had elapsed, the reaction was terminated by enzymatic heat inactivation (*i.e.* 65°C for 20 minutes).

Purification under ambient conditions. The sample mixture was purified using the protocol outlined below:
1. 500 µl of buffer PNI QIAGEN (5M guanidinium chloride) was added to the sample and mixed by gentle resuspension with a pipette.
2. The mixture was transferred into a QIAquick spin column (QIAGEN) and centrifuged for 1 min at 6000 rpm.
3. After centrifugation, flow-through was discarded and 750 µl of buffer PE QIAGEN (10mM Tris-HCl pH 7.5 and 80% ethanol) was added into the spin column and centrifuged for 1min at 6000 rpm.
4. The flow-through was discarded and the spin column was centrifuged for an additional 1 min at 13000 rpm to remove residual PE buffer.
5. The spin column was then placed in a sterile 1.5ml Eppendorf tube.
6. For DNA elution, 50 µl of Buffer EB QIAGEN (10mM Tris.CL, pH 8.5) was added to the centre of the column membrane and left to stand for 1 min at room temperature.
7. The tube was then centrifuged at 13000 rpm for 1 minutes. Eluted DNA concentration was measured and stored at -20°C for subsequent use.

Measurement of purified DNA concentration was determined using the protocol below:
1. NanoDrop one (Thermo Scientific) was equilibrated by adding 2 µl of sterile distilled water (ELGA VEOLIA) onto the pedestal.
2. After equilibration, the water was gently wiped off using a lint-free lens cleaning tissue (Whatman).
3. NanoDrop one was blanked by adding 2 µl of Buffer EB QIAGEN (10mM Tris.CL, pH 8.5). Then step 2 was repeated after blanking.
4. DNA concentration was measured by adding 2 µl of the sample onto the pedestal and selecting the measure icon on the touch screen.

Cleavage of the generated abasic site was carried out using the procedure below:
1. 2 µl (10-100ng/µl) DNA was added into a sterile 1.5ml Eppendorf tube.
2. 40 µl (0.2M) NaOH or 1.5 µl Endo VIII NEB (10units/µl) and 5 µl 10X Reaction Buffer NEB (10 mM Tris-HCl, 75 mM NaCl, 1 mM EDTA, pH 8 @ 25°C) was also added into the same tube and gently mixed by resuspension and centrifugation at 13000 rpm for 5 sec.
3. The resulting mixture was incubated at room temperature for 5 minutes for the NaOH treated sample while Endo VIII reaction mixture was incubated at 37°C for 1hr.
4. After incubation time had elapsed, the reaction mixture was purified using steps 1-7 of purification protocol as outlined above.

Gel Electrophoresis and DNA Visualization:
1. 5 µl of DNA and TBE-Urea sample buffer (Novex) was added into a sterile 1.5 ml Eppendorf tube and heated to 95°C for 2 minutes using a heat thermoblock (Eppendorf).
2. The DNA mixtures were then loaded into the wells of a 15% TBE-Urea gel 1.0mm x 10 well (Invitrogen) which contained preheated 1X TBE buffer Thermo Scientific (89mM Tris, 89mM boric acid and 2mM EDTA).
3. X-cell sure lock module (Novex) was fastened in place and electrophoresis performed at the following conditions; 260V, 90 Amps for 40 minutes at room temperature.
4. Detection and visualization of DNA in the gel was carried out with ChemiDoc MP (BioRad) using Cy3 LEDS. Visualization and analysis was carried out on the Image lab 2.0 platform.

### Results and Conclusion

The cleavage reaction without a helper strand showed a low percentage yield of cleaved to uncleaved DNA ratio of ~7% : 93% (Figure 18b-d).

Cleavage results showed that in this specific example, and based on the specific reagents used, a low yield of cleaved DNA is obtained in the absence of a helper strand in comparison to the positive control. In addition the use of a chemical base for cleavage of the abasic site was less time-consuming compared to EndoVIII cleavage.

### Step 3: Ligation

The third step describes ligation of polynucleotides with DNA ligase in the absence of a helper strand. A diagrammatic illustration is shown in Figure 19.

### Materials and Methods

### Materials

1. Oligonucleotides utilized in Example 1 were designed in-house and synthesised by Sigma Aldrich (see table in Figure 19c for sequences).
2. The oligonucleotides were diluted to a stock concentration of 100uM using sterile distilled water (ELGA VEOLIA).

### Methods

Ligation reaction on oligonucleotides was carried out using the procedure below:
1. A pipette (Gilson) was used to transfer 16µl sterile distilled water (ELGA VEOLIA) into a 1.5ml Eppendorf tube.
2. 10µl of 2X Quick Ligation Reaction buffer NEB (132 mM Tris-HCl, 20mM MgCl₂, 2mM dithiothreitol, 2mM ATP, 15% Polyethylene glycol (PEG6000) and pH 7.6 at 25°C) was then added into the same Eppendorf tube.
3. 1µl each of oligonucleotides (Figure 19c); TAMRA or any fluorescently tagged phosphate strand (SEQ ID NO: 7), primer with T (SEQ ID NO: 8) and inosine strand (SEQ ID NO: 9), all at 5 pmols, was added into the same tube.
4. 1µl of Quick T4 DNA Ligase NEB (400units/µl) was added into the same tube.
5. The reaction mixture was then gently mixed by resuspension with a pipette, centrifuged at 13,000rpm for 5 seconds and incubated at room temperature for 20 minutes.
6. Typically after incubation time had elapsed, reaction was terminated with the addition of TBE-Urea sample Buffer (Novex).
7. The reaction mixture was purified using the protocol outlined in purification steps 1-7 as described above.

Measurement of purified DNA concentration was determined using the protocol below:
1. NanoDrop one (Thermo Scientific) was equilibrated by adding 2 µl of sterile distilled water (ELGA VEOLIA) onto the pedestal.
2. After equilibration, the water was gently wiped off using a lint-free lens cleaning tissue (Whatman).
3. NanoDrop one was blanked by adding 2 µl of Buffer EB QIAGEN (10mM Tris.CL, pH 8.5), then step 2 was repeated after blanking.
4. DNA concentration was measured by adding 2 µl of the sample onto the pedestal and selecting the measure icon on the touch screen.
5. Purified DNA was run on a polyacrylamide gel and visualized in accordance with the procedure in steps 5-8 described above. No change in conditions or reagents was introduced.

### Results and Conclusion

In this specific example, and based on the specific reagents used, ligation of oligonucleotides with DNA ligase, in this particular case quick T4 DNA ligase, at room temperature (24°C) in the absence of a helper strand results in a reduced amount of ligation product (Figure 19b).

### Example 2. Version 1 Chemistry with a Helper Strand.

This example describes the synthesis of polynucleotides using 4 steps: incorporation of 3'-O-modified dNTPs from a nick site, cleavage, ligation and deprotection, with the first step taking place opposite a universal nucleotide, in this particular case inosine. The method uses a helper strand which improves the efficiency of the ligation and cleavage steps.

### Step1: Incorporation

The first step describes controlled addition of 3'-O-protected single nucleotide to oligonucleotide by enzymatic incorporation using DNA polymerase (Figure 20a).

### Materials and Methods

### Materials

1. 3'-O-modified dNTPs were synthesised in-house according to the protocol described in PhD thesis Jian Wu: Molecular Engineering of Novel Nucleotide Analogues for DNA Sequencing by Synthesis. Columbia University, 2008. The protocol for synthesis is also described in the patent application publication: J. William Efcavitch, Juliesta E. Sylvester, Modified Template-Independent Enzymes for Polydeoxynucleotide Synthesis, Molecular Assemblies US2016/0108382A1.
2. Oligonucleotides were designed in house and obtained from Sigma-Aldrich. The stock solutions were prepared at a concentration of 100 µM. Oligonucleotides are shown in Figure 20b.
3. Therminator IX DNA polymerase was used that has been engineered by New England BioLabs with enhanced ability to incorporate 3-*O*-modified dNTPs.

Two types of reversible terminators were tested:

| | |
|---|---|
| 3'-*O*-azidomethyl-dTTP: | 3'-*O*-allyl-dTTP: |
| 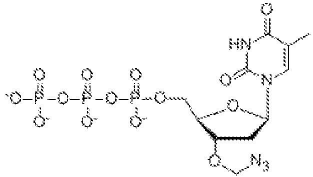 | 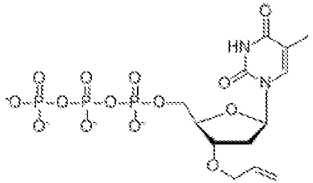 |

### Methods

1. 2 µl of 10x Thermopol^{®} buffer (20 mM Tris-HCl, 10 mM (NH₄)₂SO₄, 10 mM KCl, 2 mM MgSO₄, 0.1% Triton^{®} X-100, pH 8.8, New England BioLabs) was mixed with 10.25 µl of sterile deionized water (ELGA VEOLIA) in 1.5ml Eppendorf tube.
2. 0.5 µl of 10 µM primer (5 pmol, 1 equiv) (SEQ ID NO: 10, Table in Figure 20(b)), 0.75 µl of 10 µM template (6 pmol, 1.5equiv) (SEQ ID NO: 11, Table in Figure 20(b)), 2 µl of 10 µM of helper strand (SEQ ID NO: 12, Table in Figure 20(b)) were added to the reaction mixture.
3. 3'-O-modified-dTTP (2 µl of 100 µM) and MnCl₂ (1 µl of 40 mM) were added.
4. 1.5 µl of Therminator IX DNA polymerase (15 U, New England BioLabs) was then added.
5. The reaction was incubated for 20 minutes at 65°C.
6. The reaction was stopped by addition of TBE-Urea sample buffer (Novex).
7. The reaction was separated on polyacrylamide gel (15%) TBE buffer and visualized by ChemiDoc MP imaging system (BioRad).

Gel Electrophoresis and DNA Visualization:
1. 5 µl of reaction mixture was added to 5 µl of TBE-Urea sample buffer (Novex) in a sterile 1.5 ml Eppendorf tube and heated to 95°C for 5 minutes using a heat ThermoMixer (Eppendorf).
2. 5 µl of the sample were then loaded into the wells of a 15% TBE-Urea gel 1.0mm x 10 well (Invitrogen) which contained preheated 1X TBE buffer Thermo Scientific (89mM Tris, 89mM Boric acid and 2mM EDTA).
3. X-cell sure lock module (Novex) was fastened in place and electrophoresis performed at the following conditions; 260V, 90 Amps for 40 minutes at room temperature.
4. The gel was visualized by ChemiDoc MP (BioRad) using Cy3 LEDS. Visualization and analysis was carried out on the Image lab 2.0 platform.

The incorporation step can be studied according to the protocol described above.

### Step 2: Cleavage

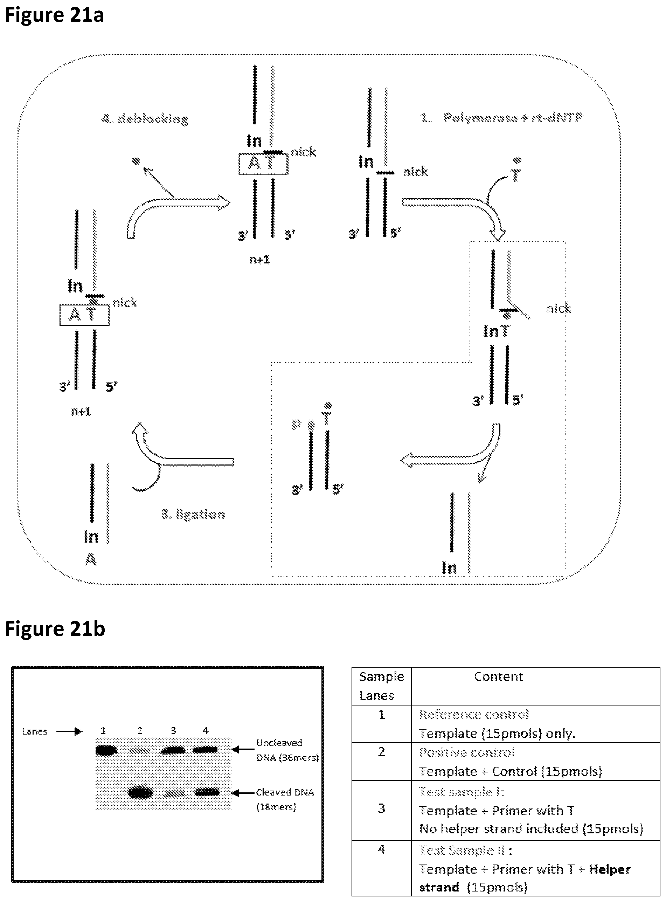

The second step describes a two-step cleavage of polynucleotides with either hAAG/Endo VIII or hAAG/chemical base (x2) (Figure 21a).

### Materials and Methods

### Materials

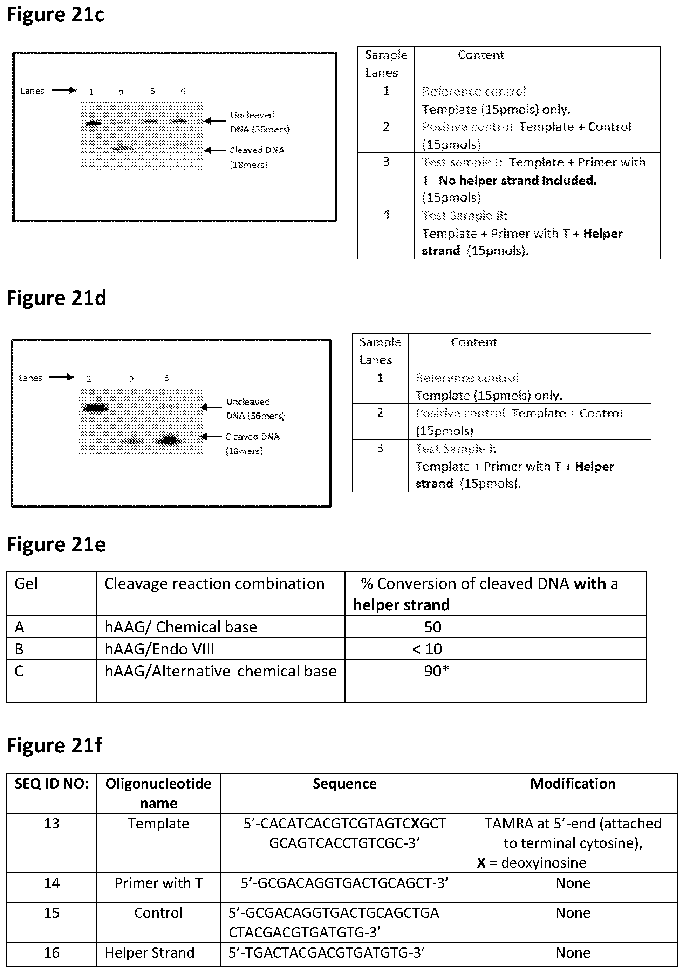

1. Oligonucleotides utilized in Example 2 were designed in-house and synthesised by Sigma Aldrich (see Figure 21f for sequences).
2. The oligonucleotides were diluted to a stock concentration of 100uM using sterile distilled water (ELGA VEOLIA).

### Methods

Cleavage reaction on oligonucleotides was carried out using the procedure below:
1. A pipette (Gilson) was used to transfer 41µl sterile distilled water (ELGA VEOLIA) into a 1.5 ml Eppendorf tube.
2. 5µl of 10X ThermoPol^{®} Reaction buffer NEB (20 mM Tris-HCl, 10 mM (NH₄)₂SO₄, 10 mM KCl, 2 mM MgSO₄, 0.1% Triton^{®} X-100, pH 8.8) was then added into the same Eppendorf tube.
3. 1µl each of oligonucleotides (Figure 21f); template (SEQ ID NO: 13) or any fluorescently tagged long oligo strand, primer with T (SEQ ID NO: 14), control (SEQ ID NO: 15) and helper strand (SEQ ID NO: 16), all at 5 pmols, were added into the same tube.
4. 1µl of Human Alkyladenine DNA Glycosylase (hAAG) NEB (10 units/µl) was added into the same tube.
5. In the reaction using alternative base, 1µl of Human Alkyladenine DNA Glycosylase (hAAG) NEB (100 units/µl) was added.
6. Reaction mixture was then gently mixed by resuspension with a pipette, centrifuged at 13,000 rpm for 5 seconds and incubated at 37°C for 1 hour.
7. Typically after incubation time had elapsed, the reaction was terminated by enzymatic heat inactivation (*i.e.* 65°C for 20 minutes).

Purification under ambient conditions. The sample mixture was purified using the protocol outlined below:
1. 500 µl of buffer PNI QIAGEN (5M guanidinium chloride) was added to the sample and mixed by gentle resuspension with a pipette.
2. The mixture was transferred into a QIAquick spin column (QIAGEN) and centrifuged for 1 min at 6000 rpm.
3. After centrifugation, flow-through was discarded and 750 µl of buffer PE QIAGEN (10mM Tris-HCl pH 7.5 and 80% ethanol) was added into the spin column and centrifuged for 1 min at 6000 rpm.
4. The flow-through was discarded and the spin column was centrifuged for an additional 1 min at 13000 rpm to remove residual PE buffer.
5. The spin column was then placed in a sterile 1.5 ml Eppendorf tube.
6. For DNA elution, 50 µl of Buffer EB QIAGEN (10mM Tris.CL, pH 8.5) was added to the centre of the column membrane and left to stand for 1min at room temperature.
7. The tube was then centrifuged at 13000 rpm for 1 minute. Eluted DNA concentration was measured and stored at -20°C for subsequent use.

Measurement of purified DNA concentration was determined using the protocol below:
1. NanoDrop one (Thermo Scientific) was equilibrated by adding 2 µl of sterile distilled water (ELGA VEOLIA) onto the pedestal.
2. After equilibration, the water was gently wiped off using a lint-free lens cleaning tissue (Whatman).
3. NanoDrop one was blanked by adding 2 µl of Buffer EB QIAGEN (10mM Tris.CL, pH 8.5). Then step 2 was repeated after blanking.
4. DNA concentration was measured by adding 2 µl of the sample onto the pedestal and selecting the measure icon on the touch screen.

Cleavage of generated abasic site was carried out using the procedure below:
1. 2 µl (10-100ng/µl) DNA was added into a sterile 1.5ml Eppendorf tube.
2. 40 µl (0.2M) NaOH or 1.5 µl Endo VIII NEB (10units/µl) and 5 µl 10X Reaction Buffer NEB (10 mM Tris-HCl, 75 mM NaCl, 1 mM EDTA, pH 8 @ 25°C) was also added into the same tube and gently mixed by resuspension and centrifugation at 13000 rpm for 5 sec.
3. The resulting mixture was incubated at room temperature for 5 minutes for the 0.2 M NaOH treated sample while Endo VIII reaction mixture was incubated at 37°C for 1hr.
4. After incubation time had elapsed, the reaction mixture was purified using steps 1-7 of purification protocol as stated above.

Cleavage of generated abasic site using alternative basic chemical was carried out using the procedure below:
1. 1 µl (10-100 ng/µl) DNA was added into a sterile 1.5 ml Eppendorf tube. 2 µl of N,N' dimethylethylenediamine Sigma (100mM) which had been buffered at room temperature with acetic acid solution sigma (99.8%) to pH 7.4 was then added into the same tube.
2. 20 µl of sterile distilled water (ELGA VEOLIA) was added into the tube and gently mixed by resuspension and centrifugation at 13000 rpm for 5 sec.
3. The resulting mixture was incubated at 37°C for 20 minutes.
4. After incubation time had elapsed, the reaction mixture was purified using steps 1-7 of the purification protocol stated above.

Gel Electrophoresis and DNA Visualization:
1. 5 µl of DNA and TBE-Urea sample buffer (Novex) was added into a sterile 1.5 ml Eppendorf tube and heated to 95°C for 2 minutes using a heat thermoblock (Eppendorf).
2. The DNA mixtures were then loaded into the wells of a 15% TBE-Urea gel 1.0mm x 10 well (Invitrogen) which contained preheated 1X TBE buffer Thermo Scientific (89mM Tris, 89mM boric acid and 2mM EDTA).
3. X-cell sure lock module (Novex) was fastened in place and electrophoresis performed at the following conditions; 260V, 90 Amps for 40 minutes at room temperature.
4. Detection and visualization of DNA in the gel was carried out with ChemiDoc MP (BioRad) using Cy3 LEDS. Visualization and analysis was carried out on the Image lab 2.0 platform.

### Results

Cleavage efficiency at a cleavage site comprising a universal nucleotide, in this particular case inosine, by hAAG DNA glycosylase was significantly increased from 10% in absence of helper strand to 50% in presence of helper strand (Figure 21b). hAAG and Endonuclease VIII cleave inosine with lower efficiency (10%) than hAAG and NaOH (50%). Chemical cleavage using 0.2M NaOH was shown to be preferable for cleavage of AP sites than Endonuclease VIII in the described system using nicked DNA (Figure 21c). Mild N,N'-dimethylethylenediamine at neutral pH has high efficiency to cleave abasic sites as 0.2M NaOH, and therefore it is preferable compared with Endonuclease VIII and NaOH (Figures 21d-e).

### Conclusion

Three methods were evaluated for cleavage of DNA containing inosine. One full enzymatic method - hAAG/Endonuclease VIII, and two methods combining chemical and enzymatic cleavage - hAAG/NaOH and hAAG/dimethylethylamine were studied for DNA cleavage in Example 2.

hAAG/NaOH results showed a much higher yield of cleaved DNA (50%) in the presence of a helper strand in comparison to the absence of a helper strand (10%). In these specific examples, and based on the specific reagents used, helper strands increase yield of DNA cleavage.

Enzymatic cleavage using Endonuclease VIII as a substitute for NaOH was less efficient (10%) compared to NaOH (50%) in the presence of a helper strand.

The inclusion of an alternative mild chemical base N,N'-dimethylethylenediamine led to high cleavage efficiency of AP sites, as efficient as for NaOH, and, together with addition of 10x hAAG enzyme, had a significant increase on cleaved DNA (see Figure 21e).

### Step 3: Ligation

The third step describes ligation of polynucleotides with DNA ligase in the presence of a helper strand. A diagrammatic illustration is shown in Figure 22a.

### Materials and Methods

### Materials

1. Oligonucleotides were designed in-house and synthesised by Sigma Aldrich (see Figure 22d for sequences).
2. The oligonucleotides were diluted to a stock concentration of 100uM using sterile distilled water (ELGA VEOLIA).

### Methods

Ligation reaction on oligonucleotides was carried out using the procedure below:
1. A pipette (Gilson) was used to transfer 16µl sterile distilled water (ELGA VEOLIA) into a 1.5 ml Eppendorf tube.
2. 10µl of 2X Quick Ligation Reaction buffer NEB (132 mM Tris-HCl, 20mM MgCl₂, 2mM dithiothreitol, 2mM ATP, 15% Polyethylene glycol (PEG6000) and pH 7.6 at 25°C) was then added into the same Eppendorf tube.
3. 1µl each of oligonucleotides (Figure 22d); TAMRA or any fluorescently tagged phosphate strand (SEQ ID NO: 18), primer with T (SEQ ID NO: 19) and inosine strand (SEQ ID NO: 20) and helper strand (SEQ ID NO: 21), all at of 5 pmols, was added into the same tube.
4. 1µl of Quick T4 DNA Ligase NEB (400 units/µl) was added into the same tube.
5. Reaction mixture was then gently mixed by resuspension with a pipette, centrifuged at 13,000 rpm for 5seconds and incubated at room temperature for 20 minutes.
6. Typically after incubation time had elapsed, reaction was terminated with the addition of TBE-Urea sample Buffer (Novex).
7. The reaction mixture was purified using the protocol outlined in purification steps 1-7 as described above.

Measurement of purified DNA concentration was determined using the protocol below:
1. NanoDrop one (Thermo Scientific) was equilibrated by adding 2 µl of sterile distilled water (ELGA VEOLIA) onto the pedestal.
2. After equilibration, the water was gently wiped off using a lint-free lens cleaning tissue (Whatman).
3. NanoDrop one was blanked by adding 2 µl of Buffer EB QIAGEN (10mM Tris.CL, pH 8.5). Then step 2 was repeated after blanking.
4. DNA concentration was measured by adding 2 µl of the sample onto the pedestal and selecting the measure icon on the touch screen.
5. Purified DNA was run on a polyacrylamide gel and visualized in accordance with the procedure in steps 5-8 above. No change in conditions or reagents was introduced.

### Results and Conclusion

In this specific example, and based on the specific reagents used, reduced ligation activity is observed in the absence of a helper strand (Figure 22b), whereas ligation proceeds with high efficiency in presence of a helper strand (Figure 22c) and the product is formed in high yield.

### Example 3. Version 2 Chemistry with a Helper Strand.

This example describes the synthesis of polynucleotides using 4 steps: incorporation of 3'-*O*- modified dNTPs on partial double-stranded DNA; cleavage, ligation and deprotection with the first step of incorporation taking place opposite a naturally complementary nucleotide which is positioned in the support strand adjacent to a universal nucleotide, in this particular case inosine.

### Step1: Incorporation

### Materials and Methods

### Materials

The first step describes controlled addition of 3'-O-protected single nucleotide to oligonucleotide by enzymatic incorporation by DNA polymerase (Figure 23a).
1. 3'-O-modified dNTPs were synthesised in-house according to the protocol described in PhD thesis Jian Wu: Molecular Engineering of Novel Nucleotide Analogues for DNA Sequencing by Synthesis. Columbia University, 2008. The protocol for synthesis is also described in the patent application publication: J. William Efcavitch, Juliesta E. Sylvester, Modified Template-Independent Enzymes for Polydeoxynucleotide Synthesis, Molecular Assemblies US2016/0108382A1.
2. Oligonucleotides were designed in house and obtained from Sigma-Aldrich (Figure 23j). The stock solutions are prepared in concentration of 100 µM.
3. Therminator IX DNA polymerase was used that has been engineered by New England BioLabs with enhanced ability to incorporate 3-*O*-modified dNTPs.

3'-*O*-azidomethyl reversible terminators of all dNTPs were tested independently for incorporation:

| | |
|---|---|
| 3'-*O*-azidomethyl-dTTP: | 3'-*O*-azidomethyl-dCTP: |
| 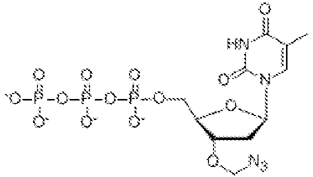 | 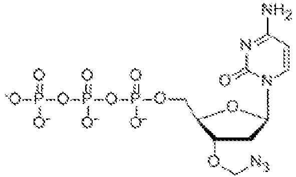 |
| 3'-*O*-azidomethyl-dATP: | 3'-*O*-azidomethyl-dGTP: |
| 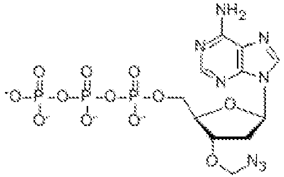 | 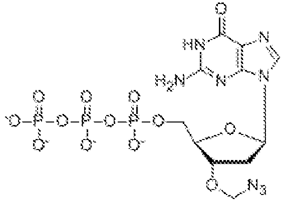 |

### Methods

1. 2 µl of 10x Thermopol^{®} buffer (20 mM Tris-HCl, 10 mM (NH₄)₂SO₄, 10 mM KCl, 2 mM MgSO₄, 0.1% Triton^{®} X-100, pH 8.8, New England BioLabs) was mixed with 12.25 µl of sterile deionized water (ELGA VEOLIA) in 1.5ml Eppendorf tube.
2. 0.5 µl of 10 µM primer (5 pmol, 1 equiv) (SEQ ID NO: 22, Figure 23j) and 0.75 µl of 10 µM template-A/G/T/C (6 pmol, 1.5equiv) (SEQ ID NOS: 23 to 26, Figure 23j) and 1 µl of 10 µM helper strand-T/C/A/G (10 pmol, 2 equiv) (SEQ ID NOS: 27 to 30, Figure 23j) were added to the reaction mixture.
3. 3'-*O*-modified-dTTP/dCTP/ dATP/dGTP (2 µl of 100 µM) and MnCl₂ (1 µl of 40 mM) were added.
4. 1.5 µl of Therminator IX DNA polymerase (15 U, New England BioLabs) was then added.
5. The reaction was incubated for 20 minutes at 65°C.
6. The reaction was stopped by addition of TBE-Urea sample buffer (Novex).
7. The reaction was separated on polyacrylamide gel (15%) TBE buffer and visualized by ChemiDoc MP imaging system (BioRad).

Gel Electrophoresis and DNA Visualization:
1. 5 µl of reaction mixture was added to 5 µl of TBE-Urea sample buffer (Novex) in a sterile 1.5 ml Eppendorf tube and heated to 95°C for 5 minutes using a heat ThermoMixer (Eppendorf).
2. 5 µl of the sample were then loaded into the wells of a 15% TBE-Urea gel 1.0mm x 10 well (Invitrogen) which contained preheated 1X TBE buffer Thermo Scientific (89mM Tris, 89mM boric acid and 2mM EDTA).
3. X-cell sure lock module (Novex) was fastened in place and electrophoresis performed at the following conditions; 260V, 90 Amps for 40 minutes at room temperature.
4. The gel was visualized by ChemiDoc MP (BioRad) using Cy3 LEDS. Visualization and analysis was carried out on the Image lab 2.0 platform.

### Results and Conclusions

Regarding the evaluation of the temperature on the incorporation of 3-*O*-azidomethyl-dTTP using Therminator IX DNA polymerase, the results indicate that incorporation of 3'-*O*-azidomethyl-dTTP in the presence of a helper strand for ligation goes to 90% after 5 minutes. 10 % of primer remains unextended after 20 minutes at 37°C and 47°C.

Therminator IX DNA polymerase at 2mM Mn²⁺ ions and a temperature of 37°C provide good conditions for incorporation of 3'-*O*-modified-dNTPs opposite a complementary base in DNA with high efficiency in the presence of the helper strand (from the ligation step from the previous cycle).

### Step 2: Cleavage

The second step describes a one-step cleavage of polynucleotides with Endonuclease V (Figure 24a).

### Materials and Methods

### Materials

1. Oligonucleotides utilized in Example 3 were designed in-house and synthesised by Sigma Aldrich (see table in Figure 24d for sequences).
2. The oligonucleotides were diluted to a stock concentration of 100uM using sterile distilled water (ELGA VEOLIA).

### Methods

Cleavage reaction on oligonucleotides was carried out using the procedure below:
1. A pipette (Gilson) was used to transfer 41 µl sterile distilled water (ELGA VEOLIA) into a 1.5 ml Eppendorf tube.
2. 5µl of 10X Reaction buffer^{®} NEB (50 mM Potassium Acetate, 20 mM Tris-acetate, 10 mM Magnesium Acetate, 1 mM DTT, pH 7.9 @ 25°C) was then added into the same Eppendorf tube.
3. 1µl each of oligonucleotides (Figure 24d); Template (SEQ ID NO: 31) or any fluorescently tagged long oligo strand, Primer with T (SEQ ID NO: 32) and control (SEQ ID NO: 33) and helper strand (SEQ ID NO: 34), all at 5pmols, were added into the same tube.
4. 1µl of Human Endonuclease V (Endo V) NEB (10 units/µl) was added into the same tube.
5. Reaction mixture was then gently mixed by resuspension with a pipette, centrifuged at 13,000 rpm for 5 seconds and incubated at 37°C for 1hour.
6. Typically after incubation time had elapsed, reaction was terminated by enzymatic heat inactivation (*i.e.* 65°C for 20 minutes).

The sample mixture was purified using the protocol outlined below:
1. 500 µl of buffer PNI QIAGEN (5M guanidinium chloride) was added to the sample and mixed by gentle resuspension with a pipette.
2. The mixture was transferred into a QIAquick spin column (QIAGEN) and centrifuged for 1 min at 6000 rpm.
3. After centrifugation, flow-through was discarded and 750 µl of buffer PE QIAGEN (10mM Tris-HCl pH 7.5 and 80% ethanol) was added into the spin column and centrifuged for 1 min at 6000 rpm.
4. The flow-through was discarded and the spin column was centrifuged for an additional 1min at 13000 rpm to remove residual PE buffer.
5. The spin column was then placed in a sterile 1.5 ml Eppendorf tube.
6. For DNA elution, 50 µl of Buffer EB QIAGEN (10mM Tris.CL, pH 8.5) was added to the centre of the column membrane and left to stand for 1min at room temperature.
7. The tube was then centrifuged at 13000 rpm for 1 minutes. Eluted DNA concentration was measured and stored at -20°C for subsequent use.

Measurement of purified DNA concentration was determined using the protocol below:
1. NanoDrop one (Thermo Scientific) was equilibrated by adding 2 µl of sterile distilled water (ELGA VEOLIA) onto the pedestal.
2. After equilibration, the water was gently wiped off using a lint-free lens cleaning tissue (Whatman).
3. NanoDrop one was blanked by adding 2 µl of Buffer EB QIAGEN (10mM Tris.CL, pH 8.5). Then step 2 was repeated after blanking.
4. DNA concentration was measured by adding 2 µl of the sample onto the pedestal and selecting the measure icon on the touch screen.

Gel Electrophoresis and DNA Visualization:
1. 5 µl of DNA and TBE-Urea sample buffer (Novex) was added into a sterile 1.5ml Eppendorf tube and heated to 95°C for 2 minutes using a heat thermoblock (Eppendorf).
2. The DNA mixtures were then loaded into the wells of a 15% TBE-Urea gel 1.0mm x 10 well (Invitrogen) which contained preheated 1X TBE buffer Thermo Scientific (89mM Tris, 89mM boric acid and 2mM EDTA).
3. X-cell sure lock module (Novex) was fastened in place and electrophoresis performed at the following conditions; 260V, 90 Amps for 40 minutes at room temperature.
4. Detection and visualization of DNA in the gel was carried out with Chemidoc MP (BioRad) using Cy3 LEDS. Visualization and analysis was carried out on the Image lab 2.0 platform.

### Results and Conclusions

Cleavage results from Example 3 showed that a significantly high yield of cleaved DNA could be obtained with Endonuclease V in the presence or absence of the helper strand (Figure 24c).

### Step 3: Ligation

The third step describes ligation of polynucleotides with DNA ligase in the presence of a helper strand. A diagrammatic illustration is shown in Figure 25a.

### Materials and Methods

### Materials

1. Oligonucleotides utilized in Example 3 were designed in-house and synthesised by Sigma Aldrich (see table in Figure 25b for sequences).
2. The oligonucleotides were diluted to a stock concentration of 100 uM using sterile distilled water (ELGA VEOLIA).

### Methods

Ligation reaction on oligonucleotides was carried out using the procedure below
1. A pipette (Gilson) was used to transfer 16 µl sterile distilled water (ELGA VEOLIA) into a 1.5ml Eppendorf tube.
2. 10 µl of 2X Quick Ligation Reaction buffer NEB (132 mM Tris-HCl, 20mM MgCl₂, 2mM dithiothreitol, 2mM ATP, 15% Polyethylene glycol (PEG6000) and pH 7.6 at 25°C) was then added into the same Eppendorf tube.
3. 1 µl each of oligonucleotides (Figure 25b); TAMRA or any fluorescently tagged phosphate strand (SEQ ID NO: 35), primer with T (SEQ ID NO: 36) and inosine strand (SEQ ID NO: 37) and helper strand (SEQ ID NO: 38) all having an amount of 5 pmols was added into the same tube.
4. 1 µl of Quick T4 DNA Ligase NEB (400units/µl) was added into the same tube.
5. Reaction mixture was then gently mixed by resuspension with a pipette, centrifuged at 13,000 rpm for 5 seconds and incubated at room temperature for 20 minutes.
6. Typically after the incubation time had elapsed, the reaction was terminated with the addition of TBE-Urea sample Buffer (Novex).
7. The reaction mixture was purified using the protocol outlined in purification steps 1-7 as described above.

Measurement of purified DNA concentration was determined using the protocol below:
1. NanoDrop one (Thermo Scientific) was equilibrated by adding 2 µl of sterile distilled water (ELGA VEOLIA) onto the pedestal.
2. After equilibration, the water was gently wiped off using a lint-free lens cleaning tissue (Whatman).
3. NanoDrop one was blanked by adding 2 µl of Buffer EB QIAGEN (10mM Tris.CL, pH 8.5). Then step 2 was repeated after blanking.
4. DNA concentration was measured by adding 2 µl of the sample onto the pedestal and selecting the measure icon on the touch screen.
5. Purified DNA was run on a polyacrylamide gel and visualized in accordance with the procedure in steps 5-8 described above. No change in conditions or reagents was introduced.

Gel Electrophoresis and DNA Visualization:
1. 5 µl of DNA and TBE-Urea sample buffer (Novex) was added into a sterile 1.5 ml Eppendorf tube and heated to 95°C for 2 minutes using a heat thermoblock (Eppendorf).
2. The DNA mixtures were then loaded into the wells of a 15% TBE-Urea gel 1.0mm x 10 well (Invitrogen) which contained preheated 1X TBE buffer Thermo Scientific (89mM Tris, 89mM boric acid and 2mM EDTA).
3. X-cell sure lock module (Novex) was fastened in place and electrophoresis performed at the following conditions; 260V, 90 Amps for 40 minutes at room temperature.
4. Detection and visualization of DNA in the gel was carried out with ChemiDoc MP (BioRad) using Cy3 LEDS. Visualization and analysis was carried out on the Image lab 2.0 platform.

### Step 4: Deprotection

Deprotection step (Figure 26a) was studied on DNA model bearing 3'-*O*-azidomethyl group that is introduced to DNA by incorporation of 3'-*O*-azidomethyl-dNTPs by Therminator IX DNA polymerase. Deprotection was carried out by tris(carboxyethyl)phosphine (TCEP) and monitored by extension reaction when mixture of all natural dNTPs is added to the solution of the purified deprotected DNA.

### Materials and Methods

### Materials

1. Oligonucleotides utilized in Example 3 were designed in-house and synthesised by Sigma Aldrich (see Figure 26i for sequences).
2. The oligonucleotides were diluted to a stock concentration of 100 uM using sterile distilled water (ELGA VEOLIA).
3. Enzymes were purchased from New England BioLabs.

### Methods

1. 2 µl of 10x Thermopol^{®} buffer (20 mM Tris-HCl, 10 mM (NH₄)₂SO₄, 10 mM KCl, 2 mM MgSO₄, 0.1% Triton^{®} X-100, pH 8.8, New England BioLabs) was mixed with 12.25 µl of sterile deionized water (ELGA VEOLIA) in 1.5 ml Eppendorf tube.
2. 1 µl of 10 µM primer (10 pmol, 1 equiv) (SEQ ID NO: 39, Figure 26i) and 1.5 µl of either 10 µM template-A/G/T/C (15 pmol, 1.5equiv) (SEQ ID NOS: 40 to 43, Figure 26i) were added to the reaction mixture.
3. 3'-*O*-modified-dTTP/dCTP/dATP/dGTP (2 µl of 100 µM) and MnCl₂ (1 µl of 40mM) were added.
4. 1.5 µl of Therminator IX DNA polymerase (15 U, New England BioLabs) was then added.
5. The reaction was incubated for 5 minutes at 37°C.
6. 4 µL of the sample was taken out and mixed with 0.5 ul of 5mM dNTP mix and allowed to react for 10 minutes for control reaction.
7. 40 µL of the 500 mM TCEP in 1M TRIS buffer pH 7.4 was added to the reaction mixture and allowed to react for 10 minutes at 37°C.
8. The reaction mixture was purified using QIAGEN Nucleotide removal kit eluting by 20 µL of 1x Thermopol^{®} buffer.
9. 1 µL of 5mM dNTP mix and 1 µL of DeepVent (exo-) DNA polymerase were added to the purified reaction mixture and allowed to react 10 minutes.
10. The reaction was stopped by addition of TBE-Urea sample buffer (Novex).
11. The reaction was separated on polyacrylamide gel (15%) TBE buffer and visualized by ChemiDoc MP imaging system (BioRad).

### Results and Conclusion

50mM TCEP was not sufficient to cleave 3'-O-azidomethyl group with high efficiency on 0.2 µM DNA model (Figure 26h). In contrast, 300mM TCEP successfully cleaved 3'-O-azidomethyl group with 95% efficiency on 0.2 µM DNA model (Figure 26h).

### Example 4. Version 2 Chemistry with Double Hairpin Model.

This Example describes the synthesis of polynucleotides using 4 steps on a two-hairpin model: incorporation of 3'-O- modified dNTPs from a nick site; cleavage, ligation and deprotection with the first step taking place opposite a naturally complementary nucleotide which is positioned in the support strand adjacent to a universal nucleotide, in this particular case inosine.

### Step: Incorporation

The first step describes controlled addition of 3'-*O*-protected single nucleotide to oligonucleotide by enzymatic incorporation by DNA polymerase (Figure 27a).

### Materials and Methods

### Materials

1. 3'-*O*-modified dNTPs were synthesised in-house according to the protocol described in PhD thesis Jian Wu: Molecular Engineering of Novel Nucleotide Analogues for DNA Sequencing by Synthesis. Columbia University, 2008. The protocol for synthesis is also described in the patent application publication: J. William Efcavitch, Juliesta E. Sylvester, Modified Template-Independent Enzymes for Polydeoxynucleotide Synthesis, Molecular Assemblies US2016/0108382A1.
2. Oligonucleotides were designed in house and obtained from Sigma-Aldrich (Figure 27c). The stock solutions were prepared in concentration of 100 µM.
3. Therminator IX DNA polymerase was used that has been engineered by New England BioLabs with enhanced ability to incorporate 3-*O*-modified dNTPs.

3'-*O*-azidomethyl-dTTP was tested for incorporation:
3'-*O*-azidomethyl-dTTP:

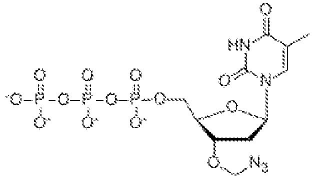

### Method

1. 2 µl of 10x Thermopol^{®} buffer (20 mM Tris-HCl, 10 mM (NH₄)₂SO₄, 10 mM KCl, 2 mM MgSO₄, 0.1% Triton^{®} X-100, pH 8.8, New England BioLabs) was mixed with 10.25 µl of sterile deionized water (ELGA VEOLIA) in 1.5ml Eppendorf tube.
2. 0.5 µl of 10 µM hairpin oligonucleotide (5 pmol, 1 equiv) (SEQ ID NO: 44, Figure 27c) was added to the reaction mixture.
3. 3'-*O*-modified-dTTP (2 µl of 100 µM) and MnCl₂ (1 µl of 40 mM) were added.
4. 1.5 µl of Therminator IX DNA polymerase (15 U, New England BioLabs) was then added.
5. The reaction was incubated for 20 minutes at 65°C.
6. The reaction was stopped by addition of TBE-Urea sample buffer (Novex).
7. The reaction was separated on polyacrylamide gel (15%) TBE buffer and visualized by ChemiDoc MP imaging system (BioRad).

Gel Electrophoresis and DNA Visualization:
1. 5 µl of reaction mixture was added to 5 µl of TBE-Urea sample buffer (Novex) in a sterile 1.5ml Eppendorf tube and heated to 95°C for 5 minutes using a heat ThermoMixer (Eppendorf).
2. 5 µl of the sample were then loaded into the wells of a 15% TBE-Urea gel 1.0mm x 10 well (Invitrogen) which contained preheated 1X TBE buffer Thermo Scientific (89mM Tris, 89mM boric acid and 2mM EDTA).
3. X-cell sure lock module (Novex) was fastened in place and electrophoresis performed at the following conditions; 260V, 90 Amps for 40 minutes at room temperature.
4. The gel was visualized by ChemiDoc MP (BioRad) using Cy3 LEDS. Visualization and analysis was carried out on the Image lab 2.0 platform.

### Results

DNA polymerases incorporate 3'-*O*-modified-dTTPs opposite its naturally complementary base in a hairpin construct.

### Step2: Cleavage

The second step describes a one-step cleavage of a hairpin model in this particular case with Endonuclease V (Figure 28a).

### Materials and Methods

### Materials

1. Oligonucleotides utilized in Example 4 were designed in-house and synthesised by Sigma Aldrich (see Figure 28c for sequences).
2. The oligonucleotides were diluted to a stock concentration of 100uM using sterile distilled water (ELGA VEOLIA).

### Methods

Cleavage reaction on hairpin oligonucleotides was carried out using the procedure below:
1. A pipette (Gilson) was used to transfer 43 µl sterile distilled water (ELGA VEOLIA) into a 1.5ml Eppendorf tube.
2. 5µl of 10X Reaction buffer^{®} NEB (50 mM potassium acetate, 20 mM Tris-acetate, 10 mM magnesium acetate, 1 mM DTT, pH 7.9 @ 25°C) was then added into the same Eppendorf tube.
3. 1µl of hairpin oligonucleotide (SEQ ID NO: 45, Figure 28c) having an amount of 5pmols was added into the same tube.
4. 1µl of Human Endonuclease V (Endo V) NEB (30 units/µl) was added into the same tube.
5. The reaction mixture was then gently mixed by resuspension with a pipette, centrifuged at 13,000 rpm for 5 seconds and incubated at 37°C for 1hour.
6. Typically after incubation time had elapsed, the reaction was terminated by enzymatic heat inactivation (*i.e.* 65°C for 20 minutes).

The sample mixture was purified using the protocol outlined below:
1. 500 µl of buffer PNI QIAGEN (5M guanidinium chloride) was added to the sample and mixed by gentle resuspension with a pipette.
2. The mixture was transferred into a QIAquick spin column (QIAGEN) and centrifuged for 1min at 6000 rpm.
3. After centrifugation, flow-through was discarded and 750 µl of buffer PE QIAGEN (10mM Tris-HCl pH 7.5 and 80% ethanol) was added into the spin column and centrifuged for 1min at 6000 rpm.
4. The flow-through was discarded and the spin column was centrifuged for an additional 1min at 13000 rpm to remove residual PE buffer.
5. The spin column was then placed in a sterile 1.5ml Eppendorf tube.
6. For DNA elution, 50 µl of Buffer EB QIAGEN (10mM Tris.CL, pH 8.5) was added to the centre of the column membrane and left to stand for 1min at room temperature.
7. The tube was then centrifuged at 13000 rpm for 1 minute. Eluted DNA concentration was measured and stored at -20°C for subsequent use.

Measurement of purified DNA concentration was determined using the protocol below:
1. NanoDrop One (Thermo Scientific) was equilibrated by adding 2 µl of sterile distilled water (ELGA VEOLIA) onto the pedestal.
2. After equilibration, the water was gently wiped off using a lint-free lens cleaning tissue (Whatman).
3. NanoDrop One was blanked by adding 2 µl of Buffer EB QIAGEN (10mM Tris.CL, pH 8.5). Then step 2 was repeated after blanking.
4. DNA concentration was measured by adding 2 µl of the sample onto the pedestal and selecting the measure icon on the touch screen.

Gel Electrophoresis and DNA Visualization:
1. 5 µl of DNA and TBE-Urea sample buffer (Novex) was added into a sterile 1.5ml Eppendorf tube and heated to 95°C for 2 minutes using a heat ThermoMixer(Eppendorf).
2. The DNA mixtures were then loaded into the wells of a 15% TBE-Urea gel 1.0mm x 10 well (Invitrogen) which contained preheated 1X TBE buffer Thermo Scientific (89mM Tris, 89mM boric acid and 2mM EDTA).
3. X-cell sure lock module (Novex) was fastened in place and electrophoresis performed at the following conditions; 260V, 90 Amps for 40 minutes at room temperature.
4. Detection and visualization of DNA in the gel was carried out with ChemiDoc MP (BioRad) using Cy3 LEDS. Visualization and analysis was carried out on the Image lab 2.0 platform.

### Results and Conclusion

Cleavage results from Example 4 showed that a significantly high yield of digested hairpin DNA was obtained with Endonuclease V at 37°C (Figure 28b).

### Step 3: Ligation

The third step describes ligation of a hairpin model with DNA ligase. Diagrammatic illustration is shown in Figure 29a.

### Materials and Methods

### Materials

1. Oligonucleotides utilized in Example 4 were designed in-house and synthesised by Sigma Aldrich (see Figure 29c for sequences).
2 The oligonucleotides were diluted to a stock concentration of 100uM using sterile distilled water (ELGA VEOLIA).

### Method

Ligation reaction on oligonucleotides was carried out using the procedure below:
1. A pipette (Gilson) was used to transfer 1µl (5pmols) of TAMRA or any fluorescently tagged phosphate hairpin oligo (SEQ ID NO: 46) into a 1.5ml Eppendorf tube.
2. 15µl (100pmols) of inosine-containing hairpin construct (SEQ ID NO: 47) was then added into the same tube and gently mixed by resuspension with a pipette for 3 seconds.
3. 40µl of Blunt/TA DNA Ligase NEB (180 units/µl) was added into the same tube.
4. Reaction mixture was then gently mixed by resuspension with a pipette, centrifuged at 13,000 rpm for 5 seconds and incubated at room temperature for 20 minutes.
5. Typically after incubation time had elapsed, the reaction was terminated with the addition of TBE-Urea sample buffer (Novex).
6. The reaction mixture was purified using the protocol outlined in purification steps 1-7 above.

Measurement of purified DNA concentration was determined using the protocol below:
1. NanoDrop One (Thermo Scientific) was equilibrated by adding 2 µl of sterile distilled water (ELGA VEOLIA) onto the pedestal.
2. After equilibration, the water was gently wiped off using a lint-free lens cleaning tissue (Whatman).
3. NanoDrop One was blanked by adding 2 µl of Buffer EB QIAGEN (10mM Tris.CL, pH 8.5). Then step 2 was repeated after blanking.
4. DNA concentration was measured by adding 2 µl of the sample onto the pedestal and selecting the measure icon on the touch screen.
5. Purified DNA was run on a polyacrylamide gel and visualized in accordance with the procedure in steps 5-8 as described above. No change in conditions or reagents was introduced.

Gel Electrophoresis and DNA Visualization.
1. 5 µl of DNA and TBE-Urea sample buffer (Novex) was added into a sterile 1.5 ml Eppendorf tube and heated to 95°C for 2 minutes using a heat ThermoMixer (Eppendorf).
2. The DNA mixtures were then loaded into the wells of a 15% TBE-Urea gel 1.0mm x 10 well (Invitrogen) which contained preheated 1X TBE buffer Thermo Scientific (89mM Tris, 89mM boric acid and 2mM EDTA).
3. X-cell sure lock module (Novex) was fastened in place and electrophoresis performed at the following conditions; 260V, 90 Amps for 40 minutes at room temperature.
4. Detection and visualization of DNA in the gel was carried out with ChemiDoc MP (BioRad) using Cy3 LEDS. Visualization and analysis was carried out on the Image lab 2.0 platform.

### Results

Ligation of hairpin oligonucleotides with blunt/TA DNA ligase at room temperature (24°C) in the presence of a helper strand resulted high yield of ligated product. Ligated hairpin oligonucleotide after 1 minute showed a high yield of ligated DNA product with a ratio of ~ 85%. The ligated hairpin oligonucleotide after 2 minutes showed a high yield of ligated DNA with a ratio of ~ 85%. The ligated hairpin oligonucleotide after 3 minutes showed a high yield of ligated DNA product with a ratio of ~ 85%. The ligated hairpin oligonucleotide after 4 minutes showed a high yield of ligated DNA product with a ratio of ~ >85% (Figure 29b).

### Example 5. Version 2 Chemistry - Complete Cycle on Double Hairpin Model.

This Example describes the synthesis of polynucleotides using 4 steps on a double hairpin model: incorporation of 3'-*O*-modified dNTPs from the nick site; cleavage, ligation and deprotection with the first step taking place opposite a naturally complementary nucleotide which is positioned in the support strand adjacent to a universal nucleotide, in this particular case inosine. One end of the hairpin serves as an attachment anchor.

The method starts by controlled addition of a 3'-*O*-protected single nucleotide to an oligonucleotide by enzymatic incorporation by DNA polymerase followed by inosine cleavage, ligation and deprotection (Figure 30a).

### Materials and Methods

### Materials

1. 3'-*O*-modified dNTPs were synthesised in-house according to the protocol described in PhD thesis Jian Wu: Molecular Engineering of Novel Nucleotide Analogues for DNA Sequencing by Synthesis. Columbia University, 2008. The protocol for synthesis is also described in the patent application publication: J. William Efcavitch, Juliesta E. Sylvester, Modified Template-Independent Enzymes for Polydeoxynucleotide Synthesis, Molecular Assemblies US2016/0108382A1.
2. Oligonucleotides were designed in house and obtained from Sigma-Aldrich (Figure 30c). The stock solutions are prepared in concentration of 100 µM.
3. Therminator IX DNA polymerase was used that has been engineered by New England BioLabs with enhanced ability to incorporate 3-*O*-modified dNTPs.

3'-*O*-azidomethyl-dTTP was tested for incorporation:
3'-*O*-azidomethyl-dTTP:

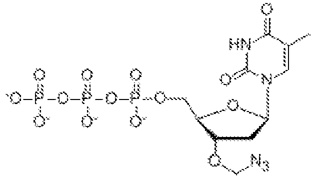

### Method

1. 2 µl of 10x Thermopol^{®} buffer (20 mM Tris-HCl, 10 mM (NH₄)₂SO₄, 10 mM KCl, 2 mM MgSO₄, 0.1% Triton^{®} X-100, pH 8.8, New England BioLabs) was mixed with 12.5 µl of sterile deionized water (ELGA VEOLIA) in 1.5ml Eppendorf tube.
2. 2 µl of 10 µM double hairpin model oligonucleotide (20 pmol, 1 equiv) (SEQ ID NO: 48, Figure 30c) were added to the reaction mixture.
3. 3'-*O*-modified-dTTP (2 µl of 100 µM) and MnCl₂ (1 µl of 40 mM) were added.
4. 1.5 µl of Therminator IX DNA polymerase (15 U, New England BioLabs) was then added.
5. The reaction was incubated for 10 minutes at 37°C.
6. The aliquot (5 µl) was taken out of the reaction mixture and 0.5 µl of natural dNTP mix was added and allowed to react for 10 minutes. The reaction was analysed by gel electrophoresis.
7. The reaction mixture was purified using the protocol outlined in purification steps 1-7.
8. The DNA sample was eluted by 20 µl of NEB reaction buffer^{®} (50 mM potassium acetate, 20 mM Tris-acetate, 10 mM magnesium acetate, 1 mM DTT, pH 7.9 @ 25°C) into clean Eppendorf tube.
9. 1 µl of Human Endonuclease V (Endo V) NEB (30 units/µl) was added into the same tube.
10. Reaction mixture was then gently mixed by resuspension with a pipette, centrifuged at 13,000 rpm for 5 seconds and incubated at 37°C for 1 hour.
11. After incubation time had elapsed, reaction was terminated by enzymatic heat inactivation (*i.e.* 65°C for 20 minutes).
12. The aliquot (5 µl) was taken out of the reaction mixture and analysed on polyacrylamide gel (15%) using TBE buffer and visualized by ChemiDoc MP imaging system (BioRad).
13. Reaction mixture was purified using the protocol outlined in purification steps 1-7 above.
14. The DNA sample was eluted by 20 µl of NEB Reaction buffer^{®} (50 mM potassium acetate, 20 mM Tris-acetate, 10 mM magnesium acetate, 1 mM DTT, pH 7.9 @ 25°C) into a clean Eppendorf tube.
15. 10 µl of 100 µM strand for ligation (1 nmol) (SEQ ID NO: 49, Figure 30c) were added to the reaction mixture.
16. 40µl of Blunt/TA DNA Ligase NEB (180 units/µl) was added into the purified DNA sample.
17. Reaction mixture was then gently mixed by resuspension with a pipette, centrifuged at 13,000 rpm for 5 seconds and incubated at room temperature for 20 minutes.
18. 40 µL of the 500 mM TCEP in 1M TRIS buffer pH 7.4 was added to the reaction mixture and allowed to react for 10 minutes at 37°C.
19. The reaction mixture was purified using QIAGEN nucleotide removal kit eluting by 20 µL of 1x Thermopol^{®} buffer.

Gel Electrophoresis and DNA Visualization:
1. 5 µl of reaction mixture was added to 5 µl of TBE-Urea sample buffer (Novex) in a sterile 1.5ml Eppendorf tube and heated to 95°C for 5 minutes using a heat ThermoMixer (Eppendorf).
2. 5 µl of the sample were then loaded into the wells of a 15% TBE-Urea gel 1.0mm x 10 well (Invitrogen) which contained preheated 1X TBE buffer Thermo Scientific (89mM Tris, 89mM boric acid and 2mM EDTA).
3. X-cell sure lock module (Novex) was fastened in place and electrophoresis performed at the following conditions; 260V, 90 Amps for 40 minutes at room temperature.
4. The gel was visualized by ChemiDoc MP (BioRad) using Cy3 LEDS. Visualization and analysis was carried out on the Image lab 2.0 platform.

Measurement of purified DNA concentration was determined using the protocol below:
1. NanoDrop One (Thermo Scientific) was equilibrated by adding 2 µl of sterile distilled water (ELGA VEOLIA) onto the pedestal.
2. After equilibration, the water was gently wiped off using a lint-free lens cleaning tissue (Whatman).
3. NanoDrop One was blanked by adding 2 µl of Buffer EB QIAGEN (10mM Tris.CL, pH 8.5). Then step 2 was repeated after blanking.
4. DNA concentration was measured by adding 2 µl of the sample onto the pedestal and selecting the measure icon on the touch screen.
5. Purified DNA was run on a polyacrylamide gel and visualized in accordance with the procedure in section 2 steps 5-8. No change in conditions or reagents was introduced.

The sample mixture was purified after each step using the protocol outlined below:
1. 500 µl of buffer PNI QIAGEN (5M guanidinium chloride) was added to the sample and mixed by gentle resuspension with a pipette.
2. The mixture was transferred into a QIAquick spin column (QIAGEN) and centrifuged for 1 min at 6000 rpm.
3. After centrifugation, flow-through was discarded and 750 µl of buffer PE QIAGEN (10mM Tris-HCl pH 7.5 and 80% ethanol) was added into the spin column and centrifuged for 1 min at 6000 rpm.
4. The flow-through was discarded and the spin column was centrifuged for an additional 1min at 13000 rpm to remove residual PE buffer.
5. The spin column was then placed in a sterile 1.5 ml Eppendorf tube.
6. For DNA elution, 20 µl of appropriate buffer for the reaction was added to the centre of the column membrane and left to stand for 1min at room temperature.
7. The tube was then centrifuged at 13000 rpm for 1 minute. Eluted DNA concentration was measured and stored at -20°C for subsequent use.

### Results

DNA polymerase incorporates 3'-*O*-modified-dTTPs opposite its naturally complementary base in a double hairpin construct (Figure 30b).

### Example 6. Version 2 Chemistry - Complete Cycle on Single Hairpin Model Using Helper Strand.

This Example describes the synthesis of polynucleotides using 4 steps on single-hairpin model: incorporation of 3'-*O*-modified dNTPs from nick site; cleavage, ligation and deprotection with the first step taking place opposite a naturally complementary base. The DNA synthesis uses a helper strand in the process.

The method starts by controlled addition of a 3'-*O*-protected single nucleotide to an oligonucleotide by enzymatic incorporation by DNA polymerase followed by inosine cleavage, ligation and deprotection (Figure 31a).

### Materials and Methods

### Materials

1. 3'-O-modified dNTPs were synthesised in-house according to the protocol described in PhD thesis Jian Wu: Molecular Engineering of Novel Nucleotide Analogues for DNA Sequencing by Synthesis. Columbia University, 2008. The protocol for synthesis is also described in the patent application publication: J. William Efcavitch, Juliesta E. Sylvester, Modified Template-Independent Enzymes for Polydeoxynucleotide Synthesis, Molecular Assemblies US2016/0108382A1.
2. Oligonucleotides were designed in house and obtained from Sigma Aldrich (Figure 31b). The stock solutions are prepared in concentration of 100 µM.
3. Therminator IX DNA polymerase was used that has been engineered by New England BioLabs with enhanced ability to incorporate 3-*O*-modified dNTPs.

3'-*O*-azidomethyl-dTTP was tested for incorporation:
3'-*O*-azidomethyl-dTTP:

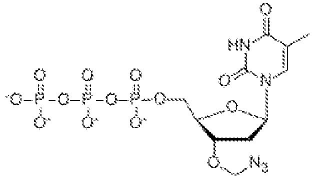

### Method

1. 2 µl of 10x Thermopol^{®} buffer (20 mM Tris-HCl, 10 mM (NH₄)₂SO₄, 10 mM KCl, 2 mM MgSO₄, 0.1% Triton^{®} X-100, pH 8.8, New England BioLabs) was mixed with 12.5 µl of sterile deionized water (ELGA VEOLIA) in 1.5ml Eppendorf tube.
2. 2 µl of 10 µM Single hairpin model oligonucleotide (20 pmol, 1 equiv) (SEQ ID NO: 50, Figure 31b) and Helper strand (30 pmol, 1.5 equiv) (SEQ ID NO: 51, Figure 31b) were added to the reaction mixture.
3. 3'-*O*-modified-dTTP (2 µl of 100 µM) and MnCl₂ (1 µl of 40 mM) were added
4. 1.5 µl of Therminator IX DNA polymerase (15 U, New England BioLabs) was then added.
5. The reaction was incubated for 10 minutes at 37°C.
6. The aliquot (5 µl) was taken out of the reaction mixture and 0.5 µl of natural dNTP mix was added and allowed to react for 10 minutes. The reaction was analysed by gel electrophoresis.
7. The reaction mixture was purified using the protocol outlined in purification steps 1-7 above.
8. The DNA sample was eluted by 20µl of NEB reaction buffer^{®} (50 mM potassium acetate, 20 mM Tris-acetate, 10 mM magnesium acetate, 1 mM DTT, pH 7.9 @ 25°C) into a clean Eppendorf tube.
9. 1µl of Human Endonuclease V (Endo V) NEB (30units/µl) was added into the same tube.
10. Reaction mixture was then gently mixed by resuspension with a pipette, centrifuged at 13,000 rpm for 5 seconds and incubated at 37°C for 1 hour.
11. After incubation time had elapsed, the reaction was terminated by enzymatic heat inactivation (*i.e.* 65°C for 20 minutes).
12. The aliquot (5 µl) was taken out of the reaction mixture and analysed on polyacrylamide gel (15%) using TBE buffer and visualized by ChemiDoc MP imaging system (BioRad).
13. The reaction mixture was purified using the protocol outlined in purification steps 1-7 above.
14. The DNA sample was eluted by 20 µl of NEB reaction buffer^{®} (50 mM potassium acetate, 20 mM Tris-acetate, 10 mM magnesium acetate, 1 mM DTT, pH 7.9 @ 25°C) into clean Eppendorf tube.
15. 10 µl of 100 µM strand for ligation (1 nmol) (SEQ ID NO: 52, Figure 31b) and 10 µl of 100 µM helper strand for ligation (1 nmol) (SEQ ID NO: 53, Figure 31b) were added to the reaction mixture.
16. 40µl of Blunt/TA DNA Ligase NEB (180 units/µl) was added into the same tube.
17. Reaction mixture was then gently mixed by resuspension with a pipette, centrifuged at 13,000rpm for 5 seconds and incubated at room temperature for 20 minutes.
18. 40 µL of the 500 mM TCEP in 1M TRIS buffer pH 7.4 was added to the reaction mixture and allowed to react for 10 minutes at 37°C.
19. The reaction mixture was purified using QIAGEN Nucleotide removal kit eluting by 20 µL of 1x NEB Thermopol^{®} buffer.
20. Typically after incubation time had elapsed, reaction was terminated with the addition of TBE-Urea sample Buffer (Novex).

Gel Electrophoresis and DNA Visualization:
1. 5 µl of reaction mixture was added to 5 µl of TBE-Urea sample buffer (Novex) in a sterile 1.5 ml Eppendorf tube and heated to 95°C for 5 minutes using a heat ThermoMixer (Eppendorf).
2. 5 µl of the sample were then loaded into the wells of a 15% TBE-Urea gel 1.0mm x 10 well (Invitrogen) which contained preheated 1X TBE buffer Thermo Scientific (89mM Tris, 89mM boric acid and 2mM EDTA).
3. X-cell sure lock module (Novex) was fastened in place and electrophoresis performed at the following conditions; 260V, 90 amps for 40 minutes at room temperature.
4. The gel was visualized by ChemiDoc MP (BioRad) using Cy3 LEDS. Visualization and analysis was carried out on the Image lab 2.0 platform.

Measurement of purified DNA concentration was determined using the protocol below:
1. NanoDrop One (Thermo Scientific) was equilibrated by adding 2 µl of sterile distilled water (ELGA VEOLIA) onto the pedestal.
2. After equilibration, the water was gently wiped off using a lint-free lens cleaning tissue (Whatman).
3. NanoDrop One was blanked by adding 2 µl of Buffer EB QIAGEN (10mM Tris.CL, pH 8.5). Then step 2 was repeated after blanking.
4. DNA concentration was measured by adding 2 µl of the sample unto the pedestal and selecting the measure icon on the touch screen.
5. Purified DNA was run on a polyacrylamide gel and visualized in accordance with the procedure noted above in steps 5-8. No change in conditions or reagents was introduced.

The sample mixture was purified after each step using the protocol outlined below:
1. 500 µl of buffer PNI QIAGEN (5M guanidinium chloride) was added to the sample and mixed by gentle resuspension with a pipette.
2. The mixture was transferred into a QIAquick spin column (QIAGEN) and centrifuged for 1min at 6000 rpm.
3. After centrifugation, flow-through was discarded and 750 µl of buffer PE QIAGEN (10mM Tris-HCl pH 7.5 and 80% ethanol) was added into the spin column and centrifuged for 1min at 6000 rpm.
4. The flow-through was discarded and the spin column was centrifuged for an additional 1min at 13000 rpm to remove residual PE buffer.
5. The spin column was then placed in a sterile 1.5ml Eppendorf tube.
6. For DNA elution, 20 µl of appropriate buffer for the reaction was added to the centre of the column membrane and left to stand for 1 minute at room temperature.
7. The tube was then centrifuged at 13000 rpm for 1 minute. Eluted DNA concentration was measured and stored at -20°C for subsequent use.

### Example 7. Version 3 Chemistry - Complete Cycle on Double Hairpin Model.

This Example describes the synthesis of polynucleotides using 4 steps on a double- hairpin construct model: incorporation of 3'-O-modified dNTPs from the nick site; cleavage, ligation and deprotection with the first step taking place opposite a universal nucleotide, in this particular case an inosine base.

The method starts by controlled addition of a 3'-*O*-protected single nucleotide to an oligonucleotide by enzymatic incorporation by DNA polymerase followed by inosine cleavage, ligation and deprotection (Figure 32a).

### Materials and Methods

### Materials

1. 3'-*O*-modified dNTPs were synthesised in-housed according to the protocol described in PhD thesis Jian Wu: Molecular Engineering of Novel Nucleotide Analogues for DNA Sequencing by Synthesis. Columbia University, 2008. The protocol for synthesis is also described in the patent application publication: J. William Efcavitch, Juliesta E. Sylvester, Modified Template-Independent Enzymes for Polydeoxynucleotide Synthesis, Molecular Assemblies US2016/0108382A1.
2. Oligonucleotides were designed in house and obtained from Sigma-Aldrich (Figure 32b). The stock solutions are prepared in concentration of 100 µM.
3. Therminator IX DNA polymerase that has been engineered by New England BioLabs has enhanced ability to incorporate 3-*O*-modified dNTPs.

3'-*O*-azidomethyl-dTTP was tested for incorporation:
3'-*O*-azidomethyl-dTTP:

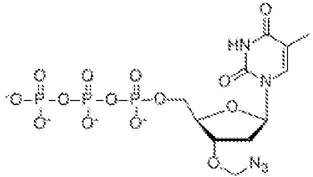

### Method

1. 2 µl of 10x Thermopol^{®} buffer (20 mM Tris-HCl, 10 mM (NH₄)₂SO₄, 10 mM KCl, 2 mM MgSO₄, 0.1% Triton^{®} X-100, pH 8.8, New England BioLabs) was mixed with 12.5 µl of sterile deionized water (ELGA VEOLIA) in 1.5ml Eppendorf tube.
2. 2 µl of 10 µM double hairpin model oligonucleotide (20 pmol, 1 equiv) (SEQ ID NO: 54, Figure 32b) were added to the reaction mixture.
3. 3'-*O*-modified-dTTP (2 µl of 100 µM) and MnCl₂ (1 µl of 40 mM) were added.
4. 1.5 µl of Therminator IX DNA polymerase (15 U, New England BioLabs) was then added.
5. The reaction was incubated for 10 minutes at 37°C.
6. The aliquot (5 µl) was taken out of the reaction mixture and 0.5 µl of natural dNTP mix was added and allowed to react for 10 minutes. The reaction was analysed by gel electrophoresis.
7. The reaction mixture was purified using the protocol outlined in purification steps 1-7.
8. The DNA sample was eluted by 20 µl of NEB Reaction buffer^{®} (50 mM potassium acetate, 20 mM Tris-acetate, 10 mM magnesium acetate, 1 mM DTT, pH 7.9 @ 25°C) into clean Eppendorf tube.
9. 1 µl of Human Endonuclease V (Endo V) NEB (30units/µl) was added into the same tube.
10. Reaction mixture was then gently mixed by resuspension with a pipette, centrifuged at 13,000 rpm for 5 seconds and incubated at 37°C for 1 hour.
11. After the incubation time had elapsed, the reaction was terminated by enzymatic heat inactivation (*i.e.* 65°C for 20 minutes).
12. The aliquot (5 µl) was taken out of the reaction mixture and analysed on polyacrylamide gel (15%) using TBE buffer and visualized by ChemiDoc MP imaging system (BioRad).
13. Reaction mixture was purified using the protocol outlined in purification steps 1-7 above.
14. The DNA sample was eluted by 20 µl of NEB Reaction buffer^{®} (50 mM potassium acetate, 20 mM Tris-acetate, 10 mM magnesium acetate, 1 mM DTT, pH 7.9 @ 25°C) into a clean Eppendorf tube.
15. 10 µl of 100 µM strand for ligation (1 nmol) (SEQ ID NO: 55, Figure 32b), were added to the reaction mixture.
16. 40µl of Blunt/TA DNA Ligase NEB (180 units/µl) was added into the same tube.
17. Reaction mixture was then gently mixed by resuspension with a pipette, centrifuged at 13,000rpm for 5 seconds and incubated at room temperature for 20 minutes.
18. 40 µL of the 500 mM TCEP in 1M TRIS buffer pH 7.4 was added to the reaction mixture and allowed to react for 10 minutes at 37°C.
19. The reaction mixture was purified using QIAGEN Nucleotide removal kit eluting by 20 µL of 1x NEB Thermopol^{®} buffer.
20. Typically after incubation time had elapsed, reaction was terminated with the addition of TBE-Urea sample Buffer (Novex).

Gel Electrophoresis and DNA Visualization:
1. 5 µl of reaction mixture was added to 5 µl of TBE-Urea sample buffer (Novex) in a sterile 1.5ml Eppendorf tube and heated to 95°C for 5 minutes using a heat ThermoMixer (Eppendorf).
2. 5 µl of the sample were then loaded into the wells of a 15% TBE-Urea gel 1.0mm x 10 well (Invitrogen) which contained preheated 1X TBE buffer Thermo Scientific (89mM Tris, 89mM boric acid and 2mM EDTA).
3. X-cell sure lock module (Novex) was fastened in place and electrophoresis performed at the following conditions; 260V, 90 amps for 40 minutes at room temperature.
4. The gel was visualized by ChemiDoc MP (BioRad) using Cy3 LEDS. Visualization and analysis was carried out on the Image lab 2.0 platform.

Measurement of purified DNA concentration was determined using the protocol below:
1. NanoDrop One (Thermo Scientific) was equilibrated by adding 2 µl of sterile distilled water (ELGA VEOLIA) onto the pedestal.
2. After equilibration, the water was gently wiped off using a lint-free lens cleaning tissue (Whatman).
3. NanoDrop One was blanked by adding 2 µl of Buffer EB QIAGEN (10mM Tris.CL, pH 8.5). Step 2 was then repeated after blanking.
4. DNA concentration was measured by adding 2 µl of the sample unto the pedestal and selecting the measure icon on the touch screen.
5. Purified DNA was run on a polyacrylamide gel and visualized in accordance with the procedure in section 2 steps 5-8. No change in conditions or reagents was introduced.

The sample mixture was purified after each step using the protocol outlined below:
1. 500 µl of buffer PNI QIAGEN (5M guanidinium chloride) was added to the sample and mixed by gentle resuspension with a pipette.
2. The mixture was transferred into a QIAquick spin column (QIAGEN) and centrifuged for 1min at 6000 rpm.
3. After centrifugation, flow-through was discarded and 750 µl of buffer PE QIAGEN (10mM Tris-HCl pH 7.5 and 80% ethanol) was added into the spin column and centrifuged for 1min at 6000 rpm.
4. The flow-through was discarded and the spin column was centrifuged for an additional 1min at 13000 rpm to remove residual PE buffer.
5. The spin column was then placed in a sterile 1.5ml Eppendorf tube.
6. For DNA elution, 20 µl of appropriate buffer for the reaction was added to the centre of the column membrane and left to stand for 1min at room temperature.
7. The tube was then centrifuged at 13000 rpm for 1minutes. Eluted DNA concentration was measured and stored at -20°C for subsequent use.

### Example 8. Version 2 Chemistry - Complete Two-Cycle Experiment on Double-Hairpin Model.

This example describes a complete two-cycle experiment for the synthesis of polynucleotides using 4 steps on a double-hairpin model: incorporation of 3'-*O*-modified dNTPs from the nick site; deprotection, cleavage, and ligation with the first step taking place opposite a complementary base.

The method starts by controlled addition of a 3'-*O*-protected single nucleotide to an oligonucleotide by enzymatic incorporation by DNA polymerase followed by deprotection, inosine cleavage and ligation, as depicted in the reaction schematic for the first cycle shown in Figure 33a. Figure 33b shows a reaction schematic for the second cycle.

### Materials and Methods

### Materials

1. 3'-*O*-modified dNTPs were synthesised in-house according to the protocol described in PhD thesis Jian Wu: Molecular Engineering of Novel Nucleotide Analogues for DNA Sequencing by Synthesis. Columbia University, 2008. The protocol for synthesis is also described in the patent application publication: J. William Efcavitch, Juliesta E. Sylvester, Modified Template-Independent Enzymes for Polydeoxynucleotide Synthesis, Molecular Assemblies US2016/0108382A1.
2. Oligonucleotides were designed in house and obtained from Sigma-Aldrich (Figure 33d). The stock solutions are prepared in concentration of 100 µM.
3. Therminator IX DNA polymerase that has been engineered by New England BioLabs has enhanced ability to incorporate 3'-*O*-modified dNTPs.

3'-*O*-azidomethyl-dTTP and 3'-O-azidomethyl-dCTP were used for incorporation:

| | |
|---|---|
| 3'-*O*-azidomethyl-dTTP: | 3'-*O*-azidomethyl-dCTP: |
| 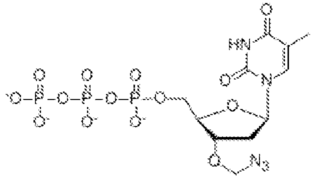 | 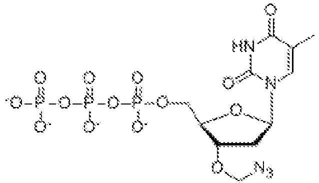 |

### Method

### 1^{st} cycle:

1. 2 µl of 10x Thermopol^{®} buffer (20 mM Tris-HCl, 10 mM (NH₄)₂SO₄, 10 mM KCl, 2 mM MgSO₄, 0.1% Triton^{®} X-100, pH 8.8, New England BioLabs) was mixed with 12.5 µl of sterile deionized water (ELGA VEOLIA) in 1.5ml Eppendorf tube.
2. 2 µl of 10 µM double hairpin model oligonucleotide (20 pmol, 1 equiv) (SEQ ID NO: 56, Figure 33d) were added to the reaction mixture.
3. 3'-*O*-modified-dTTP (2 µl of 100 µM) and MnCl₂ (1 µl of 40 mM) were added.
4. 1.5 µl of Therminator IX DNA polymerase (15 U, New England BioLabs) was then added.
5. The reaction was incubated for 10 minutes at 37°C.
6. The aliquot (5 µl) was taken out of the reaction mixture and 0.5 µl of natural dNTP mix was added and allowed to react for 10 min. The reaction was analysed by gel electrophoresis.
7. 40 µL of the 500 mM TCEP in 1M TRIS buffer pH=7.4 was added to the reaction mixture and allowed to react for 10 minutes at 37°C.
8. The reaction mixture was purified using the protocol outlined in purification steps 1-7.
9. The DNA sample was eluted by 20 µl of NEB Reaction buffer^{®} (50 mM potassium acetate, 20 mM Tris-acetate, 10 mM magnesium acetate, 1 mM DTT, pH 7.9 @ 25°C) into a clean Eppendorf tube.
10. 1µl of Human Endonuclease V (Endo V) NEB (30units/µl) was added into the same tube.
11. Reaction mixture was then gently mixed by resuspension with a pipette, centrifuged at 13,000 rpm for 5 seconds and incubated at 37°C for 1 hour.
12. After incubation time had elapsed, the reaction was terminated by enzymatic heat inactivation (*i.e.* 65°C for 20mins).
13. The aliquot (5 µl) was taken out of the reaction mixture and analysed on polyacrylamide gel (15%) using TBE buffer and visualized by ChemiDoc MP imaging system (BioRad).
14. Reaction mixture was purified by QIAGEN Nucleotide Removal kit using the protocol outlined in purification steps 1-7.
15. The DNA sample was eluted by 20 µl of NEB Reaction buffer^{®} (50 mM potassium acetate, 20 mM Tris-acetate, 10 mM magnesium acetate, 1 mM DTT, pH 7.9 @ 25°C) into a clean Eppendorf tube.
16. 10 µl of 100 µM strand for ligation (1 nmol) (SEQ ID NO: 57, Figure 33d), were added to the reaction mixture.
17. 40µl of Blunt/TA DNA Ligase NEB (180 units/µl) was added into the same tube.
18. Reaction mixture was then gently mixed by resuspension with a pipette, centrifuged at 13,000rpm for 5 seconds and incubated at room temperature for 20 mins.
19. Reaction mixture was purified by Streptavidin Magnetic Beads kit using the protocol outlined in purification steps 1-5.
20. Unligated oligonucleotide was digested by Lambda Exonuclease.
21. Reaction mixture was purified by QIAGEN Nucleotide Removal kit using the protocol outlined in purification steps 1-7.
22. The DNA sample was eluted by 20 µl of NEB Reaction buffer^{®} (50 mM potassium acetate, 20 mM Tris-acetate, 10 mM magnesium acetate, 1 mM DTT, pH 7.9 @ 25°C) into a clean Eppendorf tube.

### 2^{nd} cycle:

23. 3'-O-modified-dCTP (2 µl of 100 µM) and MnCl₂ (1 µl of 40 mM) were added.
24. 1.5 µl of Therminator IX DNA polymerase (15 U, New England BioLabs) was then added.
25. The reaction was incubated for 10 minutes at 37°C.
26. The aliquot (5 µl) was taken out of the reaction mixture and 0.5 µl of natural dNTP mix was added and reacted for 10 min. The reaction was analysed by gel electrophoresis.
27. 40 µL of the 500 mM TCEP in 1M TRIS buffer pH=7.4 was added to the reaction mixture and reacted for 10 minutes at 37°C.
28. The reaction mixture was purified using the protocol outlined in purification steps 1-7.
29. The DNA sample was eluted by 20 µl of NEB Reaction buffer^{®} (50 mM potassium acetate, 20 mM Tris-acetate, 10 mM magnesium acetate, 1 mM DTT, pH 7.9 @ 25°C) into a clean Eppendorf tube.
30. 1µl of Human Endonuclease V (Endo V) NEB (30units/µl) was added into the same tube.
31. The reaction mixture was then gently mixed by resuspension with a pipette, centrifuged at 13,000 rpm for 5 seconds and incubated at 37°C for 1 hour.
32. After incubation time had elapsed, the reaction was terminated by enzymatic heat inactivation *(i.e.* 65°C for 20mins).
33. The aliquot (5 µl) was taken out of the reaction mixture and analysed on polyacrylamide gel (15%) using TBE buffer and visualized by ChemiDoc MP imaging system (BioRad).
34. The reaction mixture was purified using the protocol outlined in purification steps 1-7.
35. The DNA sample was eluted by 20 µl of NEB Reaction buffer^{®} (50 mM potassium acetate, 20 mM Tris-acetate, 10 mM magnesium acetate, 1 mM DTT, pH 7.9 @ 25°C) into clean Eppendorf tube.
36. 10 µl of 100 µM strand for ligation (1 nmol) (SEQ ID NO: 58, Figure 33d), were added to the reaction mixture.
37. 40µl of Blunt/TA DNA Ligase NEB (180 units/µl) was added into the same tube.
38. Reaction mixture was then gently mixed by resuspension with a pipette, centrifuged at 13,000rpm for 5seconds and incubated at room temperature for 10mins.
39. After incubation time had elapsed, the reaction was terminated with the addition of TBE-Urea sample Buffer (Novex).

Gel Electrophoresis and DNA Visualization:
1. 5 µl of reaction mixture was added to 5 µl of TBE-Urea sample buffer (Novex) in a sterile 1.5ml Eppendorf tube and heated to 95°C for 5 mins using a heat ThermoMixer (Eppendorf).
2. 5 µl of the sample were then loaded into the wells of a 15% TBE-Urea gel 1.0mm x 10 well (Invitrogen) which contained preheated 1X TBE buffer Thermo Scientific (89mM Tris, 89mM boric acid and 2mM EDTA).
3. X-cell sure lock module (Novex) was fastened in place and subjected to electrophoresis by applying the following conditions; 260V, 90 amps for 40 mins at room temperature.
4. The gel was visualized by ChemiDoc MP (BioRad) using Cy3 LEDS. Visualization and analysis was carried out on the Image lab 2.0 platform.

Measurement of purified DNA concentration was determined using the protocol below:
1. NanoDrop One (Thermo Scientific) was equilibrated by adding 2 µl of sterile distilled water (ELGA VEOLIA) onto the pedestal.
2. After equilibration, the water was gently wiped off using a lint-free lens cleaning tissue (Whatman).
3. NanoDrop One was blanked by adding 2 µl of Buffer EB QIAGEN (10mM Tris.CL, pH 8.5). Then step 2 was repeated after blanking.
4. DNA concentration was measured by adding 2 µl of the sample unto the pedestal and selecting the measure icon on the touch screen.

The sample mixture was purified by QIAGEN Nucleotide Removal kit using the protocol outlined below:
1. 500 µl of buffer PNI QIAGEN (5M guanidinium chloride) was added to the sample and mixed by gentle resuspension with a pipette.
2. The mixture was transferred into a QIAquick spin column (QIAGEN) and centrifuged for 1min at 6000 rpm.
3. After centrifugation, flow-through was discarded and 750 µl of buffer PE QIAGEN (10mM Tris-HCl pH 7.5 and 80% ethanol) was added into the spin column and centrifuged for 1min at 6000 rpm.
4. The flow-through was discarded and the spin column was centrifuged for an additional 1min at 13000 rpm to remove residual PE buffer.
5. The spin column was then placed in a sterile 1.5ml Eppendorf tube.
6. For DNA elution, 20 µl of appropriate buffer for the reaction was added to the centre of the column membrane and left to stand for 1min at room temperature.
7. The tube was then centrifuged at 13000 rpm for 1min.

After the ligation step, the sample mixture was purified using Streptavidin Magnetic Beads via the protocol outlined below:
1. 100 µl of Streptavidin Magnetic Beads (New England BioLabs) were washed 3 times by 200 µl of binding buffer (20mM TRIS, 500 mM NaCl, pH = 7.4).
2. Reaction mixture after ligation step is mixed with 10 volumes of binding buffer (20mM TRIS, 500 mM NaCl, pH = 7.4) and incubated with Streptavidin Magnetic Beads for 15 minutes at 20 °C.
3. Streptavidin Magnetic Beads were washed 3 times by 200 µl of binding buffer (20mM TRIS, 500 mM NaCl, pH = 7.4).
4. Streptavidin Magnetic Beads were washed 3 times by deionized water.
5. The oligonucleotides were eluted by 40 µl of deionized water by heating to 95°C for 3 minutes.

The results shown in Figure 33c demonstrate the performance two complete synthesis cycles using an exemplary method of the invention.

### Example 9. Version 2 Chemistry - Complete Three-Cycle Experiment on Single-Hairpin Model.

This example describes a complete three-cycle experiment for the synthesis of polynucleotides using 5 steps on a double-hairpin model: incorporation of 3'-O-modified dNTPs from the nick site, deprotection, cleavage, ligation and denaturation step with the first step taking place opposite a complementary base.

Exemplary schematic overviews of the method are shown in Figures 38, 39 and 40.

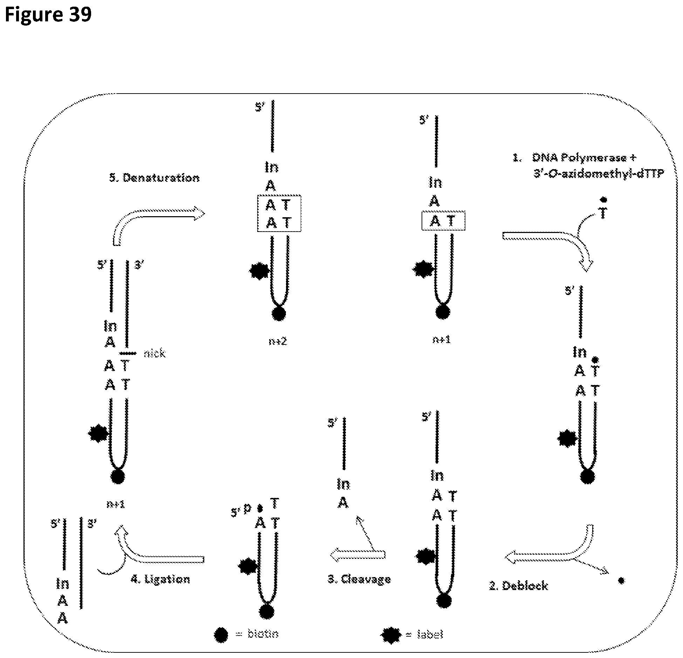

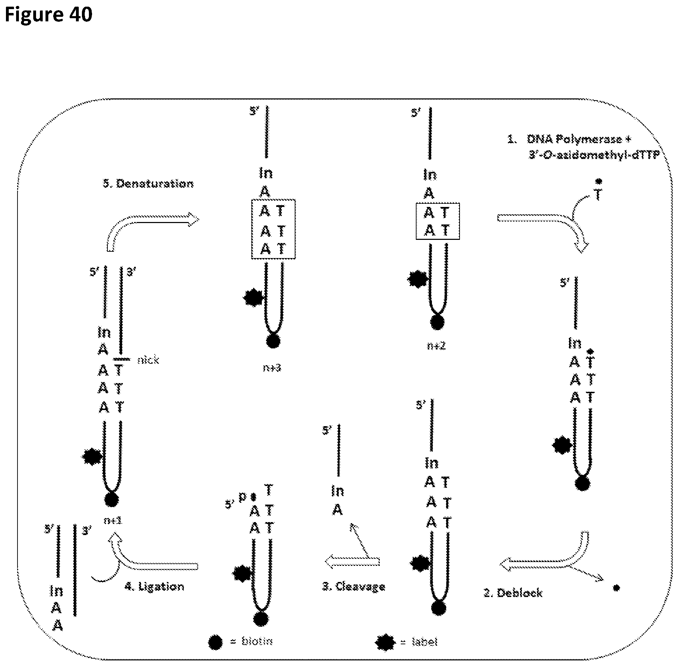

The method starts by the controlled addition of a 3'-O-protected single nucleotide to an oligonucleotide by enzymatic incorporation by DNA polymerase followed by deprotection, cleavage, ligation, and denaturation of the helper strand. Figure 38 shows the 1st full cycle involving enzymatic incorporation, deprotection, cleavage, ligation and denaturation steps. In this example the oligonucleotide is extended by T nucleotide. Figure 39 shows the 2nd full cycle following the 1st cycle involving enzymatic incorporation, deprotection, cleavage, ligation steps, and denaturation steps. In this example the oligonucleotide is extended by T nucleotide. Figure 40 shows the 3rd full cycle following the 2nd cycle involving enzymatic incorporation, deprotection, cleavage, ligation, and denaturation steps. In this example the oligonucleotide is extended by T nucleotide.

### Materials and Methods

### Materials

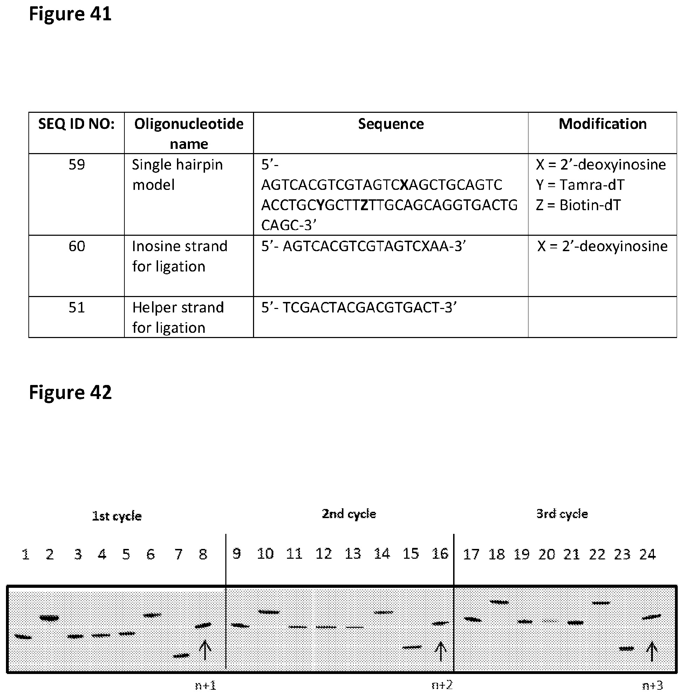

1. 3'-O-modified dNTPs were synthesised in-house according to the protocol described in PhD thesis Jian Wu: Molecular Engineering of Novel Nucleotide Analogues for DNA Sequencing by Synthesis, Columbia University, 2008. The protocol for synthesis is also described in the patent application publication: J. William Efcavitch, Juliesta E. Sylvester, Modified Template-Independent Enzymes for Polydeoxynucleotide Synthesis, Molecular Assemblies US2016/0108382A1.
2. Oligonucleotides were designed in house and obtained from Integrated DNA Technologies, Sigma-Aldrich (Figure 41). The stock solutions are prepared in concentration of 100 µM.
3. Therminator X DNA polymerase was used that has been engineered by New England BioLabs with enhanced ability to incorporate 3-O-modified dNTPs. Any DNA polymerase or other enzyme that could incorporate modified dNTPs could alternatively be used.

3'-O-azidomethyl-dTTP was used for incorporation:

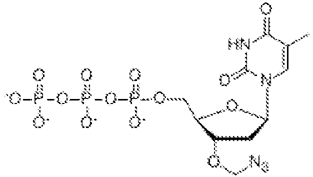

### Method

### 1^{st} cycle:

1. 20 µl of 10x Thermopol^{®} buffer (20 mM Tris-HCl, 10 mM (NH₄)₂SO₄, 10 mM KCl, 2 mM MgSO₄, 0.1% Triton^{®} X-100, pH 8.8, New England BioLabs) and MnCl₂ solution (10 µl of 40 mM) were mixed with 139 µl of sterile deionized water (ELGA VEOLIA) in 1.5ml Eppendorf tube.
2. 20 µl of 100 µM single hairpin model oligonucleotide (2 nmol, 1 equiv) (SEQ ID NO: 59, Figure 41) was added to the reaction mixture.
3. The aliquot (4 µl) was taken out of the reaction mixture and 0.5 µl of natural dNTP mix (4mM) and 0.5 µl of Bst DNA polymerase and 0.5 µl of Sulfolobus DNA polymerase IV were added and allowed to react for 10 min. The reaction was analysed by gel electrophoresis.
4. 3'-O-modified-dTTP (10 µl of 2 mM) was added.
5. 5 µl of Therminator X DNA polymerase (50 U, New England BioLabs) was then added. However, any DNA polymerase or other enzyme that could incorporate modified dNTPs could be used.
6. The reaction was incubated for 30 minutes at 37°C.
7. The reaction mixture was purified using QIAGEN Nucleotide Removal kit outlined in purification steps 66-72.
8. The DNA sample was eluted by 200 µl of TE buffer into a clean Eppendorf tube.
9. The aliquot (4 µl) was taken out of the reaction mixture and 0.5 µl of natural dNTP mix (4mM) and 0.5 µl of Bst DNA polymerase and 0.5 µl of Sulfolobus DNA polymerase IV were added and allowed to react for 10 min. The reaction was analysed by gel electrophoresis.
10. 400 µL of the 500 mM TCEP was added to the reaction mixture and allowed to react for 10 minutes at 37°C.
11. The reaction mixture was purified using QIAGEN Nucleotide Removal kit outlined in purification steps 66-72.
12. The DNA sample was eluted by 150 µl of NEB Reaction buffer^{®} (50 mM potassium acetate, 20 mM Tris-acetate, 10 mM magnesium acetate, 1 mM DTT, pH 7.9 @ 25°C) into clean Eppendorf tube.
13. The aliquot (4 µl) was taken out of the reaction mixture and 0.5 µl of natural dNTP mix (4mM) and 0.5 µl of Bst DNA polymerase and 0.5 µl of Sulfolobus DNA polymerase IV were added and allowed to react for 10 min. The reaction was analysed by gel electrophoresis.
14. 5 µl of Human Endonuclease V (Endo V) NEB (30units/µl) was added to the eluate and incubated at 37°C for 30 minutes. Any suitable alternative endonuclease could be used.
15. After incubation time had elapsed, the reaction was terminated by enzymatic heat inactivation at 65°C for 20mins.
16. An aliquot (5 µl) was taken out of the reaction mixture and analysed on a polyacrylamide gel.
17. The reaction mixture was purified by QIAGEN Nucleotide Removal kit using the protocol outlined in purification steps 66-72.
18. The DNA sample was eluted by 100 µl of T3 DNA ligase buffer (2x concentrate) into a clean Eppendorf tube.
19. 20 µl of 100 µM inosine strand for ligation (2 nmol) and 20 µl of 100 µM helper strand for ligation (2 nmol) (SEQ ID NO: 60, 51, Figure 41), and 40 µl of water were added to the reaction mixture.
20. 20 µl of T3 DNA Ligase NEB (3000 units/µl) was added into the same tube (this could include any DNA ligating enzyme) and incubated at room temperature for 30 mins.

The reaction mixture was purified using the protocol for Streptavidin Magnetic Beads kit including the denaturation step outlined in purification steps 73-78.
21. The reaction mixture was purified using the protocol for QIAGEN Nucleotide Removal kit outlined in purification steps 66-72.
22. The DNA sample was eluted by 100 µl of TE buffer into a clean Eppendorf tube.

### 2^{nd} cycle:

23. 15 µl of 10x Thermopol^{®} buffer (20 mM Tris-HCl, 10 mM (NH₄)₂SO₄, 10 mM KCl, 2 mM MgSO₄, 0.1% Triton^{®} X-100, pH 8.8, New England BioLabs), MnCl₂ solution (7.5 µl of 40 mM) and 19 µl of deionized water was added.
24. An aliquot (4 µl) was taken out of the reaction mixture and 0.5 µl of natural dNTP mix (4mM) and 0.5 µl of Bst DNA polymerase and 0.5 µl of Sulfolobus DNA polymerase IV were added and allowed to react for 10 min. The reaction was analysed by gel electrophoresis.
25. 3'-O-modified-dTTP (7.5 µl of 2 mM) was added.
26. 5 µl of Therminator X DNA polymerase (50 U, New England BioLabs) was then added. Any DNA polymerase that could incorporate modified dNTPs could be used.
27. The reaction was incubated for 30 minutes at 37°C.
28. The reaction mixture was purified using QIAGEN Nucleotide Removal kit outlined in purification steps 66-72.
29. The DNA sample was eluted by 100 µl of TE buffer into a clean Eppendorf tube.
30. An aliquot (4 µl) was taken out of the reaction mixture and 0.5 µl of natural dNTP mix (4mM) and 0.5 µl of Bst DNA polymerase and 0.5 µl of Sulfolobus DNA polymerase IV were added and allowed to react for 10 min. The reaction was analysed by gel electrophoresis.
31. 200 µL of the 500 mM TCEP was added to the reaction mixture and allowed to react for 10 minutes at 37°C.
32. The reaction mixture was purified using QIAGEN Nucleotide Removal kit outlined in purification steps 66-72.
33. The DNA sample was eluted by 100 µl of NEB Reaction buffer^{®} (50 mM potassium acetate, 20 mM Tris-acetate, 10 mM magnesium acetate, 1 mM DTT, pH 7.9 @ 25°C) into a clean Eppendorf tube.
34. The aliquot (4 µl) was taken out of the reaction mixture and 0.5 µl of natural dNTP mix (4mM) and 0.5 µl of Bst DNA polymerase and 0.5 µl of Sulfolobus DNA polymerase IV were added and allowed to react for 10 min. The reaction was analysed by gel electrophoresis.
35. 5 µl of Human Endonuclease V (Endo V) NEB (30units/µl) was added to the eluate. and incubated at 37°C for 30 minutes. Any suitable alternative endonuclease could be used.
36. After incubation time had elapsed, the reaction was terminated by enzymatic heat inactivation at 65°C for 20mins.
37. The aliquot (5 µl) was taken out of the reaction mixture and analysed on a polyacrylamide gel.
38. The reaction mixture was purified by QIAGEN Nucleotide Removal kit using the protocol outlined in purification steps 66-72.
39. The DNA sample was eluted by 60 µl of T3 DNA ligase buffer (2x concentrate) into a clean Eppendorf tube.
40. 20 µl of 100 µM inosine strand for ligation (2 nmol) and 20 µl of 100 µM helper strand for ligation (2 nmol) (SEQ ID NO: 60, 51, Figure 41), and 10 µl of deionized water were added to the reaction mixture.
41. 10 µl of T3 DNA Ligase NEB (3000 units/µl) was added into the same tube and incubated at room temperature for 30 mins. Any suitable DNA ligase could be used.
42. The reaction mixture was purified using the protocol for Streptavidin Magnetic Beads kit including denaturation step outlined in purification steps 73-78.
43. The reaction mixture was purified using the protocol for QIAGEN Nucleotide Removal kit outlined in purification steps 66-72.
44. The DNA sample was eluted by 46 µl of TE buffer into a clean Eppendorf tube.

### 3^{rd} cycle:

45. 6 µl of 10x Thermopol^{®} buffer (20 mM Tris-HCl, 10 mM (NH₄)₂SO₄, 10 mM KCl, 2 mM MgSO₄, 0.1% Triton^{®} X-100, pH 8.8, New England BioLabs), MnCl₂ solution (3 µl of 40 mM) was added.
46. An aliquot (4 µl) was taken out of the reaction mixture and 0.5 µl of natural dNTP mix (4mM) and 0.5 µl of Bst DNA polymerase and 0.5 µl of Sulfolobus DNA polymerase IV were added and allowed to react for 10 min. The reaction was analysed by gel electrophoresis.
47. 3'-O-modified-dTTP (6 µl of 200 µM) was added.
48. 3 µl of Therminator X DNA polymerase (30 U, New England BioLabs) was then added. Any DNA polymerase or other suitable enzyme that could incorporate modified dNTPs could be used.
49. The reaction was incubated for 30 minutes at 37°C.
50. The reaction mixture was purified using QIAGEN Nucleotide Removal kit outlined in purification steps 66-72.
51. The DNA sample was eluted by 50 µl of TE buffer into a clean Eppendorf tube.
52. The aliquot (4 µl) was taken out of the reaction mixture and 0.5 µl of natural dNTP mix (4mM) and 0.5 µl of Bst DNA polymerase and 0.5 µl of Sulfolobus DNA polymerase IV were added and allowed to react for 10 min. The reaction was analysed by gel electrophoresis.
53. 100 µL of the 500 mM TCEP was added to the reaction mixture and allowed to react for 10 minutes at 37°C.
54. The reaction mixture was purified using QIAGEN Nucleotide Removal kit outlined in purification steps 66-72.
55. The DNA sample was eluted by 49 µl of NEB Reaction buffer^{®} (50 mM potassium acetate, 20 mM Tris-acetate, 10 mM magnesium acetate, 1 mM DTT, pH 7.9 @ 25°C) into a clean Eppendorf tube.
56. An aliquot (4 µl) was taken out of the reaction mixture and 0.5 µl of natural dNTP mix (4mM) and 0.5 µl of Bst DNA polymerase and 0.5 µl of Sulfolobus DNA polymerase IV were added and allowed to react for 10 min. The reaction was analysed by gel electrophoresis.
57. 5 µl of Human Endonuclease V (Endo V) NEB (30units/µl) was added to the eluate and incubated at 37°C for 30 minutes. Any suitable endonuclease could alternatively be used.
58. After incubation time had elapsed, the reaction was terminated by enzymatic heat inactivation at 65°C for 20mins.
59. The aliquot (5 µl) was taken out of the reaction mixture and analysed on a polyacrylamide gel.
60. The reaction mixture was purified by QIAGEN Nucleotide Removal kit using the protocol outlined in purification steps 66-72.
61. The DNA sample was eluted by 30 µl of T3 DNA ligase buffer (2x concentrate) into a clean Eppendorf tube.
62. 10 µl of 100 µM inosine strand for ligation (2 nmol), 10 µl of 100 µM helper strand for ligation (2 nmol) (SEQ ID NO: 60, 51, Figure 41) and 5 µl of water were added to the reaction mixture.
63. 5 µl of T3 DNA Ligase NEB (3000 units/µl) was added into the same tube. (This could include any DNA ligating enzyme) and incubated at room temperature for 30 mins.
64. The reaction mixture was analysed by gel electrophoresis.

Purification of the reaction mixture by QIAGEN Nucleotide Removal kit after incorporation, deblock and cleavage steps using the protocol outlined below:
65. 10 volumes of buffer PNI QIAGEN (5M guanidinium chloride) was added to the sample and mixed by gentle resuspension with a pipette.
66. The mixture was transferred into a QIAquick spin column (QIAGEN) and centrifuged for 1min at 6000 rpm.
67. After centrifugation, flow-through was discarded and 750 µl of buffer PE QIAGEN (10mM Tris-HCl pH 7.5 and 80% ethanol) was added into the spin column and centrifuged for 1min at 6000 rpm.
68. The flow-through was discarded and the spin column was centrifuged for an additional 1min at 13000 rpm to remove residual PE buffer.
69. The spin column was then placed in a sterile 1.5ml Eppendorf tube.
70. For DNA elution, 20-200 µl of appropriate buffer for the reaction was added to the centre of the column membrane and left to stand for 1 min at room temperature.
71. The tube was then centrifuged at 13000 rpm for 1min.

Purification of the reaction after the ligation step using Streptavidin Magnetic Beads involving denaturation step was performed via the protocol outlined below:
72. 100 µl of Streptavidin Magnetic Beads (New England BioLabs) were washed 3 times by 200 µl of binding buffer (20mM TRIS, 500 mM NaCl, pH = 7.4).
73. Reaction mixture after ligation step is mixed with 10 volumes of binding buffer (20mM TRIS, 500 mM NaCl, pH = 7.4) and allowed to incubate with Streptavidin Magnetic Beads for 15 minutes at 20 °C.
74. Streptavidin Magnetic Beads were washed 3 times by 200 µl of binding buffer (20mM TRIS, 500 mM NaCl, pH = 7.4).
75. To remove the helper strand, Streptavidin Magnetic Beads were heated to 80°C in 200 µl of binding buffer (20mM TRIS, 500 mM NaCl, pH = 7.4), placed to magnet and supernatant was quickly discarded.
76. Streptavidin Magnetic Beads were washed 3 times with deionized water.
77. The oligonucleotides were eluted by 50-100 µl of deionized water by heating to 95°C for 3 minutes.

### Results and conclusion

Figure 42 depicts a gel showing reaction products corresponding to a full three-cycle experiment comprising: incorporation, deblock, cleavage and ligation steps. The results shown demonstrate the performance of three complete synthesis cycles using an exemplary method of the invention.

### Example 10. Derivatization of a polyacrylamide surface and subsequent immobilisation of molecules.

This example describes the presentation of bromoacetyl groups on a polyacrylamide surface using N-(5- bromoacetamidylpentyl) acrylamide (BRAPA) and the subsequent surface immobilisation of thiolated molecules by their covalent coupling to bromoacetyl groups.

### Materials and Methods

Glass microscope slides and coverslips were cleaned by ultrasonication in acetone, ethanol and water sequentially for 10 mins each and dried with Argon. Clean glass coverslips were silanised with Trichloro(1H,1H,2H,2H-perfluorooctyl)silane in vapor phase in a polystyrene petri dish, sonicated twice in ethanol and dried with Ar ('fluorinated coverslips' hereafter). On glass microscope slides, 4% acrylamide/N,N'-Methylenebisacrylamide (19:1) solution was mixed with 100 µl of 10% (w/v) ammonium persulphate (APS), 10 µl of tetramethylethylenediamine (TEMED) spiked with N-(5-bromoacetamidylpentyl) acrylamide (BRAPA) at 0, 0.1, 0.2, and 0.3% (w/v) and quickly dispensed into a 4 mm diameter rubber gasket and subsequently sandwiched with a fluorinated coverslip with the fluorinated side facing towards the acrylamide solution and polymerised for 10 mins. After 10 mins, the surfaces were immersed in deionised water and left immersed for a total of 4 hrs, during which time the fluorinated coverslips were carefully removed. The polymerised polyacrylamide surfaces were dried with Argon.

The polyacrylamide surfaces were subsequently exposed to thiolated polyethylene glycol (1kDa) fluorescein (FITC-PEG-SH), and carboxylated polyethylene glycol (1 kDa) fluorescein (FITC-PEG-COOH) as a negative control in sodium phosphate buffer (10mM, pH 8) for 1 hr and subsequently washed sequentially with sodium phosphate buffer (10mM, pH 7) and the same buffer containing 0.05% Tween20/0.5M NaCl to eliminate non-specifically adsorbed thiolated and carboxylated fluorophores. The surfaces were subsequently imaged by ChemiDoc (Bio-Rad) in the fluorescein channel.

### Results and conclusion

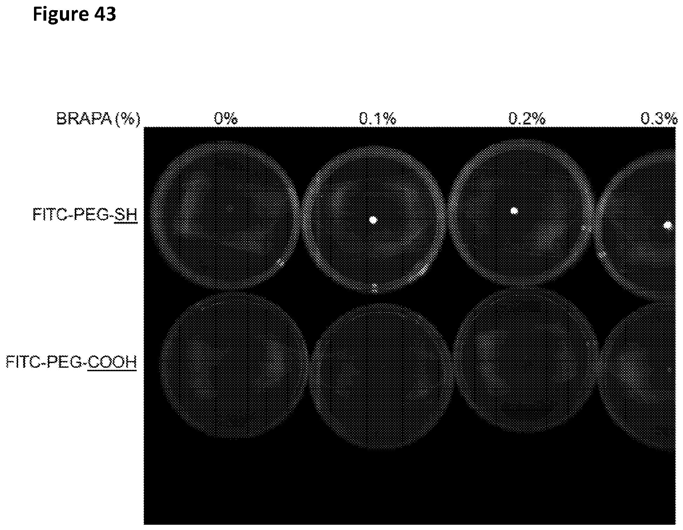

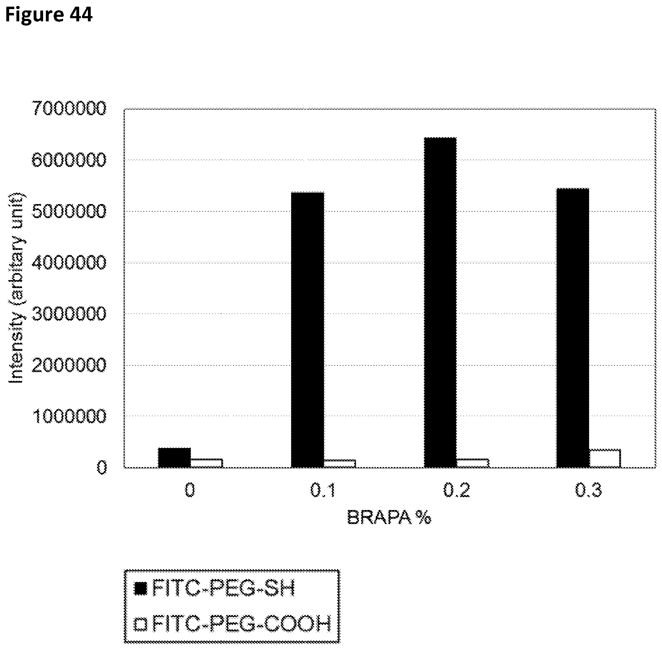

Figure 43 shows fluorescence signals and Figure 44 shown measured fluorescence from polyacrylamide gel surfaces spiked with different amount of BRAPA exposed to FITC-PEG-SH and FITC-PEG-COOH. Immobilisation of fluorescein was only successful with polyacrylamide surfaces that were spiked with BRAPA and solely with thiolated fluorescein, with close to zero non-specific adsorption of the carboxylated fluorescein.

Significantly high positive fluorescence signals were obtained from polyacrylamide surfaces containing BRAPA (BRAPA 0.1, 0.2 and 0.3%) and only from thiolated molecules (FITC-PEG-SH) compared to those polyacrylamide surfaces without BRAPA (BRAPA 0%) and those polyacrylamide surfaces containing BRAPA and carboxylated molecules (FITC-PEG-COOH). The results indicate that specific covalent coupling has occurred between the bromoacetyl moiety from the surface and the thiol moiety from the fluorescein tagged molecules.

The results demonstrate that molecules, such as a molecule comprising a support strand and a synthesis strand for use in the methods of the present invention, can readily be immobilised on a surface substrate compatible with the polynucleotide synthesis reactions described herein.

### Example 11. Surface immobilisation of hairpin DNA oligomers and subsequent incorporation of fluorescently labelled deoxynucleoside triphosphates.

This example describes:
(1) a method of presenting bromoacetyl groups on a thin polyacrylamide surface;
(2) the subsequent immobilisation of hairpin DNA via covalent coupling of thiophosphate functionalised hairpin DNA with or without a linker; and
(3) the incorporation of 2'-deoxynucleotide triphosphate (dNTP) into hairpin DNA.

The method is compatible with virtually any type of material surface (e.g. metals, polymers etc).

### (1): Fabrication of a bromoacetyl functionalised thin polyacrylamide surface

### Materials and Methods

Glass microscope slides were first cleaned by ultrasonication in neat Decon 90 (30 mins), water (30 mins), 1M NaOH (15 mins), water (30 mins), 0.1M HCl (15 mins), water (30 mins) and finally dried with Argon.

2% (w/v) acrylamide monomer solution was first made by dissolving 1 g of acrylamide monomer in 50 ml of water. The acrylamide monomer solution was vortexed and degassed in argon for 15 mins. N-(5-bromoacetamidylpentyl) acrylamide (BRAPA, 82.5 mg) was dissolved in 825 µl of DMF and added to the acrylamide monomer solution and vortexed further. Finally, 1 ml of 5% (w/v) potassium persulphate (KPS) and 115 µl of neat tetramethylethylenediamine (TEMED) were added to the acrylamide solution, vortexed and the clean glass microscope slides were exposed to this acrylamide polymerisation mixture for 90 mins. After 90 mins, the surfaces were washed with deionised water and dried with argon. These surfaces will be referred to as 'BRAPA modified surfaces' in this example hereafter. As a negative control, polyacrylamide surfaces without BRAPA was also made in a similar manner as described above by excluding the addition of BRAPA solution into the acrylamide monomer solution. These surfaces will be referred to as 'BRAPA control surface' in this example hereafter.

### (2): Covalent coupling of thiophosphate functionalised hairpin DNA onto polyacrylamide surfaces

### Materials and Methods

Rubber gaskets with a 4 mm diameter circular opening were placed and secured onto BRAPA modified and BRAPA control surfaces. The surfaces were first primed with sodium phosphate buffer (10 mM, pH 7) for 10 mins. The buffer was subsequently removed and the surfaces were exposed to 5'-fluorescently labelled (Alexa 647) hairpin DNA oligomers with and without a linker modified with six and single thiophosphates respectively at a 1 µM concentration and incubated for 1 hr in the dark. BRAPA modified surfaces were also incubated with DNA oligomers with and without linker but without thiophosphates as a control (referred to 'oligomer control surfaces' in this example hereafter). After incubation, the surfaces were rinsed in sodium phosphate (100 mM, pH 7) followed by Tris-EDTA buffer (10mM Tris, 10mM EDTA, pH 8) and finally with water. To remove any non-specifically adsorbed DNA oligomers, the surfaces were subsequently washed with water containing 1M sodium chloride and 0.05% (v/v) Tween20, washed with water and dried with argon. The surfaces were scanned on ChemiDoc imager in the Alexa 647 channel.

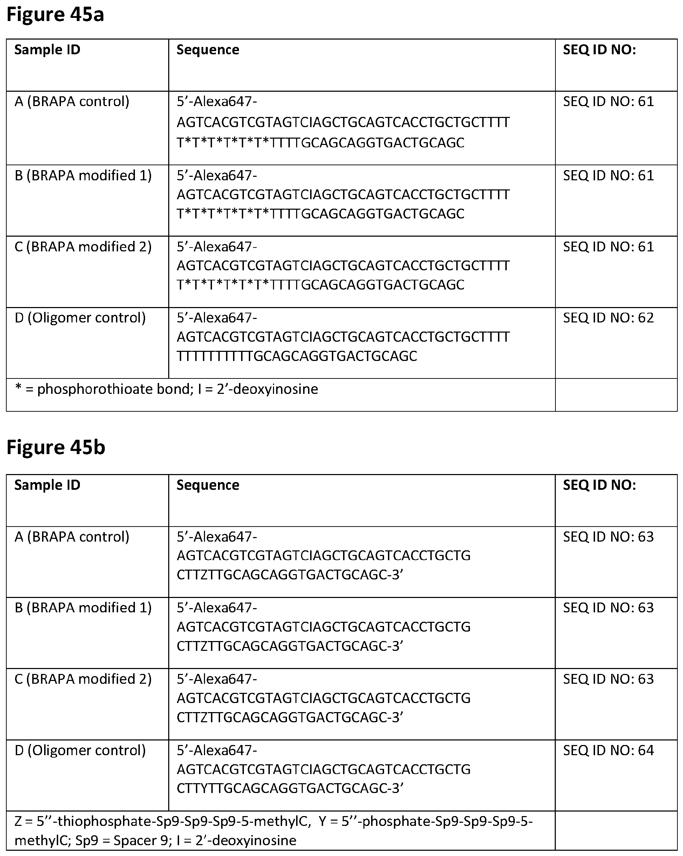

Figure 45a shows the sequences of hairpin DNA without a linker immobilised on different samples. Figure 45b shows the sequences of hairpin DNA with a linker immobilised on different samples.

### Results

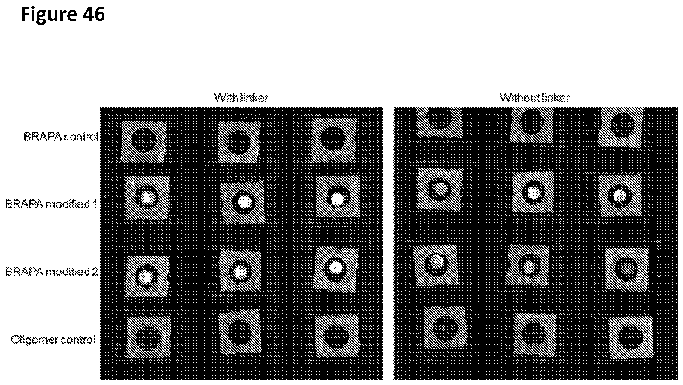

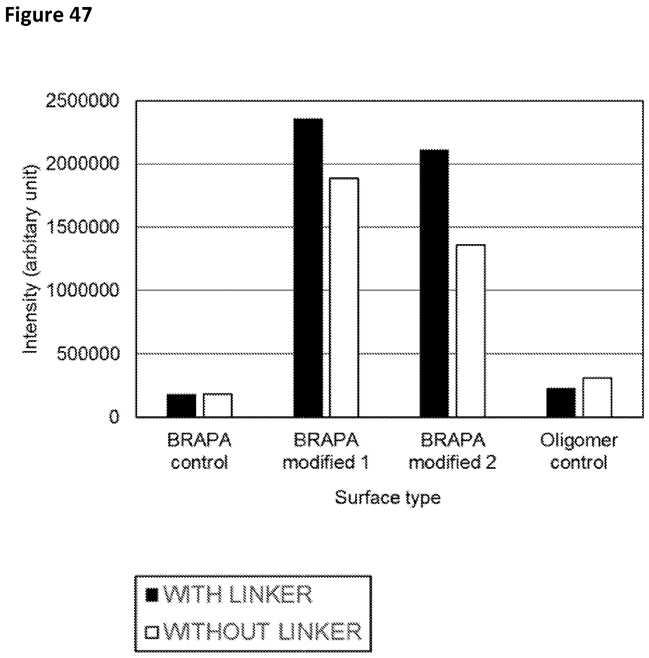

Results are shown in Figures 46 and 47. Figure 46 shows fluorescence signals originating from hairpin DNA oligomers with and without a linker immobilised onto bromoacetyl functionalised polyacrylamide surfaces, but not from BRAPA or oligomer controls.

Figure 47 shows measured fluorescence intensity following DNA immobilisation on polyacrylamide surface. The Figure shows the surface fluorescence signals obtained from various polyacrylamide surfaces and shows that significantly higher signals were obtained from hairpin DNA oligomers immobilised onto BRAPA modified surfaces compared to BRAPA and oligomer control surfaces (as described in (2)), due to successful covalent immobilisation of DNA onto bromoacetyl functionalised polyacrylamide surfaces.

### Conclusion

Fluorescence signals from DNA were only prominently present from BRAPA modified surfaces that were spiked with BRAPA, indicative of successful covalent coupling of DNA onto the surface via the thiophosphate functionality. Homogenous and higher signals were obtained from DNA with the linker compared to DNA without the linker.

### (3): Incorporation of triphosphates into hairpin DNA oligomer with a linker

### Materials and Methods

Rubber gaskets with a 9 mm diameter circular opening were placed on the BRAPA modified surfaces immobilised with the DNA oligomer with the linker and primed with incorporation buffer (50 mM TRIS pH 8, 1 mM EDTA, 6 mM MgSO₄, 0.05% tween20 and 2 mM MnCl₂) for 10 mins. The surfaces were subsequently exposed to incorporation buffer containing DNA polymerase (0.5U/µl Therminator X DNA polymerase) and triphosphates (20 µM Alexa 488 labelled dUTP) and incubated for 1 hr (referred to as 'polymerase surface' in this example hereafter). Additional set of surfaces were also exposed to incorporation buffer without Therminator X DNA polymerase for 1 hr as a negative control (referred to as 'negative surface' in this example hereafter). After 1 hr, both types of sample were washed in water, subsequently exposed to water containing 1M sodium chloride and 0.05% (v/v) Tween20, and washed again with water. Fluorescence signals from the surfaces were measured using ChemiDoc in the Alexa 647 and Alexa 488 channels to monitor both the presence of hairpin DNA (Alexa 647) and incorporation of dUTP (Alexa 488).

### Results

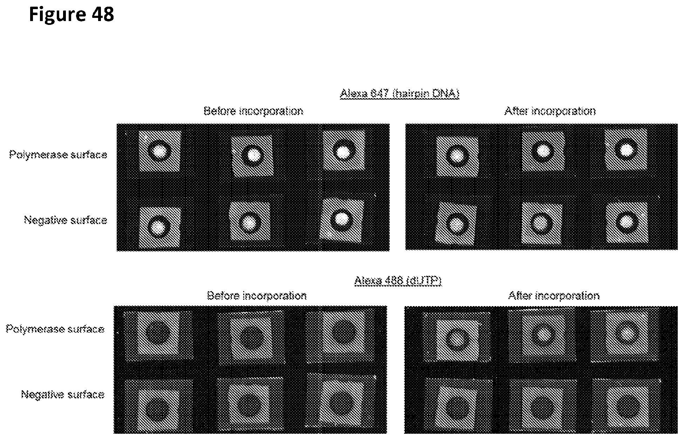

Figure 48 shows fluorescence signals detected from Alexa 647 and Alexa 488 channels before and after incorporation of Alexa 488-labelled dUTP. Unchanged positive signals from Alexa 647 before and after incorporation indicates that the surface immobilised hairpin DNA is stable during the incorporation reaction, while positive signals from Alexa 488 were only observed from the polymerase surfaces after incorporation reaction showing the successful incorporation of dUTPs only with the presence of polymerase.

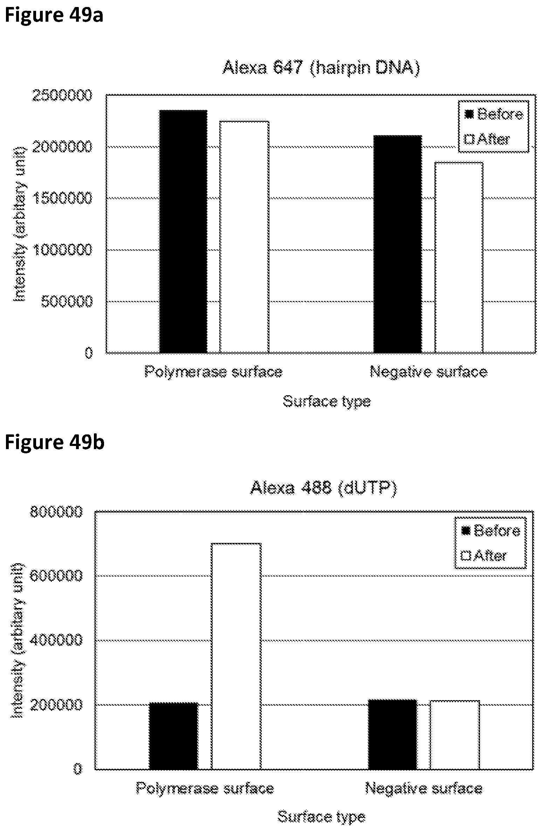

Figure 49 shows measured fluorescence signals in the Alexa 647 (hairpin DNA) and Alexa 488 (dUTP) channels obtained from 'polymerase surfaces' and 'negative surfaces' before and after incorporation of Alexa 488-labelled dUTP as described in (3). A significant increase in the Alexa 488 fluorescence signals was obtained after the incorporation reaction from the polymerase surface as a result of the successful incorporation, while the signals from negative surfaces remained the same after the incorporation reaction due to the absence of polymerase. Fluorescence signals in the Alexa 647 channel remained virtually unchanged after the incorporation reaction, indicating the presence of hairpin

DNA on the surface. The slight reduction in the fluorescence signal maybe attributed to the effect of photo-bleaching due to the second round of light exposure.

### Conclusion

The results demonstrate that a molecule comprising a support strand and a synthesis strand for use in the methods of the present invention, can readily be immobilised on a surface substrate compatible with the polynucleotide synthesis reactions described herein. The results further demonstrate that such a molecule can accept the incorporation of a new dNTP so as to extend the synthesis strand, whilst at the same time the molecule remains stable and attached to the substrate.

### Example 12. Cleavage and ligation of hairpin DNA oligomers immobilised to derivatized surfaces via a linker and thiophosphate covalent linkage.

This example describes the covalent coupling to derivatized surfaces of thiophosphate functionalised hairpin DNA with a linker, followed by cleavage and ligation reactions. The substrate preparation and coupling of hairpin DNA was carried out as described in Example 11.

### (1): Cleavage of immobilised hairpin DNA oligomers with a linker

### Materials and Methods

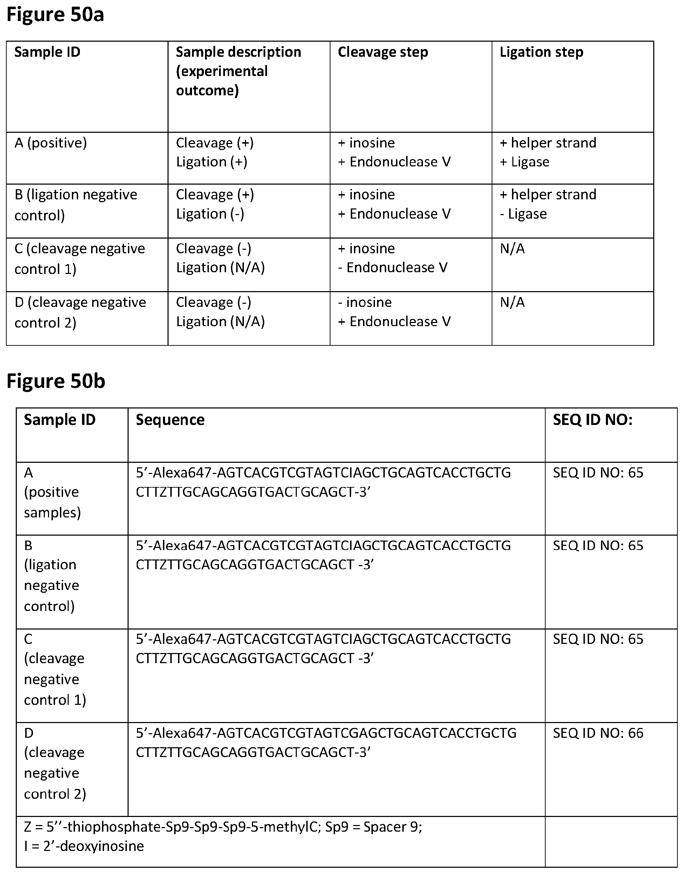

Hairpin DNA was immobilised on surface BRAPA modified surfaces as described in Example 11. Four sets of triplicate surfaces including all the experimental controls for cleavage and ligation reactions were prepared. The experimental conditions are described in Figure 50a. Figure 50b shows the sequences of hairpin DNA immobilised on different samples.

After the DNA immobilisation step, rubber gaskets with a 9 mm diameter circular opening were placed on all surfaces that were immobilised with DNA labelled with Alexa 647 at the 5' end and primed with 1X NEBuffer 4 (50 mM Potassium Acetate, 20 mM Tris-acetate, 10 mM Magnesium Acetate, 1 mM DTT, pH 7.9) for 10 mins. Note that for sample D, the immobilised hairpin DNA does not contain inosine and inosine is replaced by guanine. All the samples were subsequently exposed to either NEBuffer 4 containing 1.5 U/µl Endonuclease V (sample A, B and D) or NEBuffer 4 without Endonuclease V (sample C) for 1 hr. All the samples were subsequently washed with 1X T3 DNA Ligase buffer (66 mM Tris-HCl, 10mM MgCl2, 1 mM ATP, 7.5% PEG6000, 1 mM DTT, pH 7.6), 1X T3 DNA Ligase buffer containing 1M sodium chloride and 0.05% (v/v) Tween20, washed again with 1X T3 DNA Ligase buffer and scanned on ChemiDoc Imager in the Alexa 647 channel.

### Results

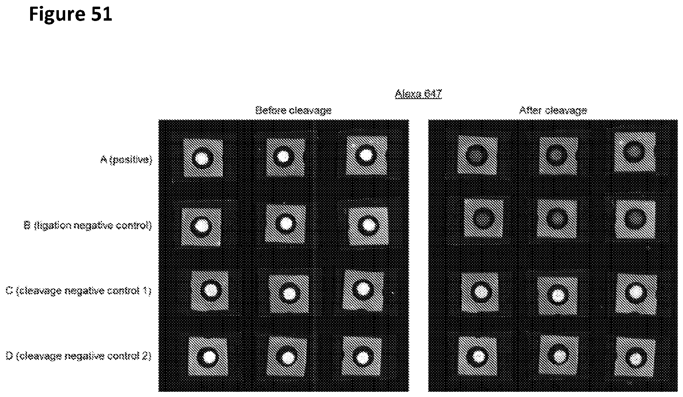

Figure 51 shows fluorescence signals from hairpin DNA oligomers before and after cleavage reactions.

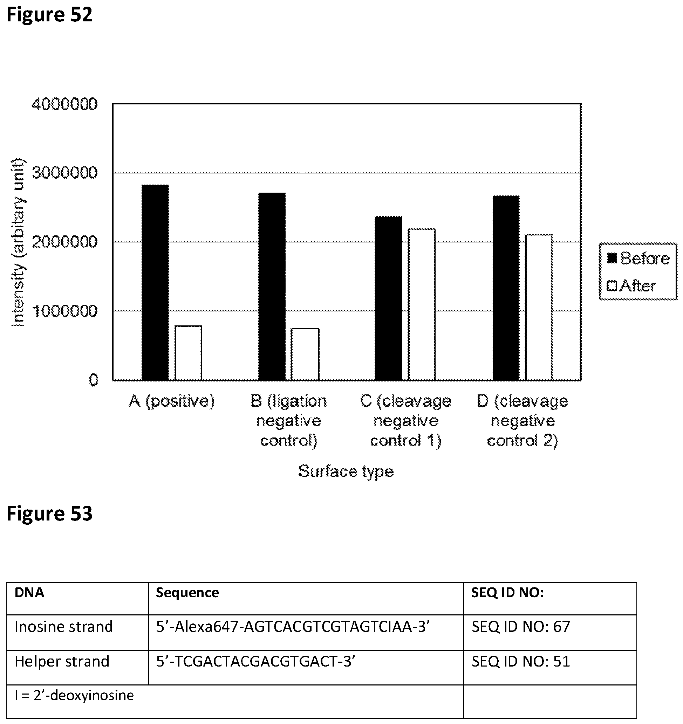

Figure 52 shows measured fluorescence signals before and after cleavage reactions obtained from DNA immobilised surfaces as described above. Successful cleavage reactions were only observed from samples A and B, while fluorescence signal intensities remained almost the same for samples C and D due to absence of either Endonuclease V (sample C) or inosine in the sequence (sample D).

Significant reductions in the fluorescence signals were observed from samples A and B as a result of successful cleavage reactions at the inosine site within the DNA strand with the presence of Endonuclease V. For samples C and D, absence of Endonuclease V and lack of inosine in the DNA respectively resulted in the fluorescence signals to remain almost the same level as the initial signals obtained after DNA immobilisation.

### (2): Ligation reactions

### Materials and Methods

After the cleavage reaction as described in (1), samples A and B (as described in Figure 50a) were exposed to 1X T3 DNA Ligase buffer containing MnCl₂ (2 mM), inosine strands labelled with Alexa 647 at the 5' end (16 µM) and complimentary 'helper' strands (16 µM) (the sequences are shown in Figure 53 below) with T3 DNA ligase (250 U/µl) for sample A, and without T3 DNA Ligase as a negative control for sample B. Samples were incubated in the respective solutions for 1 hr. After 1 hr, the surfaces were washed in water, subsequently exposed to water containing 1M sodium chloride and 0.05% (v/v) Tween20, and washed again with water. Fluorescence signals from the surfaces were measured using ChemiDoc in the Alexa 647 channels. Figure 53 shows the sequences for the inosine-containing strand and the complimentary 'helper' strand for ligation reactions.

### Results

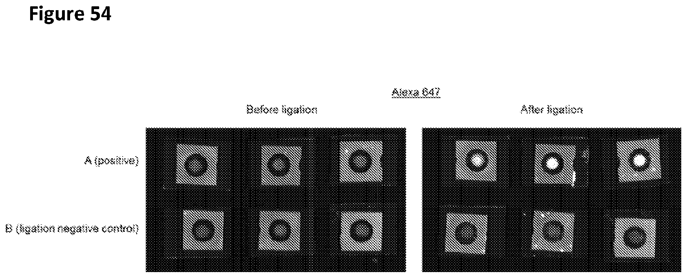

Figure 54 shows results relating to the monitoring of ligation reactions. Fluorescence signals detected from Alexa 647 channel before and after ligation reactions. An increase in fluorescence signals in the Alexa 647 channels after ligation were only obtained from sample A with T3 DNA ligase, while fluorescence signals remained at the same level after ligation reaction for sample B due to the absence of T3 DNA ligase.

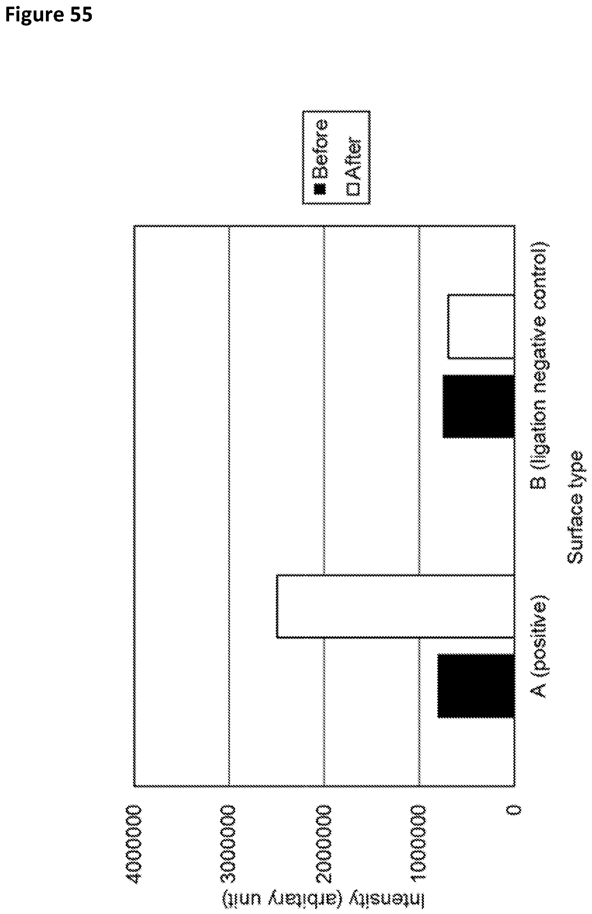

Figure 55 shows that a significant increase in the Alexa 647 fluorescence signal was obtained after ligation reaction from sample A as a result of the successful ligation, where the signal level recovers to the initial signal level after DNA immobilisation and prior to cleavage reaction as shown in Figure 52. The fluorescence signals from the sample B remained the same after the ligation reaction due to the absence of T3 DNA ligase.

### Conclusion

The results in this Example demonstrate that a molecule comprising a support strand and a synthesis strand for use in the methods of the present invention, can readily be immobilised on a surface substrate compatible with the polynucleotide synthesis reactions described herein and can be subjected to cleavage and ligation reactions whilst at the same time remaining stable and attached to the substrate.

### Example 13. Incorporation of 3'-O-azidomethyl-dNTPs to the 3' terminal end of blunt-ended DNA.

This example describes the incorporation of 3'-*O*-azidomethyl-dNTPs to the 3' end of blunt-ended double-stranded DNA.

The steps below demonstrate the controlled addition of a 3'-*O*-protected single nucleotide to a blunt-ended double-stranded oligonucleotide by enzymatic incorporation by DNA polymerase. The steps are in accordance with incorporation step 4 as shown in each of Figures 1 to 10.

### Materials and Methods

### Materials

1. In-house synthesized 3'-*O*-azidomethyl-dNTPs.
2. Therminator X DNA polymerase that has been engineered by New England Biolabs to possess enhanced ability to incorporate 3-*O*-modified dNTPs.
3. Blunt-ended double-stranded DNA oligonucleotide.

Four types of reversible terminators were tested:

| | |
|---|---|
| 3'-*O*-azidomethyl-dTTP: | 3'-*O*-azidomethyl-dCTP: |
| 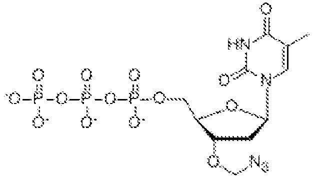 | 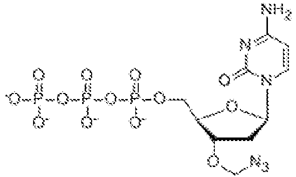 |
| 3'-*O*-azidomethyl-dATP: | 3'-*O*-azidomethyl-dGTP: |
| 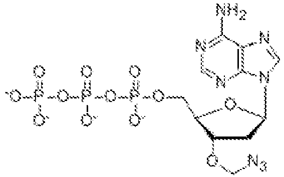 | 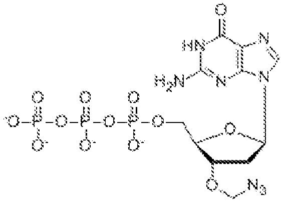 |

### Method

1. 5 µl of 10x Thermopol^{®} buffer (20 mM Tris-HCl, 10 mM (NH₄)₂SO₄, 10 mM KCl, 2 mM MgSO₄, 0.1% Triton^{®} X-100, pH 8.8, New England Biolabs) was mixed with 33.5 µl of sterile deionized water (ELGA VEOLIA) in a 1.5ml Eppendorf tube.
2. 2 µl of 20 µM primer (40 pmol, 1 equiv) (SEQ ID: NO: 68, Figure 56a) and 3 µl of 20 µM template (60 pmol, 1.5equiv) (SEQ ID: NO: 69, Figure 56a) were added to the reaction mixture.
3. 3'-O-modified-dTTP (2 µl of 100 µM) and MnCl₂ (2.5 µl of 40 mM) were added.
4. 2 µl of Therminator X DNA polymerase (20 U, New England BioLabs) was then added.
5. The reaction was incubated for 30 minutes at 37°C.
6. The reaction was stopped by addition of TBE-Urea sample buffer (Novex).
7. The reaction was separated on polyacrylamide gel (15%) TBE buffer and visualized by ChemiDoc MP imaging system (BioRad).

### Results

Figure 56b depicts a gel showing results of incorporation of 3'-*O*-modified-dNTPs by Therminator X DNA polymerase in the presence of Mn2+ ions at 37°C. The data show that Therminator X DNA polymerase was successfully able to incorporate 3'-*O*-modified-dNTPs to the 3' terminal end of the blunt ended DNA oligonucleotide to create a single base overhang.

### Example 14. Exemplary ligation of a polynucleotide ligation molecule to a scaffold polynucleotide using blunt end ligation.

This example describes the ligation of a polynucleotide ligation molecule, using DNA ligase, to a scaffold polynucleotide. This example involves ligation of molecules with blunt ends, consistent with synthesis method of the invention version 2, as depicted in Figure 2.

Figure 57 provides a scheme depicting a DNA synthesis reaction cycle. The scheme is intended to be consistent with synthesis method of the invention version 2 as depicted in Figure 2. Thus the scheme in Figure 57 shows the provision of a scaffold polynucleotide (right hand hairpin structure in the uppermost panel of the scheme) having a blunt end, the left strand corresponds to the support strand and the right strand corresponds to the synthesis strand. The terminal nucleotide of the support strand comprises a phosphate group at the 5' end. In the next step of the cycle a polynucleotide ligation molecule (far right structure in the uppermost panel of the scheme) is provided. The polynucleotide ligation molecule has a support strand (the left strand) and a helper strand (the right strand). The polynucleotide ligation molecule has a complementary ligation end which is a blunt end and which comprises a universal nucleotide which is 2-deoxyinosine (In). The terminal end of the helper strand at the complementary ligation end comprises a non-ligatible nucleotide. The terminal end of the support strand at the complementary ligation end comprises a nucleotide of the predefined sequence, depicted as "A", purely for illustration. Upon ligation of the polynucleotide ligation molecule to the scaffold polynucleotide, the terminal nucleotide of the support strand at the complementary ligation end of the polynucleotide ligation molecule is ligated to the terminal nucleotide of the support strand of the scaffold polynucleotide, and a single strand break ("nick") is created between the helper strand of the polynucleotide ligation molecule and the synthesis strand of the scaffold polynucleotide. The terminal nucleotide of the support strand of the polynucleotide ligation molecule comprises a nucleotide of the predefined sequence and occupies position n. The universal nucleotide consequently occupies position n+2. Following ligation the polynucleotide ligation molecule is cleaved by cleaving the support strand between positions n and n+1. In the reaction scheme shown in Figure 57 the helper strand is shown removed prior to cleavage, as an optional step. Following cleavage the nucleotide of the predefined sequence is retained in the scaffold polynucleotide. In the next step a further nucleotide, depicted as "T", purely for illustration, is incorporated in this case into the synthesis strand of the scaffold polynucleotide by the action of a polymerase enzyme or a nucleotide transferase enzyme. The further nucleotide comprises a reversible terminator group or blocking group. The further nucleotide pairs with the terminal nucleotide of the support strand to form a nucleotide pair. The scheme then depicts a deprotection or deblocking step wherein the reversible terminator group or blocking group is removed, thus completing a cycle of synthesis.

As detailed below, this Example 14 describes the step of ligating the polynucleotide ligation molecule to the scaffold polynucleotide as shown in the dashed box in Figure 57.

### Materials and methods

### Materials:

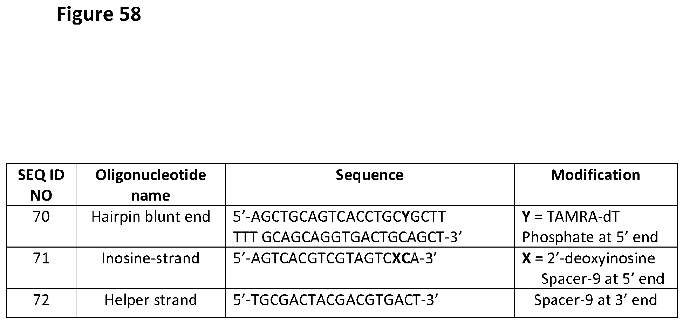

1. Oligonucleotides utilized in this example were designed in house and synthesised by Integrated DNA technologies. These are described in Figure 58.
2. The oligonucleotides were diluted to a stock concentration of 100uM using sterile distilled water (ELGA VEOLIA).

### Method:

Ligation reactions with oligonucleotides were carried out using the procedure below:
1. 12 µl of sterile distilled water (ELGA VEOLIA) was added into a 1.5ml Eppendorf tube.
2. 30 µl of 2X T3 DNA Ligase Reaction Buffer NEB (132 mM Tris-HCl, 20mM MgCl₂, 2mM Dithiothreitol, 2mM ATP, 15% Polyethylene glycol (PEG6000) and pH 7.6 at 25°C) and 2 µl of 40 mM MnCl₂ were then added into the same Eppendorf tube.
3. 5 µl of 2-deoxyinosine (In) strand (200 µmol/l) (SEQ ID: No 71) and 5 µl of helper strand (200 µmol/l) (SEQ ID: No 72) and 1 µl TAMRA or any fluorescently tagged blunt ended polynucleotide (20 µmol/l) (SEQ ID: No 70) were added into the same tube.
4. 5 µl of T3 DNA Ligase NEB (3000 units/µl) were added into the same tube.
5. The reaction mixture was then incubated at room temperature for 30 minutes.
6. After the incubation time had elapsed, the reaction was terminated with the addition of TBE-Urea sample Buffer (Novex).

### Results

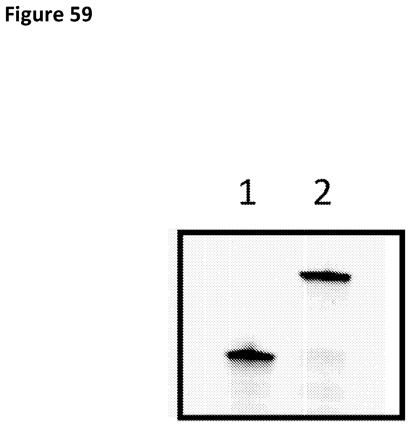

The results are shown in Figure 59.

### Example 15. Exemplary ligation of a polynucleotide ligation molecule to a scaffold polynucleotide using overhanging end ligation.

This example describes the ligation of a polynucleotide ligation molecule, using DNA ligase, to a scaffold polynucleotide. This example involves ligation of molecules with overhanging ends, consistent with synthesis method of the invention version 4 as depicted in Figure 5, and further variants of synthesis method of the invention version 4 as depicted in Figure 8.

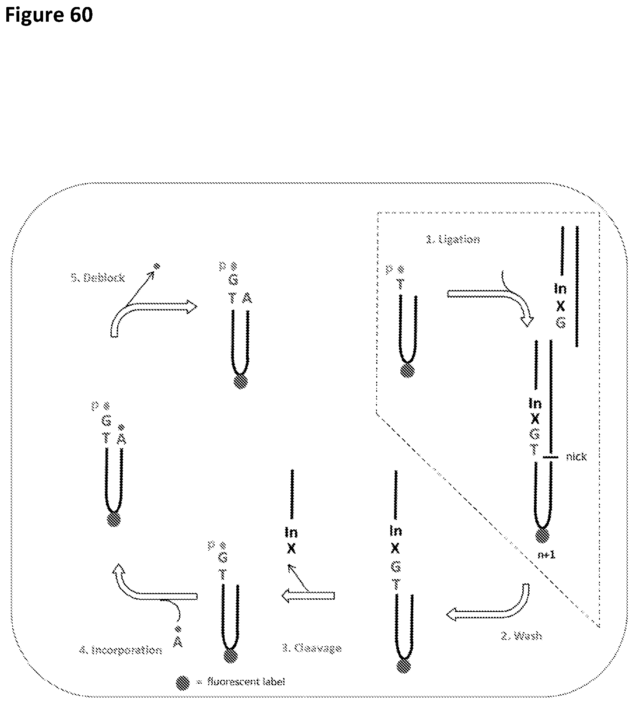

Figure 60 provides a scheme depicting a DNA synthesis reaction cycle. The scheme is intended to be consistent with synthesis method of the invention version 4 as depicted in Figure 5. Thus the scheme in Figure 60 shows the provision of a scaffold polynucleotide (right hand hairpin structure in the uppermost panel of the scheme) having an overhanging end, the left strand corresponds to the support strand and the right strand corresponds to the synthesis strand. The terminal nucleotide of the support strand occupies position n and overhangs the terminal nucleotide of the synthesis strand. The terminal nucleotide of the support strand is depicted as "T" for illustrative purposes only and comprises a phosphate group at the 5' end. In the next step of the cycle a polynucleotide ligation molecule (far right structure in the uppermost panel of the scheme) is provided. The polynucleotide ligation molecule has a support strand (the left strand) and a helper strand (the right strand). The polynucleotide ligation molecule has a complementary ligation end having an overhanging end and which comprises a universal nucleotide which is 2-deoxyinosine (In). The terminal end of the helper strand at the complementary ligation end comprises a non-ligatible nucleotide and overhangs the terminal nucleotide of the support strand. The terminal end of the support strand at the complementary ligation end comprises a nucleotide of the predefined sequence, depicted as "G", purely for illustration. Upon ligation of the polynucleotide ligation molecule to the scaffold polynucleotide, the terminal nucleotide of the support strand at the complementary ligation end of the polynucleotide ligation molecule is ligated to the terminal nucleotide of the support strand of the scaffold polynucleotide, and a single strand break ("nick") is created between the helper strand of the polynucleotide ligation molecule and the synthesis strand of the scaffold polynucleotide. The terminal nucleotide of the support strand of the polynucleotide ligation molecule comprises a nucleotide of the predefined sequence and occupies position n+1. The universal nucleotide consequently occupies position n+3. Following ligation the polynucleotide ligation molecule is cleaved by cleaving the support strand between positions n+1 and n+2. In the reaction scheme shown in Figure 60 the helper strand is shown removed prior to cleavage, as an optional step. Following cleavage the nucleotide of the predefined sequence is retained in the scaffold polynucleotide as the terminal nucleotide of the support strand in an overhanging end. In the next step a further nucleotide, depicted as "A", purely for illustration, is incorporated in this case into the synthesis strand of the scaffold polynucleotide by the action of a polymerase enzyme or a nucleotide transferase enzyme. The further nucleotide comprises a reversible terminator group or blocking group. The further nucleotide pairs with the partner nucleotide in the support strand, in this case depicted as "T", purely for illustration, thus forming a nucleotide pair. The scheme then depicts a deprotection or deblocking step wherein the reversible terminator group or blocking group is removed, thus completing a cycle of synthesis.

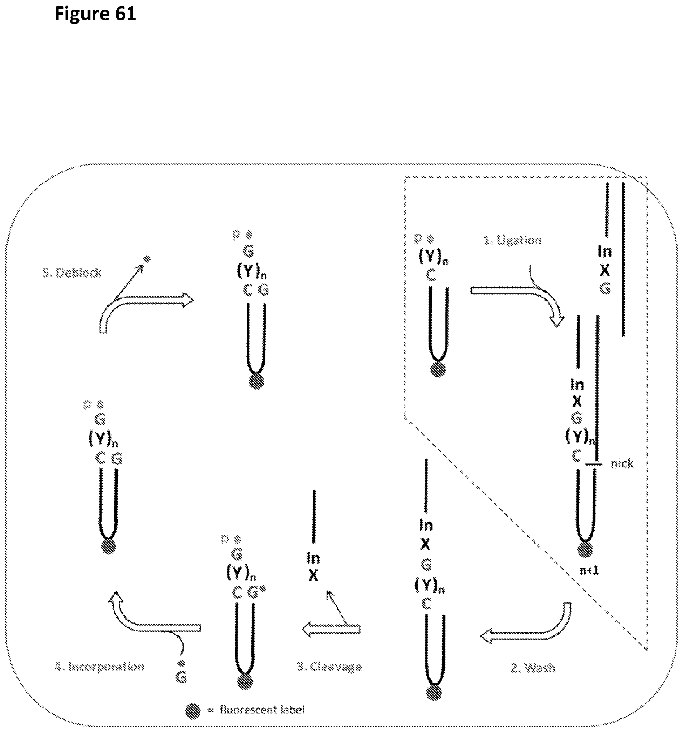

Figure 61 provides a similar scheme depicting a DNA synthesis reaction cycle. The scheme is intended to be consistent with further variants of synthesis method of the invention version 4 as depicted in Figure 8, wherein the length of the overhang can be extended from a single base overhang to a two, three, four or more base overhang.

As detailed below, this Example 15 describes the step of ligating the polynucleotide ligation molecule to the scaffold polynucleotide as shown in the dashed box in Figures 60 and 61.

### Materials and methods

### Materials:

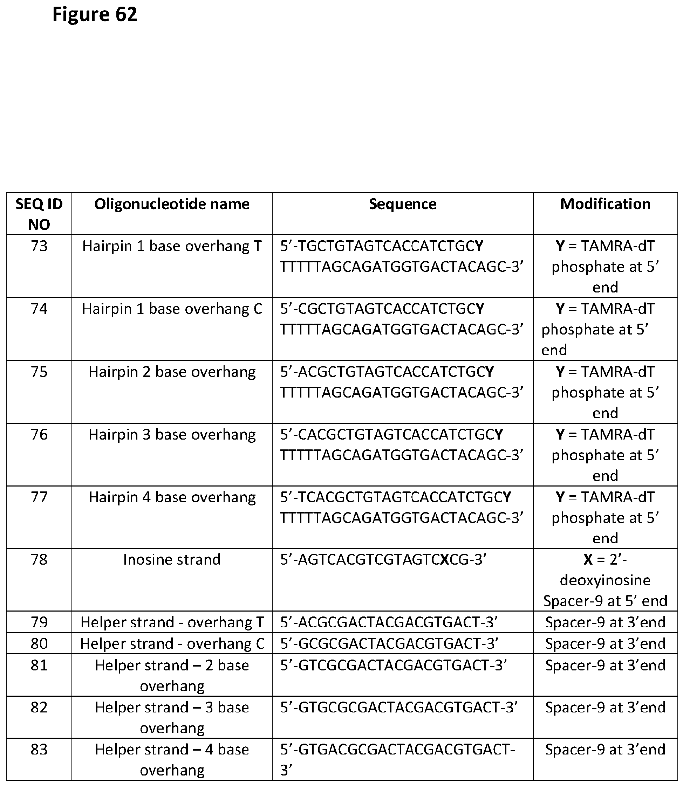

1. Oligonucleotides utilized in this example were designed in house and synthesised by Integrated DNA technologies. These are described in Figure 62.
2. The oligonucleotides were diluted to a stock concentration of 100uM using sterile distilled water (ELGA VEOLIA).

### Method:

Ligation reactions with oligonucleotides were carried out using the procedure below:
1. 12 µl of sterile distilled water (ELGA VEOLIA) was added into a 1.5ml Eppendorf tube.
2. 30 µl of 2X T3 DNA Ligase Reaction buffer NEB (132 mM Tris-HCl, 20mM MgCl₂, 2mM Dithiothreitol, 2mM ATP, 15% Polyethylene glycol (PEG6000) and pH 7.6 at 25°C) and 2 µl of 40 mM MnCl₂ were then added into the same Eppendorf tube.
3. 5 µl of 2-deoxyinosine (In) strand (200 µmol/l) (SEQ ID: No 78) and 5 µl of helper strand (200 µmol/l) (SEQ ID: No 79, 80) and 1 µl TAMRA or any fluorescently tagged polynucleotide with a 5' overhang (20 µmol/l) (SEQ ID: No 73, 74, 75, 76 or 77) were added into the same tube.
4. 5 µl of T3 DNA Ligase NEB (3000 units/µl) were added into the same tube.
5. The reaction mixture was then incubated at room temperature for 30 minutes.
6. After the incubation time had elapsed, the reaction was terminated with the addition of TBE-Urea sample Buffer (Novex).

### Results

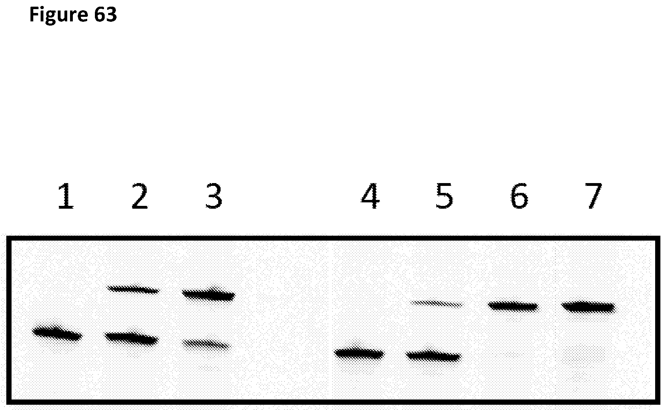

The results are shown in Figure 63.

In the above Examples, all oligonucleotides presented in SEQ ID NOS 1-83 have a hydroxyl group at the 3' terminus. All oligonucleotides presented in SEQ ID NOS 1-83 lack a phosphate group at the 5' terminus except for SEQ ID NO 7, SEQ ID NO 18, SEQ ID NO 35, SEQ ID NO 70 and SEQ ID NOS 73 to 77.

As used in this specification and the appended claims, the singular forms "a", "an", and "the" include plural referents unless the content clearly dictates otherwise. Thus, for example, reference to "a polynucleotide ligation molecule" includes two or more such polynucleotides, reference to "a scaffold polynucleotide" includes two or more such scaffold polynucleotides, and the like.

## Claims

1. An *in vitro* method of synthesising a double-stranded polynucleotide having a predefined sequence, the method comprising performing cycles of synthesis wherein in each cycle:
(A) a scaffold polynucleotide is provided comprising a synthesis strand and a support strand hybridized thereto, wherein the synthesis strand comprises a primer strand portion, and wherein the support strand is the first strand of the double-stranded polynucleotide and the synthesis strand is the strand is the second strand of the double-stranded polynucleotide;
(B) the first strand of the double-stranded polynucleotide is extended by the addition of a first nucleotide of the predefined sequence and a universal nucleotide by the action of a ligase enzyme, wherein the universal nucleotide defines a cleavage site;
(C) the double-stranded polynucleotide is then cleaved at the cleavage site; and
(D) the second strand which is hybridized to the first strand is then extended by the addition of a second nucleotide of the predefined sequence by a nucleotide transferase or polymerase enzyme; and
wherein the first and second nucleotides of the predefined sequence of each cycle are retained in the double-stranded polynucleotide following cleavage; and
wherein in a given cycle of synthesis the second nucleotide of that cycle which is added to the second strand of the double-stranded polynucleotide comprises a reversible terminator group which prevents further extension by the enzyme, and wherein the reversible terminator group is removed from the incorporated second nucleotide of that cycle prior to the addition in the next cycle of synthesis of the second nucleotide of the next cycle,
wherein in each cycle the first nucleotide is a partner nucleotide for the second nucleotide, and wherein upon incorporation into the double-stranded polynucleotide the first and second nucleotides form a nucleotide pair; optionally wherein the ligation reaction comprises a blunt-ended ligation reaction; further optionally wherein the first nucleotide and the universal nucleotide are components of a polynucleotide ligation molecule, and wherein the polynucleotide ligation molecule is ligated to the double-stranded polynucleotide during step (B) by the action of the ligase enzyme in a blunt-ended ligation reaction, and wherein upon ligation of the polynucleotide ligation molecule to the double-stranded polynucleotide the first strand of the double-stranded polynucleotide is extended and the cleavage site is created.

2. A method according to claim 1, the method comprising performing a first cycle of synthesis comprising:
(1) ligating a double-stranded polynucleotide ligation molecule to the scaffold polynucleotide by the action of the ligase enzyme in a blunt-ended ligation reaction, the polynucleotide ligation molecule comprising a support strand and a helper strand hybridised thereto and further comprising a complementary ligation end, the ligation end comprising:
(i) in the support strand a universal nucleotide and a first nucleotide of the predefined sequence; and
(ii) in the helper strand a non-ligatable terminal nucleotide;
wherein upon ligation the first strand of the double-stranded polynucleotide is extended with the first nucleotide and the cleavage site is created by the incorporation of the universal nucleotide into the first strand;
(2) cleaving the ligated scaffold polynucleotide at the cleavage site, wherein cleavage comprises cleaving the support strand and removing the universal nucleotide from the scaffold polynucleotide to provide a cleaved double-stranded scaffold polynucleotide comprising the incorporated first nucleotide;
(3) extending the terminal end of the primer strand portion of the synthesis strand of the double-stranded scaffold polynucleotide by the incorporation of a second nucleotide of the predefined sequence by the action of the nucleotide transferase or polymerase enzyme, the second nucleotide comprising a reversible terminator group which prevents further extension by the enzyme, wherein the second nucleotide is a partner for first nucleotide and wherein upon incorporation the second nucleotide and the first nucleotide form a nucleotide pair; and
(4) removing the reversible terminator group from the second nucleotide;
the method further comprising performing a further cycle of synthesis comprising:
(5) ligating a further double-stranded polynucleotide ligation molecule to the cleaved scaffold polynucleotide by the action of the ligase enzyme in a blunt-ended ligation reaction, the polynucleotide ligation molecule comprising a support strand and a helper strand hybridised thereto and further comprising a complementary ligation end, the ligation end comprising:
(i) in the support strand a universal nucleotide and the first nucleotide of the further cycle of synthesis; and
(ii) in the helper strand a non-ligatable terminal nucleotide;
wherein upon ligation the first strand of the double-stranded polynucleotide is extended with the first nucleotide of the further cycle of synthesis and the cleavage site is created by the incorporation of the universal nucleotide into the first strand;
(6) cleaving the ligated scaffold polynucleotide at the cleavage site, wherein cleavage comprises cleaving the support strand and removing the universal nucleotide from the scaffold polynucleotide to provide a cleaved double-stranded scaffold polynucleotide comprising the nucleotide pair(s) of the previous cycle(s) and the first nucleotide of the further cycle of synthesis;
(7) extending the terminal end of the primer strand portion of the synthesis strand of the double-stranded scaffold polynucleotide by the incorporation of the second nucleotide of the further cycle of synthesis by the action of the nucleotide transferase or polymerase enzyme, the second nucleotide comprising a reversible terminator group which prevents further extension by the enzyme, wherein the second nucleotide of the further cycle of synthesis is a partner for the first nucleotide of the further cycle of synthesis, and wherein upon incorporation the second and first nucleotides of the further cycle of synthesis form a further nucleotide pair;
(8) removing the reversible terminator group from the second nucleotide; and
(9) repeating steps 5 to 8 multiple times to provide the double-stranded polynucleotide having a predefined nucleotide sequence.

3. A method according to claim 2, wherein:
(I)
(a) in the ligation step of the first cycle (step 1) and in ligation steps of all further cycles the complementary ligation end of the polynucleotide ligation molecule is structured such that:
i. the first nucleotide of the predefined sequence of that cycle is the terminal nucleotide of the support strand, occupies nucleotide position n in the support strand and is paired with the terminal nucleotide of the helper strand;
ii. the universal nucleotide is the penultimate nucleotide of the support strand, occupies nucleotide position n+1 in the support strand and is paired with the penultimate nucleotide of the helper strand; and
iii. the terminal nucleotide of the helper strand is a non-ligatable nucleotide;
wherein position n is the nucleotide position which is opposite the second nucleotide of the predefined sequence of that cycle upon its incorporation, and wherein position n+1 is the next nucleotide position in the support strand relative to position n in the direction distal to the complementary ligation end; and wherein upon ligation the terminal nucleotide of the support strand of the polynucleotide ligation molecule is ligated to the terminal nucleotide of the scaffold polynucleotide proximal to the primer strand portion of the synthesis strand and a single-strand break is created between the terminal nucleotides of the helper strand and the primer strand portion of the synthesis strand;
(b) in the cleavage step of the first cycle (step 2) and in all further cycles the support strand of the ligated scaffold polynucleotide is cleaved between positions n+1 and n, thereby releasing the polynucleotide ligation molecule from the scaffold polynucleotide and retaining the first nucleotide of that cycle attached to the first strand of the cleaved scaffold polynucleotide; and
(c) in the extension step of the first cycle (step 3) and in all further cycles the second nucleotide of that cycle is incorporated into the second strand opposite the first nucleotide in the first strand and is paired therewith; and whereupon the position occupied by the first nucleotide of that cycle in the support strand of the cleaved scaffold polynucleotide is defined as nucleotide position n-1 in the next cycle of synthesis; or
(II)
(a) in the ligation step of the first cycle (step 1) and in ligation steps of all further cycles the complementary ligation end of the polynucleotide ligation molecule is structured such that:
i. the first nucleotide of the predefined sequence of that cycle is the terminal nucleotide of the support strand, occupies nucleotide position n in the support strand and is paired with the terminal nucleotide of the helper strand;
ii. the universal nucleotide occupies nucleotide position n+2 in the support strand and is paired with a partner nucleotide in the helper strand; and
iii. the terminal nucleotide of the helper strand is a non-ligatable nucleotide;
wherein position n is the nucleotide position which is opposite the second nucleotide of the predefined sequence of that cycle upon its incorporation, and wherein position n+2 is the second nucleotide position in the support strand relative to position n in the direction distal to the complementary ligation end; and wherein upon ligation the terminal nucleotide of the support strand of the polynucleotide ligation molecule is ligated to the terminal nucleotide of the scaffold polynucleotide proximal to the primer strand portion of the synthesis strand and a single-strand break is created between the terminal nucleotides of the helper strand and the primer strand portion of the synthesis strand;
(b) in the cleavage step of the first cycle (step 2) and in all further cycles the support strand of the ligated scaffold polynucleotide is cleaved between positions n+1 and n, thereby releasing the polynucleotide ligation molecule from the scaffold polynucleotide and retaining the first nucleotide of that cycle attached to the first strand of the cleaved scaffold polynucleotide; and
(c) in the extension step of the first cycle (step 3) and in all further cycles the second nucleotide of that cycle is incorporated into the second strand opposite the first nucleotide in the first strand and is paired therewith, and whereupon the position occupied by the first nucleotide of that cycle in the support strand of the cleaved scaffold polynucleotide is defined as nucleotide position n-1 in the next cycle of synthesis; or
(III)
(a) in the ligation step of the first cycle (step 1) and in ligation steps of all further cycles the complementary ligation end of the polynucleotide ligation molecule is structured such that:
i. the first nucleotide of the predefined sequence of that cycle is the terminal nucleotide of the support strand, occupies nucleotide position n in the support strand and is paired with the terminal nucleotide of the helper strand;
ii. the universal nucleotide occupies nucleotide position n+2+x in the support strand and is paired with a partner nucleotide in the helper strand; and
iii. the terminal nucleotide of the helper strand is a non-ligatable nucleotide;
wherein position n is the nucleotide position which is opposite the second nucleotide of the predefined sequence of that cycle upon its incorporation, and wherein position n+2 is the second nucleotide position in the support strand relative to position n in the direction distal to the complementary ligation end and wherein x is a number of nucleotide positions relative to position n+2 in the direction distal to the complementary ligation end wherein the number is a whole number from 1 to 10 or more; and wherein upon ligation the terminal nucleotide of the support strand of the polynucleotide ligation molecule is ligated to the terminal nucleotide of the scaffold polynucleotide proximal to the primer strand portion of the synthesis strand and a single-strand break is created between the terminal nucleotides of the helper strand and the primer strand portion of the synthesis strand;
(b) in the cleavage step of the first cycle (step 2) and in all further cycles the support strand of the ligated scaffold polynucleotide is cleaved between positions n+1 and n, thereby releasing the polynucleotide ligation molecule from the scaffold polynucleotide and retaining the first nucleotide of that cycle attached to the first strand of the cleaved scaffold polynucleotide; and
(c) in the extension step of the first cycle (step 3) and in all further cycles the second nucleotide of that cycle is incorporated into the second strand opposite the first nucleotide in the first strand and is paired therewith, and whereupon the position occupied by the first nucleotide of that cycle in the support strand of the cleaved scaffold polynucleotide is defined as nucleotide position n-1 in the next cycle of synthesis.

4. A method according to claim 1, wherein in each cycle upon incorporation the first nucleotide and the second nucleotide become partner nucleotides in different nucleotide pairs in the synthesised double-stranded polynucleotide; optionally wherein the ligation reaction comprises a sticky-ended ligation reaction; further optionally wherein the first nucleotide and the universal nucleotide are components of a polynucleotide ligation molecule, and wherein the polynucleotide ligation molecule is ligated to the double-stranded polynucleotide during step (B) by the action of the ligase enzyme in a sticky-ended ligation reaction, and wherein upon ligation of the polynucleotide ligation molecule to the double-stranded polynucleotide the first strand of the double-stranded polynucleotide is extended and the cleavage site is created.

5. A method according to claim 4, the method comprising performing a first cycle of synthesis comprising:
(1) ligating a double-stranded polynucleotide ligation molecule to the scaffold polynucleotide by the action of the ligase enzyme in a sticky-ended ligation reaction, the polynucleotide ligation molecule comprising a support strand and a helper strand hybridised thereto and further comprising a complementary ligation end, the ligation end comprising:
(i) in the support strand a universal nucleotide and a first nucleotide of the predefined sequence; and
(ii) in the helper strand a non-ligatable terminal nucleotide;
wherein upon ligation the first strand of the double-stranded polynucleotide is extended with the first nucleotide and the cleavage site is created by the incorporation of the universal nucleotide into the first strand;
(2) cleaving the ligated scaffold polynucleotide at the cleavage site, wherein cleavage comprises cleaving the support strand and removing the universal nucleotide from the scaffold polynucleotide to provide a cleaved double-stranded scaffold polynucleotide comprising the incorporated first nucleotide;
(3) extending the terminal end of the primer strand portion of the synthesis strand of the double-stranded scaffold polynucleotide by the incorporation of a second nucleotide of the predefined sequence by the action of the nucleotide transferase or polymerase enzyme, the second nucleotide comprising a reversible terminator group which prevents further extension by the enzyme, wherein the second nucleotide; and
(4) removing the reversible terminator group from the second nucleotide;
the method further comprising performing a further cycle of synthesis comprising:
(5) ligating a further double-stranded polynucleotide ligation molecule to the cleaved scaffold polynucleotide by the action of the ligase enzyme in a sticky-ended ligation reaction, the polynucleotide ligation molecule comprising a support strand and a helper strand hybridised thereto and further comprising a complementary ligation end, the ligation end comprising:
(i) in the support strand a universal nucleotide and the first nucleotide of the further cycle of synthesis; and
(ii) in the helper strand a non-ligatable terminal nucleotide;
wherein upon ligation the first strand of the double-stranded polynucleotide is extended with the first nucleotide of the further cycle of synthesis and the cleavage site is created by the incorporation of the universal nucleotide into the first strand;
(6) cleaving the ligated scaffold polynucleotide at the cleavage site, wherein cleavage comprises cleaving the support strand and removing the universal nucleotide from the scaffold polynucleotide to provide a cleaved double-stranded scaffold polynucleotide comprising the nucleotide pair(s) of the previous cycle(s) and the first nucleotide of the further cycle of synthesis;
(7) extending the terminal end of the primer strand portion of the synthesis strand of the double-stranded scaffold polynucleotide by the incorporation of the second nucleotide of the further cycle of synthesis by the action of the nucleotide transferase or polymerase enzyme, the second nucleotide comprising a reversible terminator group which prevents further extension by the enzyme;
(8) removing the reversible terminator group from the second nucleotide; and
(9) repeating steps 5 to 8 multiple times to provide the double-stranded polynucleotide having a predefined nucleotide sequence.

6. A method according to claim 5, wherein:
(a) in step (1) the terminal end of the support strand of the scaffold polynucleotide proximal to the primer strand portion comprises a nucleotide overhang, wherein the terminal nucleotide of the support strand overhangs the terminal nucleotide of the primer strand portion, and wherein the terminal nucleotide of the support strand is the partner nucleotide for the second nucleotide of that cycle;
wherein in step (1) at the complementary ligation end of the polynucleotide ligation molecule the terminal end of the helper strand comprises a nucleotide overhang, wherein the terminal nucleotide of the helper strand overhangs the terminal nucleotide of the support strand, and wherein the terminal nucleotide of the support strand is the first nucleotide of that cycle and is a partner nucleotide in a different nucleotide pair formed in the next cycle of synthesis;
wherein in step (5) in the cleaved scaffold polynucleotide the terminal end of the support strand proximal to the primer strand portion comprises a nucleotide overhang, wherein the terminal and penultimate nucleotides of the support strand overhang the terminal nucleotide of the primer strand portion, and wherein the penultimate nucleotide of the support strand is the partner nucleotide for the second nucleotide of the further cycle of synthesis incorporated in step (7);
wherein in step (5) at the complementary ligation end of the polynucleotide ligation molecule the terminal end of the helper strand comprises a nucleotide overhang, wherein the terminal nucleotide of the helper strand overhangs the terminal nucleotide of the support strand, and wherein the terminal nucleotide of the support strand is the first nucleotide of the further cycle of synthesis and is a partner nucleotide in a different nucleotide pair formed in the next cycle of synthesis; and
wherein in first and further cycles of synthesis the nucleotide overhang in the complimentary ligation end of the polynucleotide ligation molecule and the nucleotide overhang in the scaffold polynucleotide comprises the same number of nucleotides, wherein the number is one or more, provided that the number of nucleotides in the overhang in the scaffold polynucleotide is one more than the number of nucleotides in the overhang in the complimentary ligation end; or
(b) in step (1) the terminal end of the support strand of the scaffold polynucleotide proximal to the primer strand portion comprises a nucleotide overhang comprising 1+y nucleotides, wherein the 1+y nucleotides of the support strand overhang the terminal nucleotide of the primer strand portion, and wherein the first nucleotide of the overhang occupies a position in the overhang distal to the terminal end of the overhang, occupies nucleotide position n and is the partner nucleotide for the second nucleotide of that first cycle incorporated in step (3), and wherein the nucleotide of the overhang which occupies position n+1 is the partner nucleotide for the second nucleotide of the next/second cycle of synthesis incorporated in step (7);
wherein in step (1) at the complementary ligation end of the polynucleotide ligation molecule the terminal end of the helper strand comprises a nucleotide overhang comprising 1+y nucleotides, wherein the 1+y nucleotides of the helper strand overhang the terminal nucleotide of the support strand and are partner nucleotides for the 1+y overhanging nucleotides of the support strand of the scaffold polynucleotide, and wherein the terminal nucleotide of the support strand of the complementary ligation end is the first nucleotide of that cycle, occupies nucleotide position n+2+x and is a partner nucleotide in a different nucleotide pair formed in the third cycle of synthesis;
wherein in step (5) in the cleaved scaffold polynucleotide the terminal end of the support strand proximal to the primer strand portion comprises a nucleotide overhang comprising 1+y nucleotides, wherein the 1+y nucleotides of the support strand overhang the terminal nucleotide of the primer strand portion, and wherein the first nucleotide of the overhang occupies a position in the overhang distal to the terminal end of the overhang, occupies nucleotide position n and is the partner nucleotide for the second nucleotide of the further cycle of synthesis incorporated in step (7), and wherein the nucleotide of the overhang which occupies position n+1 is the partner nucleotide for the second nucleotide of the next cycle of synthesis;
wherein in step (5) at the complementary ligation end of the polynucleotide ligation molecule the terminal end of the helper strand comprises a nucleotide overhang comprising 1+y nucleotides, wherein the 1+y nucleotides of the helper strand overhang the terminal nucleotide of the support strand and are partner nucleotides for the 1+y overhanging nucleotides of the support strand of the scaffold polynucleotide, and wherein the terminal nucleotide of the support strand of the complementary ligation end is the first nucleotide of the further cycle of synthesis, occupies nucleotide position n+2+x and is a partner nucleotide in a different nucleotide pair formed in the next cycle of synthesis; and wherein:
i. position n is the nucleotide position which is opposite the second nucleotide of the predefined sequence of that cycle upon its incorporation;
ii. following ligation positions n+1 and n+2 are the first and second nucleotide positions in the support strand relative to position n in the direction proximal to the helper strand/distal to the primer strand portion;
iii. y is a whole number which is one or more;
iv. x is a whole number which is zero or more;
v. the number for y in the scaffold polynucleotide is preferably the same number as the number for y in the complementary ligation end of the polynucleotide ligation molecule, and wherein if y is a different number the number for y in the complementary ligation end of the polynucleotide ligation molecule is preferably less than the number for y in the scaffold polynucleotide; and
vi. in any given series of first and further cycles the number selected for x is the number selected for y in the scaffold polynucleotide minus one.

7. A method according to claim 6, wherein:
(i)
(a) in the ligation step of the first cycle (step 1) and in ligation steps of all further cycles the complementary ligation end of the polynucleotide ligation molecule is structured such that:
i. the first nucleotide of the predefined sequence of that cycle is the terminal nucleotide of the support strand, occupies nucleotide position n+1 in the support strand and is paired with the penultimate nucleotide of the helper strand;
ii. the universal nucleotide is the penultimate nucleotide of the support strand, occupies nucleotide position n+2 in the support strand and is paired with a partner nucleotide in the helper strand;
iii. the overhang comprises a one nucleotide overhang comprises the terminal nucleotide of the helper strand overhanging the first nucleotide of the predefined sequence of that cycle; and
iv. the terminal nucleotide of the helper strand is a non-ligatable nucleotide;
wherein position n is the nucleotide position which is opposite the second nucleotide of the predefined sequence of that cycle upon its incorporation, and positions n+1 and n+2 are respectively the first/next and second nucleotide positions in the support strand relative to position n in the direction distal to the complementary ligation end; and wherein upon ligation the terminal nucleotide of the support strand of the polynucleotide ligation molecule is ligated to the terminal nucleotide of the scaffold polynucleotide proximal to the primer strand portion of the synthesis strand and a single-strand break is created between the terminal nucleotides of the helper strand and the primer strand portion of the synthesis strand;
(b) in the cleavage step of the first cycle (step 2) and in all further cycles the support strand of the ligated scaffold polynucleotide is cleaved between positions n+2 and n+1, thereby releasing the polynucleotide ligation molecule from the scaffold polynucleotide and retaining the first nucleotide of that cycle unpaired and attached to the first strand of the cleaved scaffold polynucleotide, wherein upon cleavage a double-nucleotide overhang is created in the support strand of the cleaved scaffold polynucleotide wherein the terminal and penultimate nucleotides of the support strand overhang the terminal nucleotide of the primer strand portion of the synthesis strand; and
(c) in the extension step of the first cycle (step 3) and in all further cycles the second nucleotide of that cycle is incorporated into the second strand and is paired with the penultimate nucleotide of the overhanging support strand, and wherein following steps (7) and (8) the position occupied by the first nucleotide of that further cycle in the support strand of the cleaved scaffold polynucleotide is defined as nucleotide position n in the next cycle of synthesis; or
(ii)
(a) in the ligation step of the first cycle (step 1) and in ligation steps of all further cycles the complementary ligation end of the polynucleotide ligation molecule is structured such that:
i. the first nucleotide of the predefined sequence of that cycle is the terminal nucleotide of the support strand, occupies nucleotide position n+1 in the support strand and is paired with the penultimate nucleotide of the helper strand;
ii. the universal nucleotide occupies nucleotide position n+3 in the support strand and is paired with a partner nucleotide in the helper strand;
iii. the overhang comprises a one nucleotide overhang comprises the terminal nucleotide of the helper strand overhanging the first nucleotide of the predefined sequence of that cycle; and
iv. the terminal nucleotide of the helper strand is a non-ligatable nucleotide;
wherein position n is the nucleotide position which is opposite the second nucleotide of the predefined sequence of that cycle upon its incorporation, and positions n+1, n+2 and n+3 are respectively the next, second and third nucleotide positions in the support strand relative to position n in the direction distal to the complementary ligation end; and wherein upon ligation the terminal nucleotide of the support strand of the polynucleotide ligation molecule is ligated to the terminal nucleotide of the scaffold polynucleotide proximal to the primer strand portion of the synthesis strand and a single-strand break is created between the terminal nucleotides of the helper strand and the primer strand portion of the synthesis strand;
(b) in the cleavage step of the first cycle (step 2) and in all further cycles the support strand of the ligated scaffold polynucleotide is cleaved between positions n+2 and n+1, thereby releasing the polynucleotide ligation molecule from the scaffold polynucleotide and retaining the first nucleotide of that cycle unpaired and attached to the first strand of the cleaved scaffold polynucleotide, wherein upon cleavage a double-nucleotide overhang is created in the support strand of the cleaved scaffold polynucleotide wherein the terminal and penultimate nucleotides of the support strand overhang the terminal nucleotide of the primer strand portion of the synthesis strand; and
(c) in the extension step of the first cycle (step 3) and in all further cycles the second nucleotide of that cycle is incorporated into the second strand and is paired with the penultimate nucleotide of the overhanging support strand, and wherein following steps (7) and (8) the position occupied by the first nucleotide of that further cycle in the support strand of the cleaved scaffold polynucleotide is defined as nucleotide position n in the next cycle of synthesis; or
(iii)
(a) in the ligation step of the first cycle (step 1) and in ligation steps of all further cycles the complementary ligation end of the polynucleotide ligation molecule is structured such that:
i. the first nucleotide of the predefined sequence of that cycle is the terminal nucleotide of the support strand, occupies nucleotide position n+1 in the support strand and is paired with the penultimate nucleotide of the helper strand;
ii. the universal nucleotide occupies nucleotide position n+3+x in the support strand and is paired with a partner nucleotide in the helper strand;
iii. the overhang comprises a one nucleotide overhang comprises the terminal nucleotide of the helper strand overhanging the first nucleotide of the predefined sequence of that cycle; and
iv. the terminal nucleotide of the helper strand is a non-ligatable nucleotide;
wherein position n is the nucleotide position which is opposite the second nucleotide of the predefined sequence of that cycle upon its incorporation, and positions n+1 and n+3 are respectively the first/next and third nucleotide positions in the support strand relative to position n in the direction distal to the complementary ligation end, and wherein x is a number of nucleotide positions relative to position n+3 in the direction distal to the complementary ligation end wherein the number is a whole number from 1 to 10 or more; and wherein upon ligation the terminal nucleotide of the support strand of the polynucleotide ligation molecule is ligated to the terminal nucleotide of the scaffold polynucleotide proximal to the primer strand portion of the synthesis strand and a single-strand break is created between the terminal nucleotides of the helper strand and the primer strand portion of the synthesis strand;
(b) in the cleavage step of the first cycle (step 2) and in all further cycles the support strand of the ligated scaffold polynucleotide is cleaved between positions n+2 and n+1, thereby releasing the polynucleotide ligation molecule from the scaffold polynucleotide and retaining the first nucleotide of that cycle unpaired and attached to the first strand of the cleaved scaffold polynucleotide, wherein upon cleavage a double-nucleotide overhang is created in the support strand of the cleaved scaffold polynucleotide wherein the terminal and penultimate nucleotides of the support strand overhang the terminal nucleotide of the primer strand portion of the synthesis strand; and
(c) in the extension step of the first cycle (step 3) and in all further cycles the second nucleotide of that cycle is incorporated into the second strand and is paired with the penultimate nucleotide of the overhanging support strand, and wherein following steps (7) and (8) the position occupied by the first nucleotide of that further cycle in the support strand of the cleaved scaffold polynucleotide is defined as nucleotide position n in the next cycle of synthesis.

8. A method according to claim 6 (b), wherein:
(a) in both first and further cycles in both the polynucleotide ligation molecule and in the ligated scaffold polynucleotide the universal nucleotide occupies position n+3+x and the scaffold polynucleotide is cleaved between positions n+3+x and n+2+x; or
(b) in both first and further cycles in both the polynucleotide ligation molecule and in the ligated scaffold polynucleotide the universal nucleotide occupies position n+4+x and the scaffold polynucleotide is cleaved between positions n+3+x and n+2+x.

9. A method according to any one of claims 2 to 8, wherein the method is modified such that
(i) in step (1) the polynucleotide ligation molecule is provided with a complementary ligation end comprising a first nucleotide of the predefined sequence of the first cycle and further comprising one or more further nucleotides of the predefined sequence of the first cycle;
(ii) in step (2) following cleavage the first and further nucleotides of the predefined sequence of the first cycle are retained in the cleaved scaffold polynucleotide;
(iii) in step (3) the terminal end of the primer strand portion of the synthesis strand of the double-stranded scaffold polynucleotide is extended by the incorporation of a second nucleotide of the predefined sequence of the first cycle by the action of the nucleotide transferase or polymerase enzyme, and wherein the terminal end of the primer strand portion is further extended by the incorporation of one or more further nucleotides of the predefined sequence of the first cycle by the action of the nucleotide transferase or polymerase enzyme, wherein each one of the second and further nucleotides of the first cycle comprises a reversible terminator group which prevents further extension by the enzyme, and wherein following each further extension the reversible terminator group is removed from a nucleotide before the incorporation of the next nucleotide;
(iv) in step (5) the polynucleotide ligation molecule is provided with a complementary ligation end comprising a first nucleotide of the predefined sequence of the further cycle and further comprising one or more further nucleotides of the predefined sequence of the further cycle;
(v) in step (6) following cleavage the first and further nucleotides of the predefined sequence of the further cycle are retained in the cleaved scaffold polynucleotide;
(vi) in step (7) the terminal end of the primer strand portion of the synthesis strand of the double-stranded scaffold polynucleotide is extended by the incorporation of a second nucleotide of the predefined sequence of the further cycle by the action of the nucleotide transferase or polymerase enzyme, and wherein the terminal end of the primer strand portion is further extended by the incorporation of one or more further nucleotides of the predefined sequence of the further cycle by the action of the nucleotide transferase or polymerase enzyme, wherein each one of the second and further nucleotides of the further cycle comprises a reversible terminator group which prevents further extension by the enzyme, and wherein following each further extension the reversible terminator group is removed from a nucleotide before the incorporation of the next nucleotide.

10. A method according to claim 9, wherein:
(a) the complementary ligation end of the polynucleotide ligation molecule is structured such that in steps (2) and (6) prior to cleavage the universal nucleotide occupies a position in the support strand which is the next nucleotide position in the support strand after the nucleotide positions of the first and further nucleotides in the direction distal to the complementary ligation end, and the support strand is cleaved between the position occupied by the last further nucleotide and the position occupied by the universal nucleotide; or
(b) the complementary ligation end of the polynucleotide ligation molecule is structured such that in steps (2) and (6) prior to cleavage the universal nucleotide occupies a position in the support strand which is the next+1 nucleotide position in the support strand after the nucleotide positions of the first and further nucleotides in the direction distal to the complementary ligation end, and the support strand is cleaved between the position occupied by the last further nucleotide and the position occupied by the next nucleotide in the support strand.

11. A method according to any one of the preceding claims, wherein in any one, more or all cycles of synthesis a partner nucleotide which pairs with the first nucleotide of the predefined sequence is a nucleotide which is complementary with the first nucleotide, preferably naturally complementary.

12. A method according to any one of claims 2 to 11, wherein:
(a) in any one, more or all cycles of synthesis, prior to step (2) and/or (6) the scaffold polynucleotide is provided comprising a synthesis strand and a support strand hybridized thereto, and wherein the synthesis strand is provided without a helper strand; and/or
(b) in any one, more or all cycles of synthesis, prior to step (2) and/or (6) the helper strand portion of the synthesis strand is removed from the scaffold polynucleotide.

13. A method according to any one of claims 1 to 3(i), 4 to 6, 7(i), 6(b), 8(a), 9 and 10(a) wherein:
(a) each cleavage step comprises a two step cleavage process wherein each cleavage step comprises a first step comprising removing the universal nucleotide thus forming an abasic site, and a second step comprising cleaving the support strand at the abasic site; optionally wherein the first step is performed with a nucleotide-excising enzyme; and/or wherein the second step is performed with an enzyme having abasic site lyase activity, optionally wherein the enzyme having abasic site lyase activity is:
(i) AP Endonuclease 1;
(ii) Endonuclease III (Nth); or
(iii) Endonuclease VIII; or
(b) each cleavage step comprises a one step cleavage process comprising removing the universal nucleotide with a cleavage enzyme wherein the enzyme is:
(i) Endonuclease III;
(ii) Endonuclease VIII;
(iii) formamidopirimidine DNA glycosylase (Fpg); or
(iv) 8-oxoguanine DNA glycosylase (hOGG1).

14. A method according to any one of claims 1 to 2, 3(ii), 4 to 6, 7(ii), 8(b), 9 and 10(b), wherein the cleavage step comprises cleaving the support strand with an enzyme; optionally wherein the enzyme cleaves the support strand between nucleotide positions n+1 and n; and/or wherein the enzyme is Endonuclease V.

15. A method according to any one of claims 2 to 14, wherein in any one, more or all cycles of synthesis:
(a) in steps (1)/(5) in the scaffold polynucleotide the synthesis strand comprising the primer strand portion and the portion of the support strand hybridized thereto are connected by a hairpin loop; and
(b) in steps (1)/(5) in the polynucleotide ligation molecule the helper strand and the portion of the support strand hybridized thereto are connected by a hairpin loop at the end opposite the complementary ligation end.

16. A method according to any one of claims 2 to 15, wherein the synthesis strand comprising the primer strand portion and the portion of the support strand hybridized thereto are tethered to a common surface, optionally via one or more covalent bonds; further optionally wherein the one or more covalent bonds is formed between a functional group on the common surface and a functional group on the scaffold molecule, wherein the functional group on the scaffold molecule is an amine group, a thiol group, a thiophosphate group or a thioamide group; more preferably wherein the functional group on the common surface is a bromoacetyl group, optionally wherein the bromoacetyl group is provided on a polyacrylamide surface derived using N- (5-bromoacetamidylpentyl) acrylamide (BRAPA).

17. A method according to any one of the preceding claims, wherein:
(a) synthesis cycles are performed in droplets within a microfluidic system; optionally wherein the microfluidic system is an electrowetting system; more preferably wherein the microfluidic system is an electrowetting-on-dielectric system (EWOD); and/or
(b) following synthesis the strands of the double-stranded polynucleotides are separated to provide a single-stranded polynucleotide having a predefined sequence; and/or
(c) wherein following synthesis the double-stranded polynucleotide or a region thereof is amplified, preferably by PCR.

18. A method of assembling a polynucleotide having a predefined sequence, the method comprising performing the method of any one of the preceding claims to synthesise a first polynucleotide having a predefined sequence and one or more additional polynucleotides having a predefined sequence and joining together the first and one or more additional polynucleotides; optionally wherein the first polynucleotide and the one or more additional polynucleotides are cleaved to create compatible termini and joined together, preferably by ligation; further optionally wherein the first polynucleotide and the one or more additional polynucleotides are cleaved by a restriction enzyme at a cleavage site.

## Patentansprüche

1. In-vitro-Verfahren zum Synthetisieren eines doppelsträngigen Polynukleoids, das eine vordefinierte Sequenz aufweist, wobei das Verfahren das Durchführen von Synthesezyklen umfasst, wobei in jedem Zyklus:
(A) ein Gerüstpolynukleotid bereitgestellt wird, das einen Synthesestrang und einen daran hybridisierten Trägerstrang umfasst, wobei der Synthesestrang einen Primerstrangteil umfasst und wobei der Trägerstrang der erste Strang des doppelsträngigen Polynukleotids ist und der Synthesestrang der Strang des zweiten Strangs des doppelsträngigen Polynukleotids ist;
(B) der erste Strang des doppelsträngigen Polynukleotids durch den Zusatz eines ersten Nukleotids der vordefinierten Sequenz und eines universellen Nukleotids durch die Wirkung eines Ligaseenzyms verlängert wird, wobei das universelle Nukleotid eine Spaltstelle definiert;
(C) das doppelsträngige Polynukleotid dann an der Spaltstelle gespalten wird; und
(D) der zweite Strang, der an den ersten Strang hybridisiert ist, dann durch den Zusatz eines zweiten Nukleotids der vordefinierten Sequenz durch ein Nukleotidtransferase- oder Nukleotidpolymerase-Enzym verlängert wird; und
wobei das erste und zweite Nukleotid der vordefinierten Sequenz jedes Zyklus nach der Spaltung in dem doppelsträngigen Polynukleotid erhalten bleiben; und
wobei in einem gegebenen Synthesezyklus das zweite Nukleotid dieses Zyklus, das an den zweiten Strang des doppelsträngigen Polynukleotids hinzugefügt wird, eine reversible Terminatorgruppe umfasst, die eine weitere Verlängerung durch das Enzym verhindert, und wobei die reversible Terminatorgruppe aus dem eingebauten zweiten Nukleotid dieses Zyklus vor dem Zusatz des zweiten Nukleotids des nächsten Zyklus im nächsten Synthesezyklus entfernt wird,
wobei in jedem Zyklus das erste Nukleotid ein Partnernukleotid für das zweite Nukleotid ist, und wobei das erste und das zweite Nukleotid beim Einbau in das doppelsträngige Polynukleotid ein Nukleotidpaar bilden; wobei optional die Ligationsreaktion eine Ligationsreaktion mit stumpfen Enden umfasst; wobei ferner optional das erste Nukleotid und das universelle Nukleotid Komponenten eines Polynukleotid-Ligationsmoleküls sind und wobei das Polynukleotid-Ligationsmolekül während des Schritts (B) durch die Wirkung des Ligaseenzyms in einer Ligationsreaktion mit stumpfen Enden an das doppelsträngige Polynukleotid ligiert wird, und wobei bei der Ligation des Polynukleotid-Ligationsmoleküls an das doppelsträngige Polynukleotid der erste Strang des doppelsträngigen Polynukleotids verlängert wird und die Spaltstelle erzeugt wird.

2. Verfahren nach Anspruch 1, wobei das Verfahren die Durchführung eines ersten Synthesezyklus umfasst, umfassend:
(1) Ligieren eines doppelsträngigen Polynukleotid-Ligationsmoleküls an das GerüstPolynukleotid durch die Wirkung des Ligaseenzyms in einer Ligationsreaktion mit stumpfen Enden, wobei das Polynukleotid-Ligationsmolekül einen Trägerstrang und einen daran hybridisierten Helferstrang umfasst und ferner ein komplementäres Ligationsende umfasst, wobei das Ligationsende umfasst:
(i) in dem Trägerstrang ein universelles Nukleotid und ein erstes Nukleotid der vordefinierten Sequenz; und
(ii) in dem Helferstrang ein nicht ligierbares terminales Nukleotid;
wobei bei der Ligation der erste Strang des doppelsträngigen Polynukleotids mit dem ersten Nukleotid verlängert wird und die Spaltstelle durch den Einbau des universellen Nukleotids in den ersten Strang erzeugt wird;
(2) Spalten des ligierten Gerüstpolynukleotids an der Spaltstelle, wobei die Spaltung das Spalten des Trägerstrangs und das Entfernen des universellen Nukleotids aus dem Gerüstpolynukleotid umfasst, um ein gespaltenes doppelsträngiges Gerüstpolynukleotid bereitzustellen, das das eingebaute erste Nukleotid umfasst;
(3) Verlängern des terminalen Endes des Primerstrangteils des Synthesestrangs des doppelsträngigen Gerüstpolynukleotids durch den Einbau eines zweiten Nukleotids der vordefinierten Sequenz durch die Wirkung des Nukleotidtransferase- oder Nukleotidpolymerase-Enzyms, wobei das zweite Nukleotid eine reversible Terminatorgruppe umfasst, die eine weitere Verlängerung durch das Enzym verhindert, wobei das zweite Nukleotid ein Partner für das erste Nukleotid ist und wobei das zweite Nukleotid und das erste Nukleotid beim Einbau ein Nukleotidpaar bilden; und
(4) Entfernen der reversiblen Terminatorgruppe aus dem zweiten Nukleotid;
wobei das Verfahren ferner das Durchführen eines weiteren Synthesezyklus umfasst, umfassend:
(5) Ligieren eines weiteren doppelsträngigen Polynukleotid-Ligationsmoleküls an das gespaltene Gerüstpolynukleotid durch die Wirkung des Ligaseenzyms in einer Ligationsreaktion mit stumpfen Enden, wobei das Polynukleotid-Ligationsmolekül einen Trägerstrang und einen daran hybridisierten Helferstrang umfasst und ferner ein komplementäres Ligationsende umfasst, wobei das Ligationsende umfasst:
(i) in dem Trägerstrang ein universelles Nukleotid und das erste Nukleotid des weiteren Synthesezyklus; und
(ii) in dem Helferstrang ein nicht ligierbares terminales Nukleotid;
wobei bei der Ligation der erste Strang des doppelsträngigen Polynukleotids mit dem ersten Nukleotid des weiteren Synthesezyklus verlängert wird und die Spaltstelle durch den Einbau des universellen Nukleotids in den ersten Strang erzeugt wird;
(6) Spalten des ligierten Gerüstpolynukleotids an der Spaltstelle, wobei die Spaltung das Spalten des Trägerstrangs und das Entfernen des universellen Nukleotids vom Gerüstpolynukleotid umfasst, um ein gespaltenes doppelsträngiges Gerüstpolynukleotid bereitzustellen, das das/die Nukleotidpaar(e) des/der vorherigen Zyklus/Zyklen und das erste Nukleotid des weiteren Synthesezyklus umfasst;
(7) Verlängern des terminalen Endes des Primerstrangteils des Synthesestrangs des doppelsträngigen Gerüstpolynukleotids durch den Einbau des zweiten Nukleotids des weiteren Synthesezyklus durch die Wirkung des Nukleotidtransferase- oder Nukleotidpolymerase-Enzyms, wobei das zweite Nukleotid eine reversible Terminatorgruppe umfasst, die eine weitere Verlängerung durch das Enzym verhindert, wobei das zweite Nukleotid des weiteren Synthesezyklus ein Partner für das erste Nukleotid des weiteren Synthesezyklus ist, und wobei das zweite und das erste Nukleotid des weiteren Synthesezyklus beim Einbau ein weiteres Nukleotidpaar bilden;
(8) Entfernen der reversiblen Terminatorgruppe von dem zweiten Nukleotid; und
(9) mehrfaches Wiederholen der Schritte 5 bis 8, um das eine vordefinierte Nukleotidsequenz aufweisende doppelsträngige Polynukleotid bereitzustellen.

3. Verfahren nach Anspruch 2, wobei:
(I)
(a) im Ligationsschritt des ersten Zyklus (Schritt 1) und in den Ligationsschritten aller weiteren Zyklen das komplementäre Ligationsende des Polynukleotid-Ligationsmoleküls so strukturiert wird, dass:
i. das erste Nukleotid der vordefinierten Sequenz dieses Zyklus das terminale Nukleotid des Trägerstrangs ist, die Nukleotidposition n im Trägerstrang einnimmt und mit dem terminalen Nukleotid des Helferstrangs gepaart ist;
ii. das universelle Nukleotid das vorletzte Nukleotid des Trägerstrangs ist, die Nukleotidposition n+1 im Trägerstrang einnimmt und mit dem vorletzten Nukleotid des Helferstrangs gepaart ist; und
iii. das terminale Nukleotid des Helferstrangs ein nicht ligierbares Nukleotid ist;
wobei die Position n die Nukleotidposition ist, die dem zweiten Nukleotid der vordefinierten Sequenz dieses Zyklus bei dessen Einbau gegenüberliegt, und wobei die Position n+1 die nächste Nukleotidposition im Trägerstrang relativ zur Position n in der Richtung distal zum komplementären Ligationsende ist; und wobei bei der Ligation das terminale Nukleotid des Trägerstrangs des Polynukleotid-Ligationsmoleküls an das terminale Nukleotid des Gerüstpolynukleotids proximal zum Primerstrangteil des Synthesestrangs ligiert wird und ein Einzelstrangbruch zwischen den terminalen Nukleotiden des Helferstrangs und dem Primerstrangteil des Synthesestrangs erzeugt wird;
(b) in dem Spaltungsschritt des ersten Zyklus (Schritt 2) und in allen weiteren Zyklen der Trägerstrang des ligierten Gerüstpolynukleotids zwischen den Positionen n+1 und n gespalten wird, wodurch das Polynukleotid-Ligationsmolekül aus dem Gerüstpolynukleotid freigesetzt wird und das erste Nukleotid dieses Zyklus an den ersten Strang des gespaltenen Gerüstpolynukleotids gebunden bleibt; und
(c) im Verlängerungsschritt des ersten Zyklus (Schritt 3) und in allen weiteren Zyklen das zweite Nukleotid des Zyklus in den zweiten Strang gegenüber dem ersten Nukleotid im ersten Strang eingebaut und mit diesem gepaart wird; und woraufhin die Position, die von dem ersten Nukleotid dieses Zyklus im Trägerstrang des gespaltenen Gerüstpolynukleotids eingenommen wird, als Nukleotidposition n-1 im nächsten Synthesezyklus definiert wird; oder
(II)
(a) im Ligationsschritt des ersten Zyklus (Schritt 1) und in den Ligationsschritten aller weiteren Zyklen das komplementäre Ligationsende des Polynukleotid-Ligationsmoleküls so strukturiert wird, dass:
i. das erste Nukleotid der vordefinierten Sequenz dieses Zyklus das terminale Nukleotid des Trägerstrangs ist, die Nukleotidposition n im Trägerstrang einnimmt und mit dem terminalen Nukleotid des Helferstrangs gepaart ist;
ii. das universelle Nukleotid die Nukleotidposition n+2 im Trägerstrang einnimmt und mit einem Partnernukleotid im Helferstrang gepaart ist; und
iii. das terminale Nukleotid des Helferstrangs ein nicht ligierbares Nukleotid ist;
wobei die Position n die Nukleotidposition ist, die dem zweiten Nukleotid der vordefinierten Sequenz dieses Zyklus bei dessen Einbau gegenüberliegt, und wobei die Position n+2 die zweite Nukleotidposition im Trägerstrang relativ zur Position n in der Richtung distal zum komplementären Ligationsende ist; und wobei bei der Ligation das terminale Nukleotid des Trägerstrangs des Polynukleotid-Ligationsmoleküls an das terminale Nukleotid des Gerüstpolynukleotids proximal zum Primerstrangteil des Synthesestrangs ligiert wird und ein Einzelstrangbruch zwischen den terminalen Nukleotiden des Helferstrangs und dem Primerstrangteil des Synthesestrangs erzeugt wird;
(b) in dem Spaltungsschritt des ersten Zyklus (Schritt 2) und in allen weiteren Zyklen der Trägerstrang des ligierten Gerüstpolynukleotids zwischen den Positionen n+1 und n gespalten wird, wodurch das Polynukleotid-Ligationsmolekül aus dem Gerüstpolynukleotid freigesetzt wird und das erste Nukleotid dieses Zyklus an den ersten Strang des gespaltenen Gerüstpolynukleotids gebunden bleibt; und
(c) im Verlängerungsschritt des ersten Zyklus (Schritt 3) und in allen weiteren Zyklen das zweite Nukleotid des Zyklus in den zweiten Strang gegenüber dem ersten Nukleotid im ersten Strang eingebaut und mit diesem gepaart wird; und woraufhin die Position, die von dem ersten Nukleotid dieses Zyklus in dem Trägerstrang des gespaltenen Gerüstpolynukleotids eingenommen wird, als Nukleotidposition n-1 im nächsten Synthesezyklus definiert wird; oder
(III)
(a) im Ligationsschritt des ersten Zyklus (Schritt 1) und in den Ligationsschritten aller weiteren Zyklen das komplementäre Ligationsende des Polynukleotid-Ligationsmoleküls so strukturiert wird, dass:
i. das erste Nukleotid der vordefinierten Sequenz dieses Zyklus das terminale Nukleotid des Trägerstrangs ist, die Nukleotidposition n im Trägerstrang einnimmt und mit dem terminalen Nukleotid des Helferstrangs gepaart ist;
ii. das universelle Nukleotid die Nukleotidposition n+2+x im Trägerstrang einnimmt und mit einem Partnernukleotid im Helferstrang gepaart ist; und
iii. das terminale Nukleotid des Helferstrangs ein nicht ligierbares Nukleotid ist;
wobei die Position n die Nukleotidposition ist, die dem zweiten Nukleotid der vordefinierten Sequenz dieses Zyklus bei dessen Einbau gegenüberliegt, und wobei die Position n+2 die zweite Nukleotidposition im Trägerstrang relativ zur Position n in der Richtung distal zum komplementären Ligationsende ist und wobei x eine Anzahl von Nukleotidpositionen relativ zur Position n+2 in der Richtung distal zum komplementären Ligationsende ist, wobei die Anzahl eine ganze Zahl von 1 bis 10 oder höher ist; und wobei bei der Ligation das terminale Nukleotid des Trägerstrangs des Polynukleotid-Ligationsmoleküls an das terminale Nukleotid des Gerüstpolynukleotids proximal zum Primerstrangteil des Synthesestrangs ligiert wird und ein Einzelstrangbruch zwischen den terminalen Nukleotiden des Helferstrangs und dem Primerstrangteil des Synthesestrangs erzeugt wird;
(b) in dem Spaltungsschritt des ersten Zyklus (Schritt 2) und in allen weiteren Zyklen der Trägerstrang des ligierten Gerüstpolynukleotids zwischen den Positionen n+1 und n gespalten wird, wodurch das Polynukleotid-Ligationsmolekül aus dem Gerüstpolynukleotid freigesetzt wird und das erste Nukleotid dieses Zyklus an den ersten Strang des gespaltenen Gerüstpolynukleotids gebunden bleibt; und
(c) im Verlängerungsschritt des ersten Zyklus (Schritt 3) und in allen weiteren Zyklen das zweite Nukleotid dieses Zyklus in den zweiten Strang gegenüber dem ersten Nukleotid im ersten Strang eingebaut und mit diesem gepaart wird, und woraufhin die Position, die von dem ersten Nukleotid dieses Zyklus im Trägerstrang des gespaltenen Gerüstpolynukleotids eingenommen wird, als Nukleotidposition n-1 im nächsten Synthesezyklus definiert wird.

4. Verfahren nach Anspruch 1, wobei in jedem Zyklus das erste Nukleotid und das zweite Nukleotid beim Einbau zu Partnernukleotiden in verschiedenen Nukleotidpaaren in dem synthetisierten doppelsträngigen Polynukleotid werden; optional
wobei die Ligationsreaktion eine Ligationsreaktion mit klebrigen Enden umfasst; ferner optional, wobei das erste Nukleotid und das universelle Nukleotid Komponenten eines Polynukleotid-Ligationsmoleküls sind, und wobei das Polynukleotid-Ligationsmolekül während des Schritts (B) durch die Wirkung des Ligaseenzyms in einer Ligationsreaktion mit klebrigen Enden an das doppelsträngige Polynukleotid ligiert wird, und wobei bei der Ligation des Polynukleotid-Ligationsmoleküls an das doppelsträngige Polynukleotid der erste Strang des doppelsträngigen Polynukleotids verlängert wird und die Spaltstelle erzeugt wird.

5. Verfahren nach Anspruch 4, wobei das Verfahren das Durchführen eines ersten Synthesezyklus umfasst, umfassend:
(1) Ligieren eines doppelsträngigen Polynukleotid-Ligationsmoleküls an das Gerüstpolynukleotid durch die Wirkung des Ligaseenzyms in einer Ligationsreaktion mit klebrigen Enden, wobei das Polynukleotid-Ligationsmolekül einen Trägerstrang und einen daran hybridisierten Helferstrang umfasst und ferner ein komplementäres Ligationsende umfasst, wobei das Ligationsende umfasst:
(i) in dem Trägerstrang ein universelles Nukleotid und ein erstes Nukleotid der vordefinierten Sequenz; und
(ii) in dem Helferstrang ein nicht ligierbares terminales Nukleotid;
wobei bei der Ligation der erste Strang des doppelsträngigen Polynukleotids mit dem ersten Nukleotid verlängert wird und die Spaltstelle durch den Einbau des universellen Nukleotids in den ersten Strang erzeugt wird;
(2) Spalten des ligierten Gerüstpolynukleotids an der Spaltstelle, wobei die Spaltung das Spalten des Trägerstrangs und das Entfernen des universellen Nukleotids aus dem Gerüstpolynukleotid umfasst, um ein gespaltenes doppelsträngiges Gerüstpolynukleotid bereitzustellen, das das eingebaute erste Nukleotid umfasst;
(3) Verlängern des terminalen Endes des Primerstrangabschnitts des Synthesestrangs des doppelsträngigen Gerüstpolynukleotids durch den Einbau eines zweiten Nukleotids der vordefinierten Sequenz durch die Wirkung des Nukleotidtransferase- oder Nukleotidpolymerase-Enzyms, wobei das zweite Nukleotid eine reversible Terminatorgruppe umfasst, die eine weitere Verlängerung durch das Enzym verhindert, wobei das zweite Nukleotid; und
(4) Entfernen der reversiblen Terminatorgruppe von dem zweiten Nukleotid;
wobei das Verfahren ferner das Durchführen eines weiteren Synthesezyklus umfasst, umfassend:
(5) Ligieren eines weiteren doppelsträngigen Polynukleotid-Ligationsmoleküls an das gespaltene Gerüstpolynukleotid durch die Wirkung des Ligaseenzyms in einer Ligationsreaktion mit klebrigen Enden, wobei das Polynukleotid-Ligationsmolekül einen Trägerstrang und einen daran hybridisierten Helferstrang umfasst und ferner ein komplementäres Ligationsende umfasst, wobei das Ligationsende umfasst:
(i) in dem Trägerstrang ein universelles Nukleotid und das erste Nukleotid des weiteren Synthesezyklus; und
(ii) in dem Helferstrang ein nicht ligierbares terminales Nukleotid;
wobei bei der Ligation der erste Strang des doppelsträngigen Polynukleotids mit dem ersten Nukleotid des weiteren Synthesezyklus verlängert wird und die Spaltstelle durch den Einbau des universellen Nukleotids in den ersten Strang erzeugt wird;
(6) Spalten des ligierten Gerüstpolynukleotids an der Spaltstelle, wobei die Spaltung das Spalten des Trägerstrangs und das Entfernen des universellen Nukleotids von dem Gerüstpolynukleotid umfasst, um ein gespaltenes doppelsträngiges Gerüstpolynukleotid bereitzustellen, das das/die Nukleotidpaar(e) des/der vorherigen Zyklus/Zyklen und das erste Nukleotid des weiteren Synthesezyklus umfasst;
(7) Verlängern des terminalen Endes des Primerstrangteils des Synthesestrangs des doppelsträngigen Gerüstpolynukleotids durch den Einbau des zweiten Nukleotids des weiteren Synthesezyklus durch die Wirkung des Nukleotidtransferase- oder Nukleotidpolymerase-Enzyms, wobei das zweite Nukleotid eine reversible Terminatorgruppe umfasst, die eine weitere Verlängerung durch das Enzym verhindert;
(8) Entfernen der reversiblen Terminatorgruppe von dem zweiten Nukleotid; und
(9) mehrfaches Wiederholen der Schritte 5 bis 8, um das eine vordefinierte Nukleotidsequenz aufweisende doppelsträngige Polynukleotid bereitzustellen.

6. Verfahren nach Anspruch 5, wobei:
(a) in Schritt (1) das terminale Ende des Trägerstrangs des Gerüstpolynukleotids proximal zum Primer-Strangabschnitt einen Nukleotidüberhang umfasst, wobei das terminale Nukleotid des Trägerstrangs das terminale Nukleotid des Primerstrangteils überhängt, und wobei das terminale Nukleotid des Trägerstrangs das Partnernukleotid für das zweite Nukleotid dieses Zyklus ist;
wobei in Schritt (1) am komplementären Ligationsende des Polynukleotid-Ligationsmoleküls das terminale Ende des Helferstrangs einen Nukleotidüberhang umfasst, wobei das terminale Nukleotid des Helferstrangs das terminale Nukleotid des Trägerstrangs überhängt, und wobei das terminale Nukleotid des Trägerstrangs das erste Nukleotid dieses Zyklus ist und ein Partnernukleotid in einem im nächsten Synthesezyklus gebildeten anderen Nukleotidpaar ist;
wobei in Schritt (5) in dem gespaltenen Gerüstpolynukleotid das terminale Ende des Trägerstrangs proximal zu dem Primerstrangabschnitt einen Nukleotidüberhang umfasst, wobei das terminale und vorletzte Nukleotid des Trägerstrangs das terminale Nukleotid des Primerstrangabschnitts überhängen, und wobei das vorletzte Nukleotid des Trägerstrangs das Partnernukleotid für das in Schritt (7) eingebaute zweite Nukleotid des weiteren Synthesezyklus ist;
wobei in Schritt (5) am komplementären Ligationsende des Polynukleotid-Ligationsmoleküls das terminale Ende des Helferstrangs einen Nukleotidüberhang umfasst, wobei das terminale Nukleotid des Helferstrangs das terminale Nukleotid des Trägerstrangs überhängt, und wobei das terminale Nukleotid des Trägerstrangs das erste Nukleotid des weiteren Synthesezyklus ist und ein Partnernukleotid in einem im nächsten Synthesezyklus gebildeten anderen Nukleotidpaar ist; und
wobei im ersten und weiteren Synthesezyklen der Nukleotidüberhang im komplementären Ligationsende des Polynukleotid-Ligationsmoleküls und der Nukleotidüberhang im Gerüstpolynukleotid die gleiche Anzahl von Nukleotiden umfassen, wobei die Anzahl eins oder mehr ist, vorausgesetzt, dass die Anzahl von Nukleotiden in dem Überhang im Gerüstpolynukleotid um eins höher ist als die Anzahl von Nukleotiden im Überhang im komplementären Ligationsende; oder
(b) in Schritt (1) das terminale Ende des Trägerstrangs des Gerüstpolynukleotids proximal zum Primerstrangabschnitt einen Nukleotidüberhang umfasst, der 1+y Nukleotide umfasst, wobei die 1+y Nukleotide des Trägerstrangs das terminale Nukleotid des Primerstrangabschnitts überhängen, und wobei das erste Nukleotid des Überhangs eine Position im Überhang distal zum terminalen Ende des Überhangs einnimmt, die Nukleotidposition n einnimmt und das Partnernukleotid für das in Schritt (3) eingebaute zweite Nukleotid dieses ersten Zyklus ist, und wobei das Nukleotid des Überhangs, das die Position n+1 einnimmt, das Partnernukleotid für das in Schritt (7) eingebaute zweite Nukleotid des nächsten/zweiten Synthesezyklus ist;
wobei in Schritt (1) am komplementären Ligationsende des Polynukleotid-Ligationsmoleküls das terminale Ende des Helferstrangs einen Nukleotidüberhang umfasst, der 1+y Nukleotide umfasst, wobei die 1+y Nukleotide des Helferstrangs das terminale Nukleotid des Trägerstrangs überhängen und Partnernukleotide für die 1+y überhängenden Nukleotide des Trägerstrangs des Gerüstpolynukleotids sind, und wobei das terminale Nukleotid des Trägerstrangs des komplementären Ligationsendes das erste Nukleotid dieses Zyklus ist, die Nukleotidposition n+2+x einnimmt und ein Partnernukleotid in einem im dritten Synthesezyklus gebildeten anderen Nukleotidpaar ist;
wobei in Schritt (5) in dem gespaltenen Gerüstpolynukleotid das terminale Ende des Trägerstrangs proximal zu dem Primerstrangteil einen Nukleotidüberhang umfasst, der 1+y Nukleotide umfasst, wobei die 1+y Nukleotide des Trägerstrangs das terminale Nukleotid des Primerstrangteils überhängen, und wobei das erste Nukleotid des Überhangs eine Position in dem Überhang distal zu dem terminalen Ende des Überhangs einnimmt, die Nukleotidposition n einnimmt und das Partnernukleotid für das in Schritt (7) eingebaute zweite Nukleotid des weiteren Synthesezyklus ist, und wobei das Nukleotid des Überhangs, das die Position n+1 einnimmt, das Partnernukleotid für das zweite Nukleotid des nächsten Synthesezyklus ist;
wobei in Schritt (5) am komplementären Ligationsende des Polynukleotid-Ligationsmoleküls das terminale Ende des Helferstrangs einen Nukleotidüberhang umfasst, der 1+y Nukleotide umfasst, wobei die 1+y Nukleotide des Helferstrangs das terminale Nukleotid des Trägerstrangs überhängen und Partnernukleotide für die 1+y überhängenden Nukleotide des Trägerstrangs des Gerüstpolynukleotids sind, und wobei das terminale Nukleotid des Trägerstrangs des komplementären Ligationsendes das erste Nukleotid des weiteren Synthesezyklus ist, die Nukleotidposition n+2+x einnimmt und ein Partnernukleotid in einem im nächsten Synthesezyklus gebildeten anderen Nukleotidpaar ist; und wobei:
i. die Position n die Nukleotidposition ist, die dem zweiten Nukleotid der vordefinierten Sequenz dieses Zyklus bei dessen Einbau gegenüberliegt;
ii. die folgenden Ligationspositionen n+1 und n+2 die ersten und zweiten Nukleotidpositionen im Trägerstrang relativ zur Position n in der Richtung proximal zum Helferstrang/distal zum Primerstrangteil sind;
iii. y eine ganze Zahl ist, die eins oder höher ist;
iv. x eine ganze Zahl ist, die Null oder höher ist;
v. die Zahl für y in dem Gerüstpolynukleotid bevorzugt die gleiche Zahl ist wie die Zahl für y in dem komplementären Ligationsende des Polynukleotid-Ligationsmoleküls, und wobei, wenn y eine unterschiedliche Zahl ist, die Zahl für y in dem komplementären Ligationsende des Polynukleotid-Ligationsmoleküls bevorzugt kleiner ist als die Zahl für y in dem Gerüstpolynukleotid; und
vi. in jeder gegebenen Serie von ersten und weiteren Zyklen die für x ausgewählte Zahl die für y im Gerüstpolynukleotid ausgewählte Zahl minus eins ist.

7. Verfahren nach Anspruch 6, wobei:
(i)
(a) in dem Ligationsschritt des ersten Zyklus (Schritt 1) und in den Ligationsschritten aller weiteren Zyklen das komplementäre Ligationsende des Polynukleotid-Ligationsmoleküls so strukturiert wird, dass:
i. das erste Nukleotid der vordefinierten Sequenz dieses Zyklus das terminale Nukleotid des Trägerstrangs ist, die Nukleotidposition n+1 im Trägerstrang einnimmt und mit dem vorletzten Nukleotid des Helferstrangs gepaart ist;
ii. das universelle Nukleotid das vorletzte Nukleotid des Trägerstrangs ist, die Nukleotidposition n+2 im Trägerstrang einnimmt und mit einem Partnernukleotid im Helferstrang gepaart ist;
iii. der Überhang einen Ein-Nukleotid-Überhang umfasst, das terminale Nukleotid des Helferstrangs umfasst, das das erste Nukleotid der vordefinierten Sequenz dieses Zyklus überhängt; und
iv. das terminale Nukleotid des Helferstrangs ein nicht ligierbares Nukleotid ist;
wobei die Position n die Nukleotidposition ist, die dem zweiten Nukleotid der vordefinierten Sequenz dieses Zyklus bei dessen Einbau gegenüberliegt, und die Positionen n+1 und n+2 die erste/nächste bzw. zweite Nukleotidposition im Trägerstrang relativ zur Position n in der Richtung distal zum komplementären Ligationsende sind; und wobei bei der Ligation das terminale Nukleotid des Trägerstrangs des Polynukleotid-Ligationsmoleküls an das terminale Nukleotid des Gerüstpolynukleotids proximal zum Primerstrangteil des Synthesestrangs ligiert wird und ein Einzelstrangbruch zwischen den terminalen Nukleotiden des Helferstrangs und dem Primerstrangteil des Synthesestrangs erzeugt wird;
(b) im Spaltungsschritt des ersten Zyklus (Schritt 2) und in allen weiteren Zyklen der Trägerstrang des ligierten Gerüstpolynukleotids zwischen den Positionen n+2 und n+1 gespalten wird, wodurch das Polynukleotid-Ligationsmolekül aus dem Gerüstpolynukleotid freigesetzt wird und das erste Nukleotid dieses Zyklus ungepaart und an den ersten Strang des gespaltenen Gerüstpolynukleotids gebunden bleibt, wobei bei der Spaltung ein Doppelnukleotidüberhang in dem Trägerstrang des gespaltenen Gerüstpolynukleotids erzeugt wird, wobei das terminale und vorletzte Nukleotid des Trägerstrangs das terminale Nukleotid des Primerstrangteils des Synthesestrangs überhängen; und
(c) im Verlängerungsschritt des ersten Zyklus (Schritt 3) und in allen weiteren Zyklen das zweite Nukleotid dieses Zyklus in den zweiten Strang eingebaut wird und mit dem vorletzten Nukleotid des überhängenden Trägerstrangs gepaart wird, und wobei nach den Schritten (7) und (8) die vom ersten Nukleotid dieses weiteren Zyklus im Trägerstrang des gespaltenen Gerüstpolynukleotids eingenommene Position als Nukleotidposition n im nächsten Synthesezyklus definiert wird; oder
(ii)
(a) im Ligationsschritt des ersten Zyklus (Schritt 1) und in den Ligationsschritten aller weiteren Zyklen das komplementäre Ligationsende des Polynukleotid-Ligationsmoleküls so strukturiert wird, dass:
i. das erste Nukleotid der vordefinierten Sequenz dieses Zyklus das terminale Nukleotid des Trägerstrangs ist, die Nukleotidposition n+1 im Trägerstrang einnimmt und mit dem vorletzten Nukleotid des Helferstrangs gepaart ist;
ii. das universelle Nukleotid die Nukleotidposition n+3 in dem Trägerstrang einnimmt und mit einem Partnernukleotid in dem Helferstrang gepaart ist;
iii. der Überhang einen Ein-Nukleotid-Überhang umfasst, das terminale Nukleotid des Helferstrangs umfasst, das das erste Nukleotid der vordefinierten Sequenz dieses Zyklus überhängt; und
iv. das terminale Nukleotid des Helferstrangs ein nicht ligierbares Nukleotid ist;
wobei die Position n die Nukleotidposition ist, die dem zweiten Nukleotid der vordefinierten Sequenz dieses Zyklus bei dessen Einbau gegenüberliegt, und die Positionen n+1, n+2 und n+3 die nächste, zweite bzw. dritte Nukleotidposition im Trägerstrang relativ zur Position n in der Richtung distal zum komplementären Ligationsende sind; und wobei bei der Ligation das terminale Nukleotid des Trägerstrangs des Polynukleotid-Ligationsmoleküls an das terminale Nukleotid des Gerüstpolynukleotids proximal zum Primerstrangteil des Synthesestrangs ligiert wird und ein Einzelstrangbruch zwischen den terminalen Nukleotiden des Helferstrangs und dem Primerstrangteil des Synthesestrangs erzeugt wird;
(b) in dem Spaltungsschritt des ersten Zyklus (Schritt 2) und in allen weiteren Zyklen der Trägerstrang des ligierten Gerüstpolynukleotids zwischen den Positionen n+2 und n+1 gespalten wird, wodurch das Polynukleotid-Ligationsmolekül aus dem Gerüstpolynukleotid freigesetzt wird und das erste Nukleotid dieses Zyklus ungepaart und an den ersten Strang des gespaltenen Gerüstpolynukleotids gebunden bleibt, wobei bei der Spaltung ein Doppelnukleotidüberhang in dem Trägerstrang des gespaltenen Gerüstpolynukleotids erzeugt wird, wobei das terminale und vorletzte Nukleotid des Trägerstrangs das terminale Nukleotid des Primerstrangteils des Synthesestrangs überhängen; und
(c) im Verlängerungsschritt des ersten Zyklus (Schritt 3) und in allen weiteren Zyklen das zweite Nukleotid dieses Zyklus in den zweiten Strang eingebaut wird und mit dem vorletzten Nukleotid des überhängenden Trägerstrangs gepaart wird, und wobei nach den Schritten (7) und (8) die vom ersten Nukleotid dieses weiteren Zyklus im Trägerstrang des gespaltenen Gerüstpolynukleotids eingenommene Position als Nukleotidposition n im nächsten Synthesezyklus definiert wird; oder
(iii)
(a) im Ligationsschritt des ersten Zyklus (Schritt 1) und in den Ligationsschritten aller weiteren Zyklen das komplementäre Ligationsende des Polynukleotid-Ligationsmoleküls so strukturiert wird, dass:
i. das erste Nukleotid der vordefinierten Sequenz dieses Zyklus das terminale Nukleotid des Trägerstrangs ist, die Nukleotidposition n+1 im Trägerstrang einnimmt und mit dem vorletzten Nukleotid des Helferstrangs gepaart ist;
ii. das universelle Nukleotid die Nukleotidposition n+3+x in dem Trägerstrang einnimmt und mit einem Partnernukleotid in dem Helferstrang gepaart ist;
iii. der Überhang einen Ein-Nukleotid-Überhang umfasst, das terminale Nukleotid des Helferstrangs umfasst, das das erste Nukleotid der vordefinierten Sequenz dieses Zyklus überhängt; und
iv. das terminale Nukleotid des Helferstrangs ein nicht ligierbares Nukleotid ist;
wobei die Position n die Nukleotidposition ist, die dem zweiten Nukleotid der vordefinierten Sequenz dieses Zyklus bei dessen Einbau gegenüberliegt, und die Positionen n+1 und n+3 die erste/nächste bzw. dritte Nukleotidposition im Trägerstrang relativ zur Position n in der Richtung distal zum komplementären Ligationsende sind, und wobei x eine Anzahl von Nukleotidpositionen relativ zur Position n+3 in der Richtung distal zum komplementären Ligationsende ist, wobei die Anzahl eine ganze Zahl von 1 bis 10 oder höher ist; und wobei bei der Ligation das terminale Nukleotid des Trägerstrangs des Polynukleotid-Ligationsmoleküls an das terminale Nukleotid des Gerüstpolynukleotids proximal zum Primerstrangteil des Synthesestrangs ligiert wird und ein Einzelstrangbruch zwischen den terminalen Nukleotiden des Helferstrangs und dem Primerstrangteil des Synthesestrangs erzeugt wird;
(b) in dem Spaltungsschritt des ersten Zyklus (Schritt 2) und in allen weiteren Zyklen der Trägerstrang des ligierten Gerüstpolynukleotids zwischen den Positionen n+2 und n+1 gespalten wird, wodurch das Polynukleotid-Ligationsmolekül aus dem Gerüstpolynukleotid freigesetzt wird und das erste Nukleotid dieses Zyklus ungepaart und an den ersten Strang des gespaltenen Gerüstpolynukleotids gebunden bleibt, wobei bei der Spaltung ein Doppelnukleotidüberhang in dem Trägerstrang des gespaltenen Gerüstpolynukleotids erzeugt wird, wobei das terminale und vorletzte Nukleotid des Trägerstrangs das terminale Nukleotid des Primerstrangteils des Synthesestrangs überhängen; und
(c) im Verlängerungsschritt des ersten Zyklus (Schritt 3) und in allen weiteren Zyklen das zweite Nukleotid dieses Zyklus in den zweiten Strang eingebaut wird und mit dem vorletzten Nukleotid des überhängenden Trägerstrangs gepaart wird, und wobei nach den Schritten (7) und (8) die vom ersten Nukleotid dieses weiteren Zyklus im Trägerstrang des gespaltenen Gerüstpolynukleotids eingenommene Position als Nukleotidposition n im nächsten Synthesezyklus definiert wird.

8. Verfahren nach Anspruch 6(b), wobei:
(a) sowohl im ersten als auch in weiteren Zyklen sowohl in dem Polynukleotid-Ligationsmolekül als auch in dem ligierten Gerüstpolynukleotid das universelle Nukleotid die Position n+3+x einnimmt und das Gerüstpolynukleotid zwischen den Positionen n+3+x und n+2+x gespalten wird; oder
(b) sowohl im ersten als auch in weiteren Zyklen sowohl in dem Polynukleotid-Ligationsmolekül als auch in dem ligierten Gerüstpolynukleotid das universelle Nukleotid die Position n+4+x einnimmt und das Gerüstpolynukleotid zwischen den Positionen n+3+x und n+2+x gespalten wird.

9. Verfahren nach einem der Ansprüche 2 bis 8, wobei das Verfahren so modifiziert wird, dass
(i) in Schritt (1) das Polynukleotid-Ligationsmolekül mit einem komplementären Ligationsende bereitgestellt wird, das ein erstes Nukleotid der vordefinierten Sequenz des ersten Zyklus umfasst und ferner ein oder mehrere weitere Nukleotide der vordefinierten Sequenz des ersten Zyklus umfasst;
(ii) in Schritt (2) nach der Spaltung das erste und weitere Nukleotide der vordefinierten Sequenz des ersten Zyklus in dem gespaltenen Gerüstpolynukleotid beibehalten werden;
(iii) in Schritt (3) das terminale Ende des Primerstrangteils des Synthesestrangs des doppelsträngigen Gerüstpolynukleotids durch den Einbau eines zweiten Nukleotids der vordefinierten Sequenz des ersten Zyklus durch die Wirkung des Nukleotidtransferase- oder Nukleotidpolymerase-Enzyms verlängert wird, und wobei das terminale Ende des Primerstrangabschnitts durch den Einbau eines oder mehrerer weiterer Nukleotide der vordefinierten Sequenz des ersten Zyklus durch die Wirkung des Nukleotidtransferase- oder Nukleotidpolymerase-Enzyms weiter verlängert wird, wobei das zweite und die weiteren Nukleotide des ersten Zyklus jeweils eine reversible Terminatorgruppe umfassen, die eine weitere Verlängerung durch das Enzym verhindert, und wobei nach jeder weiteren Verlängerung die reversible Terminatorgruppe vor dem Einbau des nächsten Nukleotids von einem Nukleotid entfernt wird;
(iv) in Schritt (5) das Polynukleotid-Ligationsmolekül mit einem komplementären Ligationsende bereitgestellt wird, das ein erstes Nukleotid der vordefinierten Sequenz des weiteren Zyklus umfasst und ferner ein oder mehrere weitere Nukleotide der vordefinierten Sequenz des weiteren Zyklus umfasst;
(v) in Schritt (6) nach der Spaltung das erste und weitere Nukleotide der vordefinierten Sequenz des weiteren Zyklus in dem gespaltenen Gerüstpolynukleotid beibehalten werden;
(vi) in Schritt (7) das terminale Ende des Primerstrangteils des Synthesestrangs des doppelsträngigen Gerüstpolynukleotids durch den Einbau eines zweiten Nukleotids der vordefinierten Sequenz des weiteren Zyklus durch die Wirkung des Nukleotidtransferase- oder Nukleotidpolymerase-Enzyms verlängert wird, und wobei das terminale Ende des Primerstrangteils durch den Einbau eines oder mehrerer weiterer Nukleotide der vordefinierten Sequenz des weiteren Zyklus durch die Wirkung des Nukleotidtransferase- oder Nukleotidpolymerase-Enzyms weiter verlängert wird, wobei das zweite und weitere Nukleotide des weiteren Zyklus jeweils eine reversible Terminatorgruppe umfassen, die eine weitere Verlängerung durch das Enzym verhindert, und wobei nach jeder weiteren Verlängerung die reversible Terminatorgruppe vor dem Einbau des nächsten Nukleotids von einem Nukleotid entfernt wird.

10. Verfahren nach Anspruch 9, wobei
(a) das komplementäre Ligationsende des Polynukleotid-Ligationsmoleküls so strukturiert wird, dass in den Schritten (2) und (6) vor der Spaltung das universelle Nukleotid eine Position im Trägerstrang einnimmt, die die nächste Nukleotidposition im Trägerstrang nach den Nukleotidpositionen des ersten und weiterer Nukleotide in der Richtung distal zum komplementären Ligationsende ist, und der Trägerstrang zwischen der Position, die von dem letzten weiteren Nukleotid eingenommen wird, und der Position, die von dem universellen Nukleotid eingenommen wird, gespalten wird; oder
(b) das komplementäre Ligationsende des Polynukleotid-Ligationsmoleküls so strukturiert wird, dass in den Schritten (2) und (6) vor der Spaltung das universelle Nukleotid eine Position im Trägerstrang einnimmt, die die nächste + 1 Nukleotidposition im Trägerstrang nach den Nukleotidpositionen des ersten und der weiteren Nukleotide in der Richtung distal zum komplementären Ligationsende ist, und der Trägerstrang zwischen der Position, die das letzte weitere Nukleotid einnimmt, und der Position, die das nächste Nukleotid im Trägerstrang einnimmt, gespalten wird.

11. Verfahren nach einem der vorhergehenden Ansprüche, wobei in einem, mehreren oder allen Synthesezyklen ein Partnernukleotid, das mit dem ersten Nukleotid der vordefinierten Sequenz paart, ein Nukleotid ist, das komplementär, bevorzugt natürlich komplementär, zum ersten Nukleotid ist.

12. Verfahren nach einem der Ansprüche 2 bis 11, wobei:
(a) in einem, mehreren oder allen Synthesezyklen vor Schritt (2) und/oder (6) das Gerüstpolynukleotid bereitgestellt wird, das einen Synthesestrang und einen daran hybridisierten Trägerstrang umfasst, und wobei der Synthesestrang ohne einen Helferstrang bereitgestellt wird; und/oder
(b) in einem, mehreren oder allen Synthesezyklen vor Schritt (2) und/oder (6) der Helferstrangteil des Synthesestrangs von dem Gerüstpolynukleotid entfernt wird.

13. Verfahren nach einem der Ansprüche 1 bis 3(i), 4 bis 6, 7(i), 6(b), 8(a), 9 und 10(a), wobei:
(a) jeder Spaltungsschritt einen zweistufigen Spaltungsprozess umfasst, wobei jeder Spaltungsschritt einen ersten Schritt umfasst, der das Entfernen des universellen Nukleotids umfasst, wodurch eine abasische Stelle gebildet wird, und einen zweiten Schritt umfasst, der die Spaltung des Trägerstrangs an der abasischen Stelle umfasst; wobei optional der erste Schritt mit einem Nukleotid-ausschneidenden Enzym durchgeführt wird; und/oder wobei der zweite Schritt mit einem an der abasischen Stelle Lyaseaktivität aufweisenden Enzym durchgeführt wird, wobei optional das an der abasischen Stelle Lyaseaktivität aufweisende Enzym ist:
(i) AP Endonuklease 1;
(ii) Endonuklease III (Nth); oder
(iii) Endonuklease VIII; oder
(b) jeder Spaltungsschritt einen einstufigen Spaltungsprozess umfasst, der das Entfernen des universellen Nukleotids mit einem Spaltungsenzym umfasst, wobei das Enzym ist:
(i) Endonuklease III;
(ii) Endonuklease VIII;
(iii) Formamidopirimidin-DNA-Glykosylase (Fpg); oder
(iv) 8-Oxoguanin-DNA-Glykosylase (hOGG1).

14. Verfahren nach einem der Ansprüche 1 bis 2, 3(ii), 4 bis 6, 7(ii), 8(b), 9 und 10(b), wobei der Spaltungsschritt das Spalten des Trägerstrangs mit einem Enzym umfasst; wobei optional das Enzym den Trägerstrang zwischen den Nukleotidpositionen n+1 und n spaltet; und/oder wobei das Enzym Endonuclease V ist.

15. Verfahren nach einem der Ansprüche 2 bis 14, wobei in einem, mehreren oder allen Synthesezyklen:
(a) in den Schritten (1)/(5) im Gerüstpolynukleotid der Synthesestrang, der den Primerstrangteil umfasst, und der daran hybridisierte Teil des Trägerstrangs durch eine Haarnadelschleife verbunden sind; und
(b) in den Schritten (1)/(5) in dem Polynukleotid-Ligationsmolekül der Helferstrang und der daran hybridisierte Teil des Trägerstrangs durch eine Haarnadelschleife an dem Ende verbunden sind, das dem komplementären Ligationsende gegenüberliegt.

16. Verfahren nach einem der Ansprüche 2 bis 15, wobei der Synthesestrang, der den Primerstrangteil umfasst, und der daran hybridisierte Teil des Trägerstrangs an eine gemeinsame Oberfläche gebunden sind, optional über eine oder mehrere kovalente Bindungen; wobei ferner optional die eine oder mehreren kovalenten Bindungen zwischen einer funktionellen Gruppe auf der gemeinsamen Oberfläche und einer funktionellen Gruppe auf dem Gerüstmolekül gebildet werden, wobei die funktionelle Gruppe auf dem Gerüstmolekül eine Amingruppe, eine Thiolgruppe, eine Thiophosphatgruppe oder eine Thioamidgruppe ist; wobei stärker bevorzugt die funktionelle Gruppe auf der gemeinsamen Oberfläche eine Bromacetylgruppe ist, wobei optional die Bromacetylgruppe auf einer Polyacrylamidoberfläche bereitgestellt wird, die unter Verwendung von N-(5-Bromacetamidylpentyl)acrylamid (BRAPA) abgeleitet wird.

17. Verfahren nach einem der vorhergehenden Ansprüche, wobei:
(a) Synthesezyklen in Tröpfchen innerhalb eines mikrofluidischen Systems durchgeführt werden; wobei optional das mikrofluidische System ein elektrobenetzendes System ist; wobei stärker bevorzugt das mikrofluidische System ein auf dielektrisch beschichteter Oberfläche ektrobenetzendes System (EWOD) ist; und/oder
(b) nach der Synthese die Stränge der doppelsträngigen Polynukleotide getrennt werden, um ein eine vordefinierte Sequenz aufweisendes einzelsträngiges Polynukleotid bereitzustellen; und/oder
(c) wobei nach der Synthese das doppelsträngige Polynukleotid oder ein Bereich davon amplifiziert wird, vorzugsweise durch PCR.

18. Verfahren zum Zusammensetzen eines eine vordefinierte Sequenz aufweisenden Polynukleotids, wobei das Verfahren das Durchführen des Verfahrens nach einem der vorhergehenden Ansprüche umfasst, um ein erstes Polynukleotid, das eine vordefinierte Sequenz aufweist, und ein oder mehrere zusätzliche Polynukleotide, die eine vordefinierte Sequenz aufweisen, zu synthetisieren und das erste und das eine oder die mehreren zusätzlichen Polynukleotide miteinander zu verbinden; wobei optional das erste Polynukleotid und das eine oder die mehreren zusätzlichen Polynukleotide gespalten werden, um kompatible Enden zu erzeugen und miteinander verbunden zu werden, bevorzugt durch Ligation; wobei ferner optional das erste Polynukleotid und das eine oder die mehreren zusätzlichen Polynukleotide durch ein Restriktionsenzym an einer Spaltstelle gespalten werden.

## Revendications

1. Procédé *in vitro* de synthèse d'un polynucléotide double brin qui présente une séquence prédéfinie, le procédé comprenant la réalisation de cycles de synthèse dans lesquels, à chaque cycle :
(A) un polynucléotide d'échafaudage est fourni, comprenant un brin de synthèse et un brin de support hybridé à celui-ci, le brin de synthèse comprenant une partie de brin d'amorce, et le brin de support étant le premier brin du polynucléotide double brin et le brin de synthèse étant le second brin du polynucléotide double brin ;
(B) le premier brin du polynucléotide double brin est étendu par l'ajout d'un premier nucléotide de la séquence prédéfinie et d'un nucléotide universel sous l'action d'une enzyme ligase, le nucléotide universel définissant un site de clivage ;
(C) le polynucléotide double brin est ensuite clivé au niveau du site de clivage ; et
(D) le second brin qui est hybridé au premier brin est ensuite étendu par l'ajout d'un second nucléotide de la séquence prédéfinie par une enzyme nucléotide transférase ou polymérase ; et
dans lequel les premier et second nucléotides de la séquence prédéfinie de chaque cycle sont conservés dans le polynucléotide double brin après clivage ; et
dans lequel, dans un cycle donné de synthèse, le second nucléotide de ce cycle qui est ajouté au second brin du polynucléotide double brin comprend un groupe terminateur réversible qui empêche toute extension supplémentaire par l'enzyme, et dans lequel le groupe terminateur réversible est éliminé du second nucléotide incorporé de ce cycle avant l'ajout, dans le cycle suivant de synthèse, du second nucléotide du cycle suivant,
dans lequel, dans chaque cycle, le premier nucléotide est un nucléotide partenaire du second nucléotide, et dans lequel, lors de l'incorporation dans le polynucléotide double brin, les premier et second nucléotides forment une paire de nucléotides ; facultativement, dans lequel la réaction de ligature comprend une réaction de ligature à extrémités franches ; facultativement, dans lequel le premier nucléotide et le nucléotide universel sont des composants d'une molécule de ligature polynucléotidique, et dans lequel la molécule de ligature polynucléotidique est ligaturée au polynucléotide double brin pendant l'étape (B) sous l'action de l'enzyme ligase dans une réaction de ligature à extrémités franches, et dans lequel, lors de la ligature de la molécule de ligature polynucléotidique au polynucléotide double brin, le premier brin du polynucléotide double brin est étendu et le site de clivage est créé.

2. Procédé selon la revendication 1, le procédé comprenant la réalisation d'un premier cycle de synthèse comprenant :
(1) la ligature d'une molécule de ligature polynucléotidique double brin au polynucléotide d'échafaudage sous l'action de l'enzyme ligase dans une réaction de ligature à extrémités franches, la molécule de ligature polynucléotidique comprenant un brin de support et un brin auxiliaire hybridé à celui-ci et comprenant en outre une extrémité de ligature complémentaire, l'extrémité de ligature comprenant :
(i) dans le brin de support, un nucléotide universel et un premier nucléotide de la séquence prédéfinie ; et
(ii) dans le brin auxiliaire, un nucléotide terminal non apte à ligature ;
dans lequel, lors de la ligature, le premier brin du polynucléotide double brin est étendu avec le premier nucléotide et le site de clivage est créé par l'incorporation du nucléotide universel dans le premier brin ;
(2) le clivage du polynucléotide d'échafaudage ligaturé au niveau du site de clivage, le clivage comprenant le clivage du brin de support et l'élimination du nucléotide universel du polynucléotide d'échafaudage pour fournir un polynucléotide d'échafaudage double brin clivé comprenant le premier nucléotide incorporé ;
(3) l'extension de l'extrémité terminale de la partie brin d'amorce du brin de synthèse du polynucléotide d'échafaudage double brin par l'incorporation d'un second nucléotide de la séquence prédéfinie sous l'action de l'enzyme nucléotide transférase ou polymérase, le second nucléotide comprenant un groupe terminateur réversible qui empêche toute extension supplémentaire par l'enzyme, le second nucléotide étant un partenaire du premier nucléotide et, lors de l'incorporation, le second nucléotide et le premier nucléotide formant une paire de nucléotides ; et
(4) l'élimination du groupe terminateur réversible du second nucléotide ;
le procédé comprenant en outre la réalisation d'un cycle de synthèse supplémentaire comprenant :
(5) la ligature d'une molécule de ligature polynucléotidique double brin supplémentaire au polynucléotide d'échafaudage clivé sous l'action de l'enzyme ligase dans une réaction de ligature à extrémités franches, la molécule de ligature polynucléotidique comprenant un brin de support et un brin auxiliaire hybridé à celui-ci et comprenant en outre une extrémité de ligature complémentaire, l'extrémité de ligature comprenant :
(i) dans le brin de support, un nucléotide universel et le premier nucléotide du cycle de synthèse supplémentaire ; et
(ii) dans le brin auxiliaire, un nucléotide terminal non apte à ligature ;
dans lequel, lors de la ligature, le premier brin du polynucléotide double brin est étendu avec le premier nucléotide du cycle de synthèse supplémentaire et le site de clivage est créé par l'incorporation du nucléotide universel dans le premier brin ;
(6) le clivage du polynucléotide d'échafaudage ligaturé au niveau du site de clivage, le clivage comprenant le clivage du brin de support et l'élimination du nucléotide universel du polynucléotide d'échafaudage pour fournir un polynucléotide d'échafaudage double brin clivé comprenant la ou les paires de nucléotides du ou des cycles précédents et le premier nucléotide du cycle de synthèse supplémentaire ;
(7) l'extension de l'extrémité terminale de la partie brin d'amorce du brin de synthèse du polynucléotide d'échafaudage double brin par l'incorporation du second nucléotide du cycle de synthèse supplémentaire sous l'action de l'enzyme nucléotide transférase ou polymérase, le second nucléotide comprenant un groupe terminateur réversible qui empêche toute extension supplémentaire par l'enzyme, le second nucléotide du cycle de synthèse supplémentaire étant un partenaire du premier nucléotide du cycle de synthèse supplémentaire, et, lors de l'incorporation, les second et premier nucléotides du cycle de synthèse supplémentaire formant une paire de nucléotides supplémentaire ;
(8) l'élimination du groupe terminateur réversible du second nucléotide ; et
(9) répétition des étapes 5 à 8 plusieurs fois pour obtenir le polynucléotide double brin qui présente une séquence nucléotidique prédéfinie.

3. Procédé selon la revendication 2, dans lequel :
(I)
(a) dans l'étape de ligature du premier cycle (étape 1) et dans les étapes de ligature de tous les cycles supplémentaires, l'extrémité de ligature complémentaire de la molécule de ligature polynucléotidique est structurée de sorte que :
i. le premier nucléotide de la séquence prédéfinie de ce cycle est le nucléotide terminal du brin de support, occupe la position nucléotidique n dans le brin de support et est apparié avec le nucléotide terminal du brin auxiliaire ;
ii. le nucléotide universel est l'avant-dernier nucléotide du brin de support, occupe la position nucléotidique n+1 dans le brin de support et est apparié avec l'avant-dernier nucléotide du brin auxiliaire ; et
iii. le nucléotide terminal du brin auxiliaire est un nucléotide non apte à ligature ;
dans lequel la position n est la position nucléotidique qui est opposée au second nucléotide de la séquence prédéfinie de ce cycle lors de son incorporation, et dans lequel la position n+1 est la position nucléotidique suivante dans le brin de support par rapport à la position n dans la direction distale par rapport à l'extrémité de ligature complémentaire ; et dans lequel, lors de la ligature, le nucléotide terminal du brin de support de la molécule de ligature polynucléotidique est ligaturé au nucléotide terminal du polynucléotide d'échafaudage proximal à la partie brin d'amorce du brin de synthèse et une rupture simple brin est créée entre les nucléotides terminaux du brin auxiliaire et la partie brin d'amorce du brin de synthèse ;
(b) dans l'étape de clivage du premier cycle (étape 2) et de tous les cycles supplémentaires, le brin de support du polynucléotide d'échafaudage ligaturé est clivé entre les positions n+1 et n, libérant ainsi la molécule de ligature polynucléotidique du polynucléotide d'échafaudage et conservant le premier nucléotide de ce cycle fixé au premier brin du polynucléotide d'échafaudage clivé ; et
(c) dans l'étape d'extension du premier cycle (étape 3) et dans tous les cycles supplémentaires, le second nucléotide de ce cycle est incorporé dans le second brin opposé au premier nucléotide dans le premier brin et est apparié avec celui-ci ; et après quoi la position occupée par le premier nucléotide de ce cycle dans le brin de support du polynucléotide d'échafaudage clivé est définie en tant que position nucléotidique n-1 dans le cycle de synthèse suivant ; ou
(II)
(a) dans l'étape de ligature du premier cycle (étape 1) et dans les étapes de ligature de tous les cycles supplémentaires, l'extrémité de ligature complémentaire de la molécule de ligature polynucléotidique est structurée de sorte que :
i. le premier nucléotide de la séquence prédéfinie de ce cycle est le nucléotide terminal du brin de support, occupe la position nucléotidique n dans le brin de support et est apparié avec le nucléotide terminal du brin auxiliaire ;
ii. le nucléotide universel occupe la position nucléotidique n+2 dans le brin de support et est apparié avec un nucléotide partenaire dans le brin auxiliaire ; et
iii. le nucléotide terminal du brin auxiliaire est un nucléotide non apte à ligature ;
dans lequel la position n est la position nucléotidique qui est opposée au second nucléotide de la séquence prédéfinie de ce cycle lors de son incorporation, et dans lequel la position n+2 est la deuxième position nucléotidique dans le brin de support par rapport à la position n dans la direction distale par rapport à l'extrémité de ligature complémentaire ; et dans lequel, lors de la ligature, le nucléotide terminal du brin de support de la molécule de ligature polynucléotidique est ligaturé au nucléotide terminal du polynucléotide d'échafaudage proximal à la partie brin d'amorce du brin de synthèse et une rupture simple brin est créée entre les nucléotides terminaux du brin auxiliaire et la partie brin d'amorce du brin de synthèse ;
(b) dans l'étape de clivage du premier cycle (étape 2) et de tous les cycles supplémentaires, le brin de support du polynucléotide d'échafaudage ligaturé est clivé entre les positions n+1 et n, libérant ainsi la molécule de ligature polynucléotidique du polynucléotide d'échafaudage et conservant le premier nucléotide de ce cycle fixé au premier brin du polynucléotide d'échafaudage clivé ; et
(c) dans l'étape d'extension du premier cycle (étape 3) et dans tous les cycles supplémentaires, le second nucléotide de ce cycle est incorporé dans le second brin opposé au premier nucléotide dans le premier brin et est apparié avec celui-ci, et après quoi la position occupée par le premier nucléotide de ce cycle dans le brin de support du polynucléotide d'échafaudage clivé est définie en tant que position nucléotidique n-1 dans le cycle de synthèse suivant ; ou
(III)
(a) dans l'étape de ligature du premier cycle (étape 1) et dans les étapes de ligature de tous les cycles supplémentaires, l'extrémité de ligature complémentaire de la molécule de ligature polynucléotidique est structurée de sorte que :
i. le premier nucléotide de la séquence prédéfinie de ce cycle est le nucléotide terminal du brin de support, occupe la position nucléotidique n dans le brin de support et est apparié avec le nucléotide terminal du brin auxiliaire ;
ii. le nucléotide universel occupe la position nucléotidique n+2+x dans le brin de support et est apparié avec un nucléotide partenaire dans le brin auxiliaire ; et
iii. le nucléotide terminal du brin auxiliaire est un nucléotide non apte à ligature ;
dans lequel la position n est la position nucléotidique qui est opposée au second nucléotide de la séquence prédéfinie de ce cycle lors de son incorporation, et dans lequel la position n+2 est la deuxième position nucléotidique dans le brin de support par rapport à la position n dans la direction distale par rapport à l'extrémité de ligature complémentaire et dans lequel x est un nombre de positions nucléotidiques par rapport à la position n+2 dans la direction distale par rapport à l'extrémité de ligature complémentaire, le nombre étant un nombre entier compris entre 1 et 10 ou plus ; et dans lequel, lors de la ligature, le nucléotide terminal du brin de support de la molécule de ligature polynucléotidique est ligaturé au nucléotide terminal du polynucléotide d'échafaudage proximal à la partie brin d'amorce du brin de synthèse et une rupture simple brin est créée entre les nucléotides terminaux du brin auxiliaire et la partie brin d'amorce du brin de synthèse ;
(b) dans l'étape de clivage du premier cycle (étape 2) et de tous les cycles supplémentaires, le brin de support du polynucléotide d'échafaudage ligaturé est clivé entre les positions n+1 et n, libérant ainsi la molécule de ligature polynucléotidique du polynucléotide d'échafaudage et conservant le premier nucléotide de ce cycle fixé au premier brin du polynucléotide d'échafaudage clivé ; et
(c) dans l'étape d'extension du premier cycle (étape 3) et dans tous les cycles supplémentaires, le second nucléotide de ce cycle est incorporé dans le second brin opposé au premier nucléotide dans le premier brin et est apparié avec celui-ci, et après quoi la position occupée par le premier nucléotide de ce cycle dans le brin de support du polynucléotide d'échafaudage clivé est définie en tant que position nucléotidique n-1 dans le cycle de synthèse suivant.

4. Procédé selon la revendication 1, dans lequel, dans chaque cycle, lors de l'incorporation, le premier nucléotide et le second nucléotide deviennent des nucléotides partenaires dans différentes paires de nucléotides dans le polynucléotide double brin synthétisé ; facultativement
dans lequel la réaction de ligature comprend une réaction de ligature à extrémités cohésives ; en outre facultativement, dans lequel le premier nucléotide et le nucléotide universel sont des composants d'une molécule de ligature polynucléotidique, et dans lequel la molécule de ligature polynucléotidique est ligaturée au polynucléotide double brin pendant l'étape (B) sous l'action de l'enzyme ligase dans une réaction de ligature à extrémités cohésives, et dans lequel, lors de la ligature de la molécule de ligature de polynucléotide au polynucléotide double brin, le premier brin du polynucléotide double brin est étendu et le site de clivage est créé.

5. Procédé selon la revendication 4, le procédé comprenant la réalisation d'un premier cycle de synthèse comprenant :
(1) la ligature d'une molécule de ligature polynucléotidique double brin au polynucléotide d'échafaudage sous l'action de l'enzyme ligase dans une réaction de ligature à extrémités cohésives, la molécule de ligature polynucléotidique comprenant un brin de support et un brin auxiliaire hybridé à celui-ci et comprenant en outre une extrémité de ligature complémentaire, l'extrémité de ligature comprenant :
(i) dans le brin de support, un nucléotide universel et un premier nucléotide de la séquence prédéfinie ; et
(ii) dans le brin auxiliaire, un nucléotide terminal non apte à ligature ;
dans lequel, lors de la ligature, le premier brin du polynucléotide double brin est étendu avec le premier nucléotide et le site de clivage est créé par l'incorporation du nucléotide universel dans le premier brin ;
(2) le clivage du polynucléotide d'échafaudage ligaturé au niveau du site de clivage, le clivage comprenant le clivage du brin de support et l'élimination du nucléotide universel du polynucléotide d'échafaudage pour fournir un polynucléotide d'échafaudage double brin clivé comprenant le premier nucléotide incorporé ;
(3) l'extension de l'extrémité terminale de la partie brin d'amorce du brin de synthèse du polynucléotide d'échafaudage double brin par l'incorporation d'un second nucléotide de la séquence prédéfinie sous l'action de l'enzyme nucléotide transférase ou polymérase, le second nucléotide comprenant un groupe terminateur réversible qui empêche toute extension supplémentaire par l'enzyme, dans lequel le second nucléotide ; et
(4) l'élimination du groupe terminateur réversible du second nucléotide ;
le procédé comprenant en outre la réalisation d'un cycle de synthèse supplémentaire comprenant :
(5) la ligature d'une molécule de ligature polynucléotidique double brin supplémentaire au polynucléotide d'échafaudage clivé sous l'action de l'enzyme ligase dans une réaction de ligature à extrémités cohésives, la molécule de ligature polynucléotidique comprenant un brin de support et un brin auxiliaire hybridé à celui-ci et comprenant en outre une extrémité de ligature complémentaire, l'extrémité de ligature comprenant :
(i) dans le brin de support, un nucléotide universel et le premier nucléotide du cycle de synthèse supplémentaire ; et
(ii) dans le brin auxiliaire, un nucléotide terminal non apte à ligature ;
dans lequel, lors de la ligature, le premier brin du polynucléotide double brin est étendu avec le premier nucléotide du cycle de synthèse supplémentaire et le site de clivage est créé par l'incorporation du nucléotide universel dans le premier brin ;
(6) le clivage du polynucléotide d'échafaudage ligaturé au niveau du site de clivage, le clivage comprenant le clivage du brin de support et l'élimination du nucléotide universel du polynucléotide d'échafaudage pour fournir un polynucléotide d'échafaudage double brin clivé comprenant la ou les paires de nucléotides du ou des cycles précédents et le premier nucléotide du cycle de synthèse supplémentaire ;
(7) l'extension de l'extrémité terminale de la partie brin d'amorce du brin de synthèse du polynucléotide d'échafaudage double brin par l'incorporation du second nucléotide du cycle de synthèse supplémentaire sous l'action de l'enzyme nucléotide transférase ou polymérase, le second nucléotide comprenant un groupe terminateur réversible qui empêche toute extension supplémentaire par l'enzyme ;
(8) l'élimination du groupe terminateur réversible du second nucléotide ; et
(9) la répétition des étapes 5 à 8 plusieurs fois pour obtenir le polynucléotide double brin qui présente une séquence nucléotidique prédéfinie.

6. Procédé selon la revendication 5, dans lequel :
(a) dans l'étape (1), l'extrémité terminale du brin de support du polynucléotide d'échafaudage proximal à la partie brin d'amorce comprend un surplomb nucléotidique, dans lequel le nucléotide terminal du brin de support surplombe le nucléotide terminal de la partie brin d'amorce, et dans lequel le nucléotide terminal du brin de support est le nucléotide partenaire du second nucléotide de ce cycle ;
dans lequel, dans l'étape (1), à l'extrémité de ligature complémentaire de la molécule de ligature polynucléotidique, l'extrémité terminale du brin auxiliaire comprend un surplomb nucléotidique, dans lequel le nucléotide terminal du brin auxiliaire surplombe le nucléotide terminal du brin de support, et dans lequel le nucléotide terminal du brin de support est le premier nucléotide de ce cycle et est un nucléotide partenaire dans une paire de nucléotides différente formée dans le cycle de synthèse suivant ;
dans lequel, dans l'étape (5), dans le polynucléotide d'échafaudage clivé, l'extrémité terminale du brin de support proximale à la partie brin d'amorce comprend un surplomb nucléotidique, dans lequel les nucléotides terminal et avant-dernier du brin de support surplombent le nucléotide terminal de la partie brin d'amorce, et dans lequel l'avant-dernier nucléotide du brin de support est le nucléotide partenaire du second nucléotide du cycle de synthèse supplémentaire incorporé dans l'étape (7) ;
dans lequel, dans l'étape (5), à l'extrémité de ligature complémentaire de la molécule de ligature polynucléotidique, l'extrémité terminale du brin auxiliaire comprend un surplomb nucléotidique, dans lequel le nucléotide terminal du brin auxiliaire surplombe le nucléotide terminal du brin de support, et dans lequel le nucléotide terminal du brin de support est le premier nucléotide du cycle de synthèse supplémentaire et est un nucléotide partenaire dans une paire de nucléotides différente formée dans le cycle de synthèse suivant ; et
dans lequel, dans le premier cycle de synthèse et les cycles de synthèse supplémentaires, le surplomb nucléotidique dans l'extrémité de ligature complémentaire de la molécule de ligature polynucléotidique et le surplomb nucléotidique dans le polynucléotide d'échafaudage comprennent le même nombre de nucléotides, dans lequel le nombre est égal ou supérieur à un, à condition que le nombre de nucléotides dans le surplomb dans le polynucléotide d'échafaudage soit supérieur de un au nombre de nucléotides dans le surplomb dans l'extrémité de ligature complémentaire ; ou
(b) dans l'étape (1), l'extrémité terminale du brin de support du polynucléotide d'échafaudage proximale à la partie brin d'amorce comprend un surplomb nucléotidique comprenant 1+y nucléotides, dans lequel les 1+y nucléotides du brin de support surplombent le nucléotide terminal de la partie brin d'amorce, et dans lequel le premier nucléotide du surplomb occupe une position dans le surplomb distale à l'extrémité terminale du surplomb, occupe la position nucléotidique n et est le nucléotide partenaire du second nucléotide de ce premier cycle incorporé dans l'étape (3), et dans lequel le nucléotide du surplomb qui occupe la position n+1 est le nucléotide partenaire du second nucléotide du cycle suivant/deuxième cycle de synthèse incorporé dans l'étape (7) ;
dans lequel, dans l'étape (1), à l'extrémité de ligature complémentaire de la molécule de ligature polynucléotidique, l'extrémité terminale du brin auxiliaire comprend un surplomb nucléotidique comprenant 1+y nucléotides, dans lequel les 1+y nucléotides du brin auxiliaire surplombent le nucléotide terminal du brin de support et sont des nucléotides partenaires pour les 1+y nucléotides en surplomb du brin de support du polynucléotide d'échafaudage, et dans lequel le nucléotide terminal du brin de support de l'extrémité de ligature complémentaire est le premier nucléotide de ce cycle, occupe la position nucléotidique n+2+x et est un nucléotide partenaire dans une paire de nucléotides différente formée dans le troisième cycle de synthèse ;
dans lequel dans l'étape (5), dans le polynucléotide d'échafaudage clivé, l'extrémité terminale du brin de support proximale à la partie brin d'amorce comprend un surplomb nucléotidique comprenant 1+y nucléotides, dans lequel les 1+y nucléotides du brin de support surplombent le nucléotide terminal de la partie brin d'amorce, et dans lequel le premier nucléotide du surplomb occupe une position dans le surplomb distale à l'extrémité terminale du surplomb, occupe la position nucléotidique n et est le nucléotide partenaire du second nucléotide de ce cycle de synthèse supplémentaire incorporé dans l'étape (7), et dans lequel le nucléotide du surplomb qui occupe la position n+1 est le nucléotide partenaire du second nucléotide du cycle de synthèse suivant ;
dans lequel, dans l'étape (5), à l'extrémité de ligature complémentaire de la molécule de ligature polynucléotidique, l'extrémité terminale du brin auxiliaire comprend un surplomb nucléotidique comprenant 1+y nucléotides, dans lequel les 1+y nucléotides du brin auxiliaire surplombent le nucléotide terminal du brin de support et sont des nucléotides partenaires pour les 1+y nucléotides en surplomb du brin de support du polynucléotide d'échafaudage, et dans lequel le nucléotide terminal du brin de support de l'extrémité de ligature complémentaire est le premier nucléotide du cycle de synthèse supplémentaire, occupe la position nucléotidique n+2+x et est un nucléotide partenaire dans une paire de nucléotides différente formée dans le cycle de synthèse suivant ; et dans lequel :
i. la position n est la position nucléotidique qui est opposée au second nucléotide de la séquence prédéfinie de ce cycle lors de son incorporation ;
ii. les positions de ligature suivantes n+1 et n+2 sont les première et deuxième positions nucléotidiques dans le brin de support par rapport à la position n dans la direction proximale au brin auxiliaire/distale à la partie du brin d'amorce ;
iii. y est un nombre entier qui est égal ou supérieur à un ;
iv. x est un nombre entier qui est égal ou supérieur à zéro ;
v. le nombre pour y dans le polynucléotide d'échafaudage est de préférence le même nombre que le nombre pour y dans l'extrémité de ligature complémentaire de la molécule de ligature polynucléotidique, et dans lequel si y est un nombre différent, le nombre pour y dans l'extrémité de ligature complémentaire de la molécule de ligature polynucléotidique est de préférence inférieur au nombre pour y dans le polynucléotide d'échafaudage ; et
vi. dans toute série donnée de premier cycle et cycles supplémentaires, le nombre sélectionné pour x est le nombre sélectionné pour y dans le polynucléotide d'échafaudage moins un.

7. Procédé selon la revendication 6, dans lequel :
(i)
(a) dans l'étape de ligature du premier cycle (étape 1) et dans les étapes de ligature de tous les cycles supplémentaires, l'extrémité de ligature complémentaire de la molécule de ligature polynucléotidique est structurée de sorte que :
i. le premier nucléotide de la séquence prédéfinie de ce cycle est le nucléotide terminal du brin de support, occupe la position nucléotidique n +1 dans le brin de support et est apparié avec l'avant-dernier nucléotide du brin auxiliaire ;
ii. le nucléotide universel est l'avant-dernier nucléotide du brin de support, occupe la position nucléotidique n+2 dans le brin de support et est apparié à un nucléotide partenaire dans le brin auxiliaire ;
iii. le surplomb comprend un surplomb d'un nucléotide comprenant le nucléotide terminal du brin auxiliaire surplombant le premier nucléotide de la séquence prédéfinie de ce cycle ; et
iv. le nucléotide terminal du brin auxiliaire est un nucléotide non apte à ligature ;
dans lequel la position n est la position nucléotidique qui est opposée au second nucléotide de la séquence prédéfinie de ce cycle lors de son incorporation, et les positions n+1 et n+2 sont respectivement les première/suivante et deuxième positions nucléotidiques dans le brin de support par rapport à la position n dans la direction distale à l'extrémité de ligature complémentaire ; et dans lequel, lors de la ligature, le nucléotide terminal du brin de support de la molécule de ligature polynucléotidique est ligaturé au nucléotide terminal du polynucléotide d'échafaudage proximal à la partie brin d'amorce du brin de synthèse et une rupture simple brin est créée entre les nucléotides terminaux du brin auxiliaire et la partie brin d'amorce du brin de synthèse ;
(b) dans l'étape de clivage du premier cycle (étape 2) et dans tous les cycles supplémentaires, le brin de support du polynucléotide d'échafaudage ligaturé est clivé entre les positions n+2 et n+1, libérant ainsi la molécule de ligature polynucléotidique du polynucléotide d'échafaudage et conservant le premier nucléotide de ce cycle non apparié et fixé au premier brin du polynucléotide d'échafaudage clivé, dans lequel, lors du clivage, un surplomb binucléotidique est créé dans le brin de support du polynucléotide d'échafaudage clivé, les nucléotides terminal et avant-dernier du brin de support surplombant le nucléotide terminal de la partie brin d'amorce du brin de synthèse ; et
(c) dans l'étape d'extension du premier cycle (étape 3) et dans tous les cycles supplémentaires, le second nucléotide de ce cycle est incorporé dans le second brin et est apparié avec l'avant-dernier nucléotide du brin de support en surplomb, et dans lequel dans les étapes suivantes (7) et (8), la position occupée par le premier nucléotide de ce cycle supplémentaire dans le brin de support du polynucléotide d'échafaudage clivé est définie en tant que position nucléotidique n dans le cycle de synthèse suivant ; ou
(ii)
(a) dans l'étape de ligature du premier cycle (étape 1) et dans les étapes de ligature de tous les cycles supplémentaires, l'extrémité de ligature complémentaire de la molécule de ligature polynucléotidique est structurée de sorte que :
i. le premier nucléotide de la séquence prédéfinie de ce cycle est le nucléotide terminal du brin de support, occupe la position nucléotidique n+1 dans le brin de support et est apparié avec l'avant-dernier nucléotide du brin auxiliaire ;
ii. le nucléotide universel occupe la position nucléotidique n+3 dans le brin de support et est apparié avec un nucléotide partenaire dans le brin auxiliaire ;
iii. le surplomb comprend un surplomb d'un nucléotide comprenant le nucléotide terminal du brin auxiliaire surplombant le premier nucléotide de la séquence prédéfinie de ce cycle ; et
iv. le nucléotide terminal du brin auxiliaire est un nucléotide non apte à ligature ;
dans lequel la position n est la position nucléotidique qui est opposée au second nucléotide de la séquence prédéfinie de ce cycle lors de son incorporation, et les positions n+1, n+2 et n+3 sont respectivement les suivante, deuxième et troisième positions nucléotidiques dans le brin de support par rapport à la position n dans la direction distale à l'extrémité de ligature complémentaire ; et dans lequel, lors de la ligature, le nucléotide terminal du brin de support de la molécule de ligature polynucléotidique est ligaturé au nucléotide terminal du polynucléotide d'échafaudage proximal à la partie brin d'amorce du brin de synthèse et une rupture simple brin est créée entre les nucléotides terminaux du brin auxiliaire et la partie brin d'amorce du brin de synthèse ;
(b) dans l'étape de clivage du premier cycle (étape 2) et dans tous les cycles supplémentaires, le brin de support du polynucléotide d'échafaudage ligaturé est clivé entre les positions n+2 et n+1, libérant ainsi la molécule de ligature polynucléotidique du polynucléotide d'échafaudage et conservant le premier nucléotide de ce cycle non apparié et fixé au premier brin du polynucléotide d'échafaudage clivé, dans lequel, lors du clivage, un surplomb binucléotidique est créé dans le brin de support du polynucléotide d'échafaudage clivé, les nucléotides terminal et avant-dernier du brin de support surplombant le nucléotide terminal de la partie brin d'amorce du brin de synthèse ; et
(c) dans l'étape d'extension du premier cycle (étape 3) et dans tous les cycles supplémentaires, le second nucléotide de ce cycle est incorporé dans le second brin et est apparié avec l'avant-dernier nucléotide du brin de support en surplomb, et dans lequel dans les étapes suivantes (7) et (8), la position occupée par le premier nucléotide de ce cycle supplémentaire dans le brin de support du polynucléotide d'échafaudage clivé est définie en tant que position nucléotidique n dans le cycle de synthèse suivant ; ou
(iii)
(a) dans l'étape de ligature du premier cycle (étape 1) et dans les étapes de ligature de tous les cycles supplémentaires, l'extrémité de ligature complémentaire de la molécule de ligature polynucléotidique est structurée de sorte que :
i. le premier nucléotide de la séquence prédéfinie de ce cycle est le nucléotide terminal du brin de support, occupe la position nucléotidique n+1 dans le brin de support et est apparié avec l'avant-dernier nucléotide du brin auxiliaire ;
ii. le nucléotide universel occupe la position nucléotidique n+3+x dans le brin de support et est apparié avec un nucléotide partenaire dans le brin auxiliaire ;
iii. le surplomb comprend un surplomb d'un nucléotide comprenant le nucléotide terminal du brin auxiliaire surplombant le premier nucléotide de la séquence prédéfinie de ce cycle ; et
iv. le nucléotide terminal du brin auxiliaire est un nucléotide non apte à ligature ;
dans lequel la position n est la position nucléotidique qui est opposée au second nucléotide de la séquence prédéfinie de ce cycle lors de son incorporation, et les positions n+1 et n+3 sont respectivement les première/suivante et troisième positions nucléotidiques dans le brin de support par rapport à la position n dans la direction distale à l'extrémité de ligature complémentaire, et dans lequel x est un nombre de positions nucléotidiques par rapport à la position n+3 in la direction distale à l'extrémité de ligature complémentaire, le nombre étant un nombre entier compris entre 1 et 10 ou plus ; et dans lequel, lors de la ligature, le nucléotide terminal du brin de support de la molécule de ligature polynucléotidique est ligaturé au nucléotide terminal du polynucléotide d'échafaudage proximal à la partie brin d'amorce du brin de synthèse et une rupture simple brin est créée entre les nucléotides terminaux du brin auxiliaire et la partie brin d'amorce du brin de synthèse ;
(b) dans l'étape de clivage du premier cycle (étape 2) et dans tous les cycles supplémentaires, le brin de support du polynucléotide d'échafaudage ligaturé est clivé entre les positions n+2 et n+1, libérant ainsi la molécule de ligature polynucléotidique du polynucléotide d'échafaudage et conservant le premier nucléotide de ce cycle non apparié et fixé au premier brin du polynucléotide d'échafaudage clivé, dans lequel, lors du clivage, un surplomb binucléotidique est créé dans le brin de support du polynucléotide d'échafaudage clivé, les nucléotides terminal et avant-dernier du brin de support surplombant le nucléotide terminal de la partie brin d'amorce du brin de synthèse ; et
(c) dans l'étape d'extension du premier cycle (étape 3) et dans tous les cycles supplémentaires, le second nucléotide de ce cycle est incorporé dans le second brin et est apparié avec l'avant-dernier nucléotide du brin de support en surplomb, et dans lequel dans les étapes suivantes (7) et (8), la position occupée par le premier nucléotide de ce cycle supplémentaire dans le brin de support du polynucléotide d'échafaudage clivé est définie en tant que position nucléotidique n dans le cycle de synthèse suivant.

8. Procédé selon la revendication 6 (b), dans lequel :
(a) à la fois dans le premier cycle et les cycles supplémentaires, à la fois dans la molécule de ligature polynucléotidique et dans le polynucléotide d'échafaudage ligaturé, le nucléotide universel occupe la position n+3+x et le polynucléotide d'échafaudage est clivé entre les positions n+3+x et n+2+x ; ou
(b) à la fois dans le premier cycle et les cycles supplémentaires, à la fois dans la molécule de ligature polynucléotidique et dans le polynucléotide d'échafaudage ligaturé, le nucléotide universel occupe la position n+4+x et le polynucléotide d'échafaudage est clivé entre les positions n+3+x et n+2+x.

9. Procédé selon l'une quelconque des revendications 2 à 8, le procédé étant modifié de sorte que
(i) dans l'étape (1), la molécule de ligature polynucléotidique est pourvue d'une extrémité de ligature complémentaire comprenant un premier nucléotide de la séquence prédéfinie du premier cycle et comprenant en outre un ou plusieurs nucléotides supplémentaires de la séquence prédéfinie du premier cycle ;
(ii) dans l'étape (2), après clivage, le premier nucléotide et les nucléotides supplémentaires de la séquence prédéfinie du premier cycle sont conservés dans le polynucléotide d'échafaudage clivé ;
(iii) dans l'étape (3), l'extrémité terminale de la partie brin d'amorce du brin de synthèse du polynucléotide d'échafaudage double brin est étendue par l'incorporation d'un second nucléotide de la séquence prédéfinie du premier cycle sous l'action de l'enzyme nucléotide transférase ou polymérase, et dans lequel l'extrémité terminale de la partie brin d'amorce est davantage étendue par l'incorporation d'un ou plusieurs nucléotides supplémentaires de la séquence prédéfinie du premier cycle sous l'action de l'enzyme nucléotide transférase ou polymérase, dans lequel chacun des second nucléotide et nucléotides supplémentaires du premier cycle comprend un groupe terminateur réversible qui empêche toute extension supplémentaire par l'enzyme, et dans lequel, après chaque extension supplémentaire, le groupe terminateur réversible est éliminé d'un nucléotide avant l'incorporation du nucléotide suivant ;
(iv) dans l'étape (5), la molécule de ligature polynucléotidique est pourvue d'une extrémité de ligature complémentaire comprenant un premier nucléotide de la séquence prédéfinie du cycle supplémentaire et comprenant en outre un ou plusieurs nucléotides supplémentaires de la séquence prédéfinie du cycle supplémentaire ;
(v) dans l'étape (6), après clivage, le premier nucléotide et les nucléotides supplémentaires de la séquence prédéfinie du cycle supplémentaire sont conservés dans le polynucléotide d'échafaudage clivé ;
(vi) dans l'étape (7), l'extrémité terminale de la partie brin d'amorce du brin de synthèse du polynucléotide d'échafaudage double brin est étendue par l'incorporation d'un second nucléotide de la séquence prédéfinie du cycle supplémentaire sous l'action de l'enzyme nucléotide transférase ou polymérase, et dans lequel l'extrémité terminale de la partie du brin d'amorce est davantage étendue par l'incorporation d'un ou plusieurs nucléotides supplémentaires de la séquence prédéfinie du cycle supplémentaire sous l'action de l'enzyme nucléotide transférase ou polymérase, dans lequel chacun des second nucléotide et nucléotides supplémentaires du cycle supplémentaire comprend un groupe terminateur réversible qui empêche toute extension supplémentaire par l'enzyme, et dans lequel, après chaque extension supplémentaire, le groupe terminateur réversible est éliminé d'un nucléotide avant l'incorporation du nucléotide suivant.

10. Procédé selon la revendication 9, dans lequel :
(a) l'extrémité de ligature complémentaire de la molécule de ligature polynucléotidique est structurée de sorte que, dans les étapes (2) et (6) avant le clivage, le nucléotide universel occupe une position dans le brin de support qui est la position nucléotidique suivante dans le brin de support après les positions nucléotidiques du premier nucléotide et des nucléotides supplémentaires dans la direction distale à l'extrémité de ligature complémentaire, et le brin de support est clivé entre la position occupée par le dernier nucléotide supplémentaire et la position occupée par le nucléotide universel ; ou
(b) l'extrémité de ligature complémentaire de la molécule de ligature polynucléotidique est structurée de sorte que, dans les étapes (2) et (6) avant le clivage, le nucléotide universel occupe une position dans le brin de support qui est la position nucléotidique suivante+1 dans le brin de support après les positions nucléotidiques du premier nucléotide et des nucléotides supplémentaires dans la direction distale à l'extrémité de ligature complémentaire, et le brin de support est clivé entre la position occupée par le dernier nucléotide supplémentaire et la position occupée par le nucléotide suivant dans le brin de support.

11. Procédé selon l'une quelconque des revendications précédentes, dans lequel, dans l'un quelconque, plusieurs ou tous les cycles de synthèse, un nucléotide partenaire qui s'apparie avec le premier nucléotide de la séquence prédéfinie est un nucléotide qui est complémentaire du premier nucléotide, de préférence naturellement complémentaire.

12. Procédé selon l'une quelconque des revendications 2 à 11, dans lequel :
(a) dans l'un quelconque, plusieurs ou tous les cycles de synthèse, avant l'étape (2) et/ou (6), le polynucléotide d'échafaudage est fourni comprenant un brin de synthèse et un brin de support hybridé à celui-ci, et dans lequel le brin de synthèse est fourni sans brin auxiliaire ; et/ou
(b) dans l'un quelconque, plusieurs ou tous les cycles de synthèse, avant l'étape (2) et/ou (6), la partie brin auxiliaire du brin de synthèse est éliminée du polynucléotide d'échafaudage.

13. Procédé selon l'une quelconque des revendications 1 à 3(i), 4 à 6, 7(i), 6(b), 8(a), 9 et 10(a), dans lequel :
(a) chaque étape de clivage comprend un procédé de clivage en deux étapes, dans lequel chaque étape de clivage comprend une première étape comprenant l'élimination du nucléotide universel, formant ainsi un site abasique, et une seconde étape comprenant le clivage du brin de support au niveau du site abasique ; facultativement, dans lequel la première étape est réalisée avec une enzyme excisant les nucléotides ; et/ou dans lequel la seconde étape est réalisée avec une enzyme présentant une activité de lyase de site abasique, facultativement dans lequel l'enzyme présentant une activité de lyase de site abasique est :
(i) l'AP endonucléase 1 ;
(ii) l'endonucléase III (Nth) ; ou
(ii) l'endonucléase VIII ; ou
(b) chaque étape de clivage comprend un procédé de clivage en une seule étape comprenant l'élimination du nucléotide universel à l'aide d'une enzyme de clivage, dans lequel l'enzyme est :
(i) l'endonucléase III ;
(ii) l'endonucléase VIII ;
(ii) la formamidopyrimidine ADN glycosylase (Fpg) ; ou
(iv) la 8-oxoguanine ADN glycosylase (hOGG1).

14. Procédé selon l'une quelconque des revendications 1 à 2, 3(ii), 4 à 6, 7(ii), 8(b), 9 et 10(b), dans lequel l'étape de clivage comprend le clivage du brin de support avec une enzyme ; facultativement dans lequel l'enzyme clive le brin de support entre les positions nucléotidiques n+1 et n ; et/ou dans lequel l'enzyme est l'endonucléase V.

15. Procédé selon l'une quelconque des revendications 2 à 14, dans lequel, dans l'un quelconque, plusieurs ou tous les cycles de synthèse :
(a) dans les étapes (1)/(5), dans le polynucléotide d'échafaudage, le brin de synthèse comprenant la partie brin d'amorce et la partie du brin de support hybridée à celui-ci sont reliés par une boucle en épingle à cheveux ; et
(b) dans les étapes (1)/(5), dans la molécule de ligature polynucléotidique, le brin auxiliaire et la partie du brin de support hybridée à celui-ci sont reliés par une boucle en épingle à cheveux à l'extrémité opposée à l'extrémité de ligature complémentaire.

16. Procédé selon l'une quelconque des revendications 2 à 15, dans lequel le brin de synthèse comprenant la partie brin d'amorce et la partie du brin de support hybridée à celui-ci sont fixés à une surface commune, facultativement via une ou plusieurs liaisons covalentes ; en outre facultativement dans lequel les une ou plusieurs liaisons covalentes sont formées entre un groupe fonctionnel sur la surface commune et un groupe fonctionnel sur la molécule d'échafaudage, dans lequel le groupe fonctionnel sur la molécule d'échafaudage est un groupe amine, un groupe thiol, un groupe thiophosphate ou un groupe thioamide ; plus préférablement, dans lequel le groupe fonctionnel sur la surface commune est un groupe bromacétyle, facultativement dans lequel le groupe bromacétyle est fourni sur une surface en polyacrylamide dérivée à l'aide de N-(5-bromoacétamidylpentyl)acrylamide (BRAPA).

17. Procédé selon l'une quelconque des revendications précédentes, dans lequel :
(a) les cycles de synthèse sont réalisés dans des gouttelettes au sein d'un système microfluidique ; facultativement dans lequel le système microfluidique est un système d'électromouillage ; plus préférablement dans lequel le système microfluidique est un système d'électromouillage sur diélectrique (EWOD) ; et/ou
(b) après la synthèse, les brins des polynucléotides double brin sont séparés pour fournir un polynucléotide simple brin qui présente une séquence prédéfinie ; et/ou
(c) dans lequel, après la synthèse, le polynucléotide double brin ou une région de celui-ci est amplifié, de préférence par PCR.

18. Procédé d'assemblage d'un polynucléotide présentant une séquence prédéfinie, le procédé comprenant la réalisation du procédé de l'une quelconque des revendications précédentes pour synthétiser un premier polynucléotide présentant une séquence prédéfinie et un ou plusieurs polynucléotides additionnels présentant une séquence prédéfinie et la jonction ensemble du premier polynucléotide et des un ou plusieurs polynucléotides additionnels ; facultativement, dans lequel le premier polynucléotide et les un ou plusieurs polynucléotides additionnels sont clivés pour créer des extrémités compatibles et joints ensemble, de préférence par ligature ; en outre facultativement, dans lequel le premier polynucléotide et les un ou plusieurs polynucléotides additionnels sont clivés par une enzyme de restriction au niveau d'un site de clivage.
